(19) Europäisches Patentamt
European Patent Office
Office européen des brevets

(11) EP 3 544 978 B1

(12) # EUROPÄISCHE PATENTSCHRIFT

(45) Veröffentlichungstag und Bekanntmachung des Hinweises auf die Patenterteilung:
**04.11.2020 Patentblatt 2020/45**

(21) Anmeldenummer: **17798261.8**

(22) Anmeldetag: **22.11.2017**

(51) Int Cl.:
***C07D 471/04*** (2006.01) ***A01N 43/56*** (2006.01)
***A01N 43/90*** (2006.01)

(86) Internationale Anmeldenummer:
**PCT/EP2017/080009**

(87) Internationale Veröffentlichungsnummer:
**WO 2018/095953 (31.05.2018 Gazette 2018/22)**

(54) **2-[3-(ALKYLSULFONYL)-2H-INDAZOL-2-YL]-3H-IMIDAZO[4,5-B]PYRIDIN-DERIVATE UND ÄHNLICHE VERBINDUNGEN ALS SCHÄDLINGSBEKÄMPFUNGSMITTEL**

2-[3-(ALKYLSULFONYL)-2H-INDAZOL-2-YL]-3H-IMIDAZO[4,5-B]PYRIDINE DERIVATIVES AND RELATED COMPOUNDS AS PESTICIDES

DÉRIVÉS DE 2-[3-(ALKYLSULFONYL)-2H-INDAZOL-2-YL]-3H-IMIDAZO[4,5-B]PYRIDINE ET COMPOSÉS SIMILAIRES EN TANT QUE PESTICIDES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.11.2016 EP 16200177**

(43) Veröffentlichungstag der Anmeldung:
**02.10.2019 Patentblatt 2019/40**

(73) Patentinhaber:
- **Bayer CropScience Aktiengesellschaft
40789 Monheim am Rhein (DE)**
- **Bayer Aktiengesellschaft
51373 Leverkusen (DE)**

(72) Erfinder:
- **KAUSCH-BUSIES, Nina
51467 Bergisch Gladbach (DE)**
- **FISCHER, Rüdiger
50259 Pulheim (DE)**
- **HOFFMEISTER, Laura
40593 Düsseldorf (DE)**
- **WILCKE, David
40235 Düsseldorf (DE)**
- **HAGER, Dominik
40789 Monheim (DE)**
- **WILLOT, Matthieu
40215 Düsseldorf (DE)**
- **MOSRIN, Marc
50935 Köln (DE)**
- **ILG, Kerstin
50670 Köln (DE)**
- **GÖRGENS, Ulrich
40882 Ratingen (DE)**
- **TURBERG, Andreas
42781 Haan (DE)**

(74) Vertreter: **BIP Patents
c/o Bayer Intellectual Property GmbH
Alfred-Nobel-Straße 10
40789 Monheim am Rhein (DE)**

(56) Entgegenhaltungen:
**WO-A1-2016/142327 WO-A1-2016/162318**

- **FATHALLA, O. A. ET AL: "Synthyses, reaction and characterization of quinoline derivatives", INTERNATIONAL JOURNAL OF PHARMACY (HYDERABAD, INDIA) , 2(2), 299-305 CODEN: IJPNL6; ISSN: 2249-1848 URL: HTTP://WWW.PHARMASCHOLARS.COM/ADMIN 1/FILES /22010.PDF, 2012, XP002767321,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **DATABASE CA [Online] CHEMICAL ABSTRACTS SERVICE, COLUMBUS, OHIO, US; 2005, ABASS, MOHAMED: "Chemistry of substituted quinolinones. part 8. Synthesis and cyclization reactions of ethyl 5-amino-1-(1-methyl-2-oxoquinolin-4-yl)-3-methylsulfanylpyrazole-4-carboxylate", XP002767322, gefunden im STN Database accession no. 2005:618431 & ABASS, MOHAMED: "Chemistry of substituted quinolinones. part 8. Synthesis and cyclization reactions of ethyl 5-amino-1-(1-methyl-2-oxoquinolin-4-yl)-3 methylsulfanylpyrazole-4-carboxylate", INTERNATIONAL ELECTRONIC CONFERENCES ON SYNTHETIC ORGANIC CHEMISTRY; 5TH, 6TH, SEPT. 1-30, 2001 AND 2002 [AND] 7TH, 8TH, NOV. 1-30, 2003 AND 2004 (2004), 1630-1638, 5. September 2001 (2001-09-05), - 8. November 2004 (2004-11-08), Editor(s): Seijas, Julio A. Publisher: Molecular Diversity Preservation International, Basel, Switzerland**

## Beschreibung

Die vorliegende Erfindung betrifft neue kondensierte bicyclische Heterocyclen-Derivate der Formel (Ia) oder (Ib), agrochemische Formulierungen enthaltend die Verbindungen gemäß Formel (Ia) oder (Ib) und deren Anwendung als Akarizide und/oder Insektizide zur Bekämpfung tierischer Schädlinge, vor allem von Arthropoden und insbesondere von Insekten und Spinnentieren.

Kondensierte bicyclische Heterocyclen-Derivate mit insektiziden Eigenschaften sind in der Literatur bereits beschrieben, z.B. in WO 2010/125985, WO 2012/074135, WO 2016/162318, WO 2017/093180, WO 2017/072039, WO 2017/125340, WO 2017/144341, EP 16184163.0, EP 16189445.6, EP16163912.5, WO2016/107742, WO2016/091731, WO 2016/129684, WO2016/142326, WO2016/142327 und WO2017/155103.

Moderne Insektizide und Akarizide müssen vielen Anforderungen genügen, beispielsweise in Bezug auf Höhe, Dauer und Breite ihrer Wirkung und möglichen Verwendung. Es spielen Fragen der Toxizität, der Nützling- und Bestäuberschonung, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen zur Verwendung zur Bekämpfung von tierischen Schädlingen bereitzustellen, durch die das Spektrum der Schädlingsbekämpfungsmittel unter verschiedenen Aspekten ergänzt wird.

Es wurden nun neue kondensierte bicyclische Heterocyclen-Derivate gefunden, welche gegenüber den bereits bekannten Verbindungen Vorteile aufweisen, z.B. seien bessere biologische oder ökologische Eigenschaften, breitere Anwendungsmethoden, eine bessere insektizide, akarizide Wirkung, sowie eine gute Verträglichkeit gegenüber Nutzpflanzen beispielhaft genannt. Die kondensierten bicyclischen Heterocyclen-Derivate können in Kombination mit weiteren Mitteln zur Verbesserung der Wirksamkeit insbesondere gegen schwierig zu bekämpfende Insekten eingesetzt werden.

Gelöst wird die Aufgabe, sowie weitere nicht explizit genannte Aufgaben, die aus den hierin diskutierten Zusammenhängen ableitbar oder erschließbar sind, daher durch neue Verbindungen der Formel (Ia) oder (Ib)

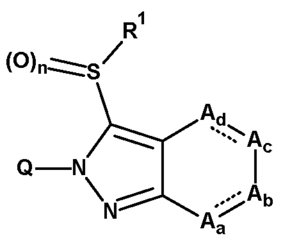

*Ia*

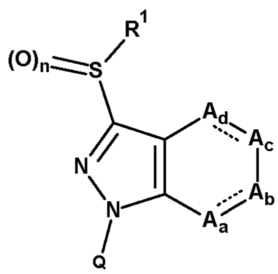

*Ib*

wobei 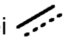 für Einfachbindungen oder Doppelbindungen stehen,
in welchen (Ausgestaltung 1),

für den Fall, dass 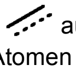 ausschließlich für Doppelbindungen stehen und somit Aa bis Ad mit den jeweils zu Aa und Ad benachbarten C-Atomen einen aromatischen Ring ausbilden,,

Aa für Stickstoff oder $C(R^{10})$ steht,
Ab für Stickstoff oder $C(R^{11})$ steht,
Ac für Stickstoff oder $C(R^{12})$ steht und
Ad für Stickstoff oder $C(R^{13})$ steht,

wobei Aa, Ab, Ac und Ad für maximal zwei Stickstoffe stehen können,

oder für den Fall, dass 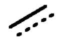 ausschließlich für Einfachbindungen stehen,

Aa für $C(R^{10})(R^{14})$ steht,
Ab für $C(R^{11})(R^{15})$ steht,

Ac für $C(R^{12})(R^{16})$ steht und

Ad für $C(R^{13})(R^{17})$ steht,

$R^1$ für $(C_1-C_6)$Alkyl, $(C_1-C_6)$Halogenalkyl, $(C_1-C_6)$Cyanoalkyl, $(C_1-C_6)$Hydroxyalkyl, $(C_1-C_6)$Alkoxy-$(C_1-C_6)$alkyl, $(C_1-C_6)$Halogenalkoxy-$(C_1-C_6)$alkyl, $(C_2-C_6)$Alkenyl, $(C_2-C_6)$Alkenyloxy-$(C_1-C_6)$alkyl, $(C_2-C_6)$Halogenalkenyloxy-$(C_1-C_6)$alkyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_6)$alkyl, $(C_2-C_6)$Halogenalkinyloxy-$(C_1-C_6)$alkyl, $(C_2-C_6)$Halogenalkinyl, $(C_2-C_6)$Cyanoalkinyl, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, Amino, $(C_1-C_6)$Alkylamino, Di-$(C_1-C_6)$alkyl-amino, $(C_3-C_8)$Cycloalkylamino, $(C_1-C_6)$Alkylcarbonyl-amino, $(C_1-C_6)$Alkylthio-$(C_1-C_6)$alkyl, $(C_1-C_6)$Halogenalkylthio-$(C_1-C_6)$alkyl, $(C_1-C_6)$Alkylsulfinyl-$(C_1-C_6)$alkyl, $(C_1-C_6)$Halogenalkylsulfinyl-$(C_1-C_6)$alkyl, $(C_1-C_6)$Alkylsulfonyl-$(C_1-C_6)$alkyl, $(C_1-C_6)$Halogenalkylsulfonyl-$(C_1-C_6)$alkyl, $(C_1-C_6)$Alkoxy-$(C_1-C_6)$alkylthio-$(C_1-C_6)$alkyl, $(C_1-C_6)$Alkoxy-$(C_1-C_6)$alkylsulfinyl-$(C_1-C_6)$alkyl, $(C_1-C_6)$Alkoxy-$(C_1-C_6)$alkylsulfonyl-$(C_1-C_6)$alkyl, $(C_1-C_6)$Alkylcarbonyl-$(C_1-C_6)$alkyl, $(C_1-C_6)$Halogenalkylcarbonyl-$(C_1-C_6)$alkyl, $(C_1-C_6)$Alkoxycarbonyl-$(C_1-C_6)$alkyl, $(C_1-C_6)$Halogenalkoxycarbonyl-$(C_1-C_6)$alkyl, $(C_1-C_6)$Alkylsulfonylamino, Aminosulfonyl-$(C_1-C_6)$alkyl, $(C_1-C_6)$Alkylaminosulfonyl-$(C_1-C_6)$alkyl, Di-$(C_1-C_6)$alkyl-aminosulfonyl-$(C_1-C_6)$alkyl,

oder für jeweils einfach oder mehrfach, gleich oder verschieden durch Aryl, Hetaryl oder Heterocyclyl substituiertes $(C_1-C_6)$Alkyl, $(C_1-C_6)$Alkoxy, $(C_2-C_6)$Alkenyl, $(C_2-C_6)$Alkinyl, $(C_3-C_8)$Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, $(C_1-C_6)$Alkyl, $(C_3-C_6)$Cycloalkyl, $(C_1-C_6)$Alkoxy, $(C_1-C_6)$Halogenalkyl, $(C_1-C_6)$Halogenalkoxy, $(C_1-C_6)$Alkylthio, $(C_1-C_6)$Alkylsulfinyl, $(C_1-C_6)$Alkylsulfonyl, $(C_1-C_6)$Alkylsulfimino, $(C_1-C_6)$Alkylsulfimino-$(C_1-C_6)$alkyl, $(C_1-C_6)$Alkylsulfimino-$(C_2-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkylsilyl oder Benzyl substituiert sein können, oder

$R^1$ für jeweils gegebenenfalls einfach oder mehrfach, gleich oder verschieden durch Halogen, Cyano, Nitro, Hydroxy, Amino, Carboxy, Carbamoyl, $(C_1-C_6)$Alkyl, $(C_3-C_8)$Cycloalkyl, $(C_1-C_6)$-Alkoxy, $(C_1-C_6)$Halogenalkyl, $(C_1-C_6)$Halogenalkoxy, $(C_1-C_6)$Alkylthio, $(C_1-C_6)$Alkyl-sulfinyl, $(C_1-C_6)$Alkylsulfonyl, $(C_1-C_6)$Alkylsulfimino, $(C_1-C_6)$Alkylsulfimino-$(C_1-C_6)$alkyl, $(C_1-C_6)$Alkylsulfimino-$(C_2-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkylsilyl, (=O) (nur im Fall von Heterocyclyl) oder $(=O)_2$ (nur im Fall von Heterocyclyl) substituiertes Aryl, Hetaryl oder Heterocyclyl steht,

$R^{10}$, $R^{11}$ $R^{12}$, $R^{13}$, $R^{14}$, $R^{15}$, $R^{16}$, $R^{17}$ unabhängig voneinander für Wasserstoff, Cyano, Halogen, Nitro, Hydroxy, Amino, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfnyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylthiocarbonyl,

$(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylammothiocarbonyl, Di-$(C_1-C_6)$alkyl-am$^i$nothiocarbonyl, $(C_3-C_8)$Cycloalkylamino, $(C_1-C_6)$Alkylcarbamoyl (umfasst -NHCOO$(C_1-C_6)$alkyl, -N$(C_1-C_6)$alkylCOO$(C_1-C_6)$alkyl, -OCONH$(C_1-C_6)$alkyl oder -OCON$(C_1-C_6)$dialkyl), $(C_1-C_6)$Alkylcarbonylamino ($(C_1-C_6)$alkylCONH), $(C_1-C_6)$Alkylharnstoff (umfasst -NHCONH$(C_1-C_6)$alkyl, und -NHCON$(C_1-C_6)$dialkyl) oder

für jeweils gegebenenfalls einfach oder mehrfach, gleich oder verschieden substituiertes Aryl oder Hetaryl stehen, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Hydroxy, Amino, Tri-$(C_1-C_6)$alkylsilyl, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, Di-$(C_1-C_6)$alkyl-aminocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkylaminosulfonyl, $(C_1-C_6)$Alkylsulfoximino, Aminothiocarbonyl, $(C_1-C_6)$Alkylaminothiocarbonyl, Di-$(C_1-C_6)$alkylaminothiocarbonyl, $(C_3-C_8)$Cycloalkylamino oder $(C_1-C_6)$Alkylcarbonylamino,

wobei nur einer oder zwei der Reste $R^{10}$, $R^{11}$, $R^{12}$, $R^{13}$, $R^{14}$, $R^{15}$, $R^{16}$ und $R^{17}$ für einen Substituenten ungleich Wasserstoff stehen,

und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl oder $(C_1-C_6)$Cyanoalkyl stehen,

Q für ein teilweise gesättigtes oder gesättigtes heterozyklisches oder heteroaromatisches 8-, 9-, 11-oder 12-gliedriges annelliertes bicyclisches oder tricyclisches Ringsystem steht, wobei gegebenenfalls mindestens eine Carbonylgruppe enthalten sein kann und wobei das Ringsystem gegebenenfalls einfach oder mehrfach, gleich oder verschieden substituiert ist, und wobei die Substituenten unabhängig voneinander ausgewählt sein können aus Cyano, Halogen, Nitro, Hydroxy, Amino, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl,

$(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_4)$alkyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Halogenalkoxy-$(C_1-C_6)$alkyl, $(C_2-C_6)$Alkenyloxy-$(C_1-C_6)$alkyl, $(C_2-C_6)$Halogenalkenyloxy-$(C_1-C_6)$alkyl, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylcarbonyl-$(C_1-C_6)$alkyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino oder $(C_1-C_6)$Alkylcarbonylamino

oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $C_1$-$C_4$-Alkoxy, $C_1$-$C_4$-Haloalkoxy substituiert sein können,

n für 0, 1 oder 2 steht.

Weiterhin wurde gefunden, dass die Verbindungen der Formeln (Ia) und (Ib) eine sehr gute Wirksamkeit als Schädlingsbekämpfungsmittel, vorzugsweise als Insektizide und/oder Akarizide aufweisen und darüber hinaus in der Regel insbesondere gegenüber Kulturpflanzen sehr gut pflanzenverträglich sind.

Die erfindungsgemäßen Verbindungen sind durch die Formeln (Ia) und (Ib) allgemein definiert. Bevorzugte Substituenten bzw. Bereiche der in der oben und nachstehend erwähnten Formeln aufgeführten Reste werden im Folgenden erläutert:

Ausgestaltung 2

Verbindungen der Formel I(a) oder I(b), in welchen bevorzugt,

für den Fall, dass 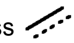 ausschließlich für Doppelbindungen stehen und somit Aa bis Ad mit den jeweils zu Aa und Ad benachbarten C-Atomen einen aromatischen Ring ausbilden,

Aa für Stickstoff oder $C(R^{10})$ steht,
Ab für Stickstoff oder $C(R^{11})$ steht,
Ac für Stickstoff oder $C(R^{12})$ steht und
Ad für Stickstoff oder $C(R^{13})$ steht,

wobei Aa, Ab, Ac und Ad für maximal zwei Stickstoffe stehen können,
oder für den Fall, dass ausschließlich für Einfachbindungen stehen,

Aa für $C(R^{10})(R^{14})$ steht,

Ab für $C(R^{11})(R^{15})$ steht,

Ac für $C(R^{12})(R^{16})$ steht und

Ad für $C(R^{13})(R^{17})$ steht,

$R^1$ bevorzugt für $(C_1\text{-}C_4)$Alkyl, $(C_1\text{-}C_4)$Hydroxyalkyl, $(C_1\text{-}C_4)$Halogenalkyl, $(C_1\text{-}C_4)$Cyanoalkyl, $(C_1\text{-}C_4)$Alkoxy-$(C_1\text{-}C_4)$alkyl, $(C_1\text{-}C_4)$Halogenalkoxy-$(C_1\text{-}C_4)$alkyl, $(C_2\text{-}C_4)$Alkenyl, $(C_2\text{-}C_4)$Alkenyloxy-$(C_1\text{-}C_4)$alkyl, $(C_2\text{-}C_4)$Halogenalkenyloxy-$(C_1\text{-}C_4)$alkyl, $(C_2\text{-}C_4)$Halogenalkenyl, $(C_2\text{-}C_4)$Cyanoalkenyl, $(C_2\text{-}C_4)$Alkinyl, $(C_2\text{-}C_4)$Alkinyloxy-$(C_1\text{-}C_4)$alkyl, $(C_2\text{-}C_4)$Halogenalkinyloxy-$(C_1\text{-}C_4)$alkyl, $(C_2\text{-}C_4)$Halogenalkinyl, $(C_2\text{-}C_4)$Cyanoalkinyl, $(C_3\text{-}C_6)$Cycloalkyl, $(C_3\text{-}C_6)$Cycloalkyl$(C_3\text{-}C_6)$cycloalkyl, $(C_1\text{-}C_4)$Alkyl-$(C_3\text{-}C_6)$cycloalkyl, Halogen$(C_3\text{-}C_6)$cycloalkyl, $(C_1\text{-}C_4)$Alkylamino, Di-$(C_1\text{-}C_4)$alkyl-amino, $(C_3\text{-}C_6)$Cycloalkylamino, $(C_1\text{-}C_4)$Alkylcarbonyl-amino, $(C_1\text{-}C_4)$Alkylthio-$(C_1\text{-}C_4)$alkyl, $(C_1\text{-}C_4)$Halogenalkylthio-$(C_1\text{-}C_4)$alkyl, $(C_1\text{-}C_4)$Alkylsulfinyl-$(C_1\text{-}C_4)$alkyl, $(C_1\text{-}C_4)$Halogenalkylsulfinyl-$(C_1\text{-}C_4)$alkyl, $(C_1\text{-}C_4)$Alkylsulfonyl-$(C_1\text{-}C_4)$alkyl, $(C_1\text{-}C_4)$Alkylcarbonyl-$(C_1\text{-}C_4)$alkyl, $(C_1\text{-}C_4)$Halogenalkylcarbonyl-$(C_1\text{-}C_4)$alkyl oder $(C_1\text{-}C_4)$Alkylsulfonylamino steht,

$R^{10}$, $R^{11}$, $R^{12}$, $R^{13}$, $R^{14}$, $R^{15}$, $R^{16}$, $R^{17}$ unabhängig voneinander bevorzugt für Wasserstoff, Cyano, Halogen, Nitro, Hydroxy, Amino, Tri-$(C_1\text{-}C_4)$alkylsilyl, $(C_3\text{-}C_6)$Cycloalkyl, $(C_3\text{-}C_6)$Cycloalkyl-$(C_3\text{-}C_6)$cycloalkyl, $(C_1\text{-}C_4)$Alkyl-$(C_3\text{-}C_6)$cycloalkyl, Halogen$(C_3\text{-}C_6)$cycloalkyl, $(C_1\text{-}C_4)$Alkyl, $(C_1\text{-}C_4)$Halogenalkyl, $(C_1\text{-}C_4)$Cyanoalkyl, $(C_1\text{-}C_4)$Hydroxyalkyl, $(C_1\text{-}C_4)$Alkoxy-$(C_1\text{-}C_4)$alkyl, $(C_2\text{-}C_4)$Alkenyl, $(C_2\text{-}C_4)$Halogenalkenyl, $(C_2\text{-}C_4)$Cyanoalkenyl, $(C_2\text{-}C_4)$Alkinyl, $(C_2\text{-}C_4)$Halogenalkinyl, $(C_2\text{-}C_4)$Cyanoalkinyl, $(C_1\text{-}C_4)$Alkoxy, $(C_1\text{-}C_4)$Halogenalkoxy, $(C_1\text{-}C_4)$Cyanoalkoxy, $(C_1\text{-}C_4)$Alkoxy-$(C_1\text{-}C_4)$alkoxy, $(C_1\text{-}C_4)$Alkylhydroxyimino, $(C_1\text{-}C_4)$Alkoxyimino, $(C_1\text{-}C_4)$Alkyl-$(C_1\text{-}C_4)$alkoxyimino, $(C_1\text{-}C_4)$Halogenalkyl-$(C_1\text{-}C_4)$alkoxyimino, $(C_1\text{-}C_4)$Alkylthio, $(C_1\text{-}C_4)$Halogenalkylthio, $(C_1\text{-}C_4)$Alkylthio-$(C_1\text{-}C_4)$alkyl, $(C_1\text{-}C_4)$Alkylsulfinyl, $(C_1\text{-}C_4)$Halogenalkylsulfinyl, $(C_1\text{-}C_4)$Alkylsulfinyl-$(C_1\text{-}C_4)$alkyl, $(C_1\text{-}C_4)$Alkylsulfonyl, $(C_1\text{-}C_4)$Halogenalkylsulfonyl, $(C_1\text{-}C_4)$Alkylsulfonyl-$(C_1\text{-}C_4)$alkyl, $(C_1\text{-}C_4)$Alkylsulfonyloxy, $(C_1\text{-}C_4)$Alkylcarbonyl, $(C_1\text{-}C_4)$Halogenalkylcarbonyl, Aminocarbonyl, Aminothiocarbonyl, $(C_1\text{-}C_4)$Alkylaminocarbonyl, Di-$(C_1\text{-}C_4)$alkyl-aminocarbonyl, $(C_3\text{-}C_6)$Cycloalkylaminocarbonyl, $(C_1\text{-}C_4)$Alkylsulfonylamino, $(C_1\text{-}C_4)$Alkylamino, Di-$(C_1\text{-}C_4)$Alkylamino, Aminosulfonyl, $(C_1\text{-}C_4)$Alkylaminosulfonyl, Di-$(C_1\text{-}C_4)$alkyl-aminosulfonyl, Aminothiocarbonyl, $(C_1\text{-}C_4)$Alkylcarbamoyl (umfasst -NHCOO$(C_1\text{-}C_4)$alkyl, -N$(C_1\text{-}C_4)$alkylCOO$(C_1\text{-}C_4)$alkyl, -OCONH$(C_1\text{-}C_4)$alkyl oder -OCON$(C_1\text{-}C_4)$dialkyl), $(C_1\text{-}C_4)$Alkylcarbonylamino, $(C_1\text{-}C_4)$Alkylhamstoff (umfasst -NHCONH$(C_1\text{-}C_4)$alkyl, und -NHCON$(C_1\text{-}C_4)$dialkyl)
oder bevorzugt für jeweils gegebenenfalls einfach oder zweifach, gleich oder verschieden substituiertes Phenyl oder Hetaryl stehen, wobei (im Fall von Hetaryl) gegebenenfalls mindestens eine Carbonylgruppe enthalten sein kann und wobei als Substituenten jeweils in Frage kommen: Cyano, Halogen, Nitro, Acetyl, Amino, $(C_3\text{-}C_6)$Cycloalkyl, $(C_3\text{-}C_6)$Cycloalkyl-$(C_3\text{-}C_6)$cycloalkyl, $(C_1\text{-}C_4)$Alkyl-$(C_3\text{-}C_6)$cycloalkyl, Halogen$(C_3\text{-}C_6)$cycloalkyl, $(C_1\text{-}C_4)$Alkyl, $(C_1\text{-}C_4)$Halogenalkyl, $(C_1\text{-}C_4)$Cyanoalkyl, $(C_1\text{-}C_4)$Hydroxyalkyl, $(C_1\text{-}C_4)$Alkoxy-$(C_1\text{-}C_4)$alkyl, $(C_2\text{-}C_4)$Alkenyl, $(C_2\text{-}C_4)$Halogenalkenyl, $(C_2\text{-}C_4)$Cyanoalkenyl, $(C_2\text{-}C_4)$Alkinyl, $(C_2\text{-}C_4)$Halogenalkinyl, $(C_2\text{-}C_4)$Cyanoalkinyl, $(C_1\text{-}C_4)$Alkoxy, $(C_1\text{-}C_4)$Halogenalkoxy, $(C_1\text{-}C_4)$Cyanoalkoxy, $(C_1\text{-}C_4)$Alkoxy-$(C_1\text{-}C_4)$alkoxy, $(C_1\text{-}C_4)$Alkylhydroxyimino, $(C_1\text{-}C_4)$Alkoxyimino, $(C_1\text{-}C_4)$Alkyl-$(C_1\text{-}C_4)$alkoxyimino, $(C_1\text{-}C_4)$Halogenalkyl-$(C_1\text{-}C_4)$alkoxyimino, $(C_1\text{-}C_4)$Alkylthio, $(C_1\text{-}C_4)$Halogenalkylthio, $(C_1\text{-}C_4)$Alkylthio-$(C_1\text{-}C_4)$alkyl, $(C_1\text{-}C_4)$Alkylsulfinyl, $(C_1\text{-}C_4)$Halogenalkylsulfinyl, $(C_1\text{-}C_4)$Alkylsulfinyl-$(C_1\text{-}C_4)$alkyl, $(C_1\text{-}C_4)$Alkylsulfonyl, $(C_1\text{-}C_4)$Halogenalkylsulfonyl, $(C_1\text{-}C_4)$Alkylsulfonyl-$(C_1\text{-}C_4)$alkyl, $(C_1\text{-}C_4)$Alkylsulfonyloxy, $(C_1\text{-}C_4)$Alkylcarbonyl, $(C_1\text{-}C_4)$Halogenalkylcarbonyl, Aminocarbonyl, $(C_1\text{-}C_4)$Alkylaminocarbonyl, Di-$(C_1\text{-}C_4)$alkyl-aminocarbonyl, $(C_1\text{-}C_4)$Alkylsulfonylamino, $(C_1\text{-}C_4)$Alkylamino, Di-$(C_1\text{-}C_4)$Alkylamino, Aminosulfonyl, $(C_1\text{-}C_4)$Alkylaminosulfonyl, Di-$(C_1\text{-}C_4)$alkylaminosulfonyl oder $(C_1\text{-}C_4)$Alkylcarbonylamino,

wobei nur einer oder zwei der Reste $R^{10}$, $R^{11}$, $R^{12}$, $R^{13}$, $R^{14}$, $R^{15}$, $R^{16}$, $R^{17}$ für einen Substituenten ungleich Wasserstoff stehen,
und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkyl, $(C_1-C_4)$Halogenalkyl oder $(C_1-C_4)$Cyanoalkyl stehen,

Q bevorzugt für ein heteroaromatisches 9-gliedriges oder 12-gliedriges annelliertes bicyclisches oder tricyclisches Ringsystem aus der Reihe Q1 bis Q15 steht:

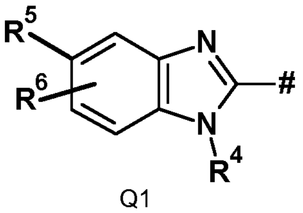
Q1

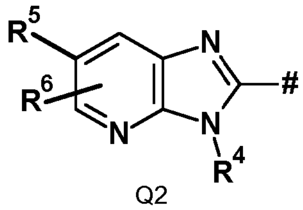
Q2

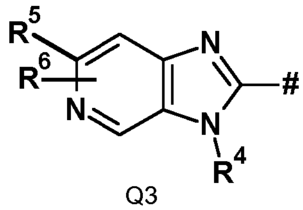
Q3

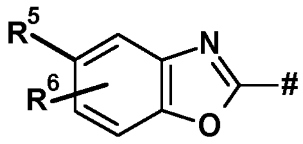
Q4

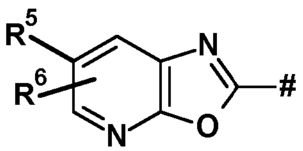
Q5

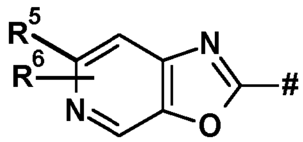
Q6

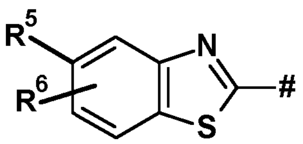
Q7

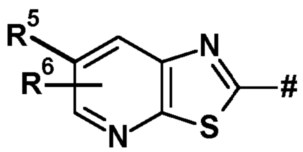
Q8

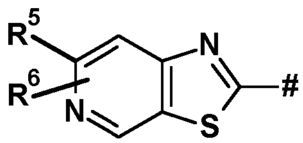
Q9

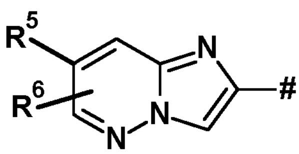
Q10

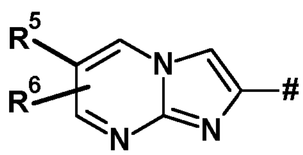
Q11

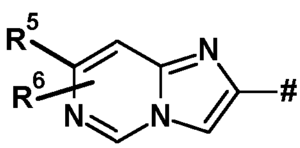
Q12

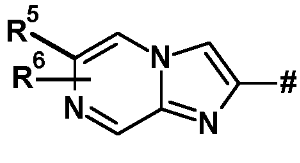
Q13

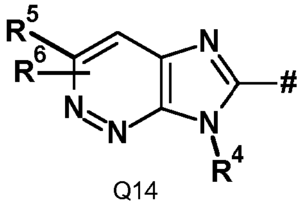
Q14

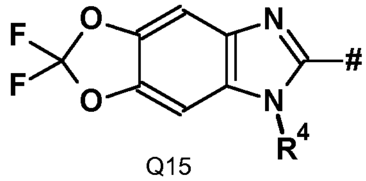
Q15

wobei

$R^4$ für Wasserstoff, $(C_1-C_4)$Alkyl, $(C_1-C_4)$Halogenalkyl, $(C_1-C_4)$Cyanoalkyl, $(C_1-C_4)$Hydroxyalkyl, $(C_1-C_4)$Alkoxy-$(C_1-C_4)$alkyl, $(C_1-C_4)$Halogenalkoxy-$(C_1-C_4)$alkyl, $(C_2-C_4)$Alkenyl, $(C_2-C_4)$Alkenyloxy-$(C_1-C_4)$alkyl,

($C_2$-$C_4$)Halogenalkenyloxy-($C_1$-$C_4$)alkyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl oder ($C_3$-$C_6$)Cycloalkyl steht und

$R^5$, $R^6$ unabhängig voneinander für Wasserstoff, Cyano, Halogen, ($C_1$-$C_4$)Alkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, ($C_1$-$C_4$)Halogenalkyl-($C_3$-$C_6$)cycloalkyl, Cyano-($C_3$-$C_6$)cycloalkyl, Halogen-($C_3$-$C_6$)cycloalkyl, ($C_1$-$C_4$)Alkoxy, ($C_1$-$C_4$)Halogenalkoxy, ($C_1$-$C_4$)Alkoxyimino, ($C_1$-$C_4$)Halogenalkoxyimino ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Halogenalkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl oder Di-($C_1$-$C_4$)alkylaminosulfonyl stehen und

n für 0, 1 oder 2 steht.

Ausgestaltung 3

Verbindungen der Formel I(a) oder I(b), in welchen besonders bevorzugt,

für den Fall, dass 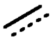 ausschließlich für Doppelbindungen stehen und somit Aa bis Ad mit den jeweils zu Aa und Ad benachbarten C-Atomen einen aromatischen Ring ausbilden,

Aa für Stickstoff oder C($R^{10}$) steht,
Ab für Stickstoff oder C($R^{11}$) steht,
Ac für Stickstoff oder C($R^{12}$) steht und
Ad für Stickstoff oder C($R^{13}$) steht,

wobei Aa, Ab, Ac und Ad für maximal zwei Stickstoffe stehen können,
oder für den Fall, dass ausschließlich für Einfachbindungen stehen,

Aa für C($R^{10}$)($R^{14}$) steht,
Ab für C($R^{11}$)($R^{15}$) steht,
Ac für C($R^{12}$)($R^{16}$) steht und
Ad für C($R^{13}$)($R^{17}$) steht,

$R^1$ besonders bevorzugt für ($C_1$-$C_4$)Alkyl, ($C_1$-$C_4$)Hydroxyalkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_1$-$C_4$)Alkylthio-($C_1$-$C_4$)alkyl, ($C_1$-$C_4$)Alkylsulfinyl-($C_1$-$C_4$)alkyl oder ($C_1$-$C_4$)Alkylsulfonyl-($C_1$-$C_4$)alkyl steht,

$R^{10}$, $R^{11}$, $R^{12}$, $R^{13}$, $R^{14}$, $R^{15}$, $R^{16}$, $R^{17}$ unabhängig voneinander besonders bevorzugt für Wasserstoff, Cyano, Halogen, Nitro, Hydroxy, Amino, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_3$-$C_6$)Cycloalkylaminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl, ($C_1$-$C_4$)Alkylcarbamoyl (umfasst-NHCOO($C_1$-$C_4$)alkyl und -N($C_1$-$C_4$)alkylCOO($C_1$-$C_4$)alkyl), ($C_1$-$C_4$)Alkylcarbonylamino, ($C_1$-$C_4$)Alkylhamstoff (umfasst -NHCONH($C_1$-$C_4$)alkyl und -NHCON($C_1$-$C_4$)dialkyl)
oder besonders bevorzugt für jeweils gegebenenfalls einfach oder zweifach, gleich oder verschieden substituiertes Phenyl oder Hetaryl stehen, wobei (im Fall von Hetaryl) gegebenenfalls mindestens eine Carbonylgruppe enthalten sein kann und wobei als Substituenten jeweils in Frage kommen: Cyano, Ha-

logen, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkyl, $(C_1-C_4)$Halogenalkyl, $(C_1-C_4)$Cyanoalkyl, $(C_2-C_4)$Alkenyl, $(C_2-C_4)$Halogenalkenyl, $(C_2-C_4)$Cyanoalkenyl, $(C_2-C_4)$Alkinyl, $(C_2-C_4)$Halogenalkinyl, $(C_2-C_4)$Cyanoalkinyl, $(C_1-C_4)$Alkoxy, $(C_1-C_4)$Halogenalkoxy, $(C_1-C_4)$Alkylthio, $(C_1-C_4)$Halogenalkylthio, $(C_1-C_4)$Alkylsulfinyl, $(C_1-C_4)$Halogenalkylsulfinyl, $(C_1-C_4)$Alkylsulfonyl, $(C_1-C_4)$Halogenalkylsulfonyl, $(C_1-C_4)$Alkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl, Di-$(C_1-C_4)$alkylaminosulfonyl, $(C_1-C_4)$Alkylcarbonylamino,

wobei nur einer oder zwei der Reste $R^{10}$, $R^{11}$, $R^{12}$, $R^{13}$, $R^{14}$, $R^{15}$, $R^{16}$, $R^{17}$ für einen Substituenten ungleich Wasserstoff stehen,

und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, Halogen$(C_3-C_6)$cycloalkyl, $(C_1-C_4)$Alkyl, $(C_1-C_4)$Halogenalkyl oder $(C_1-C_4)$Cyanoalkyl stehen,

Q besonders bevorzugt für ein heteroaromatisches 9-gliedriges oder 12-gliedriges annelliertes bicyclisches oder tricyclisches Ringsystem aus der Reihe Q1 bis Q15 steht, wobei

$R^4$ für Wasserstoff, $(C_1-C_4)$Alkyl, $(C_3-C_6)$Cycloalkyl, $(C_1-C_4)$Halogenalkyl, $(C_1-C_4)$Cyanoalkyl, $(C_1-C_4)$Alkoxy-$(C_1-C_4)$alkyl, $(C_1-C_4)$Halogenalkoxy-$(C_1-C_4)$alkyl, $(C_2-C_4)$Alkenyl, $(C_2-C_4)$Halogenalkenyl, $(C_2-C_4)$Alkinyl oder $(C_2-C_4)$Halogenalkinyl steht und

$R^5$, $R^6$ unabhängig voneinander für Wasserstoff, Cyano, Halogen, $(C_1-C_4)$Alkyl, $(C_1-C_4)$Halogenalkyl, $(C_2-C_4)$Alkenyl, $(C_2-C_4)$Halogenalkenyl, $(C_2-C_4)$Alkinyl, $(C_2-C_4)$Halogenalkinyl, $(C_3-C_6)$Cycloalkyl, $(C_3-C_6)$Cycloalkyl-$(C_3-C_6)$cycloalkyl, $(C_1-C_4)$Alkyl-$(C_3-C_6)$cycloalkyl, $(C_1-C_4)$Halogenalkyl-$(C_3-C_6)$cycloalkyl, Cyano-$(C_3-C_6)$cycloalkyl, Halogen-$(C_3-C_6)$cycloalkyl, $(C_1-C_4)$Alkoxy, $(C_1-C_4)$Halogenalkoxy, $(C_1-C_4)$Alkoxyimino, $(C_1-C_4)$Halogenalkoxyimino $(C_1-C_4)$Alkylthio, $(C_1-C_4)$Halogenalkylthio, $(C_1-C_4)$Alkylsulfinyl, $(C_1-C_4)$Halogenalkylsulfinyl, $(C_1-C_4)$Alkylsulfonyl, $(C_1-C_4)$Halogenalkylsulfonyl, $(C_1-C_4)$Alkylsulfonyloxy, $(C_1-C_4)$Halogenalkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl oder Di-$(C_1-C_4)$alkylaminosulfonyl stehen

und

n für 0, 1 oder 2 steht.

Ausgestaltung 4

Verbindungen der Formel I(a) oder I(b), in welchen ganz besonders bevorzugt,

für den Fall, dass 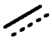 ausschließlich für Doppelbindungen stehen und somit Aa bis Ad mit den jeweils zu Aa und Ad benachbarten C-Atomen einen aromatischen Ring ausbilden,

Aa für Stickstoff oder C($R^{10}$) steht,

Ab für Stickstoff oder C($R^{11}$) steht,

Ac für Stickstoff oder C($R^{12}$) steht und

Ad für Stickstoff oder C($R^{13}$) steht,

wobei Aa, Ab, Ac und Ad für maximal zwei Stickstoffe stehen können,

oder für den Fall, dass ausschließlich für Einfachbindungen stehen,

Aa für $C(R^{10})(R^{14})$ steht,

Ab für $C(R^{11})(R^{15})$ steht,

Ac für $C(R^{12})(R^{16})$ steht und

Ad für $C(R^{13})(R^{17})$ steht,

$R^1$ ganz besonders bevorzugt für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl steht,

$R^{10}$, $R^{11}$, $R^{12}$, $R^{13}$, $R^{14}$, $R^{15}$, $R^{16}$, $R^{17}$ unabhängig voneinander ganz besonders bevorzugt für Wasserstoff, Cyano, Halogen, $(C_1-C_4)$Alkyl, $(C_1-C_4)$Halogenalkyl, $(C_1-C_4)$Alkoxy, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Halogenalkylthio, $(C_1-C_4)$Halogenalkylsulfinyl, $(C_1-C_4)$Halogenalkylsulfonyl, Aminocarbonyl, $(C_1-C_4)$Alkylaminocarbonyl, $(C_3-C_6)$Cycloalkylaminocarbonyl, $(C_1-C_4)$Alkylcarbamoyl (umfasst-NHCOO$(C_1-C_4)$alkyl und -N$(C_1-C_4)$alkylCOO$(C_1-C_4)$alkyl), $(C_1-C_4)$Alkylcarbonylamino oder $(C_1-C_4)$Alkylharnstoff (umfasst -NHCONH$(C_1-C_4)$alkyl und -NHCON$(C_1-C_4)$dialkyl) stehen, wobei nur einer oder zwei der Reste $R^{10}$, $R^{11}$, $R^{12}$, $R^{13}$, $R^{14}$, $R^{15}$, $R^{16}$, $R^{17}$ für einen Substituenten ungleich Wasserstoff stehen, und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, $(C_1-C_4)$Alkyl oder $(C_1-C_4)$Halogenalkyl stehen,

Q ganz besonders bevorzugt für ein heteroaromatisches 9-gliedriges oder 12-gliedriges annelliertes bicyclisches oder tricyclisches Ringsystem aus der Reihe Q2, Q3, Q5, Q6, Q8, Q9, Q14 oder Q15 steht,

wobei

$R^4$ ganz besonders bevorzugt für $(C_1-C_4)$Alkyl, $(C_1-C_4)$Halogenalkyl, $(C_3-C_6)$Cycloalkyl oder $(C_1-C_4)$Alkoxy-$(C_1-C_4)$alkyl steht,

$R^5$ ganz besonders bevorzugt für Cyano, Halogen, $(C_1-C_4)$Halogenalkyl, Halogen-$(C_3-C_6)$cycloalkyl, $(C_1-C_4)$Halogenalkoxy, $(C_1-C_4)$Halogenalkylthio, $(C_1-C_4)$Halogenalkylsulfinyl, $(C_1-C_4)$Alkylsulfonyl, $(C_1-C_4)$Halogenalkylsulfonyl, $(C_1-C_4)$Halogenalkylcarbonyl oder $(C_1-C_4)$Halogenalkylsulfonyloxy steht,

$R^6$ ganz besonders bevorzugt für Wasserstoff, Cyano, Halogen, $(C_1-C_4)$Alkyl, $(C_1-C_4)$Halogenalkyl oder $(C_3-C_6)$Cycloalkyl steht und

n für 0, 1 oder 2 steht.

Ausgestaltung 5-1

Verbindungen der Formel I(a) oder I(b), in welchen hervorgehoben,

für den Fall, dass 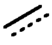 ausschließlich für Doppelbindungen stehen und somit Aa bis Ad mit den jeweils zu Aa und Ad benachbarten C-Atomen einen aromatischen Ring ausbilden,

Aa für $C(R^{10})$ steht,
Ab für Stickstoff oder $C(R^{11})$ steht,
Ac für $C(R^{12})$ steht und

Ad für $C(R^{13})$ steht,

oder für den Fall, dass ausschließlich für Einfachbindungen stehen,

Aa für $C(R^{10})(R^{14})$ steht,
Ab für $C(R^{11})(R^{15})$ steht,
Ac für $C(R^{12})(R^{16})$ steht und
Ad für $C(R^{13})(R^{17})$ steht,
$R^1$ hervorgehoben für Ethyl steht,
$R^{10}$, $R^{11}$, $R^{12}$, $R^{13}$ unabhängig voneinander hervorgehoben für Wasserstoff, Fluor, Chlor, Brom, Iod, Cyano, Methyl, Ethyl, Trifluormethyl, Trifluormethoxy, 2,2,2-Trifluoroethoxy, Acetylamino (Methylcarbonylamino,-NHCOMe), Cyclopropylamido (Cyclopropylaminocarbonyl), Methylcarbamoyl (-NHCOOMe), Methylharnstoff (-NHCONHMe) oder Cyclopropyl stehen, wobei nur einer oder zwei der Reste $R^{10}$, $R^{11}$, $R^{12}$, $R^{13}$ für einen Substituenten ungleich Wasserstoff stehen,
$R^{14}$, $R^{15}$, $R^{16}$, $R^{17}$ unabhängig voneinander hervorgehoben für Wasserstoff oder $(C_1\text{-}C_4)$Alkyl, bevorzugt für Wasserstoff stehen,
Q hervorgehoben für ein heteroaromatisches 9-gliedriges annelliertes bicyclisches Ringsystem aus der Reihe Q2, Q3 oder Q14 steht

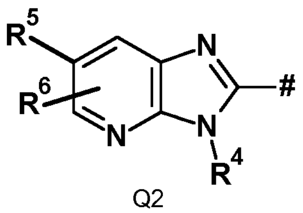
Q2

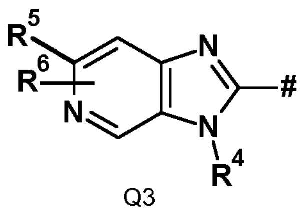
Q3

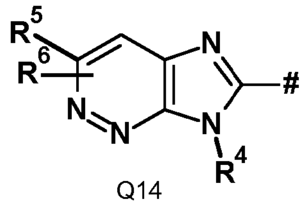
Q14

.

wobei

$R^4$ hervorgehoben für Methyl steht,
$R^5$ hervorgehoben für Trifluormethyl oder Pentafluorethyl steht,
$R^6$ hervorgehoben für Wasserstoff steht und
n für 0, 1 oder 2 steht.

Ausgestaltung 5-2

Verbindungen der Formel I(a) oder I(b), in welchen hervorgehoben,

für den Fall, dass 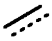 ausschließlich für Doppelbindungen stehen und somit Aa bis Ad mit den jeweils zu Aa und Ad benachbarten C-Atomen einen aromatischen Ring ausbilden,

Aa für $C(R^{10})$ steht,
Ab für $C(R^{11})$ steht,
Ac für $C(R^{12})$ steht und
Ad für $C(R^{13})$ steht,

oder für den Fall, dass 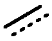 ausschließlich für Einfachbindungen stehen,

Aa für $C(R^{10})(R^{14})$ steht,
Ab für $C(R^{11})(R^{15})$ steht,
Ac für $C(R^{12})(R^{16})$ steht und
Ad für $C(R^{13})(R^{17})$ steht,
$R^1$ steht hervorgehoben für Ethyl,
$R^{10}$, $R^{11}$, $R^{12}$, $R^{13}$ stehen unabhängig voneinander hervorgehoben für Wasserstoff, Fluor, Chlor, Brom, Iod, Cyano, Methyl, Ethyl, Trifluormethyl, Trifluormethoxy, 2,2,2-Trifluoroethoxy, Acetylamino (Methylcarbonylamino), Cyclopropylamido (Cyclopropylaminocarbonyl), Methylcarbamoyl (-NHCOOMe), Methylharnstoff (-NHCONHMe) oder Cyclopropyl,
wobei nur einer oder zwei der Reste $R^{10}$, $R^{11}$, $R^{12}$, $R^{13}$ für einen Substituenten ungleich Wasserstoff stehen,

$R^{14}$, $R^{15}$, $R^{16}$, $R^{17}$ stehen unabhängig voneinander hervorgehoben für Wasserstoff oder $(C_1\text{-}C_4)$Alkyl, bevorzugt für Wasserstoff,

Q steht hervorgehoben für ein heteroaromatisches 9-gliedriges annelliertes bicyclisches Ringsystem aus der Reihe Q2, Q3, Q5 oder Q14 steht,

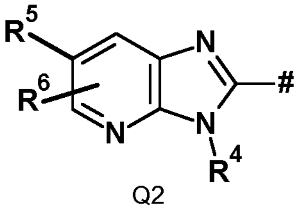
Q2

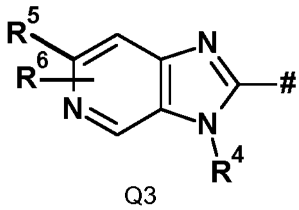
Q3

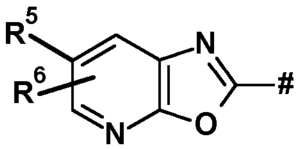
Q5

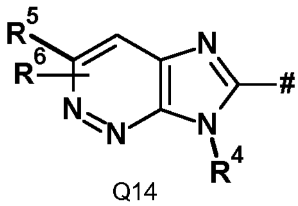
Q14

wobei

$R^4$ hervorgehoben für Methyl steht,
$R^5$ hervorgehoben für Trifluormethyl oder Pentafluorethyl steht,
$R^6$ hervorgehoben für Wasserstoff steht und
n steht für 0, 1 oder 2.

Ausgestaltung 6-1

Verbindungen der Formel I(a) oder I(b), in welchen insbesonders,

für den Fall, dass 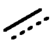 ausschließlich für Doppelbindungen stehen und somit Aa bis Ad mit den jeweils zu Aa und Ad benachbarten C-Atomen einen aromatischen Ring ausbilden,

Aa für $C(R^{10})$ steht,
Ab für Stickstoff oder $C(R^{11})$ steht,
Ac für $C(R^{12})$ steht und
Ad für $C(R^{13})$ steht, wobei
$R^{10}$ insbesonders für Wasserstoff, Chlor oder Trifluormethyl steht,
$R^{11}$ insbesonders für Wasserstoff, Chlor, -NHCOMe oder Trifluormethyl steht,
$R^{12}$ insbesonders für Wasserstoff, Chlor oder Trifluormethyl steht,
$R^{13}$ insbesonders für Wasserstoff, Chlor oder Trifluormethyl steht,

oder für den Fall, dass 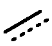 ausschließlich für Einfachbindungen stehen,

Aa für $C(R^{10})(R^{14})$ steht,
Ab für $C(R^{11})(R^{15})$ steht,
Ac für $C(R^{12})(R^{16})$ steht und
Ad für $C(R^{13})(R^{17})$ steht, wobei
$R^{10}$, $R^{12}$, $R^{13}$, $R^{14}$, $R^{16}$, $R^{17}$ insbesonders für Wasserstoff stehen,
$R^{11}$ insbesonders für Wasserstoff oder Methyl steht und
$R^{15}$ insbesondere für Wasserstoff oder Methyl steht,
$R^1$ insbesonders für Ethyl steht,

Q insbesonders für ein heteroaromatisches 9-gliedriges annelliertes bicyclisches Ringsystem aus der Reihe Q2 oder Q3 steht,

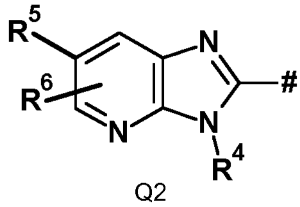

Q2

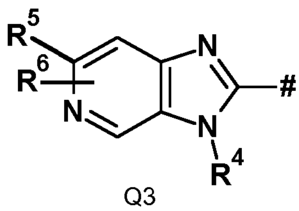

Q3

wobei

$R^4$ insbesonders für Methyl steht,
$R^5$ insbesonders für Trifluormethyl oder Pentafluorethyl steht,
$R^6$ insbesonders für Wasserstoff steht und
n für 0, 1 oder 2 steht.

Ausgestaltung 6-2:

Verbindungen der Formel I(a) oder I(b), in welchen insbesonders,

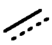 ausschließlich für Doppelbindungen stehen und somit Aa bis Ad mit den jeweils zu Aa und Ad benachbarten C-Atomen einen aromatischen Ring ausbilden,

Aa für $C(R^{10})$ steht,
Ab für $C(R^{11})$ steht,
Ac für $C(R^{12})$ steht und
Ad für $C(R^{13})$ steht,
$R^1$ steht insbesonders für Ethyl,
$R^{10}$ steht insbesonders für Wasserstoff, Chlor oder Trifluormethyl,
$R^{11}$ steht insbesonders für Wasserstoff, Chlor oder Trifluormethyl,
$R^{12}$ steht insbesonders für Wasserstoff, Chlor oder Trifluormethyl,
$R^{13}$ steht insbesonders für Wasserstoff, Chlor oder Trifluormethyl, wobei nur einer der Reste $R^{10}$, $R^{11}$, $R^{12}$ oder $R^{13}$ für einen Substituenten ungleich Wasserstoff steht,
Q steht insbesonders für ein heteroaromatisches 9-gliedriges annelliertes bicyclisches Ringsystem aus der Reihe Q2 oder Q3

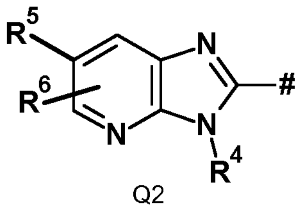

Q2

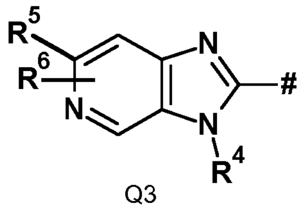

Q3

wobei

$R^4$ insbesonders für Methyl steht,
$R^5$ insbesonders für Trifluormethyl oder Pentafluorethyl steht,
$R^6$ insbesonders für Wasserstoff steht und
n für 0, 1 oder 2 steht.

Im Folgenden steht der Begriff *Ausgestaltung (5)* gleichbedeutend mit *Ausgestaltung 5-1 oder Ausgestaltung 5-2* und *Ausgestaltung (6)* gleichbedeutend mit *Ausgestaltung 6-1 oder 6-2.*

Der Substituent Aa ist identisch mit dem Substituenten $A_a$; der Substituent Ab ist identisch mit dem Substituenten $A_b$, der Substituent Ac ist identisch mit dem Substituenten $A_c$, der Substituent Ad ist identisch mit dem Substituenten $A_d$.

In einer bevorzugten Ausführungsform betrifft die Erfindung Verbindungen der Formel (Ia) oder (Ib), wobei Q für Q2, Q3, Q5, Q6, Q8, Q9, Q14 oder Q15 steht, wobei

$R^4$ für $(C_1-C_4)$Alkyl, $(C_1-C_4)$Halogenalkyl, $(C_3-C_6)$Cycloalkyl oder $(C_1-C_4)$Alkoxy-$(C_1-C_4)$alkyl steht,

$R^5$ für Cyano, Halogen, $(C_1-C_4)$Halogenalkyl, Halogen-$(C_3-C_6)$cycloalkyl, $(C_1-C_4)$Halogenalkoxy, $(C_1-C_4)$Halogenalkylthio, $(C_1-C_4)$Halogenalkylsulfinyl, $(C_1-C_4)$Alkylsulfonyl, $(C_1-C_4)$Halogenalkylsulfonyl, $(C_1-C_4)$Halogenalkylcarbonyl oder $(C_1-C_4)$Halogenalkylsulfonyloxy steht,

$R^6$ für Wasserstoff, Cyano, Halogen, $(C_1-C_4)$Alkyl, $(C_1-C_4)$Halogenalkyl oder $(C_3-C_6)$Cycloalkyl steht und

Aa, Ab, Ac, Ad, $R^1$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (5) oder Ausgestaltung (6) beschriebenen Bedeutungen haben.

In einer besonders bevorzugten Ausführungsform betrifft die Erfindung Verbindungen der Formel (Ia) oder (Ib), wobei Q für Q2, Q3 oder Q14 steht, wobei

$R^4$ für Methyl steht,

$R^5$ für Trifluormethyl oder Pentafluorethyl steht,

$R^6$ für Wasserstoff steht

und Aa, Ab, Ac, Ad, $R^1$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (6) beschriebenen Bedeutungen haben.

In einer ganz besonders bevorzugten Ausführungsform betrifft die Erfindung Verbindungen der Formel (Ia) oder (Ib), wobei Q für Q2 oder Q3 steht, wobei

$R^4$ für Methyl steht,

$R^5$ für Trifluormethyl oder Pentafluorethyl steht,

$R^6$ für Wasserstoff steht

und Aa, Ab, Ac, Ad, $R^1$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) beschriebenen Bedeutungen haben.

In einer bevorzugten Ausführungsform betrifft die Erfindung Verbindungen der Formel (Ia) oder (Ib), wobei Q für Q2, Q3, Q5, Q6, Q8, Q9, Q14 oder Q15 steht, wobei

$R^4$ für $(C_1-C_4)$Alkyl, $(C_1-C_4)$Halogenalkyl, $(C_3-C_6)$Cycloalkyl oder $(C_1-C_4)$Alkoxy-$(C_1-C_4)$alkyl steht,
$R^6$ für Wasserstoff, Cyano, Halogen, $(C_1-C_4)$Alkyl, $(C_1-C_4)$Halogenalkyl oder $(C_3-C_6)$Cycloalkyl steht und

Aa, Ab, Ac, Ad, $R^1$, $R^5$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder oder Ausgestaltung (5) oder Ausgestaltung (6) beschriebenen Bedeutungen haben.

In einer besonders bevorzugten Ausführungsform betrifft die Erfindung Verbindungen der Formel (Ia) oder (Ib), wobei Q für Q2, Q3 oder Q14 steht, wobei

$R^4$ für Methyl steht,

$R^6$ für Wasserstoff steht

und Aa, Ab, Ac, Ad, $R^1$, $R^5$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (6) beschriebenen Bedeutungen haben.

In einer ganz besonders bevorzugten Ausführungsform betrifft die Erfindung Verbindungen der Formel (Ia) oder (Ib), wobei Q für Q2 oder Q3 steht, wobei

$R^4$ für Methyl steht,

$R^6$ für Wasserstoff steht

und Aa, Ab, Ac, Ad, $R^1$, $R^5$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) beschriebenen Bedeutungen haben.

In einer bevorzugten Ausführungsform betrifft die Erfindung Verbindungen der Formel (Ia) oder (Ib), wobei Q für Q2, Q3, Q5, Q6, Q8, Q9, Q14 oder Q15 steht, wobei

$R^4$ für $(C_1-C_4)$Alkyl, bevorzugt für Methyl steht und

Aa, Ab, Ac, Ad, $R^1$, $R^5$, $R^6$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

In einer besonders bevorzugten Ausführungsform betrifft die Erfindung Verbindungen der Formel (Ia) oder (Ib), wobei Q für Q2, Q3 oder Q14 steht, wobei

$R^4$ für $(C_1-C_4)$Alkyl, bevorzugt für Methyl steht und

Aa, Ab, Ac, Ad, $R^1$, $R^5$, $R^6$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

In einer ganz besonders bevorzugten Ausführungsform betrifft die Erfindung Verbindungen der Formel (Ia) oder (Ib), wobei Q für Q2 oder Q3 steht, wobei

$R^4$ für $(C_1-C_4)$Alkyl, bevorzugt für Methyl steht und

Aa, Ab, Ac, Ad, $R^1$, $R^5$, $R^6$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

In einer bevorzugten Ausführungsform betrifft die Erfindung Verbindungen der Formel (Ia) oder (Ib), wobei Q für Q2, Q3, Q5, Q6, Q8, Q9, Q14 oder Q15 steht, wobei

$R^6$ für Wasserstoff steht und

Aa, Ab, Ac, Ad, $R^1$, $R^4$, $R^5$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

In einer besonders bevorzugten Ausführungsform betrifft die Erfindung Verbindungen der Formel (Ia) oder (Ib), wobei Q für Q2, Q3 oder Q14 steht, wobei

$R^6$ für Wasserstoff steht und

Aa, Ab, Ac, Ad, $R^1$, $R^4$, $R^5$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

In einer ganz besonders bevorzugten Ausführungsform betrifft die Erfindung Verbindungen der Formel (Ia) oder (Ib), wobei Q für Q2 oder Q3 steht, wobei

$R^6$ für Wasserstoff steht und

Aa, Ab, Ac, Ad, $R^1$, $R^4$, $R^5$, $R^{10}$ $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

In einer weiteren bevorzugten Ausführungsform betrifft die Erfindung Verbindungen der Formel (Ia) oder (Ib), wobei $R^1$ für Ethyl steht und Aa, Ab, Ac, Ad, $R^{10}$, $R^{11}$, $R^{12}$, $R^{13}$, $R^{14}$, $R^{15}$, $R^{16}$, $R^{17}$, Q, $R^4$, $R^5$, $R^6$ und n die in Ausgestaltung (1) oder Ausgestaltung (2) oder Ausgestaltung (3) oder Ausgestaltung (4) beschriebenen Bedeutungen haben.

Besonders bevorzugt ergeben sich für die Formeln (Ia) und (Ib) die folgenden Strukturen (Ia1) bis (Ia4) und (Ibl) bis (Ib4),

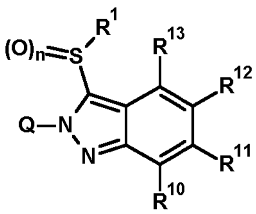
(Ia1)

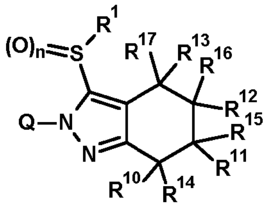
(Ia2)

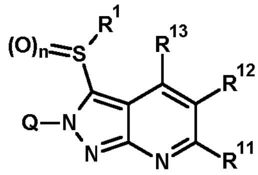
(Ia3)

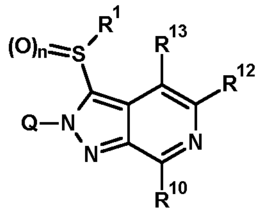
(Ia4)

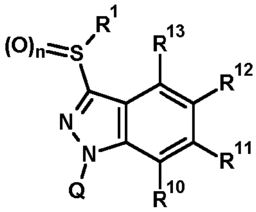
(Ib1)

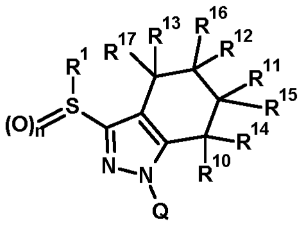
(Ib2)

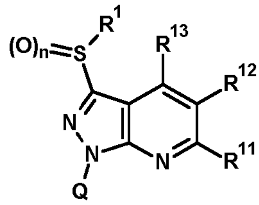
(Ib3)

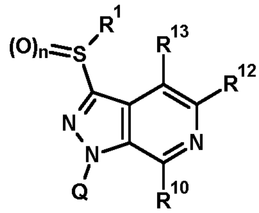
(Ib4)

wobei $R^1$, $R^{10}$, $R^{11}$, $R^{12}$, $R^{13}$, $R^{14}$, $R^{15}$, $R^{16}$, $R^{17}$, Q, $R^4$, $R^5$, $R^6$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

Ganz besonders bevorzugt ergeben sich die Verbindungen der Formeln (Ia5) bis (Ia16),

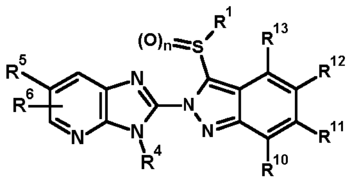
(Ia5)

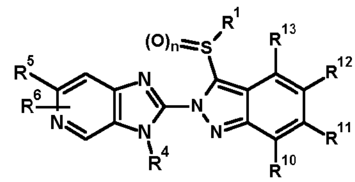
(Ia6)

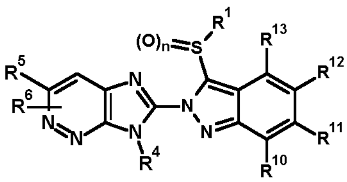
(Ia7)

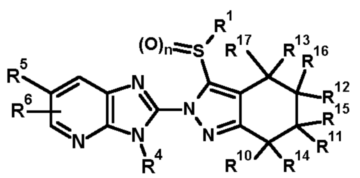
(Ia8)

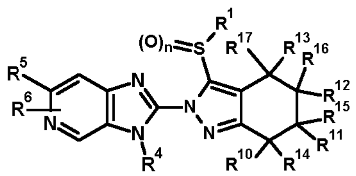
(Ia9)

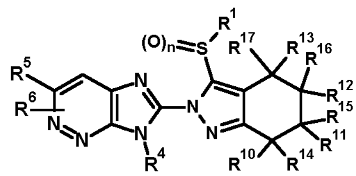
(Ia10)

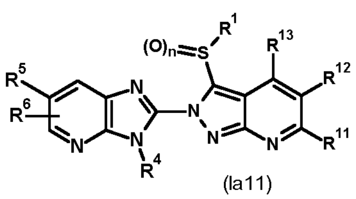
(Ia11)

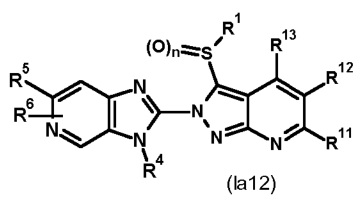
(Ia12)

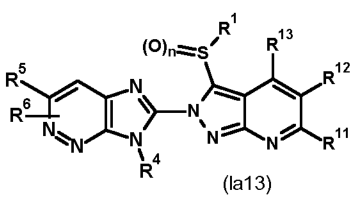
(Ia13)

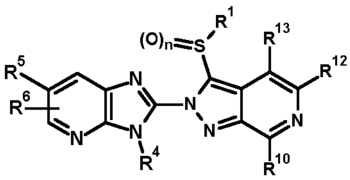

(Ia14)

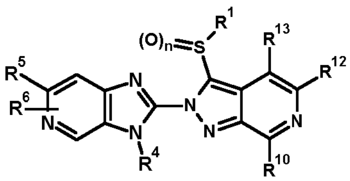

(Ia15)

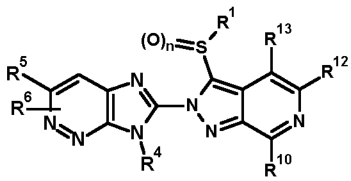

(Ia16)

wobei $R^1$, $R^{10}$, $R^{11}$, $R^{12}$, $R^{13}$, $R^{14}$, $R^{15}$, $R^{16}$, $R^{17}$, $R^4$, $R^5$, $R^6$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

Insbesondere bevorzugt sind dabei die Verbindungen der Formeln (Ia5) bis (Ia10) und Formel (Ia14) und hervorgehoben die Verbindungen (Ia5) bis (Ia7) und (Ia14).

Insbesondere ergeben sich die Strukturen (Ia17) bis (Ia25),

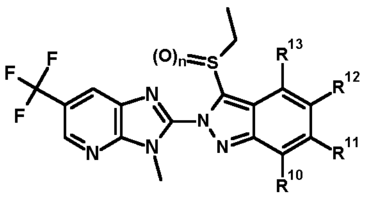

(Ia17)

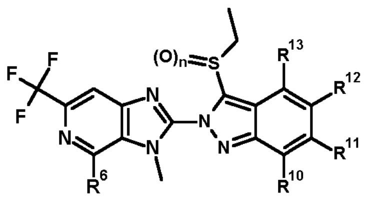

(Ia18)

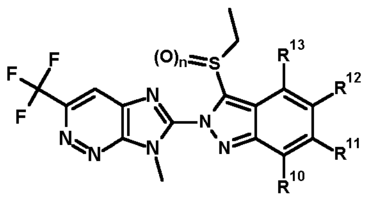

(Ia19)

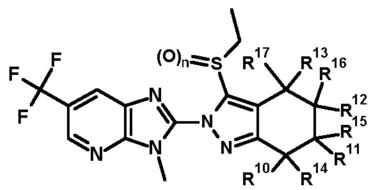

(Ia20)

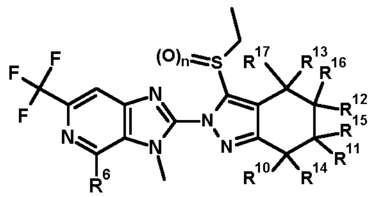

(Ia21)

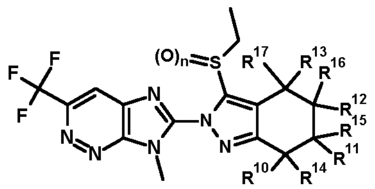

(Ia22)

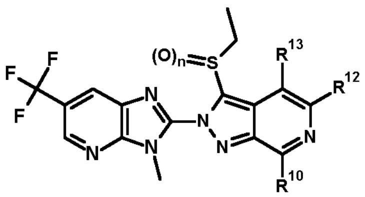

(Ia23)

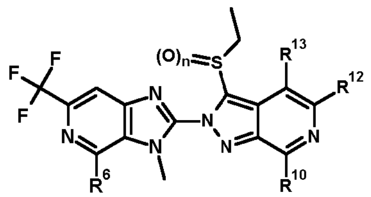

(Ia24)

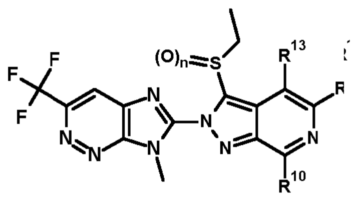

(Ia25)

wobei $R^6$, $R^{10}$, $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

Insbesondere bevorzugt sind dabei die Verbindungen der Formeln (Ia17) bis (Ia19) und (Ia23).

Weiterhin bevorzugt steht in den Formeln (Ia) und (Ib) für den Fall, dass 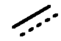 ausschließlich für Einfachbindungen stehen,

Aa für $C(R^{10})(R^{14})$ steht,

Ab für $C(R^{11})(R^{15})$ steht,

Ac für $C(R^{12})(R^{16})$ steht und

Ad für $C(R^{13})(R^{17})$ steht,

Q nicht für Q10, Q11, Q12 oder Q13.

Bevorzugt handelt es sich bei den erfindungsgemäßen Verbindungen um Verbindungen der Formel (Ia), besonders bevorzugt der Formeln (Ia1) oder (Ia2) und ganz besonders bevorzugt der Formel (Ia1).

Weiterhin besonders bevorzugt sind die Verbindungen der Formel 1-1 bis I-5 und I-7 bis I-19.

Weiterhin ganz besonders bevorzugt sind die Verbindung der Formel I-1, I-2, I-3, I-4, I-5, I-7, I-8, I-9, I-10, I-11, I-18, I-19.

| | |
|---|---|
| I-1 | 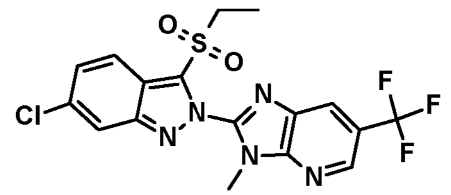 |
| I-2 | 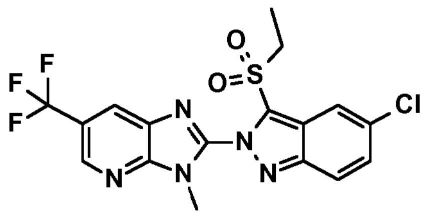 |
| I-3 | 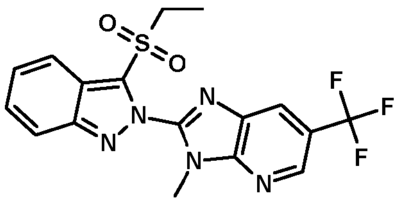 |
| I-4 | 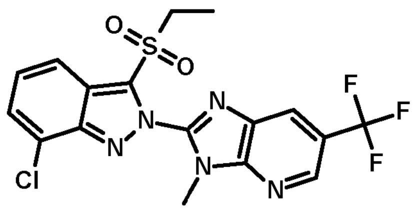 |
| I-5 | 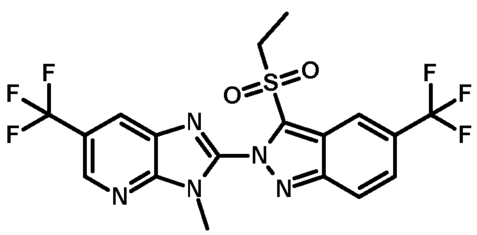 |
| I-7 | 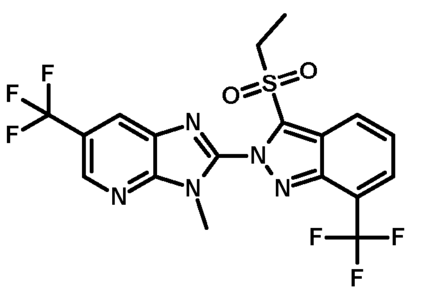 |

(fortgesetzt)

| | |
|---|---|
| I-8 | 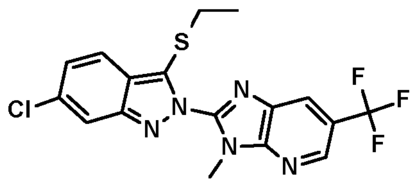 |
| I-9 | 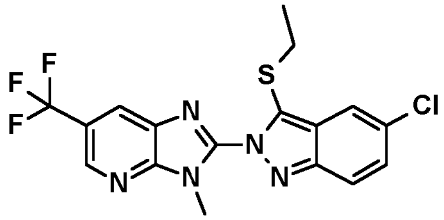 |
| I-10 | 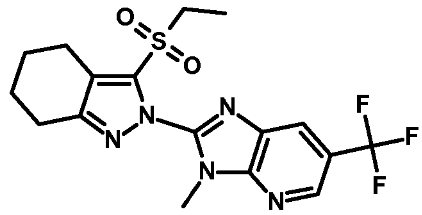 |
| I-11 | 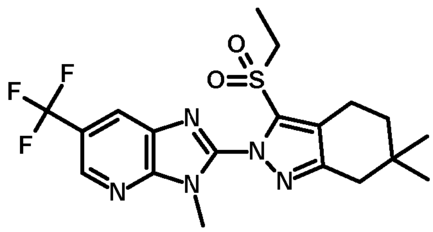 |
| I-12 | 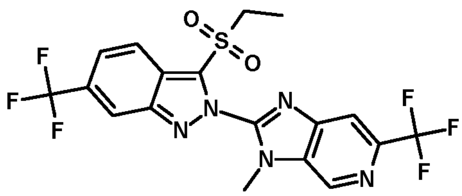 |
| I-13 | 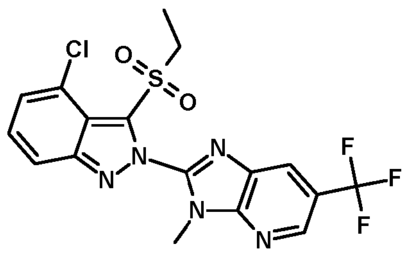 |
| I-14 | 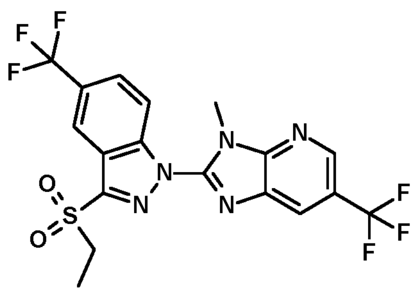 |

(fortgesetzt)

| | |
|---|---|
| I-15 | 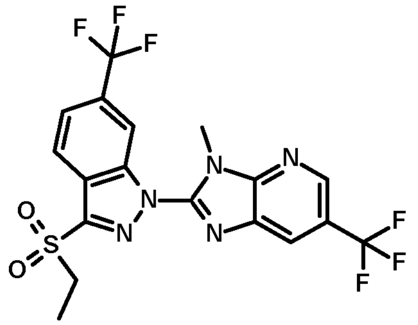 |
| I-16 | 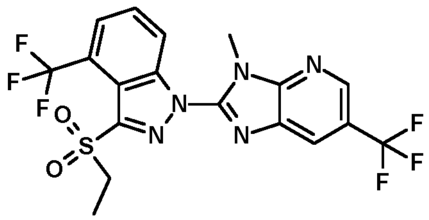 |
| I-17 | 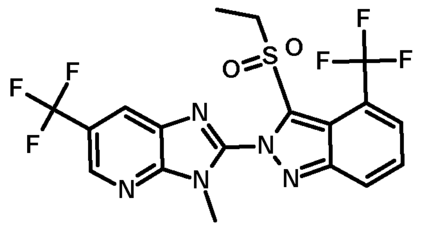 |
| I-18 | 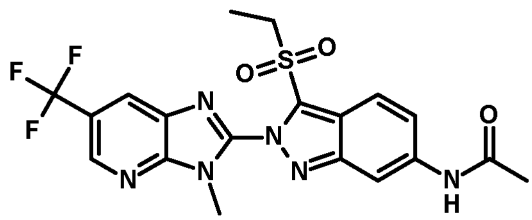 |
| I-19 | 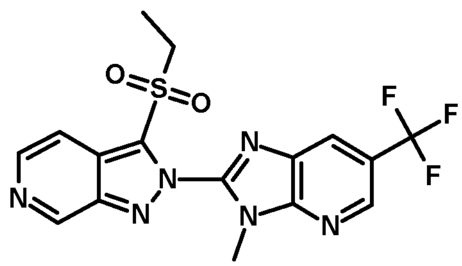 |

Besonders bevorzugt sind die Verbindungen der Formeln (Ia) oder (Ib), bevorzugt der Formel (Ia), gemäß der oben beschriebenen Ausführungsformen, insbesondere gemäß Ausgestaltung (1) oder Ausgestaltung (2) oder Ausgestaltung (3) oder Ausgestaltung (4) oder Ausgestaltung (5) oder Ausgestaltung (6), wobei für den Fall, dass für die Verbindungen der Formel (Ia)

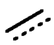 ausschließlich für Doppelbindungen steht,

$R^1$ für $C_1$-$C_6$ Alkyl steht,

Aa und Ad für CH stehen,

Ab für $C(R^{11})$ steht,

Ac für $C(R^{12})$ stehtund

Q für Q2 steht, wobei
$R^4$ für $C_1$-$C_6$ Alkyl,
$R^6$ für Wasserstoff und
$R^5$ für ($C_1$-$C_6$)Halogenalkyl, ($C_1$-$C_6$)Halogenalkylthio, ($C_1$-$C_6$)Halogenalkylsulfinyl oder ($C_1$-$C_6$)Haolgenalkylsulfonyl steht,
wenigstens einer der Reste $R^{11}$ oder $R^{12}$ nicht für Wasserstoff, Halogen oder ($C_1$-$C_6$)Halogenalkyl steht.

Weiterhin besonders bevorzugt sind die Verbindungen der Formeln (Ia) oder (Ib), bevorzugt der Formel (Ia), gemäß der oben beschriebenen Ausführungsformen, insbesondere gemäß Ausgestaltung (1) oder Ausgestaltung (2) oder Ausgestaltung (3) oder Ausgestaltung (4) oder Ausgestaltung (5) oder Ausgestaltung (6), wobei für den Fall, dass für die Verbindungen der Formel (Ia) 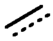 ausschließlich für Doppelbindungen steht,

$R^1$ für $C_1$-$C_6$ Alkyl steht,

Aa und Ad für CH stehen,

Ab für C($R^{11}$) steht,

Ac für C($R^{12}$) steht

$R^{11}$ und $R^{12}$ unabhängig voneinander für Wasserstoff, Halogen oder ($C_1$-$C_6$)Halogenalkyl stehen

und

Q für Q2 steht,

$R^4$ für $C_1$-$C_6$ Alkyl steht und

$R^6$ für Wasserstoff steht,

$R^5$ nicht für ($C_1$-$C_6$)Halogenalkyl, ($C_1$-$C_6$)Halogenalkylthio, ($C_1$-$C_6$)Halogenalkylsulfinyl oder ($C_1$-$C_6$)Halogenalkylsulfonyl steht.

Weiterhin besonders bevorzugt sind die Verbindungen der Formeln (Ia) oder (Ib), bevorzugt der Formel (Ia), gemäß der oben beschriebenen Ausführungsformen, insbesondere gemäß Ausgestaltung (1) oder Ausgestaltung (2) oder Ausgestaltung (3) oder Ausgestaltung (4) oder Ausgestaltung (5) oder Ausgestaltung (6), wobei Verbindungen der Formel (Y), in welchen

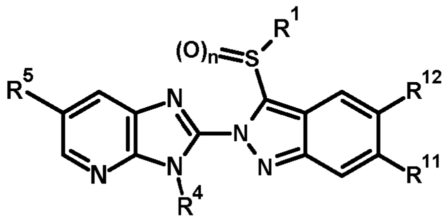

(Y)

| | |
|---|---|
| $R^1$ | für ($C_1$-$C_6$)Alkyl, |
| $R^4$ | für ($C_1$-$C_6$)Alkyl, |
| $R^5$ | für ($C_1$-$C_6$)Halogenalkyl, ($C_1$-$C_6$)Halogenalkylthio, ($C_1$-$C_6$) Halogenalkylsulfinyl oder ($C_1$-$C_6$)Halogenalkylsulfonyl, |
| $R^{11}$ und $R^{12}$ | unabhängig voneinander jeweils für Wasserstoff, Halogen oder ($C_1$-$C_6$) Halogenalkyl und |
| n | für 0, 1 oder 2 |

steht, ausgenommen sind.

Weiterhin besonders bevorzugt sind die Verbindungen der Formeln (Ia) oder (Ib), bevorzugt der Formel (Ia),

gemäß der oben beschriebenen Ausführungsformen, insbesondere gemäß Ausgestaltung (1) oder Ausgestaltung (2) oder Ausgestaltung (3) oder Ausgestaltung (4) oder Ausgestaltung (5) oder Ausgestaltung (6), wobei Verbindungen der Formel (Y), in welchen

$R^1$ für Ethyl,
$R^4$ für Methyl,
$R^5$ für Trifluormethyl,
$R^{11}$ für Wasserstoff,
$R^{12}$ für Trifluormethyl und
n für 0 oder 2

steht, ausgenommen sind.

Ein besonders bevorzugter Aspekt der Erfindung umfasst die Verbindungen der Formeln (Ia) oder (Ib), bevorzugt der Formel (Ia), in welche 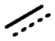 ausschließlich für Einfachbindungen steht.

Insbesondere bevorzugt ergibt sich dabei für die Formel (Ia) die folgende Struktur (Ia2),

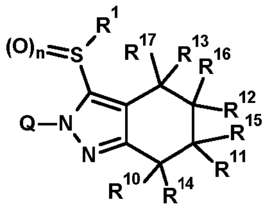

(Ia2)

wobei $R^1$, $R^{10}$, $R^{11}$, $R^{12}$, $R^{13}$, $R^{14}$, $R^{15}$, $R^{16}$, $R^{17}$, Q, $R^4$, $R^5$, $R^6$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

Ganz besonders bevorzugt ergeben sich die Verbindungen der Formeln (Ia8) bis (Ia10)

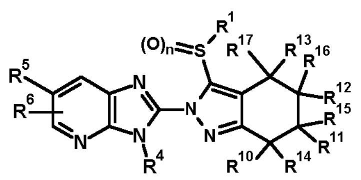

(Ia8)

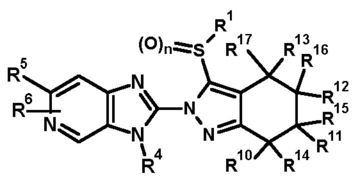

(Ia9)

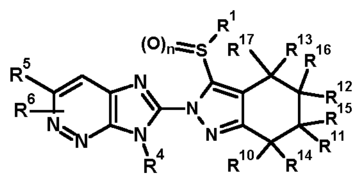

(Ia10)

wobei $R^1$, $R^{10}$, $R^{11}$, $R^{12}$, $R^{13}$, $R^{14}$, $R^{15}$, $R^{16}$, $R^{17}$, $R^4$, $R^5$, $R^6$ und n die in Ausgestaltung (1) oder Ausgestaltung (2) oder Ausgestaltung (3) oder Ausgestaltung (4) oder Ausgestaltung (5) oder Ausgestaltung (6) beschriebenen Bedeutungen haben.

Hervorgehoben sind dabei die Verbindungen der Formeln (Ia8) und (Ia9).

Insbesondere ergeben sich die Strukturen (Ia20) bis (Ia22)

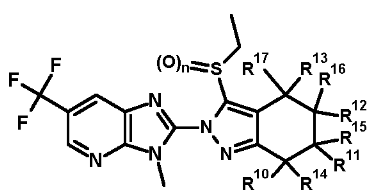

(Ia20)

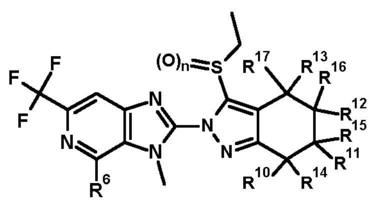

(Ia21)

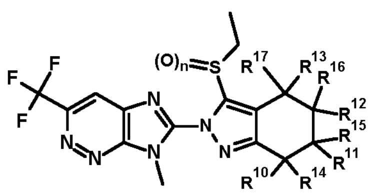

(Ia22)

wobei $R^6$, $R^{10}$, $R^{11}$, $R^{12}$, $R^{13}$, $R^{14}$, $R^{15}$, $R^{16}$, $R^{17}$ und n die in Ausgestaltung (1) oder Ausgestaltung (2) oder Ausgestaltung

(3) oder Ausgestaltung (4) oder Ausgestaltung (5) oder Ausgestaltung (6) beschriebenen Bedeutungen haben.

Insbesondere bevorzugt sind dabei weiterhin die Strukturen der Formeln I-10 und I-11.

Weiterhin bevorzugt sind Verbindungen der Formeln (Ia) oder (Ib), bevorzugt der Formel (Ia), wobei mindestens eine der Variablen Aa, Ab, Ac und Ad für Stickstoff steht, besonders bevorzugt steht eine der Variablen Aa, Ab,Ac und Ad für Stickstoff. Ganz besonders bevorzugt steht dabei ausschließlich die Variable Ab für Stickstoff.

Definitionsgemäß ist, sofern nichts anderes angegeben ist, Halogen ausgewählt aus der Reihe Fluor, Chlor, Brom und Iod, bevorzugt wiederum aus der Reihe Fluor, Chlor und Brom.

Sofern nicht an anderer Stelle anders definiert, wird unter dem Begriff "Alkyl", entweder in Alleinstellung oder aber in Kombination mit weiteren Begriffen, wie beispielsweise Halogenalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für $C_1$-$C_{12}$-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl.

Sofern nicht an anderer Stelle anders definiert, wird unter dem Begriff "Alkenyl", entweder in Alleinstellung oder aber in Kombination mit weiteren Begriffen, erfindungsgemäß ein linearer oder verzweigter $C_2$-$C_{12}$-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Sofern nicht an anderer Stelle anders definiert, wird unter dem Begriff "Alkinyl", entweder in Alleinstellung oder aber in Kombination mit weiteren Begriffen, erfindungsgemäß ein linearer oder verzweigter $C_2$-$C_{12}$-Alkinylrest, welcher mindestens eine Dreifachbindung aufweist, beispielsweise Ethinyl, 1-Propinyl und Propargyl, verstanden. Der Alkinylrest kann dabei auch mindestens eine Doppelbindung aufweisen.

Sofern nicht an anderer Stelle anders definiert, wird unter dem Begriff "Cycloalkyl", entweder in Alleinstellung oder aber in Kombination mit weiteren Begriffen, erfindungsgemäß ein $C_3$-$C_8$-Cycloalkylrest verstanden, beispielsweise Cyclopropyl, Cyclobutyl, Cyclopentyl, Cyclohexyl, Cycloheptyl und Cyclooctyl, verstanden. Unter dem Begriff "Alkoxy", entweder in Alleinstellung oder aber in Kombination mit weiteren Begriffen, wie beispielsweise Halogenalkoxy, wird vorliegend ein Rest O-Alkyl verstanden, wobei der Begriff "Alkyl" die oben stehende Bedeutung aufweist.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Sofern nicht an anderer Stelle anders definiert, wird unter dem Begriff "Aryl" erfindungsgemäß ein aromatischer Rest mit 6 bis 14 Kohlenstoffatomen, vorzugsweise Phenyl, Naphthyl, Anthryl oder Phenanthrenyl, besonders bevorzugt Phenyl, verstanden.

Sofern nicht an anderer Stelle anders definiert, wird unter dem Begriff "Arylalkyl" eine Kombination von erfindungsgemäß definierten Resten "Aryl" und "Alkyl" verstanden, wobei der Rest im Allgemeinen über die Alkylgrupe gebunden wird, Beispiele hierfür sind Benzyl, Phenylethyl oder $\alpha$-Methylbenzyl, wobei Benzyl besonders bevorzugt ist.

Sofern nicht an anderer Stelle anders definiert, bedeutet "Hetaryl" eine mono-, bi- oder tricyclische heterocyclische Gruppe aus C-Atomen und mindestens einem Heteroatom, wobei mindestens ein Zyklus aromatisch ist. Bevorzugt enthält die Hetaryl-Gruppe 3, 4, 5, 6, 7 oder 8 C-Atome und ist ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl, Imidazopyridinyl und Indolizinyl.

Die Verbindungen der Formel (Ia) oder (Ib) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere.

Die Verbindungen der Formel (I) können auch als Salze, insbesondere Säureadditionssalze und Metallsalzkomplexe, vorliegen. Die Verbindungen der Formel (I) und deren Säureadditionssalze und Metallsalzkomplexe besitzen gute Wirksamkeit, insbesondere zur Bekämpfung von tierischen Schädlingen.

Als geeignete Salze der Verbindungen der allgemeinen Formel (I) können übliche nicht toxische Salze, d. h. Salze mit entsprechenden Basen und Salze mit zugesetzten Säuren genannt werden. Vorzugsweise sind Salze mit anorganischen Basen, wie Alkalimetallsalze, beispielsweise Natrium-, Kalium- oder Cäsiumsalze, Erdalkalimetallsalze, beispielsweise Calzium- oder Magnesiumsalze, Ammoniumsalze, Salze mit organischen Basen sowie mit anorganischen Aminen, beispielsweise Triethylammonium-, Dicyclohexylammonium-, N,N'-Dibenzylethylendiammonium-, Pyridinium-, Picolinium- oder Ethanolammoniumsalze, Salze mit anorganischen Säuren, beispielsweise Hydrochloride, Hydrobromide, Dihydrosulfate, Trihydrosulfate, oder Phosphate, Salze mit organischen Carbonsäuren oder organischen Sulfosäure, beispielsweise Formiate, Acetate, Trifluoracetate, Maleate, Tartrate, Methansulfonate, Benzolsulfonate oder para-Toluolsulfonate, Salze mit basischen Aminosäuren, beispielsweise Arginate, Aspartate oder Glutamate und Ähnliches zu nennen.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (Ia) oder (Ib), in welchen eine Kombination der vorstehend als bevorzugt aufgeführten Bedeutungen vorliegt.

Erfindungsgemäß besonders bevorzugt verwendet werden Verbindungen der Formel (Ia) oder (Ib), in welchen eine Kombination der vorstehend als besonders bevorzugt aufgeführten Bedeutungen vorliegt.

Erfindungsgemäß ganz besonders bevorzugt verwendet werden Verbindungen der Formel (Ia) oder (Ib), in welchen eine Kombination der vorstehend als ganz besonders bevorzugt aufgeführten Bedeutungen vorliegt.

Erfindungsgemäß hervorgehoben verwendet werden Verbindungen der Formel (Ia) oder (Ib), in welchen eine Kombination der vorstehend als hervorgehoben aufgeführten Bedeutungen vorliegt.

Erfindungsgemäß insbesonders verwendet werden Verbindungen der Formel (Ia) oder (Ib), in welchen eine Kombination der vorstehend als insbesonders aufgeführten Bedeutungen vorliegt.

Die erfindungsgemäßen Verbindungen der Formel (Ia) oder (Ib) können durch die in den folgenden Schemata dargestellten Verfahren erhalten werden:

Verfahren A

Die Verbindungen der Formel (Ia), bei welchen Q für Q1, Q2, Q3, Q4, Q5, Q6, Q7, Q8, Q9, Q14 oder Q15 steht, können, wenn ^ ausschließlich für Doppelbindungen, Aa für $C(R^{10})$, Ab für $C(R^{11})$, Ac für $C(R^{12})$ und Ad für $C(R^{13})$ steht, wie unten in Schema 1 beschrieben, nach bekannten Methoden hergestellt werden.

**Schema 1**

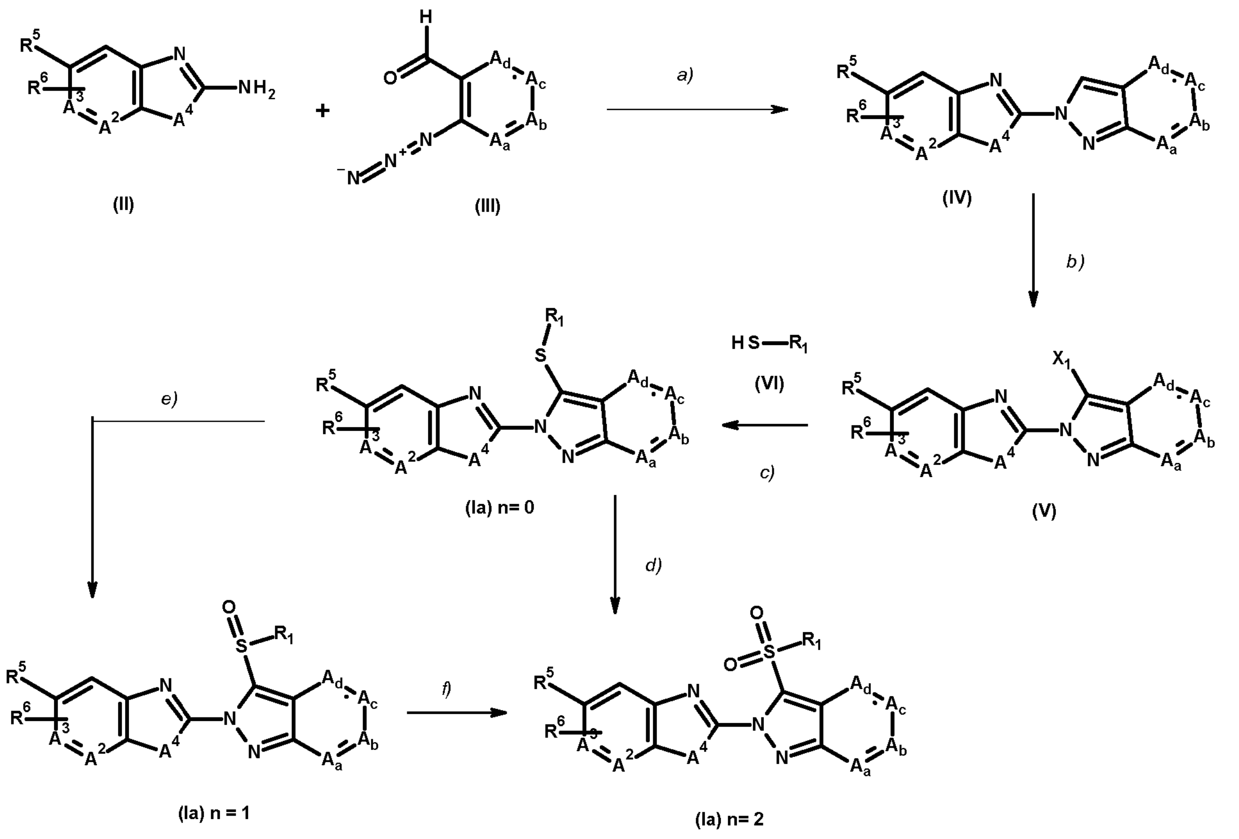


Ganz analog erfolgt die Herstellung von Verbindungen der Formel (Ia) bei denen Q für Q10, Q11, Q12 oder

Q13 steht.

**Schritt a)**

Die Verbindungen der Formel (IV) lassen sich herstellen durch Umsetzung der Verbindungen der Formel (II) und (III) (US2009/18132 A1, 2009; US3966760 A1, 1976; Chemical Communications, 2011, 47, 10133.

Verbindungen der Formel (III) sind entweder kommerziell erhältlich oder können nach bekannten Verfahren hergestellt warden. Siehe hierzu zum Beispiel US2012/214837 A1, 2012, WO2015/116882 A1, 2015, Journal of Organic Chemistry, 1995, vol. 60, # 7 p. 2254 - 2256.

Verbindungen der Formel (II) sind entweder kommerziell erhältlich oder können nach bekannten Verfahren hergestellt werden. Siehe hierzu zum Beispiel Bioorganic and medicinal chemistry letters, 2002, vol. 12, # 16 p. 2221 - 2224, WO2011/71725 A1, 2011, US2016/31875 A1, 2016 oder beispielsweise WO2010/19899 A1, 2010, Journal of Medicinal Chemistry, 1999 , vol. 42, # 1 p. 50 - 59

Die Bildung der Imin-Zwischenstufe wird durch wasserziehende Zusätze wie z.B. Molekularsieb begünstigt. Geeignete Katalysatoren sind Broenstedt- und Lewissäuren beispielsweise Essigsäure und Tetraisopropylorthotitanat. Die Cyclisierung kann durch Zugabe von Kupfersalzen wie CuI und Aminliganden wie N,N,N',N,-tetramethylethylenediamine beschleunigt werden.

Die Umsetzung zu Verbindung der Formel (IV) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden hochsiedende Lösemittel wie Dimethylformamid oder Toluol.

Die Reaktion kann im Vakuum, bei Normaldruck oder unter Überdruck und bei Temperaturen von 0 °C bis 200 °C durchgeführt werden.

Verbindung II lässt sich im Falle Q = Q1 nach bekannten Methoden herstellen. Siehe hierzu beispielsweise European Journal of Medicinal Chemistry, 2014 , vol. 78, p. 35 - 42. Ganz analog lässt sich auch Verbindung IIa im Falle Q = Q15 herstellen.

Verbindung II lässt sich im Falle Q = Q2 nach bekannten Methoden herstellen. Siehe hierzu beispielsweise Journal of Heterocyclic Chemistry, 1995 , vol. 32, # 2 p. 467 - 471. Ganz analog lässt sich auch Verbindung II im Falle Q = Q14 herstellen. Zur Herstellung von Pyridazinvorstufen für Q14 siehe auch WO2016/039411

Verbindung II lässt sich im Falle Q = Q3 nach bekannten Methoden herstellen. Siehe hierzu beispielsweise Journal of Heterocyclic Chemistry, 1997 , vol. 34, # 3 p. 717 - 727

Verbindung II lässt sich im Falle Q = Q4 nach bekannten Methoden herstellen. Siehe hierzu beispielsweise WO2013/23184 A1, 2013

Verbindung II lässt sich im Falle Q = Q5 nach bekannten Methoden herstellen. Siehe hierzu beispielsweise WO2009/147431 A1, 2009. Ganz analog lässt sich auch Verbindung II im Falle Q = Q6 herstellen.

Verbindung II lässt sich im Falle Q = Q7 nach bekannten Methoden herstellen. Siehe hierzu beispielsweise Synlett, 2016, vol. 27, # 12 art. no. ST-2015-B0983-L, p. 1798 - 1802

Verbindung II lässt sich im Falle Q = Q8 nach bekannten Methoden herstellen. Siehe hierzu beispielsweise Chemical and pharmaceutical bulletin, 1979, vol. 27, # 2 p. 410 - 418. Ganz analog lässt sich auch Verbindung IIa im Falle Q = Q9 herstellen.

Verbindung II lässt sich im Falle Q = Q10 nach bekannten Methoden herstellen. Siehe hierzu beispielsweise WO2010/19899 A1, 2010

Verbindung II lässt sich im Falle Q = Q11 nach bekannten Methoden herstellen. Siehe hierzu beispielsweise WO2009/10530 A1, 2009

Verbindung II lässt sich im Falle Q = Q12 nach bekannten Methoden herstellen. Siehe hierzu beispielsweise WO2005/89763 A1, 2005. Ganz analog lässt sich auch Verbindung II im Falle Q = Q13 herstellen.

**Schritt b)**

Die Herstellung der Verbindungen der Formel (V), wobei $X^1$ für F, Cl oder Br stehen kann, erfolgt analog allgemein bekannter Bedingungen mit Hilfe eines entsprechenden Halogenierungsreagenzes. Geeignete Halogenierungsreagenzien sind beispielsweise N-Chlorsuccinimid ($X^1$ = Cl), N-Bromsuccinimid, Brom ($X^1$ = Br).

Die Umsetzung zu Verbindung der Formel (V) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Lösemittel wie zum Beispiel Tetrachlormethan, Tetrahydrofuran, Essigsäure.

Die Reaktion kann im Vakuum, bei Normaldruck oder unter Überdruck und bei Temperaturen von 0 °C bis 200 °C durchgeführt werden.

**Schritt c)**

Die Verbindungen der Formel (Ia), wobei n für 0 steht, lassen sich herstellen durch Umsetzung der Verbindungen der Formel (V) mit den Verbindungen der Formel (VI) in Gegenwart einer Base.

Mercaptanderivate der Formel (VI) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), 1329 beschriebenen Verfahren.

Die Umsetzung zu Verbindung der Formel (Ia), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten Hydrogencarbonaten und Carbonaten von Alkali- oder Erdalkalimetallen. Bevorzugt sind dabei Caesiumcarbonat, Natriumcarbonat Kaliumcarbonat und Natriumhydrogencarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

In der beschriebenen Reaktion steht $X^1$ bevorzugt für ein Fluor- oder Chloratom.

Alternativ kann für den Fall, dass $X^1$ für Brom steht auch mit einer geeigneten Lithiumbase transmetalliert und anschließend mit dem entsprechenden kommerziell erhältlichen Disulfid umgesetzt werden. Siehe hierzu Bioorganic and Medicinal Chemistry Letters, 20 (2010), 2770 - 2775.

Geeignete Lithiumbasen sind zum Beispiel n-Butyllithium.

Die Umsetzung zu Verbindung der Formel (Ia), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert. -Butylmethylether

Die Reaktion kann im Vakuum, bei Normaldruck oder unter Überdruck und bei Temperaturen von 0 °C bis 200 °C durchgeführt werden.

Die Reaktion kann in der Mikrowelle durchgeführt werden.

**Schritt d)**

Die Verbindungen der Formel (Ia), wobei n für 1 steht, lassen sich herstellen durch Oxidation der Verbindungen der Formel (Ia), wobei n für 0 steht. Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid, meta-Chlorperbenzoesäure oder Natriumperiodat.

Die Reaktion kann im Vakuum, bei Normaldruck oder unter Überdruck und bei Temperaturen von - 20°C °C bis 120 °C durchgeführt werden.

**Schritt e)**

Die Verbindungen der Formel (Ia), wobei n für 2 steht, lassen sich herstellen durch Oxidation der Verbindungen der Formel (Ia), wobei n für 1 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von - 20°C °C bis 120 °C durchgeführt werden.

**Schritt f)**

Die Verbindungen der Formel (Ia), wobei n für 2 steht, lassen sich auch in einem einstufigen Prozess herstellen

durch Oxidation der Verbindungen der Formel (Ia), wobei n für 0 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von - 20°C °C bis 120 °C durchgeführt werden.

**Verfahren B:**

Die Verbindungen der Formel (Ib), bei welchen Q für Q1, Q2, Q3, Q4, Q5, Q6, Q7, Q8, Q9, Q14, Q15 steht, können wie unten in Schema 2 beschrieben nach bekannten Methoden hergestellt werden.

**Schema 2**

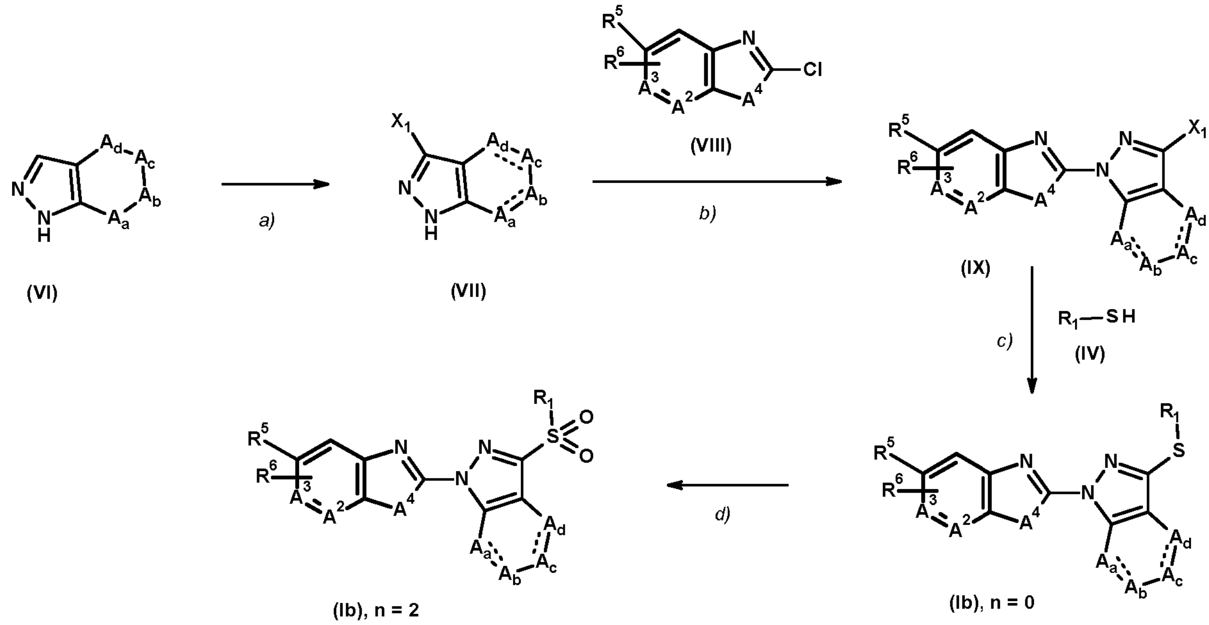


Ganz analog erfolgt die Herstellung von Verbindungen der Formel (Ib) bei denen Q für Q10, Q11, Q12, Q13 steht.

**Schritt a)**

Die Herstellung der Verbindungen der Formel (VII), wobei $X^1$ für F, Cl, Br oder Iod stehen kann, erfolgt analog allgemein bekannter Bedingungen mit Hilfe eines entsprechenden Halogenierungsreagenzes. Geeignete Halogenierungsreagenzien sind beispielsweise N-Chlorsuccinimid ($X^1$ = Cl), N-Bromsuccinimid, Brom ($X^1$ = Br) oder Iod/KOH ($X^1$ = I).

Die Umsetzung zu Verbindung der Formel (VII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Lösemittel wie zum Beispiel Tetrachlormethan, Tetrahydrofuran, Essigsäure verwendet.

Die Reaktion kann im Vakuum, bei Normaldruck oder unter Überdruck und bei Temperaturen von 0 °C bis 200 °C durchgeführt werden.

**Schritt b)**

Die Herstellung der Verbindung der Formel (IX) erfolgt wie zum Beispiel in WO2009/134750 A1 beschrieben, durch Umsetzung von Verbindung (VII) mit Verbindung (VIII) in Gegenwart einer Base. Geeignete Basen sind zum Beispiel Natriumhydrid, Cäsiumcarbonat, Kaliumcarbonat oder Triethylamin.

Die Umsetzung zu Verbindung der Formel (IX) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Lösemittel wie zum Beispiel Dimethylformamid.

Die Reaktion kann im Vakuum, bei Normaldruck oder unter Überdruck und bei Temperaturen von 0 °C bis 200 °C durchgeführt werden.

Verbindung (VIII) kann im Falle, dass Q = Q1, Q2, Q3, Q4, Q5, Q6, Q7, Q8, Q9, Q14, Q15 nach verschiedenen allgemeingültigen Verfahren hergestellt werden. Siehe hierzu zum Beispiel WO2009/134750 A1, 2009 oder Organic and Biomolecular Chemistry, 2007, vol. 5, # 16 p. 2567 - 2571 oder WO2006/116412 A2, 2006

Verbindung (VIII) kann im Falle, dass Q = Q10, Q11, Q12, Q13 Q15 nach verschiedenen allgemeingültigen Verfahren hergestellt werden. Siehe hierzu zum Beispiel WO2013/59928 A1, 2013, EP1466527 A1, 2004; KR2016/1508 A, 2016

**Schritt c)**

Im Falle das $X^1$ = Br oder I kann die Umsetzung von Verbindung (IX) in (Ib), n = 0 durch Reaktion mit Verbindung (IV) in Gegenwart eines Palladiumkatalysators und einer Base erfolgen. Ein geeigneter Palladiumkatalysator ist beispielsweise die Kombination von $Pd_2(dba)_3$ mit Xantphos als Liganden. Als Base eignet sich Diisopropylethylamin.

Im Falle das $X^1$ = F, Cl kann die Umsetzung von Verbindung (IX) in (Ib), wobei n für 0 steht durch Reaktion mit Verbindung (IV) und einer starken Base erfolgen. Beispiele für geeignete Basen sind anorganische Basen aus der Gruppe bestehend aus Acetaten, Phosphaten Hydrogencarbonaten und Carbonaten von Alkali- oder Erdalkalimetallen. Bevorzugt sind dabei Caesiumcarbonat, Natriumcarbonat Kaliumcarbonat und Natriumhydrogencarbonat. Weitere geeignete Basen sind Alkalimetallhydride wie z.B. Natriumhydrid.

Die Umsetzung zu Verbindung der Formel (Ib), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

**Schritt d)**

Die Verbindungen der Formel (Ib), wobei n für 2 oder 1 steht, lassen sich herstellen durch Oxidation der Verbindungen der Formel (Ib), wobei n für 0 steht. Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid, meta-Chlorperbenzoesäure oder Natriumperiodat.

Die Reaktion kann im Vakuum, bei Normaldruck oder unter Überdruck und bei Temperaturen von - 20°C °C bis 120 °C durchgeführt werden.

**Verfahren C**

Die Verbindungen der Formel (Ia), bei welchen Q für Q1, Q2, Q3, Q4, Q5, Q6, Q7, Q8, Q9, Q14 oder Q15 steht, können, wenn 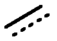 ausschließlich für Einfachbindungen stehen, Aa für $C(R^{10})(R^{14})$, Ab für $C(R^{11})(R^{15})$ Ac für $C(R^{12})(R^{16})$ und Ad für $C(R^{13})(R^{17})$ steht, wie unten in Schema 3 beschrieben, nach bekannten Methoden hergestellt werden.

**Schema 3**

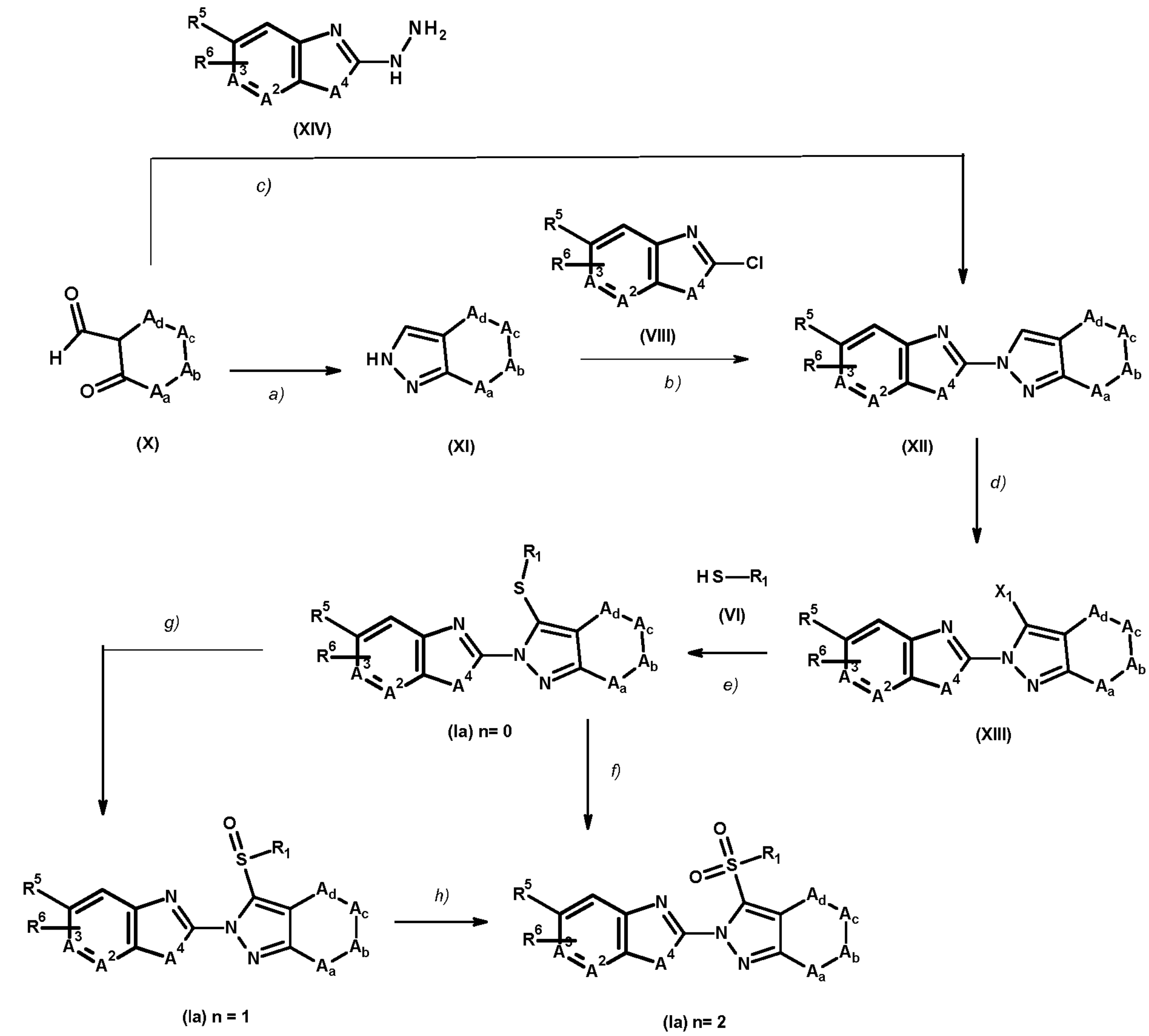


(XIV) (X) (XI) (VIII) (XII) (VI) (XIII) (Ia) n= 0 (Ia) n = 1 (Ia) n= 2

Ganz analog erfolgt die Herstellung von Verbindungen der Formel (Ia) bei denen Q für Q10, Q11, Q12 oder Q13 steht.

### Schritt a)

Verbindung (XI) ist kommerziell erhältlich oder lässt sich nach bekannten Methoden durch Cyclisierung von Verbindung (X) mit Hydrazin herstellen. Siehe hierzu zum Beispiel US 2015/0225397 A1.

Verbindung (X) ist kommerziell erhältlich oder lässt sich aus dem entsprechenden Cyclohexanon nach bekannten Methoden herstellen. Siehe hierzu zum Beispiel Tetrahedron, 1984, vol. 40, p. 1051 oder Journal of Organic Chemistry, 2006, vol. 71, # 16 p. 6149 - 6156.

### Schritt b)

Die Herstellung von Verbindungen der Formel (XII) erfolgt analog allgemein bekannter Bedingungen durch Kupplung von Verbindung (VIII) mit Verbindung (XI) in Gegenwart einer Base, siehe hierzu WO2009/134750. Eine geeignete Base ist beispielsweise Cäsiumcarbonat sowie weitere Alkalimetallcarbonate. Die Umsetzung zu Verbindung der Formel (XII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Lösemittel wie zum Beispiel Dimethylformamid.

Verbindung (VIII) kann im Falle, dass Q = Q1, Q2, Q3, Q4, Q5, Q6, Q7, Q8, Q9, Q14, Q15 nach verschiedenen allgemeingültigen Verfahren hergestellt werden. Siehe hierzu zum Beispiel WO2009/134750 A1, 2009 oder Organic and Biomolecular Chemistry, 2007, vol. 5, # 16 p. 2567 - 2571 oder WO2006/116412 A2, 2006

Verbindung (VIII) kann im Falle, dass Q = Q10, Q11, Q12, Q13 Q15 nach verschiedenen allgemeingültigen Verfahren hergestellt werden. Siehe hierzu zum Beispiel WO2013/59928 A1, 2013, EP1466527 A1, 2004; KR2016/1508 A, 2016

**Schritt c)**

Alternativ zu der zweistufigen Abfolge von Schritt a) und Schritt b) kann Verbindung (XII) auch in einer Stufe aus Verbindung (X) und Verbindung (XIV) erhalten werden. Die Reaktion erfolgt üblicherweise unter Säurekatalyse. Geeignete Säuren sind beispielsweise Schwefelsäure, Salzsäure, Essigsäure oder Toluolsulfonsäure. Als Beispiel siehe unter anderem Indian Journal of Chemistry, Section B: Organic Chemistry Including Medicinal Chemistry, 1991, vol. 30, # 3 p. 306 - 312 oder Justus Liebigs Annalen der Chemie, 1927, vol. 453, p. 228

Im Fall das Q = Q1, Q2, Q3, Q4, Q5, Q6, Q7, Q8, Q9, Q14, Q15 kann das Ausgangsmaterial (XIV) durch Umsetzung mit Hydrazin aus dem entsprechenden Halogenid (VIII) gewonnen werden. Siehe hierzu zum Beispiel Bioorganic Chemistry, 2015, vol. 60, p. 19 - 29 oder WO2009/134750 A1, 2009

Im Falle dass Q = Q10, Q11, Q12, Q13 kann das Ausgangsmaterial (XIV) beispielsweise wie in European Journal of Medicinal Chemistry, 2014, vol. 75, p. 492 - 500 oder Arzneimittel-Forschung/Drug Research, 1977, vol. 27, # 1 p. 82 - 89 beschrieben erhalten werden.

**Schritt d)**

Die Herstellung von Verbindungen der Formel (XIII), wobei $X^1$ für F, Cl oder Br stehen kann, erfolgt analog allgemein bekannter Bedingungen mit Hilfe eines entsprechenden Halogenierungsreagenzes. Geeignete Halogenierungsreagenzien sind beispielsweise N-Chlorsuccinimid ($X^1$ = Cl), N-Bromsuccinimid, Brom ($X^1$ = Br).

Die Umsetzung zu Verbindung der Formel (XIII) kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Lösemittel wie zum Beispiel Tetrachlormethan, Tetrahydrofuran, Essigsäure.

Die Reaktion kann im Vakuum, bei Normaldruck oder unter Überdruck und bei Temperaturen von 0 °C bis 200 °C durchgeführt werden.

**Schritt e)**

Die Verbindungen der Formel (Ia), wobei n für 0 steht, lassen sich herstellen durch Umsetzung der Verbindungen der Formel (XIII) mit den Verbindungen der Formel (VI) in Gegenwart einer Base.

Mercaptanderivate der Formel (VI) wie beispielsweise Methylmercaptan, Ethylmercaptan oder Isopropylmercaptan sind entweder kommerziell erhältlich oder können nach bekannten Methoden hergestellt werden, beispielsweise analog der in US2006/25633, US2006/111591, US2820062, Chemical Communications, 13 (2000), 1163-1164 oder Journal of the American Chemical Society, 44 (1922), 1329 beschriebenen Verfahren.

Die Umsetzung zu Verbindung der Formel (Ia), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise Diisopropylether, Dioxan, Tetrahydrofuran, 1,2-Dimethoxyethan, tert.-Butylmethylether; Nitrile, wie beispielsweise Acetonitril oder Propionitril; aromatische Kohlenwasserstoffe wie beispielsweise Toluol oder Xylol; aprotische polare Lösungsmittel wie beispielsweise N,N-Dimethylformamid, N-Methylpyrrolidon oder Dimethylsulfoxid.

Beispiele für geeignete Basen sind anorganische Basen aus der Gruppe bestehend aus Acetaten, Phosphaten Hydrogencarbonaten und Carbonaten von Alkali- oder Erdalkalimetallen. Bevorzugt sind dabei Caesiumcarbonat, Natriumcarbonat Kaliumcarbonat und Natriumhydrogencarbonat. Weitere geeignete Basen sind Alkalimetallhydride wie z.B. Natriumhydrid.

Die Reaktion kann im Vakuum, bei Normaldruck oder unter Überdruck und bei Temperaturen von 0 °C bis 200 °C durchgeführt werden.

In der beschriebenen Reaktion steht $X^1$ bevorzugt für ein Fluor- oder Chloratom.

Alternativ kann für den Fall, dass $X^1$ für Brom steht auch mit einer geeigneten Lithiumbase transmetalliert und anschließend mit dem entsprechenden kommerziell erhältlichen Disulfid umgesetzt werden. Siehe hierzu Bioorganic and Medicinal Chemistry Letters, 20 (2010), 2770 - 2775.

Geeignete Lithiumbasen sind zum Beispiel n-Butyllithium.

Die Umsetzung zu Verbindung der Formel (Ia), wobei n für 0 steht, kann in Substanz oder in einem Lösungsmittel erfolgen, vorzugsweise wird die Reaktion in einem Lösungsmittel durchgeführt, welches ausgewählt ist aus üblichen, bei den vorherrschenden Reaktionsbedingungen inerten Lösungsmitteln. Bevorzugt werden Ether wie beispielsweise

Diisopropylether, Dioxan, Tetrahydrofuran, 1,2-Dimethoxyethan, tert. -Butylmethylether

Die Reaktion kann im Vakuum, bei Normaldruck oder unter Überdruck und bei Temperaturen von 0 °C bis 200 °C durchgeführt werden.

Die Reaktion kann in der Mikrowelle durchgeführt werden.

**Schritt f)**

Die Verbindungen der Formel (Ia), wobei n für 1 steht, lassen sich herstellen durch Oxidation der Verbindungen der Formel (Ia), wobei n für 0 steht. Die Oxidation wird generell in einem Lösungsmittel durchgeführt, welches ausgewählt ist aus üblichen, bei den vorherrschenden Reaktionsbedingungen inerten Lösungsmitteln. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid, meta-Chlorperbenzoesäure oder Natriumperiodat.

Die Reaktion kann im Vakuum, bei Normaldruck oder unter Überdruck und bei Temperaturen von - 20°C °C bis 120 °C durchgeführt werden.

**Schritt g)**

Die Verbindungen der Formel (Ia), wobei n für 2 steht, lassen sich herstellen durch Oxidation der Verbindungen der Formel (Ia), wobei n für 1 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von - 20°C °C bis 120 °C durchgeführt werden.

**Schritt h)**

Die Verbindungen der Formel (Ia), wobei n für 2 steht, lassen sich auch in einem einstufigen Prozess herstellen durch Oxidation der Verbindungen der Formel (Ia), wobei n für 0 steht. Die Oxidation wird generell in einem Lösungsmittel durchgeführt. Bevorzugt werden halogenierte Kohlenwasserstoffe wie beispielsweise Dichlormethan, Chloroform, Tetrachlorkohlenstoff, 1,2-Dichlorethan oder Chlorbenzol; Alkohole wie Methanol oder Ethanol; Ameisensäure, Essigsäure, Propionsäure oder Wasser.

Beispiele für geeignete Oxidationsmittel sind Wasserstoffperoxid und meta-Chlorperbenzoesäure.

Die Reaktion kann im Vakuum, bei Normaldruck oder unter Überdruck und bei Temperaturen von - 20°C °C bis 120 °C durchgeführt werden.

**Verfahren und Verwendungen**

Im Folgenden ist der Begriff Formel (I) gleichbedeutend mit Formel (Ia) und Formel (Ib).

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere

Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Der Begriff "Hygienesektor" deckt alle Gebiete, technischen Felder und industriellen Anwendungen ab, bei denen diese Hygienemaßnahmen, -vorschriften und -verfahrensweisen wichtig sind, zum Beispiel im Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden. Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:

Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;

aus der Klasse der Chilopoda z. B. Geophilus spp., Scutigera spp.;

aus der Ordnung oder der Klasse der Collembola z. B. Onychiurus armatus; Sminthurus viridis;

aus der Klasse der Diplopoda z. B. Blaniulus guttulatus;

aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;

aus der Ordnung der Coleoptera z. B. Acalymma vittatum, Acanthoscelides obtectus, Adoretus spp., Aethina tumida, Agelastica alni, Agrilus spp., z. B. Agrilus planipennis, Agrilus coxalis, Agrilus bilineatus, Agrilus anxius, Agriotes spp., z. B. Agriotes linneatus, Agriotes mancus, Alphitobius diaperinus, Amphimallon solstitialis, Anobium punctatum, Anoplophora spp., z. B. Anoplophora glabripennis, Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dendroctonus spp., z. B. Dendroctonus ponderosae, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Lissorhoptrus oryzophilus, Listronotus (=Hyperodes) spp., Lixus spp., Luperodes spp., Luperomorpha xanthodera, Lyctus spp., Megacyllene spp., z. B. Megacyllene robiniae, Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affmis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Scolytus spp., z. B. Scolytus multistriatus, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;

aus der Ordnung der Dermaptera z. B. Anisolabis maritime, Forficula auricularia, Labidura riparia;

aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;

aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis

fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;

aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;

aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., z. B. Sirex noctilio, Solenopsis invicta, Tapinoma spp., Technomyrmex albipes, Urocerus spp., Vespa spp., z. B. Vespa crabro, Wasmannia auropunctata, Xeris spp.;

aus der Ordnung der Isopoda z. B. Armadillidium vulgare, Oniscus asellus, Porcellio scaber;

aus der Ordnung der Isoptera z. B. Coptotermes spp., z. B. Coptotermes formosanus, Comitermes cumulans,

Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;

aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Dioryctria spp., z. B. Dioryctria zimmermani, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens , Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Podesia spp., z. B. Podesia syringae, Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;

aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;

aus der Ordnung der Phthiraptera z. B. Damalinia spp., Haematopinus spp., Linognathus spp., Pediculus spp., Phylloxera vastatrix, Phthirus pubis, Trichodectes spp.;

aus der Ordnung der Psocoptera z. B. Lepinotus spp., Liposcelis spp.;

aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;

aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;

aus der Ordnung der Zygentoma (= Thysanura), z. B. Ctenolepisma spp., Lepisma saccharina, Lepismodes inquilinus, Thermobia domestica;

aus der Klasse der Symphyla z. B. Scutigerella spp., z. B. Scutigerella immaculata;

Schädlinge aus dem Stamm der Mollusca, z. B. aus der Klasse der Bivalvia, z. B. Dreissena spp.;

sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;

Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella omata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

**Formulierungen**

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropylguar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einer oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

## Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

## Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.

(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos,

Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.

(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.

(3) Natrium-Kanal-Modulatoren, wie beispielsweise Pyrethroide, z. B. Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(lR)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.

(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamethoxam oder Nicotin oder Sulfoxaflor oder Flupyradifurone.

(5) Allosterische Modulatoren des nicotinischen Acetylcholin-Rezeptors (nAChR), wie beispielsweise Spinosyne, z. B. Spinetoram und Spinosad.

(6) Allosterische Modulatoren des Glutamat-abhängigen Chloridkanals(GluCl), wie beispielsweise Avermectine/Milbemycine, z. B. Abamectin, Emamectin-benzoat, Lepimectin und Milbemectin.

(7) Juvenilhormon-Mimetika, wie beispielsweise Juvenilhormon-Analoge, z. B. Hydropren, Kinopren und Methopren oder Fenoxycarb oder Pyriproxyfen.

(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.

(9) Modulatoren chordotonaler Organe, z. B. Pymetrozin oder Flonicamid.

(10) Milbenwachstumsinhibitoren, wie z. B. Clofentezin, Hexythiazox und Diflovidazin oder Etoxazol.

(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und B.t.-Pflanzenproteine: Cry1Ab, CrylAc, CrylFa, CrylA.105, Cry2Ab, VIP3A, mCry3A, Cry3Ab, Cry3Bb, Cry34Ab1/35Ab1.

(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.

(13) Entkoppler der oxidativen Phoshorylierung durch Störung des Protonengradienten, wie beispielsweise Chlorfenapyr, DNOC und Sulfluramid.

(14) Blocker des nicotinischen Acetylcholinrezeptorkanals, wie beispielsweise Bensultap, Cartaphydrochlorid, Thiocyclam und Thiosultap-Natrium.

(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.

(16) Inhibitoren der Chitinbiosynthese, Typ 1, wie beispielsweise Buprofezin.

(17) Häutungsdisruptor (insbesondere bei Dipteren, d. h. Zweiflüglern), wie beispielsweise Cyromazin.

(18) Ecdyson-Rezeptor-Agonisten, wie beispielsweise Chromafenozid, Halofenozid, Methoxyfenozid und Tebufenozid.

(19) Oktopamin-Rezeptor-Agonisten, wie beispielsweise Amitraz.

(20) Mitochondriale Komplex-III-Elektronentransportinhibitoren, wie beispielsweise Hydramethylnon oder Acequinocyl oder Fluacrypyrim.

(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).

(22) Blocker des spannungsabhängigen Natriumkanals, wie z. B. Indoxacarb oder Metaflumizone.

(23) Inhibitoren der Acetyl-CoA-Carboxylase, wie beispielsweise Tetron- und Tetramsäurederivate, z. B. Spirodiclofen, Spiromesifen und Spirotetramat.

(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und Natriumcyanid.

(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.

(28) Ryanodinrezeptor-Modulatoren, wie beispielsweise Diamide, z. B. Chlorantraniliprol, Cyantraniliprol und Flubendiamid,

weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tioxazafen, Thiofluoximat, Triflumezopyrim und Iodmethan; des Weiteren Präparate auf Basis von *Bacillus firmus* (I-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-1-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfinyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H -pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethyl-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e][1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentafluorethoxy)phenyl]-1H-1,2,4-triazol-3-yl]phenyl]carbamat]-$\alpha$-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8-(2-Cyclopropylmethoxy-4-trifluormethylphenoxy)-3-(6-trifluormethylpyridazin-3-yl)-3-azabicyclo[3.2.1]octan (bekannt aus WO 2007040280 A1, WO 2007040282 A1) (CAS 934001-66-8) und N-[3-Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9).

**Fungizide**

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.

1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-1-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1 ,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4- Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4- Dichlorphenyl)-5-hydroxy-2,6,6-trimethylheptan-4-yl]-2,4-dihydro-3H-1,2,4-triazol-3-thion, (1.049) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(1H-1,2,4-triazol-1-yl)pentan-2-01, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1- {[3-(2-chlorphenyl)-2-(2,4-difluorphenyl)oxiran-2-yl]methyl}-1H-1,2,4-triazol, (1.061) 5-(Allylsulfanyl)-1- {[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4- {[3-(2,2,3,3-tetrafluorpropoxy)phenyl]sulfanyl}phenyl)-N-ethyl-N-methylimidoformamid, (1.066) N'-(2,5-Dimethyl-4- {[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4- {3-[(2,2,2-trifluorethyl)sulfanyl]phenoxy}phenyl)-N-ethyl-N-methylimidoformamid, (1.069) N'-(2,5-Dimethyl-4- {3-[(2,2,3,3-tetrafluorpropyl)sulfanyl]phenoxy}phenyl)-N-ethyl-N-methylimidoformamid, (1.070) N'-(2,5-Dimethyl-4- {3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4- {[3-(Difluormethoxy)phenyl]sulfanyl}-2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-1,3-thiazol-2-yl)oxy]-2,5-dimethylphenyl}-N-ethyl-N-methylimidoformamid, (1.076) N'-{5-Brom-6-[(1R)-1-(3,5-difluorophenyl)ethoxy]-2-methylpyridin-3-yl}-N-ethyl-N-methylimidoformamid, (1.077) N'-{5-Brom-6-[(1S)-1-(3,5-difluorphenyl)ethoxy]-2-methylpyridin-3-yl} -N-ethyl-N-methylimidoformamid, (1.078) N'- {5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'-{5-Bromo-6-[1-(3,5-difluorphenyl)ethoxy]-2-methylpyridin-3-yl}-N-ethyl-N-methylimidoformamid, (1.081) Mefentrifluconazole, (1.082) Ipfentrifluconazole.

2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4-{[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-[1-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluormethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.

3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2- {2-[(2,5-Dimethylphenoxy)methyl]phenyl} -2-methoxy-N-methylacetamid, (3.024) (2S)-2-{2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl}-2-methoxy-N-methylacetamid, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid, (3.029) Methyl {5-[3-(2,4-dimethylphenyl)-1H-pyrazol-1-yl]-2-methylbenzyl}carbamate.

4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.

5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram, (5.023) 6-Ethyl-5,7-dioxo-6,7-dihydro-5H-pyrrolo[3',4':5,6][1,4]dithiino[2,3-c][1,2]thiazole-3-carbonitrile.

6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.

7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.

(8) Inhibitoren der ATP-Produktion, beispielsweise (8.001) Silthiofam.

9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.

10) Inhibitoren der Lipid- und Membran-Synthese, beispielsweise (10.001) Propamocarb, (10.002) Propamocarbhydrochlorid, (10.003) Tolclofos-Methyl.

11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.

12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).

13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.

14) Verbindungen, die als Entkoppler wirken können, beispielsweise (14.001) Fluazinam, (14.002) Meptyldinocap.

15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-l-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2-{2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]amino}oxy)methyl]pyridin-2-yl}carbamat, (15.062) 5-Fluor-4-imino-3-methyl-1-[(4-methylphenyl)sulfonyl]-3,4-dihydropyrimidin-2(1H)-one.

**Biologische Schädlingsbekämpfungsmittel als Mischungskomponenten**

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:

*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus*, insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai*, insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:

*Beauveria bassiana*, insbesondere Stamm ATCC 74040, *Coniothyrium minitans*, insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus (*neu: *Isaria fumosorosea)*, insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride*, insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:

*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:

*Agrobacterium spp., Azorhizobium caulinodans*, *Azospirillum spp., Azotobacter spp., Bradyrhizobium spp., Burkholderia spp.,* insbesondere *Burkholderia cepacia* (ehemals bekannt als *Pseudomonas cepacia), Gigaspora spp.*, oder *Gigaspora monosporum, Glomus spp., Laccaria spp., Lactobacillus buchneri, Paraglomus spp.*, *Pisolithus tinctorus*, *Pseudomonas spp., Rhizobium spp.,* insbesondere *Rhizobium trifolii, Rhizopogon spp., Scleroderma spp.*, *Suillus spp.*, *Streptomyces spp.*.

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:

Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

**Safener als Mischungskomponenten**

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

**Pflanzen und Pflanzenteile**

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben).

Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

**Transgene Pflanze, Saatgutbehandlung und Integrationsereignisse**

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die

erhöhte Abwehrfähigkeit der Pflanzen gegen Insekten, Spinnentiere, Nematoden und Schnecken.

**Pflanzenschutz - Behandlungsarten**

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

**Saatgutbehandlung**

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische

Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

## Vektorbekämpfung

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:

1) Mücken

- Anopheles: Malaria, Filariose;
- Culex: Japanische Encephalitis, Filariasis, weitere virale Erkrankungen, Übertragung von anderen Würmern;
- Aedes: Gelbfieber, Dengue-Fieber, weitere virale Erkrankungen, Filariasis;
- Simulien: Übertragung von Würmern, insbesondere Onchocerca volvulus;
- Psychodidae: Übertragung von Leishmaniose

2) Läuse: Hautinfektionen, epidemisches Fleckfieber;

3) Flöhe: Pest, endemisches Fleckfieber, Bandwürmer;

4) Fliegen: Schlafkrankheit (Trypanosomiasis); Cholera, weitere bakterielle Erkrankungen;

5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;

6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Forsten, in Gärten und Freizeiteinrichtungen sowie im Vorrats-und Materialschutz.

**Schutz von technischen Materialen**

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

**Bekämpfung von tierischen Schädlingen auf dem Hygienesektor**

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen

bzw. passiven Verdampfungssystemen, Mottenpapieren, Mottensäckchen und Mottengelen, als Granulate oder Stäube, in Streuködern oder Köderstationen.

**Herstellungsbeispiele**

**Methoden:**

Die Bestimmung des $M^+$ mit der LC-MS im sauren Bereich erfolgte bei pH 2,7 mit 0,1 % wässriger Ameisensäure und Acetonitril (enthält 0,1% Ameisensäure) als Eluenten; linearer Gradient von 10% Acetonitril bis 95% Acetonitril, Gerät: Agilent 1100 LC-System, Agilent MSD System, HTS PAL.

Die Bestimmung des $M^+$ mit der LC-MS im neutralen Bereich erfolgte bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die NMR-Daten ausgewählter Beispiele werden entweder in klassischer Form (δ-Werte, Multiplettaufspaltung, Anzahl der H-Atome) oder als NMR-Peak-Listen aufgeführt.

Das Lösungsmittel, in welchem das NMR-Spektrum aufgenommen wurde ist jeweils angegeben.

**2-[6-Chlor-3-(ethylsulfonyl)-2H-indazol-2-yl]-3-methyl-6-(trifluormethyl)-3H-imidazo[4,5-b]pyridin (Bsp. I-1)**

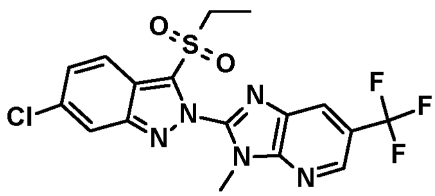

Bei Raumtemperatur wurden zu 2-[6-Chlor-3-(ethylsulfanyl)-2H-indazol-2-yl]-3-methyl-6-(trifluormethyl)-3H-imidazo[4,5-b]pyridin (175 mg, 0.43 mmol) in Dichlormethan (10 mL) Ameisensäure (122 mg, 2.9 mmol, 88%) and Wasserstoffperoxid (241 mg, 2.1 mmol, 30%) gegeben und das Gemisch für 2 h gerührt. Die Reaktionslösung wurde mit Essigester verdünnt und mit gesättigter Natriumchloridlösung gewaschen. Die organische Phase wurde über Natriumsulfat getrocknet und nach Filtration am Rotationsverdampfer eingeengt. Der Rückstand wurde durch säulenchromatographische Aufreinigung über präparative HPLC mit einem Wasser / Acetonitril Gradienten als Laufmittel gereinigt.

$MH^+$: 444; $^1$H-NMR(300 MHz, D6-DMSO) δ ppm: 9.01 (s, 1H), 8.85 (s, 1H), 8.22 (s, 1H), 8.10 - 8.07 (d, 1H), 7.59 - 7.55 (d, 1H), 3.97- 3.88 (q, 2H), 3.83 (s, 3H), 1.31 -1.26 (t, 3H).

**2-[6-Chlor-3-(ethylsulfanyl)-2H-indazol-2-yl]-3-methyl-6-(trifluormethyl)-3H-imidazo[4,5-b]pyridin (Bsp. I-8)**

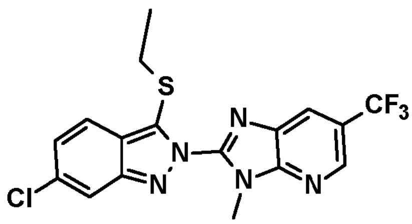

Bei Raumtemperatur wurden zu 2-(3-Brom-6-chlor-2H-indazol-2-yl)-3-methyl-6-(trifluormethyl)-3H-imidazo[4,5-b]pyridin (300 mg, 0.69 mmol), gelöst in Dioxan(8 mL), Ethylmercaptan (87 mg, 1.4 mmol), $Pd_2(dba)_3$ (36 mg, 0.035 mmol), Xantphos(40 mg, 0.069 mol) und *N*,*N*-Diisopropylethylamin (270 mg, 2.1 mmol) zugegeben und das Gemisch wurde über Nacht gerührt. Die Reaktionslösung wurde mit Essigester verdünnt und mit gesättigter Natriumchloridlösung gewaschen. Die organische Phase wurde über Natriumsulfat getrocknet und nach Filtration am Rotationsverdampfer eingeengt. Der Rückstand wurde durch säulenchromatographische Aufreinigung über präparative HPLC mit einem Wasser / Acetonitril Gradienten als Laufmittel gereinigt.

$MH^+$: 412; $^1H$-NMR(300 MHz, $D_6$-DMSO) δ ppm: 8.97 (s, 1H), 8.81 (s, 1H), 8.00 - 7.93 (m, 2H), 7.34 - 7.30 (d, 1H), 3.83 (s, 3H), 3.06-3.03 (q, 2H), 1.13 -1.08 (t, 3H).

**2-(3-Brom-6-chlor-2H-indazol-2-yl)-3-methyl-6-(trifluormethyl)-3H-imidazo[4,5-b]pyridin**

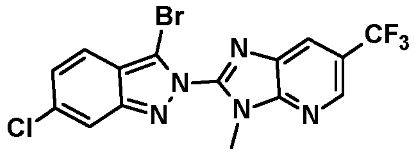

2-(6-Chlor-2H-indazol-2-yl)-3-methyl-6-(trifluormethyl)-3H-imidazo[4,5-b]pyridin (460 mg, 1.3 mmol) wurde in Essigsäure (10 ml) gelöst und es wurde N-Bromacetamid (773 mg, 13 mmol) zugegeben. Die Reaktionslösung wurde über Nacht bei Raumtemperatur gerührt. Nach Entfernen des Lösemittels am Rotationsverdampfer wurde der Rückstand chromatographisch aufgereinigt ($SiO_2$, Laufmittel Essigester/ Pentan).
$MH^+$: 432; $^1H$-NMR(300 MHz, $D_6$-DMSO) δ ppm: 8.98 (s, 1H), 8.83 (s, 1H), 8.00 (s, 1H), 7.80 - 7.77 (d, 1H), 7.33 - 7.30 (d, 1H), 3.91 (s, 3H),

**2-(6-Chlor-2H-indazol-2-yl)-3-methyl-6-(trifluormethyl)-3H-imidazo[4,5-b]pyridine**

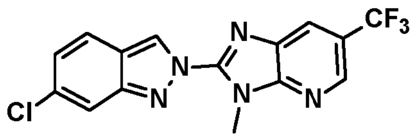

2-Azido-4-chlorbenzaldehyd (366 mg, 2.3 mmol) wurde in THF gelöst, und es wurden 3-Methyl-6-(trifluormethyl)-3H-imidazo[4,5-b]pyridin-2-amin (500 mg, 2.3 mmol) und Tetraisopropyltitanat (787 mg, 2.3 mmol) zugegeben. Das Reaktionsgemisch wurde für 2 h auf 60°C erhitzt. Dann wurde das Lösemittel abdestilliert und durch 10 ml Toluol ersetzt. Die Reaktionsmischung wurde weitere 12 h auf 110°C erhitzt. Nach Entfernen des Lösemittels wurde der Rückstand chromatographisch aufgereinigt ($SiO_2$, Laufmittel Essigester/Pentan).
$MH^+$: 352; $^1H$-NMR(300 MHz, $D_6$-DMSO) δ ppm: 9.33 (s, 1H), 8.86 (s, 1H), 8.64 (s, 1H), 7.94-7.90 (m, 2H), 7.23 - 7.20 (m, 1H), 4.24 (s, 3H)

**2-Azido-4-chlorbenzaldehyd**

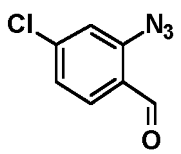

Zu einer Lösung von 4-Chlor-2-fluorobenzaldehyd (1 g, 6.3 mmol) in Dimethylformaid (10 ml) wurde Natriumazid addiert (620 mg, 10 mmol). Das Gemisch wurde für 12 h auf 60°C erhitzt. Die Reaktionslösung wurde mit Wasser verdünnt und anschließend mit Essigester extrahiert. Die vereinigten organischen Extrakte wurden mit gesättigter Natriumchloridlösung gewaschen und über Natriumsulfat getrocknet. Nach Entfernen des Lösemittels wurde das Rohprodukt ohne weitere Aufreinigung in der Folgereaktion eingesetzt.

**2-[5-Chlor-3-(ethylsulfonyl)-2H-indazol-2-yl]-3-methyl-6-(trifluormethyl)-3H-imidazo[4,5-b]pyridin (Bsp. I-2)**

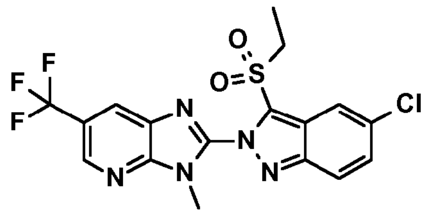

2-[5-Chlor-3-(ethylsulfanyl)-2H-indazol-2-yl]-3-methyl-6-(trifluormethyl)-3H-imidazo[4,5- b]pyridin (150 mg, 0.36 mmol) wurde in Dichlormethan (10 ml) gelöst, auf 0°C gekühlt und es wurde meta-Chlorperbenzoesäure (178 mg, 1.0 mmol) zugegeben. Das Reaktionsgemisch wurde auf Raumtemperatur erwärmt und weitere 4 h gerührt. Dann wurde die Lösung mit Dichlormethan verdünnt und mit gesättigter Natriumhydrogencarbonatlösung gewaschen. Die organische Phase wurde über Natriumsulfat getrocknet und am Rotationsverdampfer eingeengt. Der Rückstand wurde durch säulenchromatographische Aufreinigung über präparative HPLC mit einem Wasser / Acetonitril Gradienten als Laufmittel gereinigt.
$MH^+$: 444; $^1$H-NMR(300 MHz, $D_6$-DMSO) $\delta$ ppm: 9.02 (s, 1H), 8.85 (s, 1H), 8.12 - 8.06 (m, 2H), 7.68 - 7.65 (m, 1H), 3.94 - 3.89 (q, 2H), 3.83 (s, 3H), 1.32 - 1.28 (t, 3H).

**2-[5-Chlor-3-(ethylsulfanyl)-2H-indazol-2-yl]-3-methyl-6-(trifluormethyl)-3H-imidazo[4,5-b]pyridin (Bsp. I-9)**

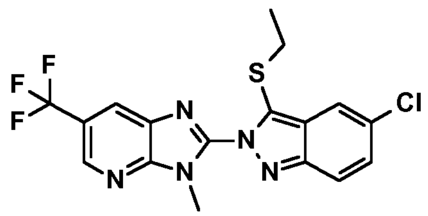

2-(3,5-Dichlor-2H-indazol-2-yl)-3-methyl-6-(trifluormethyl)-3H-imidazo[4,5-b]pyridin (300 mg, 0.78 mmol) wurde in 1,4-Dioxan (5 ml) gelöst und mit Ethylmercaptan (120 mg, 2.0 mmol), Diisopropylethylamin (258 mg, 2.0 mmol), $Pd_2(dba)_3CHCl_3$ (55 mg, 0.05 mmol) und Xantphos (55 mg, 0.1 mmol) versetzt. Das Reaktionsgemisch wurde für 4 h auf 110°C erhitzt. Nach Abkühlen auf Raumtemperatur wurde die Lösung mit Wasser gewaschen und die wässrige Phase zwei Mal mit Ethylacetat extrahiert. Die vereinigten organischen Extrakte wurden über Natriumsulfat getrocknet und am Rotationsverdampfer eingeengt. Der Rückstand wurde durch säulenchromatographische Aufreinigung über präparative TLC (Laufmittel: Dichlormethan/Pentan) gereinigt.
$MH^+$: 412; $^1$H-NMR(300 MHz, $D_6$-DMSO) $\delta$ ppm: 8.97 (s, 1H), 8.81 (s, 1H), 7.97-7.90 (m, 2H), 7.52 - 7.49 (d, 1H), 3.83 (s, 3H), 3.10 - 3.02 (q, 2H), 1.31 - 1.03 (t, 3H).

**2-(3,5-Dichlor-2H-indazol-2-yl)-3-methyl-6-(trifluormethyl)-3H-imidazo[4,5-b]pyridin**

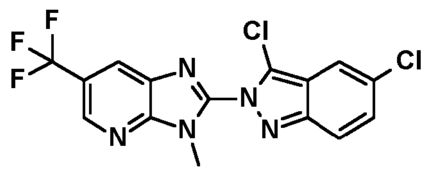

2-(5-Chlor-2H-indazol-2-yl)-3-methyl-6-(trifluormethyl)-3H-imidazo[4,5-b]pyridine (700 mg, 2.0 mmol) wurde in Essigsäure (10 ml) gelöst und mit N-Chlorsuccinimid (1.3 g, 10 mmol) versetzt. Die Reaktionsmischung wurde für 12 h bei Raumtemperatur gerührt. Nach Entfernen des Lösemittels am Rotationsverdampfer wurde der Rückstand chromatographisch aufgreinigt (Laufmittel $SiO_2$, Laufmittel Dichlormethan, Pentan).
$MH^+$: 386; $^1$H-NMR(300 MHz, $D_6$-DMSO) $\delta$ ppm: 8.97 (s, 1H), 8.81 (s, 1H), 7.93 - 7.87 (m, 2H), 7.53 - 7.49 (d, 1H), 3.96 (s, 3H),

**2-(5-Chlor-2H-indazol-2-yl)-3-methyl-6-(trifluormethyl)-3H-imidazo[4,5-b]pyridin**

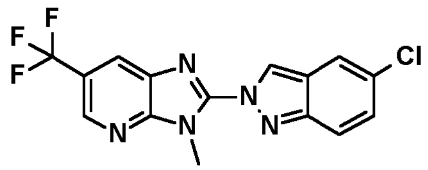

2-Azido-5-chlorbenzaldehyd (1 g, 5.5 mmol) und 3-Methyl-6-(trifluormethyl)-3H-imidazo[4,5-b]pyridin-2-amin (1.2 g, 5.5 mmol) wurden in THF (15 ml) gelöst und mit Tetraisopropyltitanat (1.6 g, 5.5 mmol) versetzt. Das Reaktionsgemisch wurde für 6 h auf 60°C erhitzt und anschließend wurde das Lösemittel abdestilliert. Der Rückstand wurde ohne weitere Aufreinigung in Toluol (15 ml) aufgenommen und für 12 h auf 110 °C erhitzt. Nach Entfernen des Lösemittels am Rotationsverdampfer wurde der Rückstand chromatographisch aufgreinigt (Laufmittel $SiO_2$, Laufmittel Dichlormethan, Pentan).
MH$^+$: 352; $^1$H-NMR(300 MHz, $D_6$-DMSO) δ ppm: 9.25 (s, 1H), 8.86 (s, 1H), 8.64 (s, 1H), 7.98 (s, 1H), 7.89 - 7.86 (d, 1H), 7.44 - 7.40 (d, 1H), 4.24 (s, 3H).

**2-Azido-5-chlorbenzaldehyd**

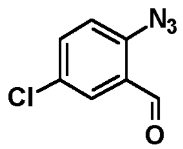

5-Chlor-2-fluorbenzaldehyd (1 g, 6.3 mmol) wurde in DMF (10 mL) gelöst und mit Natriumazid (620 mg, 10 mmol) versetzt. Das Reaktionsgemisch wurde für 12 h auf 60°C erhitzt. Die Reaktionslösung wurde mit Wasser verdünnt und anschließend mit Essigester extrahiert. Die vereinigten organischen Extrakte wurden mit gesättigter Natriumchloridlösung gewaschen und über Natriumsulfat getrocknet. Nach Entfernen des Lösemittels wurde das Rohprodukt ohne weitere Aufreinigung in der Folgereaktion eingesetzt.

**2-[3-(Ethylsulfonyl)-4,5,6,7-tetrahydro-2H-indazol-2-yl]-3-methyl-6-(trifluormethyl)-3H-imidazo[4,5-b]pyridin (Bsp. I-10)**

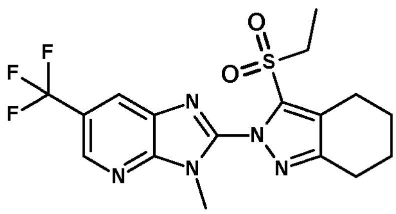

Unter einer Inertgasatmosphäre wurde 2-[3-(Ethylsulfanyl)-4,5,6,7-tetrahydro-2H-indazol-2-yl]-3-methyl-6-(trifluormethyl)-3H-imidazo[4,5-b]pyridin (400 mg, 0.99 mmol, 1.00 equiv) in 10 mL Dichlormethan gelöst und mit Ameisensäure (284 mg, 6.9 mmol, 7.00 equiv, 88%) und Wasserstoffperoxidlösung (562 mg, 4.9 mmol, 5.00 equiv, 30%) versetzt. Das Reaktionsgemisch wurde über Nacht bei Raumtemperatur gerührt und anschließend mit 100 ml Dichlormethan verdünnt. Das Gemisch wurde mit 100 ml Wasser und anschließend mit 100 ml gesättigter NaCl-Lösung gewaschen. Nach Entfernen des Lösemittels am Rotationsverdampfer wurde das Rohprodukt über präperative HPLC aufgereinigt (SiO2, Acetonitril:Wasser).
MH$^+$: 414; $^1$H-NMR(300 MHz, $D_6$-DMSO) δ ppm: 8.92 (s, 1H), 8.71 (s, 1H9, 3.76 (m, 3H) 2.78 (m, 4H), 1.855 (m, 4H), 1.28 (t, 3H).

**2-[3-(Ethylsulfanyl)-4,5,6,7-tetrahydro-2H-indazol-2-yl]-3-methyl-6-(trifluormethyl)-3H-imidazo[4,5-b]pyridin**

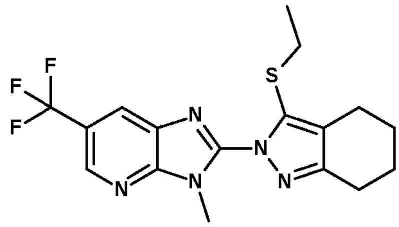

Unter einer Inertgasatmosphäre wurde 2-[3-Methyl-6-(trifluormethyl)-3H-imidazo[4,5-b]pyridin-2-yl]-4,5,6,7-tetrahydro-2H-indazol-3-yl-trifluormethansulfonat (650 mg, 1.4 mml, 1.0 eq.) in 10 ml Dioxan gelöst und mit Ethylmercaptan (171 mg, 2.77 mmol, 2.00 equiv), $Pd_2(dba_3)$ $CHCl_3$ (72 mg, 0.07 mmol, 0.05 equiv), Xantphos (80 mg, 0.14 mmol, 0.1 equiv) und Diisopropylethylamin (536 mg, 4.2 mmol, 3.00 equiv) versetzt. Das Reaktionsgemisch wurde über Nacht abei Raumtemperatur gerührt und anschließend mit 100 ml Dichlormethan verdünnt. Das Gemisch wurde mit 100 ml Wasser und anschließend mit 100 ml gesättigter NaCl-Lösung gewaschen. Nach Entfernen des Lösemittels am Rotationsverdampfer wurde das Rohprodukt säulenchromatographisch aufgereinigt (SiO2, Pentan:Ethylacetat).

**2-[3-Methyl-6-(trifluormethyl)-3H-imidazo[4,5-b]pyridin-2-yl]-4,5,6,7-tetrahydro-2H-indazol-3-yl-trifluormethansulfonat**

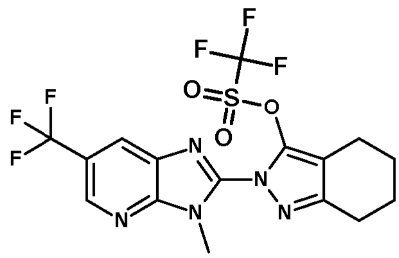

Unter Inertgasatmosphäre wurde 2-[3-Methyl-6-(trifluormethyl)-3H-imidazo[4,5-b]pyridin-2-yl]-4,5,6,7-tetrahydro-2H-indazol-3-ol (400 mg, 1.2 mmol, 1.0 eq.) in 10 ml trockenem Dioxan gelöst, und mit N-Phenyl-bis(trifluormethansulfonimid) (636 mg, 1.8 mmol, 1.50 equiv) und Diisopropylethylamin (301 mg, 2.4 mmol, 2.00 equiv) versetzt. Das Reaktionsgemisch wurde über Nacht abei Raumtemperatur gerührt und anschließend mit 100 ml Dichlomethan verdünnt. Das Gemisch wurde mit 100 ml Wasser und anschließend mit 100 ml gesättigter NaCl-Lösung gewaschen. Nach Entfernen des Lösemittels am Rotationsverdampfer wurde das Rohprodukt säulenchromatographisch aufgereinigt (SiO2, Dichlormethan:Methanol).
$MH^+$:470; $^1$H-NMR(300 MHz, $D_6$-DMSO) $\delta$ ppm: 8.86 (s, 1H), 8.59 (s, 1H), 3.98 (s, 3H), 2.75 (m, 2H), 2.58 (m, 2H), 1.80 (m, 4H).

**2-[3-Methyl-6-(trifluormethyl)-3H-imidazo[4,5-b]pyridin-2-yl]-4,5,6,7-tetrahydro-2H-indazol-3-ol**

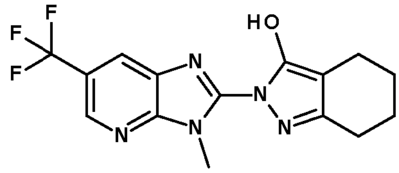

Unter Inertgasatmosphäre wurde 2-Hydrazino-3-methyl-6-(trifluormethyl)-3H-imidazo[4,5-b]pyridin (1 g, 4.3

mmol, 1.0 eq.) in 10 ml Eisessig gelöst und mit Ethyl-2-oxocyclohexancarboxylat (1.5 g, 8.7 mmol, 2.0 eq.) versetzt. Das Reaktionsgemisch wurde bei 80°C für 1 h gerührt und nach Abkühlen auf Raumtemperatur mit 100 ml Ethylacetat verdünnt. Das Gemisch wurde mit 100 ml Wasser und anschließend mit 100 ml gesättigter NaCl-Lösung gewaschen. Nach Entfernen des Lösemittels am Rotationsverdampfer wurde das Rohprodukt säulenchromatographisch aufgereinigt (SiO2, Pentan:Ethylacetat).

$MH^+$: 337; $^1H$-NMR(300 MHz, $D_6$-DMSO) δ ppm: 11.32 (s-b, 1H), 8.77 (s, 1H), 8.47 (s, 1H), 3.91 (s, 3H), 2.54 (s, 2H), 2.23 (m, 2H), 1.73 (m, 4H).

**2-Hydrazino-3-methyl-6-(trifluormethyl)-3H-imidazo[4,5-b]pyridin**

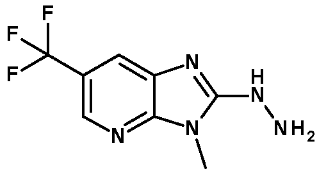

Unter Inertgasatmosphäre wurde 2-Chlor-3-methyl-6-(trifluormethyl)-3H-imidazo[4,5-b]pyridine (5 g, 21 mmol, 1.0 eq., Herstellung siehe US2015/94329 A1, 2015) in 50 ml trockenem Methanol gelöst und es wurde Hydrazin-Hydrat (5.3 g, 11 mmol, 5.0 eq.) zugegeben. Das Reaktionsgemisch würde über Nacht bei Raumtemperatur gerührt und dann mit 100 ml Dichlormethan verdünnt. Das Gemisch wurde mit 100 ml Wasser und anschließend mit 100 ml gesättigter NaCl-Lösung gewaschen. Nach Entfernen des Lösemittels am Rotationsverdampfer wurde das Rohprodukt ohne weitere Aufreinigung in der nächsten Reaktion eingesetzt.

**2-[3-(Ethylsulfonyl)-4-(trifluormethyl)-2H-indazol-2-yl]-3-methyl-6-(trifluormethyl)-3H-imidazo[4,5-b]pyridin (Bsp. I-17)**

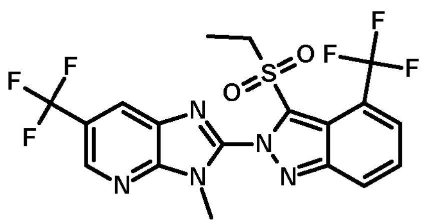

Unter Inergasatmosphäre wurde 2-[3-(Ethylsulfanyl)-4-(trifluormethyl)-2H-indazol-2-yl]-3-methyl-6-(trifluormethyl)-3H-imidazo[4,5-b]pyridin (220 mg, 0.49 mmol, 1 eq.) in Ameisensäure (142 mg, 3.5 mmol, 88%, 7 eq.) und wässriger Wasserstoffperoxidlösung (280 mg, 2.5 mmol, 5.00 eq. 30%) gelöst und bei Raumtemperatur für 4 h gerührt. Das Reaktionsgemisch wurde mit Dichlormethan (2 x 100 ml) extrahiert. Die vereinigten organischen Extrakte wurden mit Wasser und gesättigter Natriumchloridlösung gewaschen und am Rotationsverdampfer vom Lösemittel befreit. Das Rohprodukt wurde über präparative HPLC aufgereinigt (SiO2, Acetonitril:Wasser/0.1%Ameisensäure).

$^1H$-NMR(300 MHz, $D_6$-DMSO) δ ppm: 8.97-8.87 (m, 2H), 8.70 (m, 1H), 8.12-8.00 (m, 2H), 4.18 (s, 3H), 3.92-3.87 (q, 2H), 1.45-1.40 (t, 3H).

**2-[3-(Ethylsulfanyl)-4-(trifluormethyl)-2H-indazol-2-yl]-3-methyl-6-(trifluormethyl)-3H-imidazo[4,5-b]pyridin**

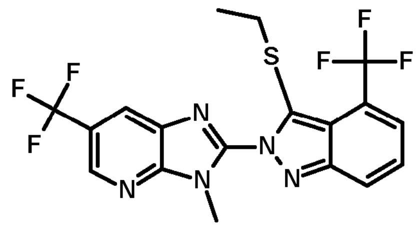

Unter Inertgasatmosphäre wurde 2-[3-Iod-4-(trifluormethyl)-2H-indazol-2-yl]-3-methyl-6-(trifluormethyl)-3H-imidazo[4,5-b]pyridin (290 mg, 0.57 mmol, 1.00 equiv) in trockenem Dioxan (10 ml) gelöst und es wurden Ethylmercaptan (70 mg, 1.4 mmol, 2.00 equiv), $Pd_2(dba)_3CHCl_3$ (29 mg, 0.03 mmol, 0.05 equiv), Xantphos (33 mg, 0.057 mmol, 0.1 equiv), und Diisopropylethylamin (220 mg, 1.7 mmol, 3.00 equiv) zugegeben. Das Reaktionsgemisch wurde für 2 h auf 80°C erwärmt und nach Abkühlen auf Raumtemperatur mit Dichlormethan (100 ml) verdünnt. Die Lösung wurde mit Wasser und gesättigter Natriumchloridlösung gewaschen und am Rotationsverdampfer vom Lösemittel befreit. Das Rohprodukt wurde über präparative HPLC aufgereinigt (SiO2, Acetonitril:Wasser/0.1%Ameisensäure).
$MH^+$: 446; $^1$H-NMR(300 MHz, $D_6$-DMSO) $\delta$ ppm: 8.93 - 8.88 (m, 1H), 8.77 (d, 1H), 8.52 (d, 1H), 4.20 (s, 3H), 3.44-3.36 (q, 2H), 1.47 (t, 3H).

**2-[3-Iod-4-(trifluormethyl)-2H-indazol-2-yl]-3-methyl-6-(trifluormethyl)-3H-imidazo[4,5-b]pyridin**

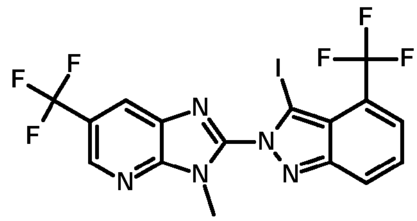

Unter einer Inergasatmosphäre wurde 3-Iod-4-(trifluormethyl)-2H-indazol (300 mg, 0.96 mmol, 1.00 equiv) in DMF (10 mL) gelöst und auf 0°C gekühlt. Über einen Zeitraum von 30 min wurde Natriumhydrid (38 mg, 0.96 mmol, 1.00 equiv) zugegeben, gefolgt von 2-Chlor-3-methyl-6-(trifluormethyl)-3H-imidazo[4,5-b]pyridin (339 mg, 1.4 mmol, 1.50 equiv). Das Reaktionsgemisch wurde für 1 h auf 80°C erhitzt. Nach Abkühlen auf Raumtemperatur wurde die Lösung mit Ethylacetat verdünnt. Das Gemisch wurde mit Wasser und gesättigter Natriumchloridlösung gewaschen und am Rotationsverdampfer vom Lösemittel befreit. Das Rohprodukt wurde säulenchromatographisch aufgereinigt (SiO2, Pentan:Ethylacetat).
$MH^+$: 512; $^1$H-NMR(300 MHz, $D_6$-DMSO) $\delta$ ppm: 8.82 (m, 2H), 8.57 (s, 1H), 7.94-7.86 (m, 2H), 4.04 (s, 3H).

**3-Iod-4-(trifluormethyl)-2H-indazol**

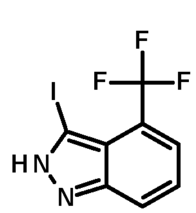

Unter einer Inertgasatmosphäre wurde 4-(Trifluormethyl)-2H-indazol (500 mg, 2.7 mmol, 1.00 equiv) in DMF (10 mL) gelöst und mit Iod (1.4 g, 5.4 mmol, 2.00 equiv) und Kaliumhydroxid (378 mg, 6.8 mmol, 2.50 equiv) versetzt. Das Gemisch wurde für 30 min bei Raumtemperatur gerührt. Die Lösung wurde mit Dichlormethan (100 ml) verdünnt und zuerst mit mit Natriumthiosulfatlösung und anschließend mit gesättigter Natriumchloridlösung gewaschen. Nach Entfernen des Lösemittels am Rotationsverdampfer wurde das Rohprodukt ohne weitere Aufreinigung in der Folgere-

aktion eingesetzt.
$MH^+$: 313; $^1H$-NMR(300 MHz, $D_6$-DMSO) δ ppm: 14.10 (s, 1H), 7.96 (d, 1H), 7.64 - 7.53 (m, 2H).

**N-{3-(Ethylsulfonyl)-2-[3-methyl-6-(trifluormethyl)-3H-imidazo[4,5-b]pyridin-2-yl]-2H-indazol-6-yl}acetamid (Bsp. I-18)**

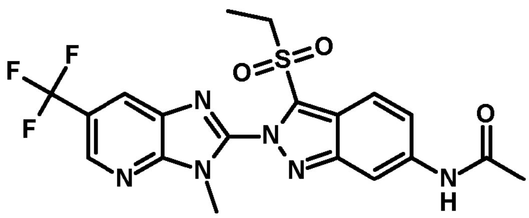

N-{3-(Ethylsulfanyl)-2-[3-methyl-6-(trifluormethyl)-3H-imidazo[4,5-b]pyridin-2-yl]-2H-indazol-6-yl}acetamid (35 mg, 0.1 mmol) wurde in Dichlormethan (2 ml) gelöst, auf 0°C gekühlt und mit wässriger Wasserstoffperoxidlösung (0.2 mL, 1.0 mmol, 30%) und Ameisensäure (60 mg, 1 mmol, 88%) versetzt. Das Reaktionsgemisch wurde für 48 h bei Raumtemperatur gerührt und dann mit Dichlormethan verdünnt. Das Gemisch wurde mit gesättigter Natriumhydrogencarbonatlösung gewaschen. Die organische Phase wurde über Natriumsulfat getrocknet und am Rotationsverdampfer vom Lösemittel befreit. Das Rohprodukt wurde durch präperative TLC aufgereinigt (SiO2, Pentan/Ethylacetat).
$MH^+$: 467; $^1H$-NMR(300 MHz, $D_6$-DMSO) δ ppm: 10.70 (s, 1H), 9.49 (s, 1H), 8.92 (s, 1H), 8.71 (s, 1H), 8.33-8.25 (m, 2H), 4.33 (s, 3H), 3.86 - 3.83 (m, 2H), 2.56 (s, 3H), 1.30-1.25 (t, 3H).

**N-{3-(Ethylsulfanyl)-2-[3-methyl-6-(trifluormethyl)-3H-imidazo[4,5-b]pyridin-2-yl]-2H-indazol-6-yl}acetamid**

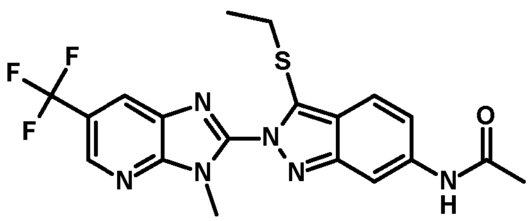

Unter einer Inertgasatmosphäre wurde N-{3-Chlor-2-[3-methyl-6-(trifluormethyl)-3H-imidazo[4,5-b]pyridin-2-yl]-2H-indazol-6-yl}acetamid (60 mg, 0.2 mmol) in 1,4-Dioxan (2 ml) gelöst und mit Ethylmercaptan (60 mg, 1.0 mmol), Diisopropylethylamin (129 mg, 1.0 mmol), $Pd_2(dba)_3CHCl_3$ (55 mg, 0.05 mmol) und Xantphos (55 mg, 0.1 mmol) versetzt. Das Reaktionsgemisch wurde für 12 h auf 110°C erhitzt. Nach Abkühlen auf Raumtemperatur wurde die Reaktionslösung mit Wasser gewaschen. Die wässrige Phase wurde mehrfach mit Ethylacetat extrahiert. Die kombinierten organischen Extrakte wurden über Natriumsulfat getrocknet und am Rotationsverdampfer vom Lösemittel befreit. Das Rohprodukt wurde durch präperative TLC aufgereinigt (SiO2, Pentan/Ethylacetat).
$MH^+$: 435; $^1H$-NMR(300 MHz, $D_6$-DMSO) δ ppm: 9.61 (s, 1H), 9.28 (s, 1H), 8.84 (s, 1H), 8.62 (s, 1H), 7.78 (m, 1H), 7.55 (m, 1H), 4.29 (s, 3H), 3.34-3.18 (q, 2H), 2.15 (s, 3H), 1.23 - 1.11 (t, 3H).

**N-{3-Chlor-2-[3-methyl-6-(trifluormethyl)-3H-imidazo[4,5-b]pyridin-2-yl]-2H-indazol-6-yl}acetamid**

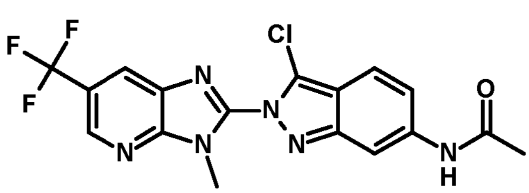

N-{2-[3-Methyl-6-(trifluormethyl)-3H-imidazo[4,5-b]pyridin-2-yl]-2H-indazol-6-yl}acetamid (150 mg, 0.4 mmol) wurde in Eisessig (5 ml) gelöst, mit N-Chlorsuccinimid (120 mg, 1 mmol) versetzt und für 12 h bei Raumtemperatur gerührt. Das Reaktionsgemisch wurde vom Lösemittel befreit und das Rohprodukt wurde säulenchromatographisch aufgereinigt (SiO2, Pentan/Dichlormethan).
$MH^+$: 409; $^1$H-NMR(300 MHz, $D_6$-DMSO) $\delta$ ppm: 8.72 (s, 1H), 8.35 - 8.32 (m, 1H), 8.26 (s, 2H) 7.71 - 7.64 (m, 2H), 3.82 (s, 3H), 2.21 (s, 3H).

**N-{2-[3-Methyl-6-(trifluormethyl)-3H-imidazo[4,5-b]pyridin-2-yl]-2H-indazol-6-yl}acetamid**

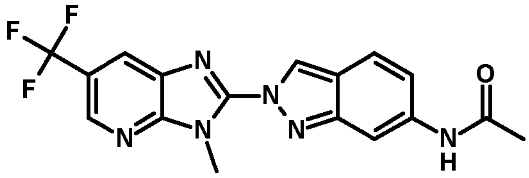

Unter einer Inertgasatmosphäre wurden in einem Druckgefäß 2-(6-Brom-2H-indazol-2-yl)-3-methyl-6-(trifluormethyl)-3H-imidazo[4,5-b]pyridin (0.8 g, 2 mmol), Acetamid (0.3 g, 5 mmol), Cäsiumcarbonat (1.6 g, 5 mmol), $Pd_2(dba)_3CHCl_3$ (0.12 g, 0.1 mmol) und Xantphos (0.11 g, 0.2 mmol) in 1,4-Dioxan (10 mL) gelöst und für 2 h auf 110°C erhitzt. Das Reaktionsgemisch wurde mit Dichlormethan verdünnt und mit gesättigter Natriumchloridlösung gewaschen. Die organische Phase wurde über Natriumsulfat getrocknet und am Rotationsverdampfer vom Lösemittel befreit. Das Rohprodukt wurde säulenchromatographisch aufgereinigt (SiO2, Pentan/Ethylacetat).
$MH^+$: 375; $^1$H-NMR(300 MHz, $D_6$-DMSO) $\delta$ ppm: 10.37 (s, 1H), 8.88 (s, 1H), 8.77 (s, 1H), 8.58 (s, 1H), 8.47 (s, 1H), 7.87 (d, 1H), 7.60 (d, 1H), 4.16 (s, 3H), 2.12 (s, 3H).

**2-(6-Brom-2H-indazol-2-yl)-3-methyl-6-(trifluormethyl)-3H-imidazo[4,5-b]pyridin**

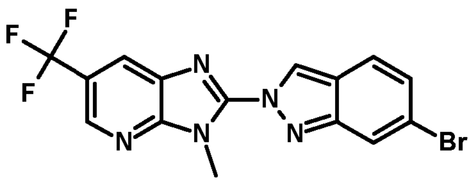

Unter Inertgasatmosphäre wurde 4-Brom-2-fluorobenzaldehyd (4 g, 20 mmol) in DMF (20 mL) gelöst und mit Natriumazid (1.240 g, 20 mmol) versetzt. Das Gemisch wurde für 12 h auf 60°C erhitzt. Die Reaktionslösung wurde mit Wasser verdünnt und zweimal mit Ethylacetat extrahiert. Die kombinierten organischen Extrakte wurden vom Lösemittel befreit.

Das Rohprodukt (4 g, 18 mmol) wurde unter Inertgasatmosphäre in THF (30 ml) gelöst und mit 3-Methyl-6-(trifluormethyl)-3H-imidazo[4,5-b]pyridin-2-amin (4.2 g, 20 mmol) und Titantriisopropylat (6 g, 21 mmol) versetzt. Das Reaktionsgemisch wurde für 6 h auf 60°C erhitzt.

Nach Entfernen des Lösemittels im Vakuum wurde der Rückstand in Toluol (30 ml) aufgenommen und für 12 h auf 110°C erhitzt. Das Lösemittel wurde am Rotationsverdampfer entfernt und das Rohprodukt wurde säulenchromatographisch aufgereinigt (SiO2, Pentan/Dichlormethan).
$MH^+$: 397; $^1$H-NMR(600 MHz, $D_6$-DMSO) $\delta$ ppm: 9.32 (s, 1H), 8.90 (s, 1H), 8.65 (s, 1H), 8.12 (s, 1H), 7.85 (d, 1H), 7.35 (d, 1H), 4.23 (s, 3H).

**3-(Ethylsulfonyl)-2-[3-methyl-6-(trifluormethyl)-3H-imidazo[4,5-b]pyridin-2-yl]-2H-pyrazolo[3,4-c]pyridin (Bsp. I-19)**

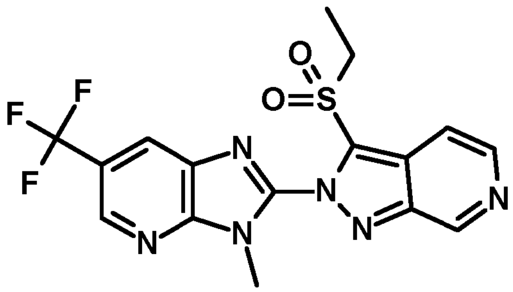

3-(Ethylsulfanyl)-2-[3-methyl-6-(trifluormethyl)-3H-imidazo[4,5-b]pyridin-2-yl]-2H-pyrazolo[3,4-c]pyridin (100 mg, 0.27 mmol) wurde in Dichlormethan (20 ml) gelöst, auf 0°C gekühlt und mit Ameisensäure (5 ml) und dann langsam mit wässriger Wasserstoffperoxidlösung (0.5 mL, 30%) versetzt. Das Reaktionsgemisch wurde für 2 h bei Raumtemperatur gerührt. Das Gemisch wurde zuerst mit gesättigter Natriumhydrogencarbonatlösung und dann mit gesättigter Natriumchloridlösung gewaschen. Die kombinierten organischen Phasen wurde über Natriumsulfat getrocknet und am Rotationsverdampfer vom Lösemittel befreit. Das Rohprodukt wurde durch präparative HPLC aufgereinigt.
$MH^+$: 411; $^1$H-NMR(600 MHz, D6-DMSO) $\delta$ ppm: 9.82 (s, 1H), 9.44 (s, 1H), 8.94 (s, 1H), 8.75 (s, 1H), 8.70 (s, 1H), 4.25 (s, 3H), 3.60 - 3.54 (m, 2H), 1.19 (t, 3H).

**3-(Ethylsulfanyl)-2-[3-methyl-6-(trifluormethyl)-3H-imidazo[4,5-b]pyridin-2-yl]-2H-pyrazolo [3,4-c] pyridin**

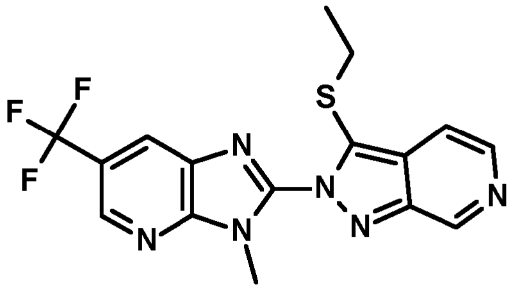

3-Brom-2-[3-methyl-6-(trifluormethyl)-3H-imidazo[4,5-b]pyridin-2-yl]-2H-pyrazolo[3,4-c]pyridin (300 mg, 0.76 mmol), Ethylmercaptan (54 mg, 0.87 mmol), $Pd_2(dba)_3CHCl_3$ (124 mg, 0.12 mmol), Xantphos(140 mg, 0.24 mmol) und Diisopropylethylamin (312 mg, 2.4 mmol) wurden in 10 ml 1,4-Dioxan gelöst und in einem geschlossenen Druckgefäß für 2h auf 80°C erhitzt. Nach Abkühlen auf Raumtemperatur wurde die Reaktionslösung auf 50 ml Wasser gegossen und das Gemisch wurde zwei Mal mit Ethylacetat extrahiert. Die kombinierten organischen Extrakte wurden mit gesättigter Natriumchlorid-Lösung gewaschen, über Natriumsulfat getrocknet und am Rotationsverdampfer eingeengt. Das Rohprodukt wurde säulenchromatographisch aufgereinigt.
$MH^+$: 379; $^1$H-NMR(600 MHz, D6-DMSO) $\delta$ ppm: 9.233 (s, 1H), 9.26 (s, 1H), 8.92 (s, 1H), 8.70 (m, 1H), 8.15 (s, 1H), 4.22 (s, 3H), 3.35 - 3.21 (m, 2H), 1.32 (t, 3H).

**3-Brom-2-[3-methyl-6-(trifluormethyl)-3H-imidazo[4,5-b]pyridin-2-yl]-2H-pyrazolo[3,4-c]pyridin**

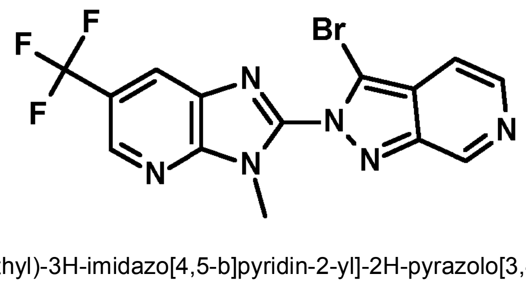

2-[3-Methyl-6-(trifluormethyl)-3H-imidazo[4,5-b]pyridin-2-yl]-2H-pyrazolo[3,4-c]pyridin (500 mg, 1.57 mmol)

wurde in 5 ml Eisessig gelöst und portionsweise bei 0°C mit N-Bromsuccinimid (280 mg, 1.57 mmol) versetzt. Das Reaktionsgemisch wurde für 2 h bei Raumtemperatur gerührt. Nach Zugabe von 50 ml Wasser wurde ein pH-Wert von 8 durch Zugabe von Natriumhydrogencarbonat eingestellt. Das Gemisch wurde drei Mal mit Ethylacetat extrahiert. Die kombinierten organischen Extrakte wurden drei Mal mit gesättigter Natriumchlorid-Lösung gewaschen, über Natriumsulfat getrocknet und am Rotationsverdampfer eingeengt. Das Rohprodukt wurde über präparative TLC aufgereinigt (Ethylacetat/Petrolether).

$MH^+$: 399; $^1$H-NMR(600 MHz, D6-DMSO) $\delta$ ppm: 9.45 (s, 1H), 9.37 (s, 1H), 8.93 (s, 1H), 8.73 (s, 1H), 8.43 (s, 1H), 4.21 (s, 3H).

**2-[3-Methyl-6-(trifluormethyl)-3H-imidazo[4,5-b]pyridin-2-yl]-2H-pyrazolo[3,4-c]pyridin**

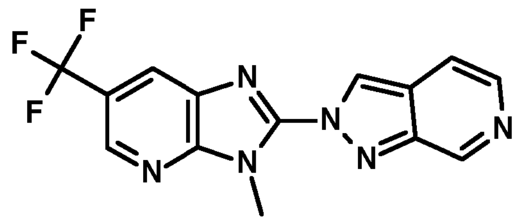

3-Fluorisonicotinaldehyd (800 mg, 6.4 mmol) und Natriumazid (416 mg, 6.4 mmol) wurden in 10 ml DMF gelöst, und für 4 h bei 50°C gerührt. Dass Gemisch wurde auf Wasser gegossen und drei Mal mit Ethylacetat extrahiert. Die kombinierten organischen Extrakte wurden über Natriumsulfat getrocknet und am Rotationsverdampfer eingeengt. Das Rohprodukt 3-Azidoisonicotinaldehyd wurde als gelber Feststoff erhalten und ohne weitere Aufreinigung in der Folgereaktion eingesetzt.

3-Azidoisonicotinaldehyd (620 mg, 4.16 mmol) und 3-Methyl-6-(trifluormethyl)-3H-imidazo[4,5-b]pyridin-2-amin (910 mg, 4.2 mmol) wurden in 30 ml Toluol gelöst und es wurde Tetraisopropylorthotitanat (2.4 g, 8.4 mmol) zugegeben. Die Reaktionslösung wurde für 2 h in der Mikrowelle auf 120°C erhitzt. Die Reaktion wurde nach Abkühlen auf Raumtemperatur durch Zugabe von 100 ml Wasser beendet. Das Gemisch wurde drei Mal mit Ethylacetat extrahiert. Die kombinierten organischen Extrakte wurden mit gesättigter Natriumchlorid-Lösung gewaschen, über Natriumsulfat getrocknet und am Rotationsverdampfer eingeengt. Das Rohprodukt wurde säulenchromatographisch aufgereinigt (Ethylacetat/Petrolether).

$MH^+$: 319; $^1$H-NMR(600 MHz, D6-DMSO) $\delta$ ppm: 9.43 (s, 1H), 9.09 (s, 1H), 8.75 (s, 1H), 8.31 - 8.255 (m, 2H), 7.64 (m, 1H), 4.44 (s, 3H).

In Analogie zu den Beispielen und gemäß den oben beschriebenen Herstellungsverfahren lassen sich auch die übrigen in Tabelle 1 aufgeführten Verbindungen der Formel (Ia) oder (Ib) erhalten:

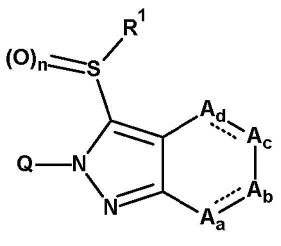

*Ia*

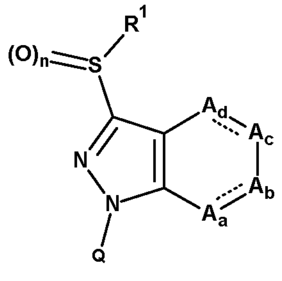

*Ib*

**Tabelle 1**

| Bsp-No | Struktur |
|---|---|
| I-1 | 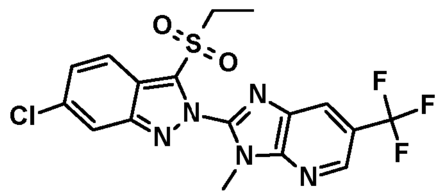 |
| I-2 | 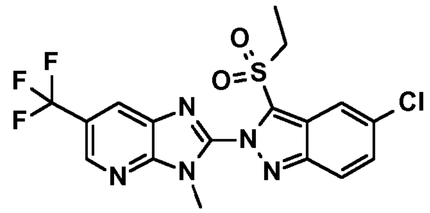 |
| I-3 | 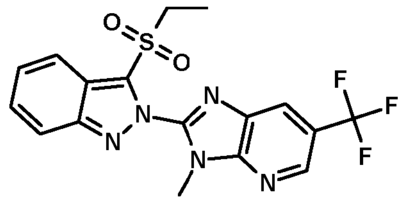 |
| I-4 | 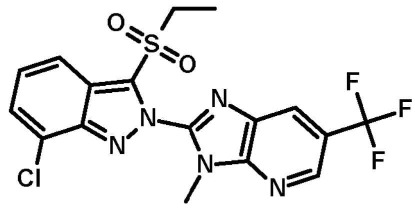 |
| I-5 | 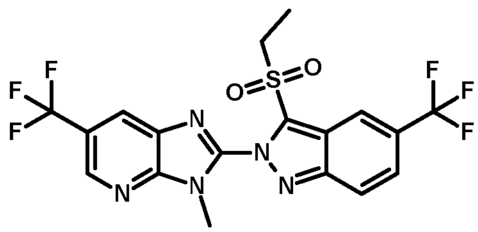 |
| I-6 | 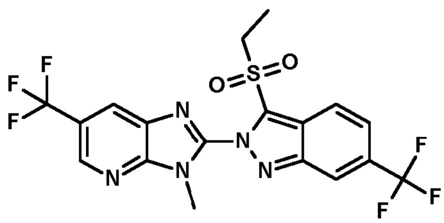 |
| I-7 | 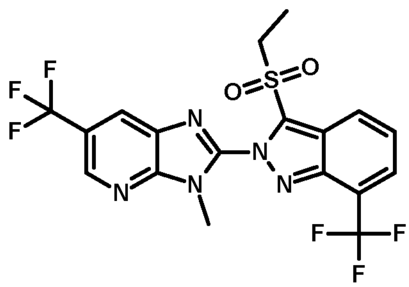 |

(fortgesetzt)

| Bsp-No | Struktur |
|---|---|
| I-8 | 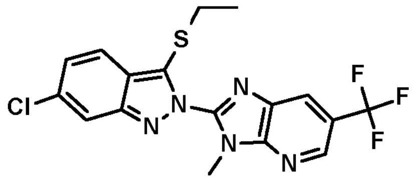 |
| I-9 | 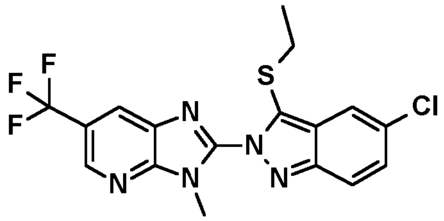 |
| I-10 | 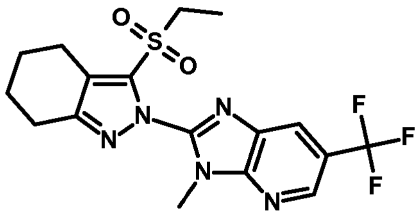 |
| I-11 | 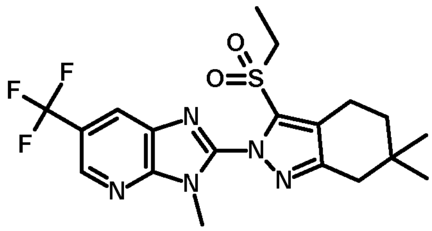 |
| I-12 | 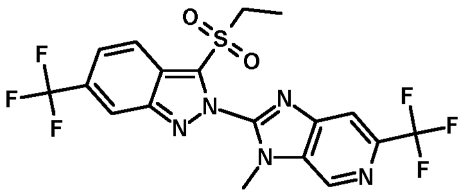 |
| I-13 | 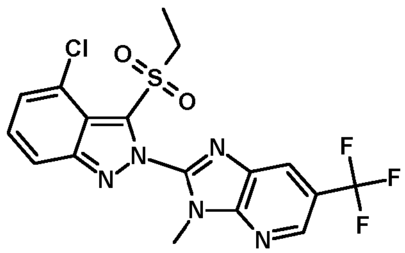 |

(fortgesetzt)

| Bsp-No | Struktur |
|---|---|
| I-14 | 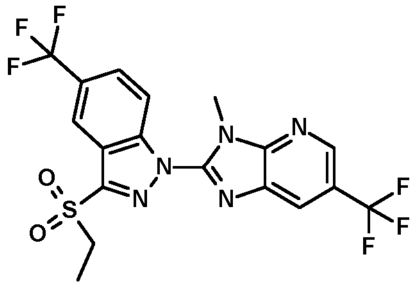 |
| I-15 | 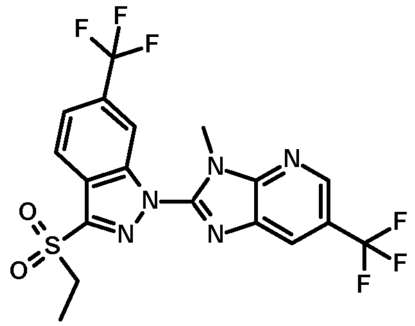 |
| I-16 | 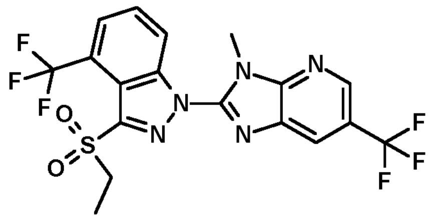 |
| I-17 | 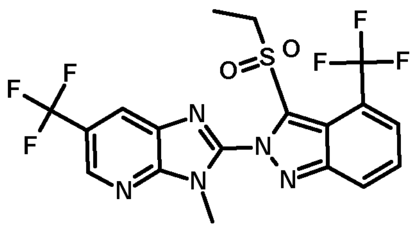 |
| I-18 | 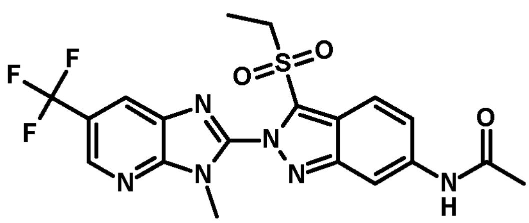 |
| I-19 | 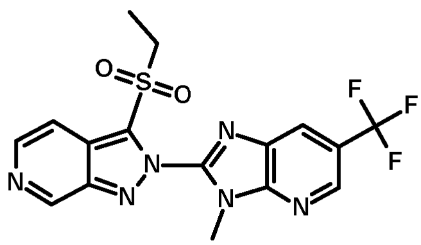 |

**NMR-Daten ausgewählter Beispiele**

NMR-Peak-Listenverfahren

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:

$$\delta_1 \text{ (Intensität}_1\text{)}; \delta_2 \text{ (Intensität}_2\text{)};\ldots\ldots; \delta_i \text{ (Intensität}_i\text{)};\ldots\ldots; \delta_n \text{ (Intensität}_n\text{)}$$

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-$D_6$ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

**Tabelle 2**

| NMR Daten ausgewählter Beispiele |
|---|
| Beispiel I-1: $^1$H-NMR(300.1 MHz, $d_6$-DMSO):<br>δ= 9.021(2.0);9.016(2.2);8.851(2.1);8.845(2.0);8.219(2.2);8.216(2.4);8.214(2.3);8.104(2.0);8.102(2.0);8.073(2.3);8.071(2.3);7.59 4(1.5);7.589(1.5);7.564(1.4);7.558(1.4);3.937(0.9);3.913(3.2);3.888(3.2);3.864(1.0);3.828(16.0);3.322(9.4);2.515(4.6);2.509(9.2); 2.503(12.3);2.497(8.4);2.492(3.8);2.076(0.3);1.311(3.3);1.286(7.7); 1.262(3.2);0.000(7.0) |
| Beispiel I-2: $^1$H-NMR(400.2 MHz, $d_6$-DMSO):<br>δ= 9 .022(2.5); 9.019(2.5);8.856(2.6);8.853(2.5);8.122(2.3);8.099(2.6);8.060(2.7);8.057(2.8);7.678(2.0);7.673(1.9);7.654(1.8);7.65 0(1.7);3.943(1.0);3.925(3.4);3.906(3.4);3.888(1.1);3.834(16.0);3.329(14.6);2.510(6.8); 2.506(9.1);2.501(6.7);1.319(3.6);1.300(7.8 );1.282(3.4);0.001(5.1) |
| Beispiel I-3: $^1$H-NMR(400.2 MHz, $d_6$-DMSO): |

(fortgesetzt)

| NMR Daten ausgewählter Beispiele |
|---|
| δ= 9.018(2.5);9.015(2.5);8.848(2.6);8.843(2.4);8.081(1.9);8.060(2.1);8.037(2.0);8.014(2.2);7.675(1.0);7.674 (1.0);7.659(1.4);7.65 7(1.4);7.637(1.2);7.590(1.4);7.568(1.4);7.552(0.9);3.968(0.3);3.906(1.1);3.888(3.5);3.870 (3.6);3.851(1.3);3.824(16.0);3.335(11.0 );2.512(4.6);2.508(5.9);2.504(4.3);2.081(1.4);1.307(3.7);1.288(7.8); 1.270(3.5);0.001(2.4) |
| Beispiel I-4: $^1$H-NMR(300.1 MHz, $d_6$-DMSO):<br>δ= 9.033(1.8);9.031(2.0);9.027(2.1);8.863(2.2);8.858(2.0);8.050(1.8);8.047(2.0);8.021(2.2);8.019(2.1);7.823 (1.8);7.820(2.0);7.79 8(2.3);7.796(2.2);7.568(1.8);7.543(1.7);7.539(1.8);7.514(1.4);5.756(1.1);3.913(0.8);3.888 (3.0);3.864(3.2);3.837(16.0);3.316(22.0 );2.514(10.8);2.508(21.5);2.502(28.6);2.496(19.5);2.490(8.8);1.305 (3.2);1.281(7.4);1.256(3.1);1.235(0.5);0.011(0.6);0.000(17.5); -0.011(0.6) |
| Beispiel I-5: $^1$H-NMR(300.1 MHz, $d_6$-DMSO):<br>δ= 9.036(2.4);9.031(2.5);8.875(2.5);8.870(2.4);8.431(2.4);8.308(1.6);8.277(1.9);7.902(1.6);7.897(1.6);7.872 (1.4);7.866(1.4);4.01 3(1.0);3.988(3.3);3.964(3.4);3.939(1.0);3.849(16.0);3.321(16.8);2.515(6.1);2.510(11.6); 2.504(15.1);2.498(10.7);2.077(0.8);1.337( 3.5);1.313(7.9);1.288(3.4);0.000(1.8) |
| Beispiel 1-6: $^1$H-NMR(300.1 MHz, $d_6$-DMSO):<br>δ=9.037(2.0);9.035(2.2);9.030(2.2);8.871(2.3);8.866(2.2);8.590(2.3);8.587(2.2);8.310(1.5);8.279(1.7);7.819 (1.5);7.814(1.5);7.78 8(1.4);7.784(1.4);3.959(0.9);3.934(3.1);3.910(3.2);3.885(1.0);3.845(16.0);3.319(14.0); 2.516(3.4);2.511(6.7);2.505(8.8);2.499(6.0 );2.493(2.7);1.325(3.3);1.300(7.6);1.276(3.1);0.000(6.8) |
| Beispiel I-7: $^1$H-NMR(400.2 MHz, $d_6$-DMSO):<br>δ= 9.036(3.2);8.874(3.3);8.384(2.2);8.362(2.3);8.139(2.0);8.122(2.1);7.730(1.3);7.711(1.8);7.691(1.2);3.967 (1.3);3.949(3.8);3.93 0(3.8);3.912(1.3);3.844(16.0);3.329(35.2);2.674(0.4);2.505(53.4);2.332(0.3);1.325(4.1); 1.306(8.2);1.288(3.8);0.002(19.8) |
| Beispiel I-10: $^1$H-NMR(300.1 MHz, $d_6$-DMSO):<br>δ= 8.9232 (2.1); 8.9189 (2.2); 8.7100 (2.2); 8.7047 (2.2); 3.7751 (16.0); 3.7594 (3.3); 3.7347 (3.2); 3.7102 (0.9); 3.3171 (13.7); 2.8629 (1.1); 2.8424 (2.4); 2.8234 (1.2); 2.8017 (1.1); 2.7824 (2.4); 2.7620 (1.2); 2.5137 (6.7); 2.5078 (13.1); 2.5019 (17.2); 2.4960 (11.8); 1.8549 (1.0); 1.8309 (1.4); 1.8138 (1.6); 1.7988 (1.4); 1.7740 (0.9); 1.3034 (3.3); 1.2789 (7.4); 1.2542 (3.1); - 0.0001 (6.9) |
| Beispiel I-11: $^1$H-NMR(300.1 MHz, $d_6$-DMSO):<br>δ= 8.9269 (1.8); 8.9225 (1.9); 8.7139 (1.9); 8.7089 (1.9); 3.7944 (0.8); 3.7635 (13.8); 3.7451 (2.8); 3.7208 (0.8); 3.3243 (4.0); 2.8691 (1.0); 2.8475 (2.1); 2.8254 (1.0); 2.5732 (4.6); 2.5144 (5.6); 2.5086 (11.1); 2.5026 (14.7); 2.4967 (10.0); 2.4910 (4.6); 2.0762 (0.7); 1.6309 (1.0); 1.6090 (2.1); 1.5870 (0.9); 1.2981 (2.7); 1.2736 (6.3); 1.2489 (2.6); 1.0318 (16.0); 0.0000 (6.7) |
| Beispiel I-12: $^1$H-NMR(400.1 MHz, $d_6$-DMSO):<br>δ= 15.9016 (0.3); 9.4083 (5.0); 8.9769 (0.4); 8.5945 (4.2); 8.4655 (5.3); 8.2965 (2.3); 8.2729 (2.7); 7.8162 (2.6); 7.7937 (2.5); 6.5178 (0.8); 3.9481 (16.0); 3.9271 (1.6); 3.9095 (4.0); 3.8904 (4.0); 3.8736 (1.4); 3.6357 (0.4); 3.5058 (0.5); 3.4200 (0.4); 3.3164 (132.2); 3.1674 (0.3); 2.7077 (0.3); 2.6710 (2.0); 2.5863 (0.5); 2.5005 (301.4); 2.3273 (1.8); 1.7702 (0.4); 1.4486 (0.4); 1.3382 (0.5); 1.3048 (4.3); 1.2865 (8.5); 1.2683 (4.3); 1.2339 (4.5); 1.1527 (0.5); 0.9399 (0.5); 0.9136 (0.4); 0.8475 (0.5); 0.8175 (0.5); 0.8038 (0.6); 0.1460 (0.7); 0.1309 (0.4); 0.0666 (0.5); -0.0003 (104.1); -0.1513 (0.4) |
| Beispiel I-13: $^1$H-NMR(300.1 MHz, $d_6$-DMSO):<br>δ= 9.0101 (2.3); 9.0057 (2.4); 8.8248 (2.5); 8.8200 (2.4); 8.0548 (2.1); 8.0283 (2.3); 8.0261 (2.3); 7.7307 (1.7); 7.7086 (2.8); 7.7066 (2.7); 7.6583 (2.2); 7.6337 (1.4); 7.6296 (2.0); 7.6051 (1.2); 3.8655 (1.1); 3.8408 (3.6); 3.8161 (3.6); 3.7916 (1.2); 3.7374 (16.0); 3.3212 (5.6); 2.5144 (5.2); 2.5088 (10.2); 2.5029 (13.3); 2.4971 (9.4); 1.3261 (3.9); 1.3014 (8.8); 1.2767 (3.8); 0.0104 (0.4); -0.0001 (7.8) |
| Beispiel I-14: $^1$H-NMR(300.1 MHz, $d_6$-DMSO):<br>δ= 8.8861 (2.4); 8.8822 (2.4); 8.7844 (1.7); 8.7546 (2.0); 8.7160 (2.6); 8.7107 (2.5); 8.4781 (2.7); 8.2267 (1.4); 8.2217 (1.4); 8.1913 (1.3); 4.1658 (16.0); 4.1430 (0.4); 3.7941 (1.1); 3.7695 (3.6); 3.7451 (3.7); 3.7207 (1.2); 3.3255 (18.8); 2.5418 (0.4); 2.5081 (22.4); 2.5023 (29.0); 2.4966 (20.2); 2.0761 (1.0); 1.3553 (3.9); 1.3309 (8.6); 1.3064 (3.7); 0.0000 (12.1); -0.0112 (0.4) |
| Beispiel I-15: $^1$H-NMR(300.1 MHz, $d_6$-DMSO): |

(fortgesetzt)

| NMR Daten ausgewählter Beispiele |
|---|
| δ= 8.9277 (2.5); 8.8793 (2.1); 8.8752 (2.2); 8.7966 (2.4); 8.7916 (2.1); 8.4293 (1.6); 8.4006 (1.8); 7.9974 (1.4); 7.9933 (1.4); 7.9685 (1.2); 7.9645 (1.2); 4.1616 (16.0); 3.7664 (1.0); 3.7419 (3.4); 3.7174 (3.5); 3.6929 (1.1); 3.3207 (6.9); 2.5141 (6.8); 2.5083 (13.1); 2.5024 (17.1); 2.4965 (11.7); 2.0758 (1.0); 1.3470 (3.8); 1.3226 (8.4); 1.2981 (3.6); 0.0108 (0.5); -0.0001 (11.5); - 0.0111 (0.4) |
| Beispiel I-16: $^1$H-NMR(300.1 MHz, $d_6$-DMSO):<br>δ= 8.9701 (1.6); 8.9419 (1.8); 8.8942 (2.2); 8.8900 (2.3); 8.8756 (0.4); 8.7063 (2.4); 8.7011 (2.3); 8.1221 (1.1); 8.0976 (2.0); 8.0528 (1.2); 8.0262 (1.6); 7.9997 (0.6); 4.1837 (1.8); 4.1671 (16.0); 3.9236 (1.0); 3.8989 (3.2); 3.8745 (3.3); 3.8502 (1.0); 3.3284 (22.6); 2.5420 (0.4); 2.5142 (9.8); 2.5085 (19.2); 2.5026 (25.2); 2.4968 (17.4); 2.0766 (0.4); 1.4551 (3.4); 1.4308 (7.8); 1.4063 (3.3); 1.3170 (0.3); 1.2923 (0.7); 1.2676 (0.3); 0.0109 (0.5); -0.0001 (12.0); -0.0111 (0.4) |
| Beispiel I-17: $^1$H-NMR(300.1 MHz, $d_6$-DMSO):<br>δ= 10.0529 (0.3); 9.0191 (2.3); 9.0037 (0.4); 8.9938 (0.4); 8.8346 (2.4); 8.3995 (1.7); 8.3701 (2.0); 8.1492 (1.5); 8.1249 (1.7); 7.8265 (1.0); 7.7981 (1.3); 7.7752 (0.9); 6.5318 (1.4); 5.7574 (0.8); 3.8205 (0.9); 3.7957 (3.7); 3.7863 (16.0); 3.7709 (3.2); 3.7464 (1.0); 3.6282 (0.4); 3.5977 (1.6); 3.5044 (0.7); 3.4591 (0.8); 3.4428 (0.9); 3.4279 (1.1); 3.3272 (2865.2); 3.2293 (0.7); 3.2216 (0.6); 2.7335 (2.7); 2.7276 (3.9); 2.7216 (3.0); 2.6179 (0.4); 2.5847 (0.8); 2.5131 (255.1); 2.5074 (494.6); 2.5015 (649.9); 2.4957 (454.5); 2.3976 (0.5); 2.3740 (0.3); 2.2770 (2.8); 2.2714 (3.8); 2.2653 (2.9); 2.0744 (1.4); 1.5058 (0.3); 1.4768 (0.4); 1.3220 (3.4); 1.2976 (7.2); 1.2729 (3.5); 1.2339 (7.3); 1.1482 (0.8); 1.1306 (0.3); 1.0771 (0.3); 0.9032 (0.4); 0.8725 (0.5); 0.8528 (0.9); 0.8267 (0.6); 0.8050 (0.6); 0.7800 (0.4); 0.1947 (1.0); 0.0107 (10.9); -0.0002 (273.0); -0.0112 (10.3); -0.0537 (0.4); -0.1988 (1.0) |
| Beispiel I-18: $^1$H-NMR(300 MHz, $D_6$-DMSO) δ = 10.70 (1), 9.49 (1), 8.92 (1), 8.71 (1), 8.33-8.25 (2), 4.33 (3), 3.86 - 3.83 (2), 2.56 (3), 1.30-1.25 (3) |
| Beispiel I-19: $^1$H-NMR(600 MHz, D6-DMSO) δ ppm: 9.82 (s, 1H), 9.44 (s, 1H), 8.94 (s, 1H), 8.75 (s, 1H), 8.70 (s, 1H), 4.25 (s, 3H), 3.60 - 3.54 (m, 2H), 1.19 (t, 3H). |

## Anwendungsbeispiele

### Ctenocephalides felis - in-vitro Kontakttests mit adulten Katzenflöhen

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht. Nach Abdampfen des Lösungsmittels werden die Gläschen mit 5-10 adulten Katzenflöhen (*Ctenocephalides felis*) besetzt, mit einem gelochten Kunststoffdeckel verschlossen und liegend bei Raumtemperatur und Umgebungsfeuchte inkubiert. Nach 48 h wird die Wirksamkeit bestimmt. Hierzu werden die Gläschen aufrecht gestellt und die Flöhe auf den Boden des Gläschens geklopft. Flöhe, die unbeweglich auf dem Boden verbleiben oder sich unkoordiniert bewegen, gelten als tot bzw. angeschlagen.

Eine Substanz zeigt gute Wirkung gegen *Ctenocephalides felis,* wenn in diesem Test bei einer Aufwandmenge von 5 $\mu g/cm^2$ mindestens 80% Wirkung erzielt wurde. Dabei bedeutet 100% Wirkung, dass alle Flöhe angeschlagen oder tot waren. 0% Wirkung bedeutet, dass keine Flöhe geschädigt wurden.

Bei diesem Test zeigen z.B. die folgenden Verbindungen der Herstellungsbeispiele eine Wirkung von 100% bei einer Aufwandmenge von 5 $\mu g/cm^2$ (= 500g/ha): I-2, I-6, I-5, I-10

### Rhipicephalus sanguineus - in-vitro Kontakttests mit Adulten der braunen Hundezecke

Für die Beschichtung der Teströhrchen werden zunächst 9 mg Wirkstoff in 1 ml Aceton p.a. gelöst und anschließend mit Aceton p.a. auf die gewünschte Konzentration verdünnt. 250 µl der Lösung werden durch Drehen und Kippen auf einem Rotationsschüttler (2 h Schaukelrotation bei 30 rpm) homogen auf den Innenwänden und dem Boden eines 25ml Glasröhrchens verteilt. Bei 900 ppm Wirkstofflösung und 44,7 $cm^2$ Innenoberfläche wird bei homogener Verteilung eine Flächendosis von 5 $\mu g/cm^2$ erreicht. Nach Abdampfen des Lösungsmittels werden die Gläschen mit 5-10 adulten Hundezecken (*Rhipicephalus sanguineus*) besetzt, mit einem gelochten Kunststoffdeckel verschlossen und liegend im Dunkeln bei Raumtemperatur und Umgebungsfeuchte inkubiert. Nach 48 h wird die Wirksamkeit bestimmt.

Hierzu werden die Zecken auf den Boden des Gläschens geklopft und auf einer Wärmeplatte bei 45-50°C maximal 5 min. inkubiert. Zecken, die unbeweglich auf dem Boden verbleiben oder sich so unkoordiniert bewegen, dass sie nicht gezielt der Wärme durch nach oben klettern ausweichen können, gelten als tot bzw. angeschlagen.

Eine Substanz zeigt gute Wirkung gegen *Rhipicephalus sanguineus,* wenn in diesem Test bei einer Aufwandmenge von 5 $\mu g/cm^2$ mindestens 80% Wirkung erzielt wurde. Dabei bedeutet 100% Wirkung, dass alle Zecken angeschlagen oder tot waren. 0% Wirkung bedeutet, dass keine Zecken geschädigt wurden.

Bei diesem Test zeigen z.B. die folgenden Verbindungen der Herstellungsbeispiele eine Wirkung von 100% bei einer Aufwandmenge von 5 $\mu g/cm^2$ (= 500g/ha): I-10

## Ctenocephalides felis - Oraltest

Lösungsmittel: Dimethylsulfoxid

Zwecks Herstellung einer zweckmäßigen Wirkstoffzubereitung vermischt man 10 mg Wirkstoff mit 0,5 ml Dimethylsulfoxid. Durch Verdünnen mit citriertem Rinderblut erhält man die gewünschte Konzentration.

Ca. 20 nüchterne adulte Katzenflöhe (*Ctenocephalides felis*) werden in eine Kammer eingesetzt, die oben und unten mit Gaze verschlossen ist. Auf die Kammer wird ein Metallzylinder gestellt, dessen Unterseite mit Parafilm verschlossen ist. Der Zylinder enthält die Blut-Wirkstoffzubereitung, die von den Flöhen durch die Parafilmmembran aufgenommen werden kann.

Nach 2 Tagen wird die Abtötung in % bestimmt. Dabei bedeutet 100 %, dass alle Flöhe abgetötet wurden; 0 % bedeutet, dass keiner der Flöhe abgetötet wurde.

Bei diesem Test zeigen z.B. die folgenden Verbindungen der Herstellungsbeispiele eine Wirkung von 80% bei einer Aufwandmenge von 100ppm: I-2

## Lucilia cuprina - Test

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Ca. 20 L1-Larven der Australischen Schafgoldfliege (*Lucilia cuprina*) werden in ein Testgefäß überführt, welches gehacktes Pferdefleisch und die Wirkstoffzubereitung der gewünschten Konzentration enthält.

Nach 2 Tagen wird die Abtötung in % bestimmt. Dabei bedeutet 100 %, dass alle Larven abgetötet wurden; 0 % bedeutet, dass keine Larven abgetötet wurden.

Bei diesem Test zeigen z.B. die folgenden Verbindungen der Herstellungsbeispiele eine Wirkung von 100% bei einer Aufwandmenge von 100ppm: I-1, I-2, I-3, I-5, I-6, I-10

## Musca domestica-Test

Lösungsmittel: Dimethylsulfoxid

Zur Herstellung einer zweckmäßigen Wirkstoffzubereitung vermischt man 10 mg Wirkstoff mit 0,5 ml Dimethylsulfoxid und verdünnt das Konzentrat mit Wasser auf die gewünschte Konzentration.

Gefäße, die einen Schwamm enthalten, der mit Zuckerlösung und der Wirkstoffzubereitung der gewünschten Konzentration behandelt wurde, werden mit 10 adulten Stubenfliegen *(Musca domestica)* besetzt.

Nach 2 Tagen wird die Abtötung in % bestimmt. Dabei bedeutet 100 %, dass alle Fliegen abgetötet wurden; 0 % bedeutet, dass keine der Fliegen abgetötet wurde.

Bei diesem Test zeigen z.B. die folgenden Verbindungen der Herstellungsbeispiele eine Wirkung von 90% bei einer Aufwandmenge von 100ppm: I-6

**Myzus persicae - Oraltest**

Lösungsmittel: 100 Gewichtsteile Aceton

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.
50 μl der Wirkstoffzubereitung werden in Mikrotiterplatten überführt und mit 150μl IPL41Insektenmedium (33% + 15% Zucker) auf eine Endvolumen von 200 μl aufgefüllt. Anschließend werden die Platten mit Parafilm verschlossen, durch den eine gemischte Population der Grünen Pfirsichblattlaus *(Myzus persicae),* die sich in einer zweiten Mikrotiterplatte befindet, hindurchstechen und die Lösung aufnehmen kann.
Nach 5 Tagen wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele Wirkung von 100% bei einer Aufwandmenge von 20ppm: I-2, I-6
Bei diesem Test zeigen z. B. die folgenden Verbindungen der Herstellungsbeispiele Wirkung von 100% bei einer Aufwandmenge von 4ppm: I-2, I-5, I-6, I-10, I-11, I-12
Bei diesem Test zeigen z. B. die folgenden Verbindungen der Herstellungsbeispiele Wirkung von 90% bei einer Aufwandmenge von 4ppm: I-1, I-3, I-4

**Myzus persicae - Sprühtest**

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.
Nach 5 Tagen wird die Wirkung in % bestimmt. Dabei bedeutet 100 %, dass alle Blattläuse abgetötet wurden; 0 % bedeutet, dass keine Blattläuse abgetötet wurden.
Bei diesem Test zeigen z. B. die folgenden Verbindungen der Herstellungsbeispiele Wirkung von 100% bei einer Aufwandmenge von 100g/ha: I-3
Bei diesem Test zeigen z. B. die folgenden Verbindungen der Herstellungsbeispiele Wirkung von 90% bei einer Aufwandmenge von 100g/ha: I-10, I-11

**Phaedon cochleariae - Sprühtest**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.
Chinakohlblattscheiben *(Brassica pekinensis)* werden mit einer Wirkstoffzubereitung der gewünschten Konzentration gespritzt und nach dem Abtrocknen mit Larven des Meerrettichblattkäfers *(Phaedon cochleariae)* besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 100g/ha: I-1, I-2, I-3, I-4, I-5, I-6, I-12

**Spodoptera frugiperda - Sprühtest**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays)* werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 100% bei einer Aufwandmenge von 100g/ha: I-1, I-2, I-5, I-6, I-12

## Patentansprüche

**1.** Verbindungen der Formel (Ia) oder (Ib)

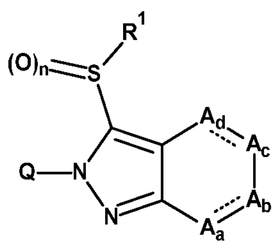

*Ia*

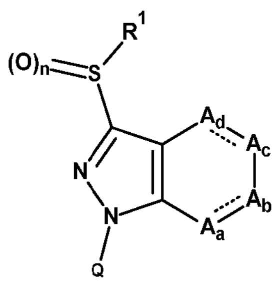

*Ib*

wobei 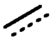 für Einfachbindungen oder Doppelbindungen stehen,
in welchen,

für den Fall, dass 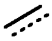 ausschließlich für Doppelbindungen stehen und somit Aa bis Ad mit den jeweils zu Aa und Ad benachbarten C-Atomen einen aromatischen Ring ausbilden,

Aa für Stickstoff oder $C(R^{10})$ steht,
Ab für Stickstoff oder $C(R^{11})$ steht,
Ac für Stickstoff oder $C(R^{12})$ steht und
Ad für Stickstoff oder $C(R^{13})$ steht,

wobei Aa, Ab, Ac und Ad für maximal zwei Stickstoffe stehen,

oder für den Fall, dass 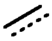 ausschließlich für Einfachbindungen stehen,

Aa für $C(R^{10})(R^{14})$ steht,

Ab für $C(R^{11})(R^{15})$ steht,
Ac für $C(R^{12})(R^{16})$ steht und
Ad für $C(R^{13})(R^{17})$ steht,
$R^1$ für $(C_1-C_6)$Alkyl, $(C_1-C_6)$Halogenalkyl, $(C_1-C_6)$Cyanoalkyl, $(C_1-C_6)$Hydroxyalkyl, $(C_1-C_6)$Alkoxy-$(C_1-C_6)$alkyl, $(C_1-C_6)$Halogenalkoxy-$(C_1-C_6)$alkyl, $(C_2-C_6)$Alkenyl, $(C_2-C_6)$Alkenyloxy-$(C_1-C_6)$alkyl, $(C_2-C_6)$Halogenalkenyloxy-$(C_1-C_6)$alkyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_6)$alkyl, $(C_2-C_6)$Halogenalkinyloxy-$(C_1-C_6)$alkyl, $(C_2-C_6)$Halogenalkinyl, $(C_2-C_6)$Cyanoalkinyl, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, Amino, $(C_1-C_6)$Alkylamino, Di-$(C_1-C_6)$alkyl-amino, $(C_3-C_8)$Cycloalkylamino, $(C_1-C_6)$Alkylcarbonyl-amino, $(C_1-C_6)$Alkylthio-$(C_1-C_6)$alkyl, $(C_1-C_6)$Halogenalkylthio-$(C_1-C_6)$alkyl, $(C_1-C_6)$Alkylsulfinyl-$(C_1-C_6)$alkyl, $(C_1-C_6)$Halogenalkylsulfinyl-$(C_1-C_6)$alkyl, $(C_1-C_6)$Alkylsulfonyl-$(C_1-C_6)$alkyl, $(C_1-C_6)$Halogenalkylsulfonyl-$(C_1-C_6)$alkyl, $(C_1-C_6)$Alkoxy-$(C_1-C_6)$alkylthio-$(C_1-C_6)$alkyl, $(C_1-C_6)$Alkoxy-$(C_1-C_6)$alkylsulfinyl-$(C_1-C_6)$alkyl, $(C_1-C_6)$Alkoxy-$(C_1-C_6)$alkylsulfonyl-$(C_1-C_6)$alkyl, $(C_1-C_6)$Alkylcarbonyl-$(C_1-C_6)$alkyl, $(C_1-C_6)$Halogenalkylcarbonyl-$(C_1-C_6)$alkyl, $(C_1-C_6)$Alkoxycarbonyl-$(C_1-C_6)$alkyl, $(C_1-C_6)$Halogenalkoxycarbonyl-$(C_1-C_6)$alkyl, $(C_1-C_6)$Alkylsulfonylamino, Aminosulfonyl-$(C_1-C_6)$alkyl, $(C_1-C_6)$Alkylaminosulfonyl-$(C_1-C_6)$alkyl, Di-$(C_1-C_6)$alkyl-aminosulfonyl-$(C_1-C_6)$alkyl,
oder für jeweils einfach oder mehrfach, gleich oder verschieden durch Aryl, Hetaryl oder Heterocyclyl substituiertes $(C_1-C_6)$Alkyl, $(C_1-C_6)$Alkoxy, $(C_2-C_6)$Alkenyl, $(C_2-C_6)$Alkinyl, $(C_3-C_8)$Cycloalkyl steht, wobei Aryl, Hetaryl oder Heterocyclyl jeweils gegebenenfalls einfach oder mehrfach, gleich oder verschieden durch Halogen, Cyano, Nitro, Hydroxy, Amino, Carboxy, Carbamoyl, Aminosulfonyl, $(C_1-C_6)$Alkyl, $(C_3-C_6)$Cycloalkyl, $(C_1-C_6)$Alkoxy, $(C_1-C_6)$Halogenalkyl, $(C_1-C_6)$Halogenalkoxy, $(C_1-C_6)$Alkylthio, $(C_1-C_6)$Alkyl-sulfinyl, $(C_1-C_6)$Alkylsulfonyl, $(C_1-C_6)$Alkylsulfimino, $(C_1-C_6)$Alkylsulfimino-$(C_1-C_6)$alkyl, $(C_1-C_6)$Alkylsulfimino-$(C_2-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkylsilyl oder Benzyl substituiert sein können, oder
$R^1$ für jeweils gegebenenfalls einfach oder mehrfach, gleich oder verschieden durch Halogen, Cyano, Nitro, Hydroxy, Amino, Carboxy, Carbamoyl, $(C_1-C_6)$Alkyl, $(C_3-C_8)$Cycloalkyl, $(C_1-C_6)$-Alkoxy, $(C_1-C_6)$Halogenalkyl, $(C_1-C_6)$Halogenalkoxy, $(C_1-C_6)$Alkylthio, $(C_1-C_6)$Alkylsulfinyl, $(C_1-C_6)$Alkylsulfonyl, $(C_1-C_6)$Alkylsulfimino, $(C_1-C_6)$Alkylsulfimino-$(C_1-C_6)$alkyl, $(C_1-C_6)$Alkylsulfimino-$(C_2-C_6)$alkylcarbonyl, $(C_1-C_6)$Alkylsulfoximino, $(C_1-C_6)$Alkylsulfoximino-$(C_1-C_6)$alkyl, $(C_1-C_6)$Alkylsulfoximino-$(C_2-C_6)$alkylcarbonyl, $(C_1-C_6)$Alkoxycarbonyl, $(C_1-C_6)$Alkylcarbonyl, $(C_3-C_6)$Trialkylsilyl, (=O) (nur im Fall von Heterocyclyl) oder $(=O)_2$ (nur im Fall von Heterocyclyl) substituiertes Aryl, Hetaryl oder Heterocyclyl steht,
$R^{10}$, $R^{11}$, $R^{12}$, $R^{13}$, $R^{14}$, $R^{15}$, $R^{16}$, $R^{17}$ unabhängig voneinander für Wasserstoff, Cyano, Halogen, Nitro, Hydroxy, Amino, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylanünothiocarbonyl, Di-$(C_1-C_6)$alkylaminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkylaminothiocarbonyl, $(C_3-C_8)$Cycloalkylamino, $(C_1-C_6)$Alkylcarbamoyl (umfasst -NHCOO$(C_1-C_6)$alkyl, -N$(C_1-C_6)$alkyl-COO$(C_1-C_6)$alkyl, -OCONH$(C_1-C_6)$alkyl oder -OCON$(C_1-C_6)$dialkyl), $(C_1-C_6)$Alkylcarbonylamino (($C_1-C_6$)alkyl-CONH), $(C_1-C_6)$Alkylharnstoff (umfasst -NHCONH$(C_1-C_6)$alkyl, und -NHCON$(C_1-C_6)$dialkyl) oder
für jeweils gegebenenfalls einfach oder mehrfach, gleich oder verschieden substituiertes Aryl oder Hetaryl stehen, wobei (im Fall von Hetaryl) gegebenenfalls mindestens eine Carbonylgruppe enthalten sein kann und wobei als Substituenten jeweils in Frage kommen: Cyano, Carboxyl, Halogen, Nitro, Hydroxy, Amino, Tri-$(C_1-C_6)$alkylsilyl, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Halogenalkinyl, $(C_2-C_6)$Cyanoalkinyl,

$(C_1-C_6)$Alkoxy, $(C_1-C_6)$Halogenalkoxy, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, Di-$(C_1-C_6)$alkyl-aminocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkylaminosulfonyl, $(C_1-C_6)$Alkylsulfoximino, Aminothiocarbonyl, $(C_1-C_6)$Alkylaminothiocarbonyl, Di-$(C_1-C_6)$alkylaminothiocarbonyl, $(C_3-C_8)$Cycloalkylamino oder $(C_1-C_6)$Alkylcarbonylamino,
wobei nur einer oder zwei der Reste $R^{10}$, $R^{11}$, $R^{12}$, $R^{13}$, $R^{14}$, $R^{15}$, $R^{16}$ und $R^{17}$ für einen Substituenten ungleich Wasserstoff stehen,
und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, $(C_3-C_8)$Cycloalkyl, $(C_3-C_8)$Cycloalkyl-$(C_3-C_8)$cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl oder $(C_1-C_6)$Cyanoalkyl stehen,
Q für ein teilweise gesättigtes oder gesättigtes heterozyklisches oder heteroaromatisches 8-, 9-, 11- oder 12-gliedriges annelliertes bicyclisches oder tricyclisches Ringsystem steht, wobei gegebenenfalls mindestens eine Carbonylgruppe enthalten sein kann und wobei das Ringsystem gegebenenfalls einfach oder mehrfach, gleich oder verschieden substituiert ist, und wobei die Substituenten unabhängig voneinander ausgewählt sein können aus Cyano, Halogen, Nitro, Hydroxy, Amino, Tri-$(C_1-C_6)$alkylsilyl, $(C_3-C_8)$Cycloalkyl, $(C_3-C_8)$Cycloalkyl-$(C_3-C_8)$Cycloalkyl, $(C_1-C_6)$Alkyl-$(C_3-C_8)$cycloalkyl, Halogen$(C_3-C_8)$cycloalkyl, $(C_1-C_6)$Alkyl, $(C_1-C_6)$Halogenalkyl, $(C_1-C_6)$Cyanoalkyl, $(C_1-C_6)$Hydroxyalkyl, Hydroxycarbonyl-$(C_1-C_6)$-alkoxy, $(C_1-C_6)$Alkoxycarbonyl-$(C_1-C_6)$alkyl, $(C_1-C_6)$Alkoxy-$(C_1-C_6)$alkyl, $(C_2-C_6)$Alkenyl, $(C_2-C_6)$Halogenalkenyl, $(C_2-C_6)$Cyanoalkenyl, $(C_2-C_6)$Alkinyl, $(C_2-C_6)$Alkinyloxy-$(C_1-C_4)$alkyl, $(C_2-C_6)$Halogenalkinyl, $(C_2-C_6)$Cyanoalkinyl, $(C_1-C_6)$Alkoxy, $(C_1-C_6)$Halogenalkoxy, $(C_1-C_6)$Halogenalkoxy-$(C_1-C_6)$alkyl, $(C_2-C_6)$Alkenyloxy-$(C_1-C_6)$alkyl, $(C_2-C_6)$Halogenalkenyloxy-$(C_1-C_6)$ alkyl, $(C_1-C_6)$Cyanoalkoxy, $(C_1-C_6)$Alkoxycarbonyl-$(C_1-C_6)$alkoxy, $(C_1-C_6)$Alkoxy-$(C_1-C_6)$alkoxy, $(C_1-C_6)$Alkylhydroxyimino, $(C_1-C_6)$Alkoxyimino, $(C_1-C_6)$Alkyl-$(C_1-C_6)$alkoxyimino, $(C_1-C_6)$Halogenalkyl-$(C_1-C_6)$alkoxyimino, $(C_1-C_6)$Alkylthio, $(C_1-C_6)$Halogenalkylthio, $(C_1-C_6)$Alkoxy-$(C_1-C_6)$alkylthio, $(C_1-C_6)$Alkylthio-$(C_1-C_6)$alkyl, $(C_1-C_6)$Alkylsulfinyl, $(C_1-C_6)$Halogenalkylsulfinyl, $(C_1-C_6)$Alkoxy-$(C_1-C_6)$alkylsulfinyl, $(C_1-C_6)$Alkylsulfinyl-$(C_1-C_6)$alkyl, $(C_1-C_6)$Alkylsulfonyl, $(C_1-C_6)$Halogenalkylsulfonyl, $(C_1-C_6)$Alkoxy-$(C_1-C_6)$alkylsulfonyl, $(C_1-C_6)$Alkylsulfonyl-$(C_1-C_6)$alkyl, $(C_1-C_6)$Alkylsulfonyloxy, $(C_1-C_6)$Alkylcarbonyl, $(C_1-C_6)$Alkylcarbonyl-$(C_1-C_6)$alkyl, $(C_1-C_6)$Alkylthiocarbonyl, $(C_1-C_6)$Halogenalkylcarbonyl, $(C_1-C_6)$Alkylcarbonyloxy, $(C_1-C_6)$Alkoxycarbonyl, $(C_1-C_6)$Halogenalkoxycarbonyl, Aminocarbonyl, $(C_1-C_6)$Alkylaminocarbonyl, $(C_1-C_6)$Alkylaminothiocarbonyl, Di-$(C_1-C_6)$alkyl-aminocarbonyl, Di-$(C_1-C_6)$alkyl-aminothiocarbonyl, $(C_2-C_6)$Alkenylaminocarbonyl, Di-$(C_2-C_6)$-alkenylaminocarbonyl, $(C_3-C_8)$Cycloalkylaminocarbonyl, $(C_1-C_6)$Alkylsulfonylamino, $(C_1-C_6)$Alkylamino, Di-$(C_1-C_6)$Alkylamino, Aminosulfonyl, $(C_1-C_6)$Alkylaminosulfonyl, Di-$(C_1-C_6)$alkyl-aminosulfonyl, $(C_1-C_6)$Alkylsulfoximino, Aminothiocarbonyl, $(C_1-C_6)$Alkylaminothiocarbonyl, Di-$(C_1-C_6)$alkyl-aminothiocarbonyl, $(C_3-C_8)$Cycloalkylamino oder $(C_1-C_6)$Alkylcarbonylamino
oder wobei die Substituenten unabhängig voneinander ausgewählt sein können aus Phenyl oder einem 5- oder 6-gliedrigen heteroaromatischen Ring, wobei Phenyl oder der Ring gegebenenfalls einfach oder mehrfach, gleich oder verschieden durch $C_1$-$C_6$-Alkyl, $C_2$-$C_6$-Alkenyl, $C_2$-$C_6$-Alkinyl, $C_3$-$C_6$-Cycloalkyl, $C_1$-$C_6$-Haloalkyl, $C_2$-$C_6$-Haloalkenyl, $C_2$-$C_6$-Haloalkinyl, $C_3$-$C_6$-Halocycloalkyl, Halogen, CN, $C_1$-$C_4$-Alkoxy, $C_1$-$C_4$-Haloalkoxy substituiert sein können,
n für 0, 1 oder 2 steht.

2. Verbindungen der Formel (Ia) oder (Ib) gemäß Anspruch 1, in welchen

für den Fall, dass 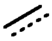 ausschließlich für Doppelbindungen stehen und somit Aa bis Ad mit den jeweils zu Aa und Ad benachbarten C-Atomen einen aromatischen Ring ausbilden,

Aa für Stickstoff oder C($R^{10}$) steht,
Ab für Stickstoff oder C($R^{11}$) steht,
Ac für Stickstoff oder C($R^{12}$) steht und
Ad für Stickstoff oder C($R^{13}$) steht,

wobei Aa, Ab, Ac und Ad für maximal zwei Stickstoffe stehen,

oder für den Fall, dass 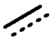 ausschließlich für Einfachbindungen stehen,

Aa für C(R$^{10}$)(R$^{14}$) steht,
Ab für C(R$^{11}$)(R$^{15}$) steht,
Ac für C(R$^{12}$)(R$^{16}$) steht und
Ad für C(R$^{13}$)(R$^{17}$) steht,
R$^1$ für (C$_1$-C$_4$)Alkyl, (C$_1$-C$_4$)Hydroxyalkyl, (C$_1$-C$_4$)Halogenalkyl, (C$_1$-C$_4$)Cyanoalkyl, (C$_1$-C$_4$)Alkoxy-(C$_1$-C$_4$)alkyl, (C$_1$-C$_4$)Halogenalkoxy-(C$_1$-C$_4$)alkyl, (C$_2$-C$_4$)Alkenyl, (C$_2$-C$_4$)Alkenyloxy-(C$_1$-C$_4$)alkyl, (C$_2$-C$_4$)Halogenalkenyloxy-(C$_1$-C$_4$)alkyl, (C$_2$-C$_4$)Halogenalkenyl, (C$_2$-C$_4$)Cyanoalkenyl, (C$_2$-C$_4$)Alkinyl, (C$_2$-C$_4$)Alkinyloxy-(C$_1$-C$_4$)alkyl, (C$_2$-C$_4$)Halogenalkinyloxy-(C$_1$-C$_4$)alkyl, (C$_2$-C$_4$)Halogenalkinyl, (C$_2$-C$_4$)Cyanoalkinyl, (C$_3$-C$_6$)Cycloalkyl, (C$_3$-C$_6$)Cycloalkyl(C$_3$-C$_6$)cycloalkyl, (C$_1$-C$_4$)Alkyl-(C$_3$-C$_6$)cycloalkyl, Halogen(C$_3$-C$_6$)cycloalkyl, (C$_1$-C$_4$)Alkylamino, Di-(C$_1$-C$_4$)alkyl-amino, (C$_3$-C$_6$)Cycloalkylamino, (C$_1$-C$_4$)Alkylcarbonyl-amino, (C$_1$-C$_4$)Alkylthio-(C$_1$-C$_4$)alkyl, (C$_1$-C$_4$)Halogenalkylthio-(C$_1$-C$_4$)alkyl, (C$_1$-C$_4$)Alkylsulfinyl-(C$_1$-C$_4$)alkyl, (C$_1$-C$_4$)Halogenalkylsulfinyl-(C$_1$-C$_4$)alkyl, (C$_1$-C$_4$)Alkylsulfonyl-(C$_1$-C$_4$)alkyl, (C$_1$-C$_4$)Alkylcarbonyl-(C$_1$-C$_4$)alkyl, (C$_1$-C$_4$)Halogenalkylcarbonyl-(C$_1$-C$_4$)alkyl oder (C$_1$-C$_4$)Alkylsulfonylamino steht,
R$^{10}$, R$^{11}$, R$^{12}$, R$^{13}$, R$^{14}$, R$^{15}$, R$^{16}$, R$^{17}$ unabhängig voneinander für Wasserstoff, Cyano, Halogen, Nitro, Hydroxy, Amino, Tri-(C$_1$-C$_4$)alkylsilyl, (C$_3$-C$_6$)Cycloalkyl, (C$_3$-C$_6$)Cycloalkyl-(C$_3$-C$_6$)cycloalkyl, (C$_1$-C$_4$)Alkyl-(C$_3$-C$_6$)cycloalkyl, Halogen(C$_3$-C$_6$)cycloalkyl, (C$_1$-C$_4$)Alkyl, (C$_1$-C$_4$)Halogenalkyl, (C$_1$-C$_4$)Cyanoalkyl, (C$_1$-C$_4$)Hydroxyalkyl, (C$_1$-C$_4$)Alkoxy-(C$_1$-C$_4$)alkyl, (C$_2$-C$_4$)Alkenyl, (C$_2$-C$_4$)Halogenalkenyl, (C$_2$-C$_4$)Cyanoalkenyl, (C$_2$-C$_4$)Alkinyl, (C$_2$-C$_4$)Halogenalkinyl, (C$_2$-C$_4$)Cyanoalkinyl, (C$_1$-C$_4$)Alkoxy, (C$_1$-C$_4$)Halogenalkoxy, (C$_1$-C$_4$)Cyanoalkoxy, (C$_1$-C$_4$)Alkoxy-(C$_1$-C$_4$)alkoxy, (C$_1$-C$_4$)Alkylhydroxyimino, (C$_1$-C$_4$)Alkoxyimino, (C$_1$-C$_4$)Alkyl-(C$_1$-C$_4$)alkoxyimino, (C$_1$-C$_4$)Halogenalkyl-(C$_1$-C$_4$)alkoxyimino, (C$_1$-C$_4$)Alkylthio, (C$_1$-C$_4$)Halogenalkylthio, (C$_1$-C$_4$)Alkylthio-(C$_1$-C$_4$)alkyl, (C$_1$-C$_4$)Alkylsulfinyl, (C$_1$-C$_4$)Halogenalkylsulfinyl, (C$_1$-C$_4$)Alkylsulfinyl-(C$_1$-C$_4$)alkyl, (C$_1$-C$_4$)Alkylsulfonyl, (C$_1$-C$_4$)Halogenalkylsulfonyl, (C$_1$-C$_4$)Alkylsulfonyl-(C$_1$-C$_4$)alkyl, (C$_1$-C$_4$)Alkylsulfonyloxy, (C$_1$-C$_4$)Alkylcarbonyl, (C$_1$-C$_4$)Halogenalkylcarbonyl, Aminocarbonyl, Aminothiocarbonyl, (C$_1$-C$_4$)Alkylaminocarbonyl, Di-(C$_1$-C$_4$)alkyl-aminocarbonyl, (C$_3$-C$_6$)Cycloalkylaminocarbonyl, (C$_1$-C$_4$)Alkylsulfonylamino, (C$_1$-C$_4$)Alkylamino, Di-(C$_1$-C$_4$)Alkylamino, Aminosulfonyl, (C$_1$-C$_4$)Alkylaminosulfonyl, Di-(C$_1$-C$_4$)alkyl-aminosulfonyl, Aminothiocarbonyl, (C$_1$-C$_4$)Alkylcarbamoyl (umfasst -NHCOO(C$_1$-C$_4$)alkyl, -N(C$_1$-C$_4$)alkylCOO(C$_1$-C$_4$)alkyl, -OCONH(C$_1$-C$_4$)alkyl oder -OCON(C$_1$-C$_4$)dialkyl), (C$_1$-C$_4$)Alkylcarbonylamino, (C$_1$-C$_4$)Alkylharnstoff (umfasst -NHCONH(C$_1$-C$_4$)alkyl und -NHCON(C$_1$-C$_4$)dialkyl) stehen
oder für jeweils gegebenenfalls einfach oder zweifach, gleich oder verschieden substituiertes Phenyl oder Hetaryl stehen, wobei (im Fall von Hetaryl) gegebenenfalls mindestens eine Carbonylgruppe enthalten sein kann und wobei als Substituenten jeweils in Frage kommen: Cyano, Halogen, Nitro, Acetyl, Amino, (C$_3$-C$_6$)Cycloalkyl, (C$_3$-C$_6$)Cycloalkyl-(C$_3$-C$_6$)cycloalkyl, (C$_1$-C$_4$)Alkyl-(C$_3$-C$_6$)cycloalkyl, Halogen(C$_3$-C$_6$)cycloalkyl, (C$_1$-C$_4$)Alkyl, (C$_1$-C$_4$)Halogenalkyl, (C$_1$-C$_4$)Cyanoalkyl, (C$_1$-C$_4$)Hydroxyalkyl, (C$_1$-C$_4$)Alkoxy-(C$_1$-C$_4$)alkyl, (C$_2$-C$_4$)Alkenyl, (C$_2$-C$_4$)Halogenalkenyl, (C$_2$-C$_4$)Cyanoalkenyl, (C$_2$-C$_4$)Alkinyl, (C$_2$-C$_4$)Halogenalkinyl, (C$_2$-C$_4$)Cyanoalkinyl, (C$_1$-C$_4$)Alkoxy, (C$_1$-C$_4$)Halogenalkoxy, (C$_1$-C$_4$)Cyanoalkoxy, (C$_1$-C$_4$)Alkoxy-(C$_1$-C$_4$)alkoxy, (C$_1$-C$_4$)Alkylhydroxyimino, (C$_1$-C$_4$)Alkoxyimino, (C$_1$-C$_4$)Alkyl-(C$_1$-C$_4$)alkoxyimino, (C$_1$-C$_4$)Halogenalkyl-(C$_1$-C$_4$)alkoxyimino, (C$_1$-C$_4$)Alkylthio, (C$_1$-C$_4$)Halogenalkylthio, (C$_1$-C$_4$)Alkylthio-(C$_1$-C$_4$)alkyl, (C$_1$-C$_4$)Alkylsulfinyl, (C$_1$-C$_4$)Halogena$_l$kylsu$_l$finyl, (C$_1$-C$_4$)Alkylsulfinyl-(C$_1$-C$_4$)alkyl, (C$_1$-C$_4$)Alkylsulfonyl, (C$_1$-C$_4$)Halogenalkylsulfonyl, (C$_1$-C$_4$)Alkylsulfonyl-(C$_1$-C$_4$)alkyl, (C$_1$-C$_4$)Alkylsulfonyloxy, (C$_1$-C$_4$)Alkylcarbonyl, (C$_1$-C$_4$)Halogenalkylcarbonyl, Aminocarbonyl, (C$_1$-C$_4$)Alkylaminocarbonyl, Di-(C$_1$-C$_4$)alkyl-aminocarbonyl, (C$_1$-C$_4$)Alkylsulfonylamino, (C$_1$-C$_4$)Alkylamino, Di-(C$_1$-C$_4$)Alkylamino, Aminosulfonyl, (C$_1$-C$_4$)Alkylaminosulfonyl, Di-(C$_1$-C$_4$)alkylaminosulfonyl oder (C$_1$-C$_4$)Alkylcarbonylamino,
wobei nur einer oder zwei der Reste R$^{10}$, R$^{11}$, R$^{12}$, R$^{13}$, R$^{14}$, R$^{15}$, R$^{16}$, R$^{17}$ für einen Substituenten ungleich Wasserstoff stehen,
und im Falle dass R$^{10}$ und R$^{14}$, R$^{11}$ und R$^{15}$, R$^{12}$ und R$^{16}$ oder R$^{13}$ und R$^{17}$ jeweils beide für ungleich Wasserstoff stehen, R$^{14}$, R$^{15}$, R$^{16}$ und R$^{17}$ jeweils unabhängig voneinander nur für Cyano, Halogen, (C$_3$-C$_6$)Cycloalkyl, (C$_3$-C$_6$)Cycloalkyl-(C$_3$-C$_6$)cycloalkyl, (C$_1$-C$_4$)Alkyl-(C$_3$-C$_6$)cycloalkyl, Halogen(C$_3$-C$_6$)cycloalkyl, (C$_1$-C$_4$)Alkyl, (C$_1$-C$_4$)Halogenalkyl oder (C$_1$-C$_4$)Cyanoalkyl stehen,
Q für ein heteroaromatisches 9-gliedriges oder 12-gliedriges annelliertes bicyclisches oder tricyclisches Ringsystem aus der Reihe Q1 bis Q15 steht:

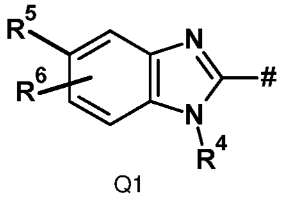
Q1

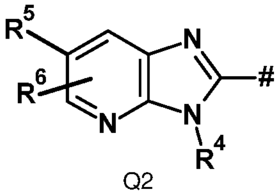
Q2

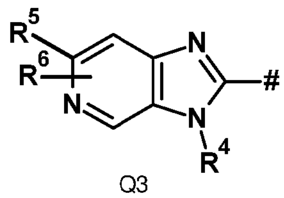
Q3

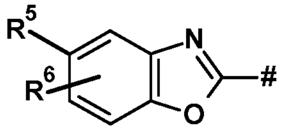
Q4

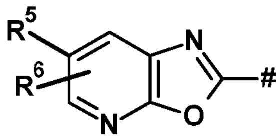
Q5

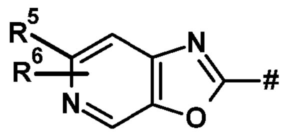
Q6

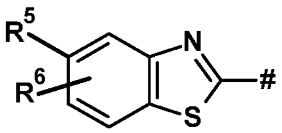
Q7

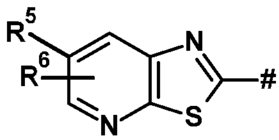
Q8

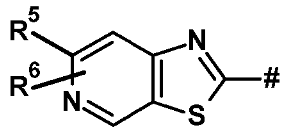
Q9

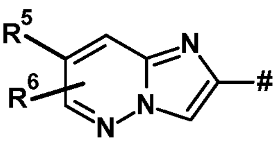
Q10

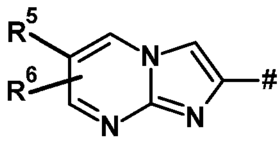
Q11

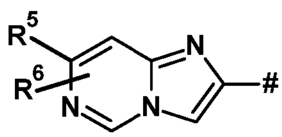
Q12

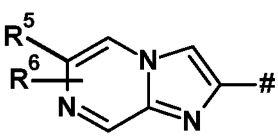
Q13

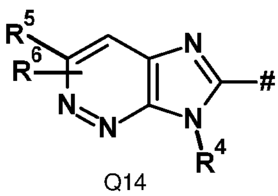
Q14

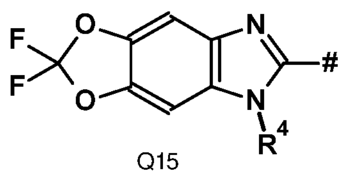
Q15

wobei

$R^4$ für Wasserstoff, ($C_1$-$C_4$)Alkyl, ($C_1$-$C_4$)Halogenalkyl, ($C_1$-$C_4$)Cyanoalkyl, ($C_1$-$C_4$)Hydroxyalkyl, ($C_1$-$C_4$)Alkoxy-($C_1$-$C_4$)alkyl, ($C_1$-$C_4$)Halogenalkoxy-($C_1$-$C_4$)alkyl, ($C_2$-$C_4$)Alkenyl, ($C_2$-$C_4$)Alkenyloxy-($C_1$-$C_4$)alkyl, ($C_2$-$C_4$)Halogenalkenyloxy-($C_1$-$C_4$)alkyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl oder ($C_3$-$C_6$)Cycloalkyl steht und

$R^5$, $R^6$ unabhängig voneinander für Wasserstoff, Cyano, Halogen, ($C_1$-$C_4$)Alkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, ($C_1$-$C_4$)Halogenalkyl-($C_3$-$C_6$)cycloalkyl, Cyano-($C_3$-$C_6$)cycloalkyl, Halogen-($C_3$-$C_6$)cycloalkyl, ($C_1$-$C_4$)Alkoxy, ($C_1$-$C_4$)Halogenalkoxy, ($C_1$-$C_4$)Alkoxyimino, ($C_1$-$C_4$)Halogenalkoxyimino ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Halogenalkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl oder Di-($C_1$-$C_4$)alkylaminosulfonyl stehen

und

n für 0, 1 oder 2 steht.

**3.** Verbindungen der Formel (Ia) oder (Ib) gemäß Anspruch 2, in welchen

für den Fall, dass 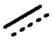 ausschließlich für Doppelbindungen stehen und somit Aa bis Ad mit den jeweils zu Aa und Ad benachbarten C-Atomen einen aromatischen Ring ausbilden,

Aa für Stickstoff oder C($R^{10}$) steht,
Ab für Stickstoff oder C($R^{11}$) steht,
Ac für Stickstoff oder C($R^{12}$) steht und
Ad für Stickstoff oder C($R^{13}$) steht,

wobei Aa, Ab, Ac und Ad für maximal zwei Stickstoffe stehen,

oder für den Fall, dass 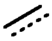 ausschließlich für Einfachbindungen stehen,

Aa für C($R^{10}$)($R^{14}$) steht,
Ab für C($R^{11}$)($R^{15}$) steht,
Ac für C($R^{12}$)($R^{16}$) steht und
Ad für C($R^{13}$)($R^{17}$) steht,
$R^1$ für ($C_1$-$C_4$)Alkyl, ($C_1$-$C_4$)Hydroxyalkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_1$-$C_4$)Alkylthio-($C_1$-$C_4$)alkyl, ($C_1$-$C_4$)Alkylsulfinyl-($C_1$-$C_4$)alkyl oder ($C_1$-$C_4$)Alkylsulfonyl-($C_1$-$C_4$)alkyl steht,
$R^{10}$, $R^{11}$, $R^{12}$, $R^{13}$, $R^{14}$, $R^{15}$, $R^{16}$, $R^{17}$ unabhängig voneinander für Wasserstoff, Cyano, Halogen, Nitro, Hydroxy, Amino, Tri-($C_1$-$C_4$)alkylsilyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_1$-$C_4$)Alkoxy-($C_1$-$C_4$)alkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Cyanoalkoxy, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylannnocarbonyl, Di-($C_1$-$C_4$)alkyl-aminocarbonyl, ($C_3$-$C_6$)Cycloalkylaminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkyl-aminosulfonyl, ($C_1$-$C_4$)Alkylcarbamoyl (umfasst -NHCOO($C_1$-$C_4$)alkyl und -N($C_1$-$C_4$)alkylCOO($C_1$-$C_4$)alkyl), ($C_1$-$C_4$)Alkylcarbonylamino, ($C_1$-$C_4$)Alkylharnstoff (umfasst -NHCONH($C_1$-$C_4$)alkyl und -NHCON($C_1$-$C_4$)dialkyl) stehen
oder für jeweils gegebenenfalls einfach oder zweifach, gleich oder verschieden substituiertes Phenyl oder Hetaryl stehen, wobei (im Fall von Hetaryl) gegebenenfalls mindestens eine Carbonylgruppe enthalten sein kann und wobei als Substituenten jeweils in Frage kommen: Cyano, Halogen, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl, ($C_1$-$C_4$)Cyanoalkyl, ($C_2$-$C_4$)Alkenyl, ($C_2$-$C_4$)Halogenalkenyl, ($C_2$-$C_4$)Cyanoalkenyl, ($C_2$-$C_4$)Alkinyl, ($C_2$-$C_4$)Halogenalkinyl, ($C_2$-$C_4$)Cyanoalkinyl, ($C_1$-$C_4$)Alkoxy, ($C_1$-$C_4$)Halogenalkoxy, ($C_1$-$C_4$)Alkylthio, ($C_1$-$C_4$)Halogenalkylthio, ($C_1$-$C_4$)Alkylsulfinyl, ($C_1$-$C_4$)Halogenalkylsulfinyl, ($C_1$-$C_4$)Alkylsulfonyl, ($C_1$-$C_4$)Halogenalkylsulfonyl, ($C_1$-$C_4$)Alkylsulfonyloxy, ($C_1$-$C_4$)Alkylcarbonyl, ($C_1$-$C_4$)Halogenalkylcarbonyl, Aminocarbonyl, ($C_1$-$C_4$)Alkylaminocarbonyl, Di-($C_1$-$C_4$)alkyl-aminocarbonyl, ($C_1$-$C_4$)Alkylsulfonylamino, ($C_1$-$C_4$)Alkylamino, Di-($C_1$-$C_4$)Alkylamino, Aminosulfonyl, ($C_1$-$C_4$)Alkylaminosulfonyl, Di-($C_1$-$C_4$)alkylaminosulfonyl, ($C_1$-$C_4$)Alkylcarbonylamino,
wobei nur einer oder zwei der Reste $R^{10}$, $R^{11}$, $R^{12}$, $R^{13}$, $R^{14}$, $R^{15}$, $R^{16}$, $R^{17}$ für einen Substituenten ungleich Wasserstoff stehen, und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, Halogen($C_3$-$C_6$)cycloalkyl, ($C_1$-$C_4$)Alkyl, ($C_1$-$C_4$)Halogenalkyl oder ($C_1$-$C_4$)Cyanoalkyl stehen,
Q für ein heteroaromatisches 9-gliedriges oder 12-gliedriges annelliertes bicyclisches oder tricyclisches Ringsystem aus der Reihe Q1 bis Q15 steht,
wobei
$R^4$ für Wasserstoff, ($C_1$-$C_4$)Alkyl, ($C_3$-$C_6$)Cycloalkyl, ($C_1$-$C_4$)Halogenalkyl, ($C_1$-$C_4$)Cyanoalkyl, ($C_1$-$C_4$)Alkoxy-($C_1$-$C_4$)alkyl, ($C_1$-$C_4$)Halogenalkoxy-($C_1$-$C_4$)alkyl, ($C_2$-$C_4$)Alkenyl, ($C_2$-$C_4$)Halogenalkenyl, ($C_2$-$C_4$)Alkinyl oder ($C_2$-$C_4$)Halogenalkinyl steht und
$R^5$, $R^6$ unabhängig voneinander für Wasserstoff, Cyano, Halogen, ($C_1$-$C_4$)Alkyl, ($C_1$-$C_4$)Halogenalkyl, ($C_2$-$C_4$)Alkenyl, ($C_2$-$C_4$)Halogenalkenyl, ($C_2$-$C_4$)Alkinyl, ($C_2$-$C_4$)Halogenalkinyl, ($C_3$-$C_6$)Cycloalkyl, ($C_3$-$C_6$)Cycloalkyl-($C_3$-$C_6$)cycloalkyl, ($C_1$-$C_4$)Alkyl-($C_3$-$C_6$)cycloalkyl, ($C_1$-$C_4$)Halogenalkyl-($C_3$-$C_6$)cycloalkyl, Cya-

no-$(C_3-C_6)$cycloalkyl, Halogen-$(C_3-C_6)$cycloalkyl, $(C_1-C_4)$Alkoxy, $(C_1-C_4)$Halogenalkoxy, $(C_1-C_4)$Alkoxyimino, $(C_1-C_4)$Halogenalkoxyimino $(C_1-C_4)$Alkylthio, $(C_1-C_4)$Halogenalkylthio, $(C_1-C_4)$Alkylsulfinyl, $(C_1-C_4)$Halogenalkylsulfinyl, $(C_1-C_4)$Alkylsulfonyl, $(C_1-C_4)$Halogenalkylsulfonyl, $(C_1-C_4)$Alkylsulfonyloxy, $(C_1-C_4)$Halogenalkylsulfonyloxy, $(C_1-C_4)$Alkylcarbonyl, $(C_1-C_4)$Halogenalkylcarbonyl, Aminocarbonyl, $(C_1-C_4)$Alkylaminocarbonyl, Di-$(C_1-C_4)$alkyl-aminocarbonyl, $(C_1-C_4)$Alkylsulfonylamino, $(C_1-C_4)$Alkylamino, Di-$(C_1-C_4)$Alkylamino, Aminosulfonyl, $(C_1-C_4)$Alkylaminosulfonyl oder Di-$(C_1-C_4)$alkylaminosulfonyl stehen
und
n für 0, 1 oder 2 steht.

**4.** Verbindungen der Formel (Ia) oder (Ib) gemäß Anspruch 2, in welchen für den Fall, dass 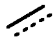 ausschließlich für Doppelbindungen stehen und somit Aa bis Ad mit den jeweils zu Aa und Ad benachbarten C-Atomen einen aromatischen Ring ausbilden,

Aa für Stickstoff oder $C(R^{10})$ steht,
Ab für Stickstoff oder $C(R^{11})$ steht,
Ac für Stickstoff oder $C(R^{12})$ steht und
Ad für Stickstoff oder $C(R^{13})$ steht,

wobei Aa, Ab, Ac und Ad für maximal zwei Stickstoffe stehen,

oder für den Fall, dass 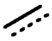 ausschließlich für Einfachbindungen stehen,

Aa für $C(R^{10})(R^{14})$ steht,
Ab für $C(R^{11})(R^{15})$ steht,
Ac für $C(R^{12})(R^{16})$ steht und
Ad für $C(R^{13})(R^{17})$ steht,
$R^1$ für Methyl, Ethyl, n-Propyl, i-Propyl, cyclo-Propyl, n-Butyl, i-Butyl, tert.-Butyl, cyclo-Butyl, Fluormethyl, Difluormethyl, Trifluormethyl, Fluorethyl, Difluorethyl, Trifluorethyl, Tetrafluorethyl oder Pentafluorethyl steht,
$R^{10}$, $R^{11}$, $R^{12}$, $R^{13}$, $R^{14}$, $R^{15}$, $R^{16}$, $R^{17}$ unabhängig voneinander für Wasserstoff, Cyano, Halogen, $(C_1-C_4)$Alkyl, $(C_1-C_4)$Halogenalkyl, $(C_1-C_4)$Alkoxy, $(C_1-C_4)$Halogenalkoxy, $(C_1-C_4)$Alkylthio, $(C_1-C_4)$Alkylsulfinyl, $(C_1-C_4)$Alkylsulfonyl, $(C_1-C_4)$Halogenalkylthio, $(C_1-C_4)$Halogenalkylsulfinyl, $(C_1-C_4)$Halogenalkylsulfonyl, Aminocarbonyl, $(C_1-C_4)$Alkylaminocarbonyl, $(C_3-C_6)$Cycloalkylaminocarbonyl, $(C_1-C_4)$Alkylcarbamoyl (umfasst -NHCOO$(C_1-C_4)$alkyl und -N$(C_1-C_4)$alkylCOO$(C_1-C_4)$alkyl), $(C_1-C_4)$Alkylcarbonylamino oder $(C_1-C_4)$Alkylharnstoff (umfasst -NHCONH$(C_1-C_4)$alkyl und -NHCON$(C_1-C_4)$dialkyl) stehen,
wobei nur einer oder zwei der Reste $R^{10}$, $R^{11}$, $R^{12}$, $R^{13}$, $R^{14}$, $R^{15}$, $R^{16}$, $R^{17}$ für einen Substituenten ungleich Wasserstoff stehen,
und im Falle dass $R^{10}$ und $R^{14}$, $R^{11}$ und $R^{15}$, $R^{12}$ und $R^{16}$ oder $R^{13}$ und $R^{17}$ jeweils beide für ungleich Wasserstoff stehen, $R^{14}$, $R^{15}$, $R^{16}$ und $R^{17}$ jeweils unabhängig voneinander nur für Cyano, Halogen, $(C_1-C_4)$Alkyl oder $(C_1-C_4)$Halogenalkyl stehen,
Q für ein heteroaromatisches 9-gliedriges oder 12-gliedriges annelliertes bicyclisches oder tricyclisches Ringsystem aus der Reihe Q2, Q3, Q5, Q6, Q8, Q9, Q14 oder Q15 steht,

wobei

$R^4$ für $(C_1-C_4)$Alkyl, $(C_1-C_4)$Halogenalkyl, $(C_3-C_6)$Cycloalkyl oder $(C_1-C_4)$Alkoxy-$(C_1-C_4)$alkyl steht,
$R^5$ für Cyano, Halogen, $(C_1-C_4)$Halogenalkyl, Halogen-$(C_3-C_6)$cycloalkyl, $(C_1-C_4)$Halogenalkoxy, $(C_1-C_4)$Halogenalkylthio, $(C_1-C_4)$Halogenalkylsulfinyl, $(C_1-C_4)$Alkylsulfonyl, $(C_1-C_4)$Halogenalkylsulfonyl, $(C_1-C_4)$Halogenalkylcarbonyl oder $(C_1-C_4)$Halogenalkylsulfonyloxy steht,
$R^6$ für Wasserstoff, Cyano, Halogen, $(C_1-C_4)$Alkyl, $(C_1-C_4)$Halogenalkyl oder $(C_3-C_6)$Cycloalkyl steht und
n für 0, 1 oder 2 steht.

**5.** Verbindungen der Formel (Ia) oder (Ib) gemäß Anspruch 2, in welchen

für den Fall, dass 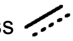 ausschließlich für Doppelbindungen stehen und somit Aa bis Ad mit den jeweils zu Aa und Ad benachbarten C-Atomen einen aromatischen Ring ausbilden,

Aa für $C(R^{10})$ steht,
Ab für $C(R^{11})$ steht,

Ac für $C(R^{12})$ steht und
Ad für $C(R^{13})$ steht,

oder für den Fall, dass 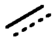 ausschließlich für Einfachbindungen stehen,

Aa für $C(R^{10})(R^{14})$ steht,
Ab für $C(R^{11})(R^{15})$ steht,
Ac für $C(R^{12})(R^{16})$ steht und
Ad für $C(R^{13})(R^{17})$ steht,
$R^1$ für Ethyl steht,
$R^{10}$, $R^{11}$, $R^{12}$, $R^{13}$ unabhängig voneinander für Wasserstoff, Fluor, Chlor, Brom, Iod, Cyano, Methyl, Ethyl, Trifluormethyl, Trifluormethoxy, 2,2,2-Trifluoroethoxy, Acetylamino (Methylcarbonylamino), Cyclopropylamido (Cyclopropylaminocarbonyl), Methylcarbamoyl (-NHCOOMe), Methylharnstoff (-NHCONHMe) oder Cyclopropyl stehen,
wobei nur einer oder zwei der Reste $R^{10}$, $R^{11}$, $R^{12}$, $R^{13}$ für einen Substituenten ungleich Wasserstoff stehen,
$R^{14}$, $R^{15}$, $R^{16}$, $R^{17}$ unabhängig voneinander für Wasserstoff oder $(C_1\text{-}C_4)$Alkyl stehen,
Q für ein heteroaromatisches 9-gliedriges annelliertes bicyclisches Ringsystem aus der Reihe Q2, Q3 oder Q14 steht

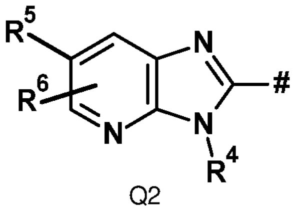
Q2

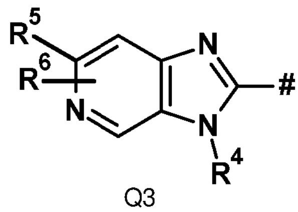
Q3

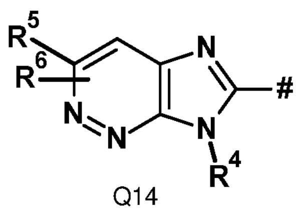
Q14
,

wobei

$R^4$ für Methyl steht,
$R^5$ für Trifluormethyl oder Pentafluorethyl steht,
$R^6$ für Wasserstoff steht und
n für 0, 1 oder 2 steht.

**6.** Verbindungen der Formel (Ia) oder (Ib) gemäß Anspruch 2, in welchen

für den Fall, dass 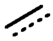 ausschließlich für Doppelbindungen stehen und somit Aa bis Ad mit den jeweils zu Aa und Ad benachbarten C-Atomen einen aromatischen Ring ausbilden,

Aa für $C(R^{10})$ steht,
Ab für Stickstoff oder $C(R^{11})$ steht,
Ac für $C(R^{12})$ steht und
Ad für $C(R^{13})$ steht, wobei
$R^{10}$ für Wasserstoff, Chlor oder Trifluormethyl steht,
$R^{11}$ für Wasserstoff, Chlor, -NHCOMe oder Trifluormethyl steht,
$R^{12}$ für Wasserstoff, Chlor oder Trifluormethyl steht,
$R^{13}$ für Wasserstoff, Chlor oder Trifluormethyl steht,

oder für den Fall, dass 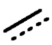 ausschließlich für Einfachbindungen stehen,

Aa für $C(R^{10})(R^{14})$ steht,
Ab für $C(R^{11})(R^{15})$ steht,
Ac für $C(R^{12})(R^{16})$ steht und
Ad für $C(R^{13})(R^{17})$ steht, wobei
$R^{10}$, $R^{12}$, $R^{13}$, $R^{14}$, $R^{16}$, $R^{17}$ für Wasserstoff stehen,
$R^{11}$ für Wasserstoff oder Methyl steht und

$R^{15}$ für Wasserstoff oder Methyl steht,
$R^1$ für Ethyl steht,
Q für ein heteroaromatisches 9-gliedriges annelliertes bicyclisches Ringsystem aus der Reihe Q2 oder Q3 steht

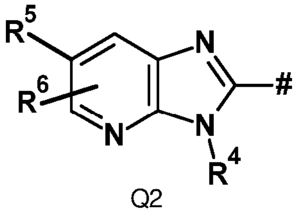
Q2

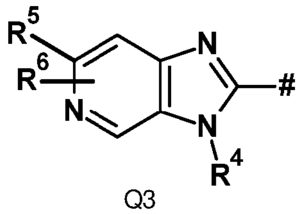
Q3
,

wobei

$R^4$ für Methyl steht,
$R^5$ für Trifluormethyl oder Pentafluorethyl steht,
$R^6$ für Wasserstoff steht und
n für 0, 1 oder 2 steht.

**7.** Verbindungen der Formel (Ia) oder (Ib) gemäß einem der Ansprüche 1 bis 6, in welchen
Q für Q2, Q3, Q5, Q6, Q8, Q9, Q14 oder Q15 steht,
wobei

$R^4$ für $(C_1\text{-}C_4)$Alkyl, $(C_1\text{-}C_4)$Halogenalkyl, $(C_3\text{-}C_6)$Cycloalkyl oder $(C_1\text{-}C_4)$Alkoxy-$(C_1\text{-}C_4)$alkyl steht,
$R^5$ für Cyano, Halogen, $(C_1\text{-}C_4)$Halogenalkyl, Halogen-$(C_3\text{-}C_6)$cycloalkyl, $(C_1\text{-}C_4)$Halogenalkoxy, $(C_1\text{-}C_4)$Halogenalkylthio, $(C_1\text{-}C_4)$Halogenalkylsulfinyl, $(C_1\text{-}C_4)$Alkylsulfonyl, $(C_1\text{-}C_4)$Halogenalkylsulfonyl, $(C_1\text{-}C_4)$Halogenalkylcarbonyl oder $(C_1\text{-}C_4)$Halogenalkylsulfonyloxy steht,
$R^6$ für Wasserstoff, Cyano, Halogen, $(C_1\text{-}C_4)$Alkyl, $(C_1\text{-}C_4)$Halogenalkyl oder $(C_3\text{-}C_6)$Cycloalkyl steht und

und Aa, Ab, Ac, Ad, $R^1$, $R^{10}$ $R^{11}$, $R^{12}$, $R^{13}$, $R^{14}$, $R^{15}$, $R^{16}$, $R^{17}$ und n die Bedeutungen gemäß Anspruch 1 oder Anspruch 2 oder Anspruch 3 oder Anspruch 5 oder Anspruch 6 haben.

**8.** Verbindungen der Formel (Ia) oder (Ib) gemäß einem der Ansprüch 1 bis 6, in welchen
Q für Q2, Q3 oder Q14 steht

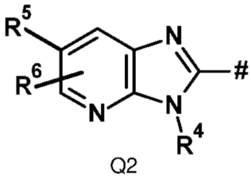
Q2

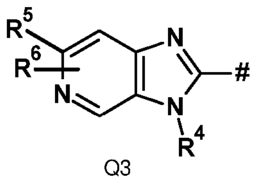
Q3

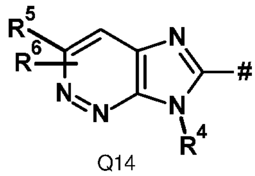
Q14
,

wobei

$R^4$ für Methyl steht,
$R^5$ für Trifluormethyl oder Pentafluorethyl steht,
$R^6$ für Wasserstoff steht

und Aa, Ab, Ac, Ad, $R^1$, $R^{10}$ $R^{11}$, $R^{12}$, $R^{13}$, $R^{14}$, $R^{15}$, $R^{16}$, $R^{17}$ und n die Bedeutungen gemäß Anspruch 1 oder Anspruch 2 oder Anspruch 3 oder Anspruch 4 oder Anspruch 6 haben.

**9.** Verbindungen der Formel (Ia) oder (Ib) gemäß einem der Ansprüche 1 bis 6, in welchen
Q für Q2 oder Q3 steht

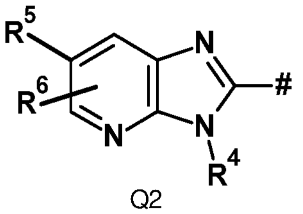

Q2

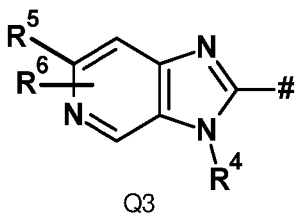

Q3

,

wobei

$R^4$ für Methyl steht,
$R^5$ für Trifluormethyl oder Pentafluorethyl steht,
$R^6$ für Wasserstoff steht

und Aa, Ab, Ac, Ad, $R^1$, $R^{10}$ $R^{11}$, $R^{12}$, $R^{13}$, $R^{14}$, $R^{15}$, $R^{16}$, $R^{17}$ und n die Bedeutungen gemäß Anspruch 1 oder Anspruch 2 oder Anspruch 3 oder Anspruch 4 oder Anspruch 5 haben.

**10.** Verbindungen der Formel (Ia) oder (Ib) gemäß einem der Ansprüche 1 bis 6, in welchen $R^1$ für Ethyl steht und Aa, Ab, Ac, Ad, $R^{10}$ $R^{11}$, $R^{12}$, $R^{13}$, $R^{14}$, $R^{15}$, $R^{16}$, $R^{17}$ Q, $R^4$, $R^5$, $R^6$ und n die Bedeutungen gemäß Anspruch 1 oder Anspruch 2 oder Anspruch 3 oder Anspruch 4 haben.

**11.** Verbindungen der Formel (Ia) oder (Ib) gemäß einem der Ansprüche 1 bis 6, wobei sich für die Formeln (Ia) und (Ib) die folgenden Strukturen (Ia1) bis (Ia4) und (Ib1) bis (Ib4) ergeben,

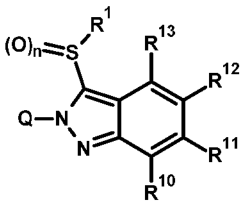

(Ia1)

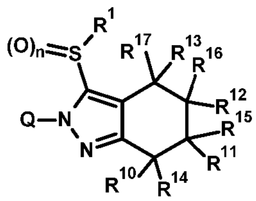

(Ia2)

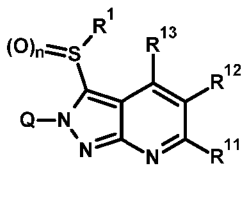

(Ia3)

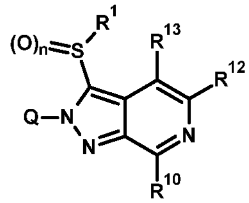

(Ia4)

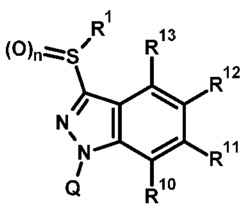

(Ib1)

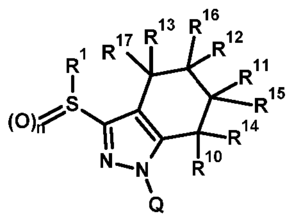

(Ib2)

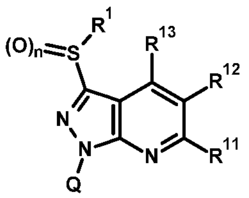

(Ib3)

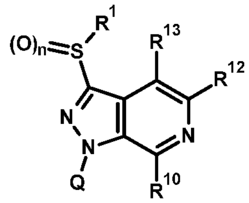

(Ib4)

wobei $R^1$, $R^{10}$, $R^{11}$, $R^{12}$, $R^{13}$, $R^{14}$, $R^{15}$, $R^{16}$, $R^{17}$, Q, $R^4$, $R^5$, $R^6$ und n die in einem der Ansprüche 1 bis 6 beschriebenen Bedeutungen haben.

**12.** Verbindungen der Formel I-1 bis I-5 und I-7 bis I-19.

| | |
|---|---|
| I-1 | 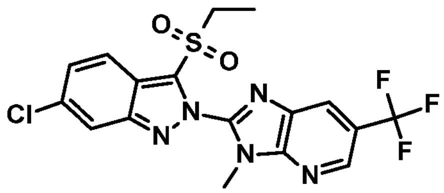 |
| I-2 | 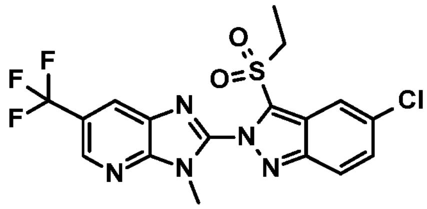 |
| I-3 | 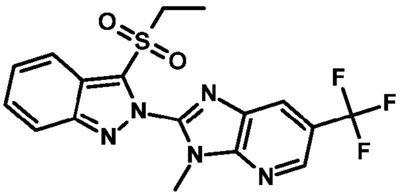 |
| I-4 | 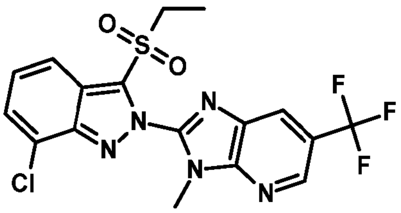 |
| I-5 | 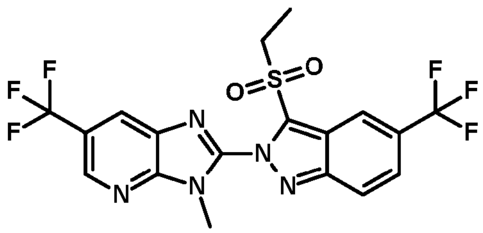 |
| I-7 | 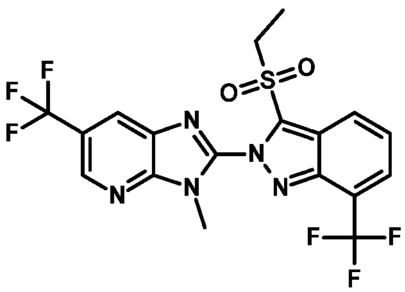 |
| I-8 | 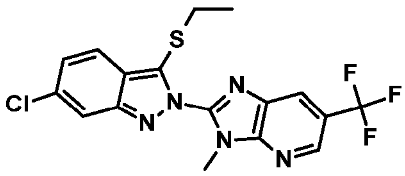 |

(fortgesetzt)

| | |
|---|---|
| I-9 | 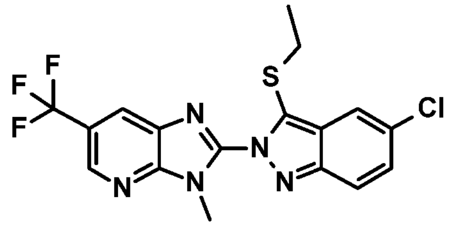 |
| I-10 | 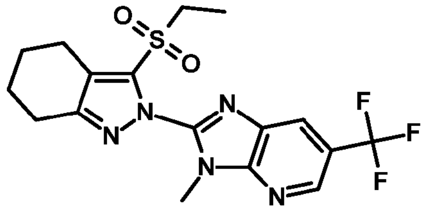 |
| I-11 | 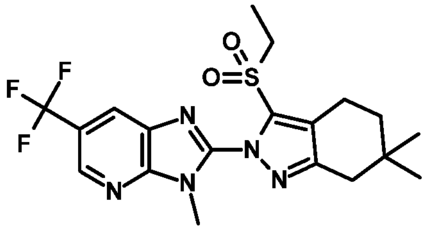 |
| I-12 | 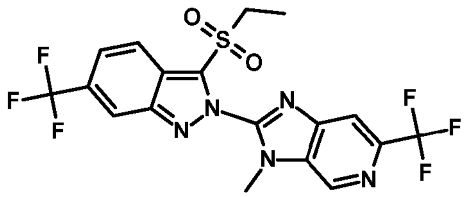 |
| I-13 | 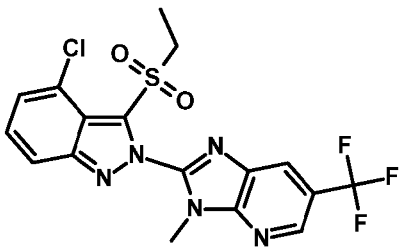 |
| I-14 | 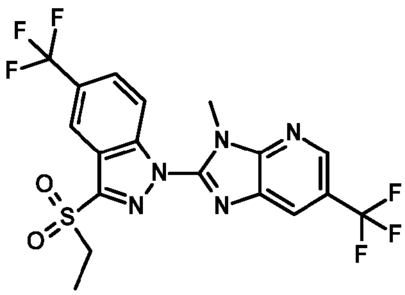 |

(fortgesetzt)

| | |
|---|---|
| I-15 | 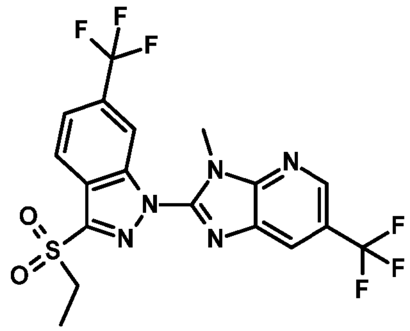 |
| I-16 | 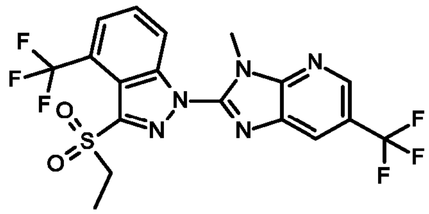 |
| I-17 | 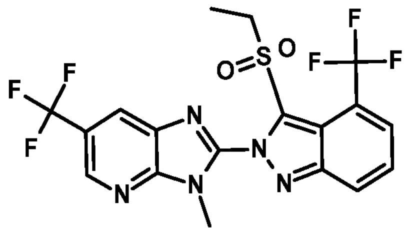 |
| I-18 | 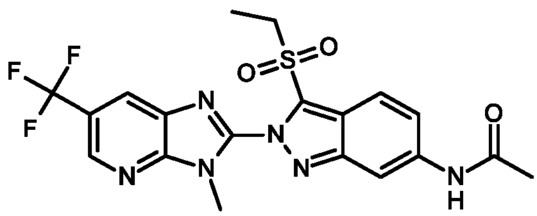 |
| I-19 | 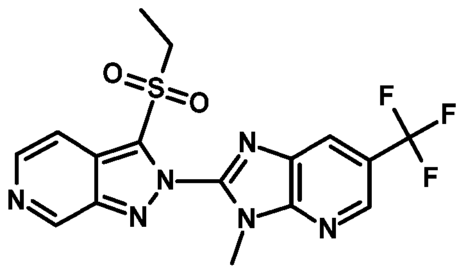 |

**13.** Verbindungen der Formeln (Ia) oder (Ib) gemäß einem der Ansprüche 1 bis 11, **dadurch gekennzeichnet, dass** für den Fall, dass für die Verbindungen der Formel (Ia) 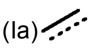 ausschließlich für Doppelbindungen steht,

$R^1$ für $C_1$-$C_6$ Alkyl steht,
Aa und Ad für CH stehen,
Ab für C($R^{11}$) steht,
Ac für C($R^{12}$) steht

und

Q für Q2 steht, wobei
$R^4$ für $C_1$-$C_6$ Alkyl,
$R^6$ für Wasserstoff und
$R^5$ für $(C_1$-$C_6)$Halogenalkyl, $(C_1$-$C_6)$Halogenalkylthio, $(C_1$-$C_6)$Halogenalkylsulfinyl oder $(C_1$-$C_6)$Haolgenalkylsulfonyl steht,
wenigstens einer der Reste $R^{11}$ oder $R^{12}$ nicht für Wasserstoff, Halogen oder $(C_1$-$C_6)$Halogenalkyl steht.

**14.** Verbindungen der Formeln (Ia) oder (Ib) gemäß einem der Ansprüche 1 bis 11, **dadurch gekennzeichnet, dass** für den Fall, dass für die Verbindungen der Formel (Ia)

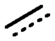 ausschließlich für Doppelbindungen steht,
$R^1$ für $C_1$-$C_6$ Alkyl steht,
Aa und Ad für CH stehen,
Ab für $C(R^{11})$ steht,
Ac für $C(R^{12})$ steht
$R^{11}$ und $R^{12}$ unabhängig voneinander für Wasserstoff, Halogen oder $(C_1$-$C_6)$Halogenalkyl stehen

und

Q für Q2 steht,
$R^4$ für $C_1$-$C_6$ Alkyl steht und
$R^6$ für Wasserstoff steht,
$R^5$ nicht für $(C_1$-$C_6)$Halogenalkyl, $(C_1$-$C_6)$Halogenalkylthio, $(C_1$-$C_6)$Halogenalkylsulfinyl oder $(C_1$-$C_6)$Halogenalkylsulfonyl steht.

**15.** Verbindungen der Formeln (Ia) oder (Ib) gemäß einem der Ansprüche 1 bis 11, **dadurch gekennzeichnet, dass** Verbindungen der Formel (Y), in welchen

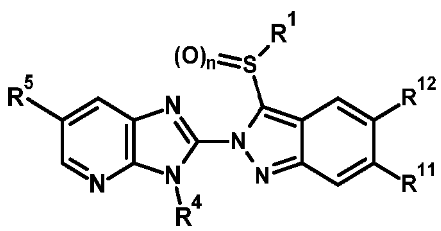

(Y)

$R^1$ für $(C_1$-$C_6)$Alkyl,
$R^4$ für $(C_1$-$C_6)$Alkyl,
$R^5$ für $(C_1$-$C_6)$Halogenalkyl, $(C_1$-$C_6)$Halogenalkylthio, $(C_1$-$C_6)$ Halogenalkylsulfinyl oder $(C_1$-$C_6)$Halogenalkylsulfonyl,
$R^{11}$ und $R^{12}$ unabhängig voneinander jeweils für Wasserstoff, Halogen oder $(C_1$-$C_6)$ Halogenalkyl und
n für 0, 1oder 2
steht, ausgenommen sind.

**16.** Verbindungen der Formeln (Ia) gemäß einem der Ansprüche 1 bis 11, **dadurch gekennzeichnet, dass**  ausschließlich für Einfachbindungen steht.

**17.** Agrochemische Formulierung enthaltend wenigstens eine Verbindung der Formel (Ia) oder(Ib) gemäß einem der Ansprüche 1 bis 16, sowie Streckmittel und/oder oberflächenaktive Substanzen.

**18.** Agrochemische Formulierung gemäß Anspruch 17, zusätzlich enthaltend einen weiteren agrochemischen Wirkstoff.

**19.** Verfahren zur Bekämpfung von tierischen Schädlingen **dadurch gekennzeichnet, dass** man eine Verbindung der Formel (Ia) oder (Ib) gemäß einem der Ansprüche 1 bis 16 oder eine agrochemische Formulierung gemäß einem der Ansprüche 17 oder 18 auf die tierischen Schädlinge und/oder ihren Lebensraum einwirken lässt, wobei Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

**20.** Verwendung von Verbindungen der Formel (Ia) oder (Ib) gemäß einem der Ansprüche 1 bis 16 oder von agrochemischen Formulierungen gemäß einem der Ansprüche 17 oder 18 zur Bekämpfung von tierischen Schädlingen, wobei Verwendungen in Verfahren zur chirurgischen oder therapeutischen Behandlung des menschlichen oder tierischen Körpers und Diagnostizierverfahren, die am menschlichen oder tierischen Körper vorgenommen werden, ausgenommen sind.

## Claims

**1.** Compounds of the formula (Ia) or (Ib)

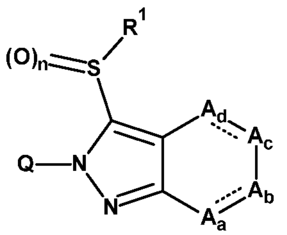

*Ia*

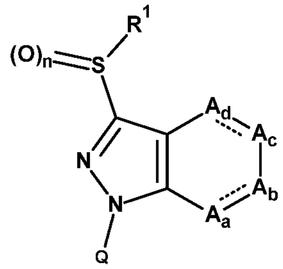

*Ib*

where 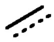 represent single bonds or double bonds, in which,

if 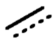 represent exclusively double bonds and hence Aa to Ad together with the carbon atoms adjacent to Aa and Ad in each case form an aromatic ring,

Aa is nitrogen or $C(R^{10})$,
Ab is nitrogen or $C(R^{11})$,
Ac is nitrogen or $C(R^{12})$, and
Ad is nitrogen or $C(R^{13})$,

where not more than two of Aa, Ab, Ac and Ad are nitrogen,

or, if 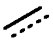 represent exclusively single bonds,

Aa is $C(R^{10})(R^{14})$,
Ab is $C(R^{11})(R^{15})$,
Ac is $C(R^{12})(R^{16})$ and
Ad is $C(R^{13})(R^{17})$,
$R^1$ is $(C_1\text{-}C_6)$alkyl, $(C_1\text{-}C_6)$haloalkyl, $(C_1\text{-}C_6)$cyanoalkyl, $(C_1\text{-}C_6)$hydroxyalkyl, $(C_1\text{-}C_6)$alkoxy-$(C_1\text{-}C_6)$alkyl, $(C_1\text{-}C_6)$haloalkoxy-$(C_1\text{-}C_6)$alkyl, $(C_2\text{-}C_6)$alkenyl, $(C_2\text{-}C_6)$alkenyloxy-$(C_1\text{-}C_6)$alkyl, $(C_2\text{-}C_6)$haloalkenyloxy-$(C_1\text{-}C_6)$alkyl, $(C_2\text{-}C_6)$haloalkenyl, $(C_2\text{-}C_6)$cyanoalkenyl, $(C_2\text{-}C_6)$alkynyl, $(C_2\text{-}C_6)$alkynyloxy-$(C_1\text{-}C_6)$alkyl, $(C_2\text{-}C_6)$haloalkynyloxy-$(C_1\text{-}C_6)$alkyl, $(C_2\text{-}C_6)$haloalkynyl, $(C_2\text{-}C_6)$cyanoalkynyl, $(C_3\text{-}C_8)$cycloalkyl, $(C_3\text{-}C_8)$cycloalkyl-$(C_3\text{-}C_8)$cycloalkyl, $(C_1\text{-}C_6)$alkyl-$(C_3\text{-}C_8)$cycloalkyl, halo$(C_3\text{-}C_8)$cycloalkyl, amino, $(C_1\text{-}C_6)$alkylamino, di$(C_1\text{-}C_6)$alkylamino, $(C_3\text{-}C_8)$cycloalkylamino, $(C_1\text{-}C_6)$alkylcarbonylamino, $(C_1\text{-}C_6)$alkylthio-$(C_1\text{-}C_6)$alkyl, $(C_1\text{-}C_6)$haloalkylthio-$(C_1\text{-}C_6)$alkyl, $(C_1\text{-}C_6)$alkylsulfinyl-$(C_1\text{-}C_6)$alkyl, $(C_1\text{-}C_6)$haloalkylsulfinyl-$(C_1\text{-}C_6)$alkyl, $(C_1\text{-}C_6)$alkylsulfonyl-$(C_1\text{-}C_6)$alkyl, $(C_1\text{-}C_6)$haloalkylsulfonyl-$(C_1\text{-}C_6)$alkyl, $(C_1\text{-}C_6)$alkoxy-$(C_1\text{-}C_6)$alkylthio-$(C_1\text{-}C_6)$alkyl, $(C_1\text{-}C_6)$alkoxy-$(C_1\text{-}C_6)$alkylsulfinyl-$(C_1\text{-}C_6)$alkyl, $(C_1\text{-}C_6)$alkoxy-$(C_1\text{-}C_6)$alkylsulfonyl-$(C_1\text{-}C_6)$alkyl, $(C_1\text{-}C_6)$alkylcarbonyl-$(C_1\text{-}C_6)$alkyl, $(C_1\text{-}C_6)$haloalkylcarbonyl-$(C_1\text{-}C_6)$alkyl, $(C_1\text{-}C_6)$alkoxycarbonyl-$(C_1\text{-}C_6)$alkyl, $(C_1\text{-}C_6)$haloalkoxycarbonyl-$(C_1\text{-}C_6)$alkyl, $(C_1\text{-}C_6)$alkylsulfonylamino, aminosulfonyl-$(C_1\text{-}C_6)$alkyl, $(C_1\text{-}C_6)$alkylaminosulfonyl-$(C_1\text{-}C_6)$alkyl, di$(C_1\text{-}C_6)$alkylaminosulfonyl-$(C_1\text{-}C_6)$alkyl, or is in each case singly or multiply, identically or differently aryl-, hetaryl- or heterocyclyl-substituted $(C_1\text{-}C_6)$alkyl, $(C_1\text{-}C_6)$alkoxy, $(C_2\text{-}C_6)$alkenyl, $(C_2\text{-}C_6)$alkynyl, $(C_3\text{-}C_8)$cycloalkyl, where aryl, hetaryl or heterocyclyl may each optionally be mono- or polysubstituted identically or differently by halogen, cyano, nitro, hydroxyl, amino, carboxyl, carbamoyl, aminosulfonyl, $(C_1\text{-}C_6)$alkyl, $(C_3\text{-}C_6)$cycloalkyl, $(C_1\text{-}C_6)$alkoxy, $(C_1\text{-}C_6)$haloalkyl, $(C_1\text{-}C_6)$haloalkoxy, $(C_1\text{-}C_6)$alkylthio, $(C_1\text{-}C_6)$alkylsulfinyl, $(C_1\text{-}C_6)$alkylsulfonyl, $(C_1\text{-}C_6)$alkylsulfimino, $(C_1\text{-}C_6)$alkylsulfimino-$(C_1\text{-}C_6)$alkyl, $(C_1\text{-}C_6)$alkylsulfimino-$(C_2\text{-}C_6)$alkylcarbonyl, $(C_1\text{-}C_6)$alkylsulfoximino,

$(C_1-C_6)$alkylsulfoximino-$(C_1-C_6)$alkyl, $(C_1-C_6)$alkylsulfoximino-$(C_2-C_6)$alkylcarbonyl, $(C_1-C_6)$alkoxycarbonyl, $(C_1-C_6)$alkylcarbonyl, $(C_3-C_6)$trialkylsilyl or benzyl, or

$R^1$ is aryl, hetaryl or heterocyclyl, each optionally mono- or polysubstituted identically or differently by halogen, cyano, nitro, hydroxyl, amino, carboxyl, carbamoyl, $(C_1-C_6)$alkyl, $(C_3-C_8)$ cycloalkyl, $(C_1-C_6)$-alkoxy, $(C_1-C_6)$haloalkyl, $(C_1-C_6)$haloalkoxy, $(C_1-C_6)$alkylthio, $(C_1-C_6)$alkylsulfinyl, $(C_1-C_6)$alkylsulfonyl, $(C_1-C_6)$alkylsulfimino, $(C_1-C_6)$alkylsulfimino-$(C_1-C_6)$alkyl, $(C_1-C_6)$alkylsulfimino-$(C_2-C_6)$alkylcarbonyl, $(C_1-C_6)$alkylsulfoximino, $(C_1-C_6)$alkylsulfoximino-$(C_1-C_6)$alkyl, $(C_1-C_6)$alkylsulfoximino-$(C_2-C_6)$alkylcarbonyl, $(C_1-C_6)$alkoxycarbonyl, $(C_1-C_6)$alkylcarbonyl, $(C_3-C_6)$trialkylsilyl, (=O) (in the case of heterocyclyl only) or $(=O)_2$ (in the case of heterocyclyl only),

$R^{10}$, $R^{11}$, $R^{12}$, $R^{13}$, $R^{14}$, $R^{15}$, $R^{16}$, $R^{17}$ are independently hydrogen, cyano, halogen, nitro, hydroxyl, amino, tri $(C_1-C_6)$alkylsilyl, $(C_3-C_8)$cycloalkyl, $(C_3-C_8)$cycloalkyl-$(C_3-C_8)$cycloalkyl, $(C_1-C_6)$alkyl-$(C_3-C_8)$cycloalkyl, halo$(C_3-C_8)$cycloalkyl, $(C_1-C_6)$alkyl, $(C_1-C_6)$haloalkyl, $(C_1-C_6)$cyanoalkyl, $(C_1-C_6)$hydroxyalkyl, hydroxycarbonyl-$(C_1-C_6)$-alkoxy, $(C_1-C_6)$alkoxycarbonyl-$(C_1-C_6)$alkyl, $(C_1-C_6)$alkoxy-$(C_1-C_6)$alkyl, $(C_2-C_6)$alkenyl, $(C_2-C_6)$haloalkenyl, $(C_2-C_6)$cyanoalkenyl, $(C_2-C_6)$alkynyl, $(C_2-C_6)$haloalkynyl, $(C_2-C_6)$cyanoalkynyl, $(C_1-C_6)$alkoxy, $(C_1-C_6)$haloalkoxy, $(C_1-C_6)$cyanoalkoxy, $(C_1-C_6)$alkoxycarbonyl-$(C_1-C_6)$alkoxy, $(C_1-C_6)$alkoxy-$(C_1-C_6)$alkoxy, $(C_1-C_6)$alkylhydroxyimino, $(C_1-C_6)$alkoxyimino, $(C_1-C_6)$alkyl-$(C_1-C_6)$alkoxyimino, $(C_1-C_6)$haloalkyl-$(C_1-C_6)$alkoxyimino, $(C_1-C_6)$alkylthio, $(C_1-C_6)$haloalkylthio, $(C_1-C_6)$alkoxy-$(C_1-C_6)$alkylthio, $(C_1-C_6)$alkylthio-$(C_1-C_6)$alkyl, $(C_1-C_6)$alkylsulfinyl, $(C_1-C_6)$haloalkylsulfinyl, $(C_1-C_6)$alkoxy-$(C_1-C_6)$alkylsulfinyl, $(C_1-C_6)$alkylsulfinyl-$(C_1-C_6)$alkyl, $(C_1-C_6)$alkylsulfonyl, $(C_1-C_6)$haloalkylsulfonyl, $(C_1-C_6)$alkoxy-$(C_1-C_6)$alkylsulfonyl, $(C_1-C_6)$alkylsulfonyl-$(C_1-C_6)$alkyl, $(C_1-C_6)$alkylsulfonyloxy, $(C_1-C_6)$alkylcarbonyl, $(C_1-C_6)$alkylthiocarbonyl, $(C_1-C_6)$haloalkylcarbonyl, $(C_1-C_6)$alkylcarbonyloxy, $(C_1-C_6)$alkoxycarbonyl, $(C_1-C_6)$haloalkoxycarbonyl, aminocarbonyl, $(C_1-C_6)$alkylaminocarbonyl, $(C_1-C_6)$alkylaminothiocarbonyl, di$(C_1-C_6)$alkylaminocarbonyl, di$(C_1-C_6)$alkylaminothiocarbonyl, $(C_2-C_6)$alkenylaminocarbonyl, di$(C_2-C_6)$alkenylaminocarbonyl, $(C_3-C_8)$cycloalkylaminocarbonyl, $(C_1-C_6)$alkylsulfonylamino, $(C_1-C_6)$alkylamino, di$(C_1-C_6)$alkylamino, aminosulfonyl, $(C_1-C_6)$alkylaminosulfonyl, di$(C_1-C_6)$alkylaminosulfonyl, $(C_1-C_6)$alkylsulfoximino, aminothiocarbonyl, $(C_1-C_6)$alkylaminothiocarbonyl, di $(C_1-C_6)$alkylaminothiocarbonyl, $(C_3-C_8)$cycloalkylamino, $(C_1-C_6)$alkylcarbamoyl (including -NHCOO$(C_1-C_6)$alkyl,-N$(C_1-C_6)$alkylCOO$(C_1-C_6)$alkyl, -OCONH$(C_1-C_6)$alkyl or -OCON$(C_1-C_6)$dialkyl), $(C_1-C_6)$alkylcarbonylamino (($(C_1-C_6)$alkylCONH), $(C_1-C_6)$alkylurea (including-NH-CONH$(C_1-C_6)$alkyl, and -NHCON$(C_1-C_6)$dialkyl) or

is in each case optionally singly or multiply, identically or differently substituted aryl or hetaryl, where (in the case of hetaryl) at least one carbonyl group may optionally be present and where possible substituents in each case are as follows: cyano, carboxyl, halogen, nitro, hydroxyl, amino, tri-$(C_1-C_6)$alkylsilyl, $(C_3-C_8)$cycloalkyl, $(C_3-C_8)$cycloalkyl-$(C_3-C_8)$cycloalkyl, $(C_1-C_6)$alkyl-$(C_3-C_8)$cycloalkyl, halo$(C_3-C_8)$cycloalkyl, $(C_1-C_6)$alkyl, $(C_1-C_6)$haloalkyl, $(C_1-C_6)$cyanoalkyl, $(C_1-C_6)$hydroxyalkyl, hydroxycarbonyl-$(C_1-C_6)$-alkoxy, $(C_1-C_6)$alkoxycarbonyl-$(C_1-C_6)$alkyl, $(C_1-C_6)$alkoxy-$(C_1-C_6)$alkyl, $(C_2-C_6)$alkenyl, $(C_2-C_6)$haloalkenyl, $(C_2-C_6)$cyanoalkenyl, $(C_2-C_6)$alkynyl, $(C_2-C_6)$haloalkynyl, $(C_2-C_6)$cyanoalkynyl, $(C_1-C_6)$alkoxy, $(C_1-C_6)$haloalkoxy, $(C_1-C_6)$cyanoalkoxy, $(C_1-C_6)$alkoxycarbonyl-$(C_1-C_6)$alkoxy, $(C_1-C_6)$alkoxy-$(C_1-C_6)$alkoxy, $(C_1-C_6)$alkylhydroxyimino, $(C_1-C_6)$alkoxyimino, $(C_1-C_6)$alkyl-$(C_1-C_6)$alkoxyimino, $(C_1-C_6)$haloalkyl-$(C_1-C_6)$alkoxyimino, $(C_1-C_6)$alkylthio, $(C_1-C_6)$haloalkylthio, $(C_1-C_6)$alkoxy-$(C_1-C_6)$alkylthio, $(C_1-C_6)$alkylthio-$(C_1-C_6)$alkyl, $(C_1-C_6)$alkylsulfinyl, $(C_1-C_6)$haloalkylsulfinyl, $(C_1-C_6)$alkoxy-$(C_1-C_6)$alkylsulfinyl, $(C_1-C_6)$alkylsulfinyl-$(C_1-C_6)$alkyl, $(C_1-C_6)$alkylsulfonyl, $(C_1-C_6)$haloalkylsulfonyl, $(C_1-C_6)$alkoxy-$(C_1-C_6)$alkylsulfonyl, $(C_1-C_6)$alkylsulfonyl-$(C_1-C_6)$alkyl, $(C_1-C_6)$alkylsulfonyloxy, $(C_1-C_6)$alkylcarbonyl, $(C_1-C_6)$haloalkylcarbonyl, $(C_1-C_6)$alkylcarbonyloxy, $(C_1-C_6)$alkoxycarbonyl, $(C_1-C_6)$haloalkoxycarbonyl, aminocarbonyl, $(C_1-C_6)$alkylaminocarbonyl, di$(C_1-C_6)$alkylaminocarbonyl, $(C_2-C_6)$alkenylaminocarbonyl, di$(C_2-C_6)$-alkenylaminocarbonyl, $(C_3-C_8)$cycloalkylaminocarbonyl, $(C_1-C_6)$alkylsulfonylamino, $(C_1-C_6)$alkylamino, di$(C_1-C_6)$alkylamino, aminosulfonyl, $(C_1-C_6)$alkylaminosulfonyl, di$(C_1-C_6)$alkylaminosulfonyl, $(C_1-C_6)$alkylsulfoximino, aminothiocarbonyl, $(C_1-C_6)$alkylaminothiocarbonyl, di$(C_1-C_6)$alkylaminothiocarbonyl, $(C_3-C_8)$cycloalkylamino or $(C_1-C_6)$alkylcarbonylamino, where only one or two of the $R^{10}$, $R^{11}$, $R^{12}$, $R^{13}$, $R^{14}$, $R^{15}$, $R^{16}$ and $R^{17}$ radicals are a substituent other than hydrogen, and, if any of $R^{10}$ and $R^{14}$, $R^{11}$ and $R^{15}$, $R^{12}$ and $R^{16}$ or $R^{13}$ and $R^{17}$ are both not hydrogen, $R^{14}$, $R^{15}$, $R^{16}$ and $R^{17}$ are each independently only cyano, halogen, $(C_3-C_8)$cycloalkyl, $(C_3-C_8)$cycloalkyl-$(C_3-C_8)$cycloalkyl, $(C_1-C_6)$alkyl-$(C_3-C_8)$cycloalkyl, halo$(C_3-C_8)$cycloalkyl,$(C_1-C_6)$alkyl, $(C_1-C_6)$haloalkyl or $(C_1-C_6)$cyanoalkyl,

Q is a partly saturated or saturated heterocyclic or heteroaromatic 8-, 9-, 11- or 12-membered fused bicyclic or tricyclic ring system, where at least one carbonyl group may optionally be present and where the ring system may optionally be mono- or polysubstituted identically or differently, and where the substituents may independently be selected from cyano, halogen, nitro, hydroxyl, amino, tri$(C_1-C_6)$alkylsilyl, $(C_3-C_8)$cycloalkyl, $(C_3-C_8)$cycloalkyl-$(C_3-C_8)$cycloalkyl, $(C_1-C_6)$alkyl-$(C_3-C_8)$cycloalkyl, halo$(C_3-C_8)$cycloalkyl, $(C_1-C_6)$alkyl, $(C_1-C_6)$haloalkyl, $(C_1-C_6)$cyanoalkyl, $(C_1-C_6)$hydroxyalkyl, hydroxycarbonyl-$(C_1-C_6)$-alkoxy, $(C_1-C_6)$alkoxycar-

bonyl-(C$_1$-C$_6$)alkyl, (C$_1$-C$_6$)alkoxy-(C$_1$-C$_6$)alkyl, (C$_2$-C$_6$)alkenyl, (C$_2$-C$_6$)haloalkenyl, (C$_2$-C$_6$)cyanoalkenyl, (C$_2$-C$_6$)alkynyl, (C$_2$-C$_6$)alkynyloxy-(C$_1$-C$_4$)alkyl, (C$_2$-C$_6$)haloalkynyl, (C$_2$-C$_6$)cyanoalkynyl, (C$_1$-C$_6$)alkoxy, (C$_1$-C$_6$)haloalkoxy, (C$_1$-C$_6$)haloalkoxy-(C$_1$-C$_6$)alkyl, (C$_2$-C$_6$)alkenyloxy-(C$_1$-C$_6$)alkyl, (C$_2$-C$_6$)haloalkenyloxy-(C$_1$-C$_6$)alkyl, (C$_1$-C$_6$)cyanoalkoxy, (C$_1$-C$_6$)alkoxycarbonyl-(C$_1$-C$_6$)alkoxy, (C$_1$-C$_6$)alkoxy-(C$_1$-C$_6$)alkoxy, (C$_1$-C$_6$)alkylhydroxyimino, (C$_1$-C$_6$)alkoxyimino, (C$_1$-C$_6$)alkyl-(C$_1$-C$_6$)alkoxyimino, (C$_1$-C$_6$)haloalkyl-(C$_1$-C$_6$)alkoxyimino, (C$_1$-C$_6$)alkylthio, (C$_1$-C$_6$)haloalkylthio, (C$_1$-C$_6$)alkoxy-(C$_1$-C$_6$)alkylthio, (C$_1$-C$_6$)alkylthio-(C$_1$-C$_6$)alkyl, (C$_1$-C$_6$)alkylsulfinyl, (C$_1$-C$_6$)haloalkylsulfinyl, (C$_1$-C$_6$)alkoxy-(C$_1$-C$_6$)alkylsulfinyl, (C$_1$-C$_6$)alkylsulfinyl-(C$_1$-C$_6$)alkyl, (C$_1$-C$_6$)alkylsulfonyl, (C$_1$-C$_6$)haloalkylsulfonyl, (C$_1$-C$_6$)alkoxy-(C$_1$-C$_6$)alkylsulfonyl, (C$_1$-C$_6$)alkylsulfonyl-(C$_1$-C$_6$)alkyl, (C$_1$-C$_6$)alkylsulfonyloxy, (C$_1$-C$_6$)alkylcarbonyl, (C$_1$-C$_6$)alkylcarbonyl-(C$_1$-C$_6$)alkyl, (C$_1$-C$_6$)alkylthiocarbonyl, (C$_1$-C$_6$)haloalkylcarbonyl, (C$_1$-C$_6$)alkylcarbonyloxy, (C$_1$-C$_6$)alkoxycarbonyl, (C$_1$-C$_6$)haloalkoxycarbonyl, aminocarbonyl, (C$_1$-C$_6$)alkylaminocarbonyl, (C$_1$-C$_6$)alkylaminothiocarbonyl, di(C$_1$-C$_6$)alkylaminocarbonyl, di(C$_1$-C$_6$)alkylaminothiocarbonyl, (C$_2$-C$_6$)alkenylaminocarbonyl, di(C$_2$-C$_6$)alkenylaminocarbonyl, (C$_3$-C$_8$)cycloalkylaminocarbonyl, (C$_1$-C$_6$)alkylsulfonylamino, (C$_1$-C$_6$)alkylamino, di(C$_1$-C$_6$)alkylamino, aminosulfonyl, (C$_1$-C$_6$)alkylaminosulfonyl, di(C$_1$-C$_6$)alkylaminosulfonyl, (C$_1$-C$_6$)alkylsulfoximino, aminothiocarbonyl, (C$_1$-C$_6$)alkylaminothiocarbonyl, di(C$_1$-C$_6$)alkylaminothiocarbonyl, (C$_3$-C$_8$)cycloalkylamino or (C$_1$-C$_6$)alkylcarbonylamino or where the substituents may independently be selected from phenyl or a 5- or 6-membered heteroaromatic ring, where phenyl or the ring may optionally be mono- or polysubstituted identically or differently by C$_1$-C$_6$-alkyl, C$_2$-C$_6$-alkenyl, C$_2$-C$_6$-alkynyl, C$_3$-C$_6$-cycloalkyl, C$_1$-C$_6$-haloalkyl, C$_2$-C$_6$-haloalkenyl, C$_2$-C$_6$-haloalkynyl, C$_3$-C$_6$-halocycloalkyl, halogen, CN, C$_1$-C$_4$-alkoxy, C$_1$-C$_4$-haloalkoxy,
n is 0, 1 or 2.

**2.** Compounds of the formula (Ia) or (Ib) according to Claim 1, in which,

if 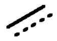 represent exclusively double bonds and hence Aa to Ad together with the carbon atoms adjacent to Aa and Ad in each case form an aromatic ring,

Aa is nitrogen or C(R$^{10}$),
Ab is nitrogen or C(R$^{11}$),
Ac is nitrogen or C(R$^{12}$) and
Ad is nitrogen or C(R$^{13}$),

where not more than two of Aa, Ab, Ac and Ad are nitrogen,

or, if 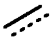 represent exclusively single bonds,

Aa is C(R$^{10}$)(R$^{14}$),
Ab is C(R$^{11}$)(R$^{15}$),
Ac is C(R$^{12}$)(R$^{16}$) and
Ad is C(R$^{13}$)(R$^{17}$),
R$^1$ is (C$_1$-C$_4$)alkyl, (C$_1$-C$_4$)hydroxyalkyl, (C$_1$-C$_4$)haloalkyl, (C$_1$-C$_4$)cyanoalkyl, (C$_1$-C$_4$)alkoxy-(C$_1$-C$_4$)alkyl, (C$_1$-C$_4$)haloalkoxy-(C$_1$-C$_4$)alkyl, (C$_2$-C$_4$)alkenyl, (C$_2$-C$_4$)alkenyloxy-(C$_1$-C$_4$)alkyl, (C$_2$-C$_4$)haloalkenyloxy-(C$_1$-C$_4$)alkyl, (C$_2$-C$_4$)haloalkenyl, (C$_2$-C$_4$)cyanoalkenyl, (C$_2$-C$_4$)alkynyl, (C$_2$-C$_4$)alkynyloxy-(C$_1$-C$_4$)alkyl, (C$_2$-C$_4$)haloalkynyloxy-(C$_1$-C$_4$)alkyl, (C$_2$-C$_4$)haloalkynyl, (C$_2$-C$_4$)cyanoalkynyl, (C$_3$-C$_6$)cycloalkyl, (C$_3$-C$_6$)cycloalkyl(C$_3$-C$_6$)cycloalkyl, (C$_1$-C$_4$)alkyl-(C$_3$-C$_6$)cycloalkyl, halo(C$_3$-C$_6$)cycloalkyl, (C$_1$-C$_4$)alkylamino, di(C$_1$-C$_4$)alkylamino, (C$_3$-C$_6$)cycloalkylamino, (C$_1$-C$_4$)alkylcarbonylamino, (C$_1$-C$_4$)alkylthio-(C$_1$-C$_4$)alkyl, (C$_1$-C$_4$)haloalkylthio-(C$_1$-C$_4$)alkyl, (C$_1$-C$_4$)alkylsulfinyl-(C$_1$-C$_4$)alkyl, (C$_1$-C$_4$)haloalkylsulfinyl-(C$_1$-C$_4$)alkyl, (C$_1$-C$_4$)alkylsulfonyl-(C$_1$-C$_4$)alkyl, (C$_1$-C$_4$)alkylcarbonyl-(C$_1$-C$_4$)alkyl, (C$_1$-C$_4$)haloalkylcarbonyl-(C$_1$-C$_4$)alkyl or (C$_1$-C$_4$)alkylsulfonylamino,
R$^{10}$, R$^{11}$, R$^{12}$, R$^{13}$, R$^{14}$, R$^{15}$, R$^{16}$, R$^{17}$ are independently hydrogen, cyano, halogen, nitro, hydroxyl, amino, tri(C$_1$-C$_4$)alkylsilyl, (C$_3$-C$_6$)cycloalkyl, (C$_3$-C$_6$)cycloalkyl-(C$_3$-C$_6$)cycloalkyl, (C$_1$-C$_4$)alkyl-(C$_3$-C$_6$)cycloalkyl, halo(C$_3$-C$_6$)cycloalkyl, (C$_1$-C$_4$)alkyl, (C$_1$-C$_4$)haloalkyl, (C$_1$-C$_4$)cyanoalkyl, (C$_1$-C$_4$)hydroxyalkyl, (C$_1$-C$_4$)alkoxy-(C$_1$-C$_4$)alkyl, (C$_2$-C$_4$)alkenyl, (C$_2$-C$_4$)haloalkenyl, (C$_2$-C$_4$)cyanoalkenyl, (C$_2$-C$_4$)alkynyl, (C$_2$-C$_4$)haloalkynyl, (C$_2$-C$_4$)cyanoalkynyl, (C$_1$-C$_4$)alkoxy, (C$_1$-C$_4$)haloalkoxy, (C$_1$-C$_4$)cyanoalkoxy, (C$_1$-C$_4$)alkoxy-(C$_1$-C$_4$)alkoxy, (C$_1$-C$_4$)alkylhydroxyimino, (C$_1$-C$_4$)alkoxyimino (C$_1$-C$_4$)alkyl-(C$_1$-C$_4$)alkoxyimino, (C$_1$-C$_4$)haloalkyl-(C$_1$-C$_4$)alkoxyimino, (C$_1$-C$_4$)alkylthio, (C$_1$-C$_4$)haloalkylthio, (C$_1$-C$_4$)alkylthio-(C$_1$-C$_4$)alkyl, (C$_1$-C$_4$)alkylsulfinyl, (C$_1$-C$_4$)haloalkylsulfinyl, (C$_1$-C$_4$)alkylsulfinyl-(C$_1$-C$_4$)alkyl, (C$_1$-C$_4$)alkylsulfonyl, (C$_1$-C$_4$)haloalkylsulfonyl, (C$_1$-C$_4$)alkylsulfonyl-(C$_1$-C$_4$)alkyl, (C$_1$-C$_4$)alkylsulfonyloxy, (C$_1$-C$_4$)alkylcarbonyl, (C$_1$-C$_4$)haloalkylcarbonyl,aminocarbonyl, aminothiocarbonyl, (C$_1$-C$_4$)alkylaminocarbonyl, di(C$_1$-C$_4$)alkylamino-

carbonyl, ($C_3$-$C_6$)cycloalkylaminocarbonyl, ($C_1$-$C_4$)alkylsulfonylamino, ($C_1$-$C_4$)alkylamino, di($C_1$-$C_4$)alkylamino, aminosulfonyl, $C_1$-$C_4$)alkylaminosulfonyl, di($C_1$-$C_4$)alkylaminosulfonyl, aminothiocarbonyl, ($C_1$-$C_4$)alkylcarbamoyl (including-NHCOO($C_1$-$C_4$)alkyl, N($C_1$-$C_4$)alkylCOO($C_1$-$C_4$)alkyl,-OCONH($C_1$-$C_4$)alkyl or -OCON($C_1$-$C_4$)dialkyl), ($C_1$-$C_4$)alkylcarbonylamino, ($C_1$-$C_4$)alkylurea (including-NHCONH($C_1$-$C_4$)alkyl, and-NHCON($C_1$-$C_4$)dialkyl) or are phenyl or hetaryl, each of which is optionally mono- or disubstituted identically or differently, where (in the case of hetaryl) at least one carbonyl group may optionally be present and where possible substituents are in each case as follows: cyano, halogen, nitro, acetyl, amino, ($C_3$-$C_6$)cycloalkyl, ($C_3$-$C_6$)cycloalkyl-($C_3$-$C_6$)cycloalkyl, ($C_1$-$C_4$)alkyl-($C_3$-$C_6$)cycloalkyl, halo($C_3$-$C_6$)cycloalkyl, ($C_1$-$C_4$)alkyl, ($C_1$-$C_4$)haloalkyl, ($C_1$-$C_4$)cyanoalkyl, ($C_1$-$C_4$) hydroxyalkyl, ($C_1$-$C_4$)alkoxy-($C_1$-$C_4$)alkyl, ($C_2$-$C_4$)alkenyl, ($C_2$-$C_4$)haloalkenyl, ($C_2$-$C_4$)cyanoalkenyl, ($C_2$-$C_4$)alkynyl, ($C_2$-$C_4$)haloalkynyl, ($C_2$-$C_4$)cyanoalkynyl, ($C_1$-$C_4$)alkoxy, ($C_1$-$C_4$)haloalkoxy, ($C_1$-$C_4$)cyanoalkoxy, ($C_1$-$C_4$)alkoxy-($C_1$-$C_4$)alkoxy, ($C_1$-$C_4$)alkylhydroxyimino, ($C_1$-$C_4$)alkoxyimino, ($C_1$-$C_4$)alkyl-($C_1$-$C_4$)alkoxyimino, ($C_1$-$C_4$)haloalkyl-($C_1$-$C_4$)alkoxyimino, ($C_1$-$C_4$)alkylthio, ($C_1$-$C_4$)haloalkylthio, ($C_1$-$C_4$)alkylthio-($C_1$-$C_4$)alkyl, ($C_1$-$C_4$)alkylsulfinyl, ($C_1$-$C_4$)haloalkylsulfinyl, ($C_1$-$C_4$)alkylsulfinyl-($C_1$-$C_4$)alkyl, ($C_1$-$C_4$)alkylsulfonyl, ($C_1$-$C_4$)haloalkylsulfonyl, ($C_1$-$C_4$)alkylsulfonyl-($C_1$-$C_4$)alkyl, ($C_1$-$C_4$)alkylsulfonyloxy, ($C_1$-$C_4$)alkylcarbonyl, ($C_1$-$C_4$)haloalkylcarbonyl, aminocarbonyl, ($C_1$-$C_4$)alkylaminocarbonyl, di($C_1$-$C_4$)alkylaminocarbonyl, ($C_1$-$C_4$)alkylsulfonylamino, ($C_1$-$C_4$)alkylamino, di($C_1$-$C_4$)alkylamino, aminosulfonyl, ($C_1$-$C_4$)alkylaminosulfonyl, di($C_1$-$C_4$)alkylaminosulfonyl or ($C_1$-$C_4$)alkylcarbonylamino, where only one or two of the $R^{10}$, $R^{11}$, $R^{12}$, $R^{13}$, $R^{14}$, $R^{15}$, $R^{16}$, $R^{17}$ radicals are a substituent other than hydrogen, and, if any of $R^{10}$ and $R^{14}$, $R^{11}$ and $R^{15}$, $R^{12}$ and $R^{16}$ or $R^{13}$ and $R^{17}$ are both not hydrogen, $R^{14}$, $R^{15}$, $R^{16}$ and $R^{17}$ are each independently only cyano, halogen, ($C_3$-$C_6$)cycloalkyl, ($C_3$-$C_6$)cycloalkyl-($C_3$-$C_6$)cycloalkyl, ($C_1$-$C_4$)alkyl-($C_3$-$C_6$)cycloalkyl, halo($C_3$-$C_6$)cycloalkyl, ($C_1$-$C_4$)alkyl, ($C_1$-$C_4$)haloalkyl or ($C_1$-$C_4$)cyanoalkyl,

Q is a heteroaromatic 9-membered or 12-membered fused bicyclic or tricyclic ring system from the group of Q1 to Q15:

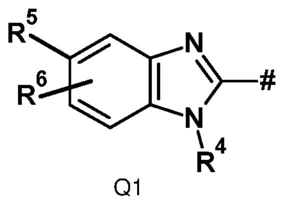
Q1

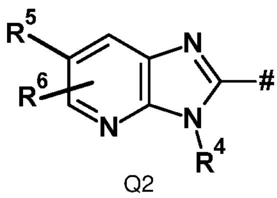
Q2

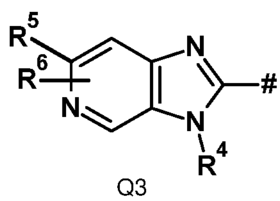
Q3

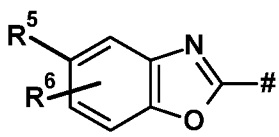
Q4

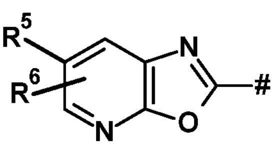
Q5

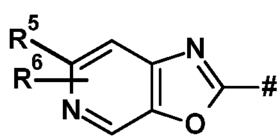
Q6

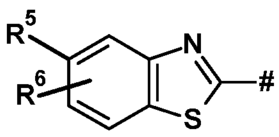
Q7

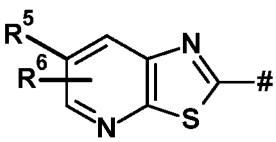
Q8

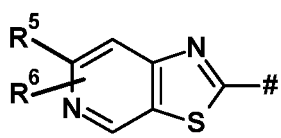
Q9

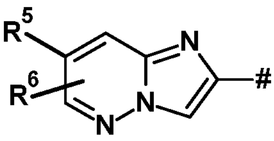
Q10

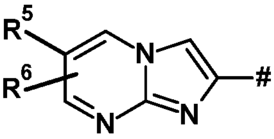
Q11

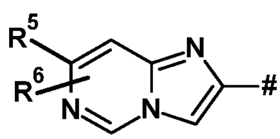
Q12

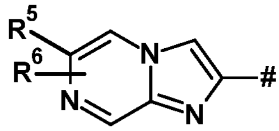

Q13

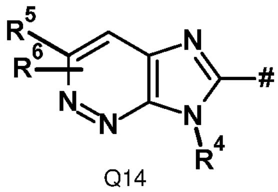

Q14

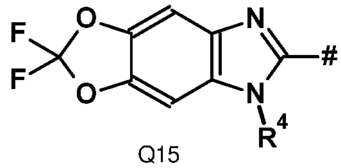

Q15

where

$R^4$ is hydrogen, $(C_1\text{-}C_4)$alkyl, $(C_1\text{-}C_4)$haloalkyl, $(C_1\text{-}C_4)$cyanoalkyl, $(C_1\text{-}C_4)$hydroxyalkyl, $(C_1\text{-}C_4)$alkoxy-$(C_1\text{-}C_4)$alkyl, $(C_1\text{-}C_4)$haloalkoxy-$(C_1\text{-}C_4)$alkyl, $(C_2\text{-}C_4)$alkenyl, $(C_2\text{-}C_4)$alkenyloxy-$(C_1\text{-}C_4)$alkyl, $(C_2\text{-}C_4)$haloalkenyloxy-$(C_1\text{-}C_4)$alkyl, $(C_2\text{-}C_4)$haloalkenyl, $(C_2\text{-}C_4)$cyanoalkenyl, $(C_2\text{-}C_4)$alkynyl, $(C_2\text{-}C_4)$haloalkynyl or $(C_3\text{-}C_6)$cycloalkyl and

$R^5$, $R^6$ are independently hydrogen, cyano, halogen, $(C_1\text{-}C_4)$alkyl, $(C_1\text{-}C_4)$haloalkyl, $(C_2\text{-}C_4)$alkenyl, $(C_2\text{-}C_4)$haloalkenyl, $(C_2\text{-}C_4)$alkynyl, $(C_2\text{-}C_4)$haloalkynyl, $(C_3\text{-}C_6)$cycloalkyl, $(C_3\text{-}C_6)$cycloalkyl-$(C_3\text{-}C_6)$cycloalkyl, $(C_1\text{-}C_4)$alkyl-$(C_3\text{-}C_6)$cycloalkyl, $(C_1\text{-}C_4)$haloalkyl-$(C_3\text{-}C_6)$cycloalkyl, cyano-$(C_3\text{-}C_6)$cycloalkyl, halo-$(C_3\text{-}C_6)$cycloalkyl, $(C_1\text{-}C_4)$alkoxy, $(C_1\text{-}C_4)$haloalkoxy, $(C_1\text{-}C_4)$alkoxyimino, $(C_1\text{-}C_4)$haloalkoxyimino $(C_1\text{-}C_4)$alkylthio, $(C_1\text{-}C_4)$haloalkylthio, $(C_1\text{-}C_4)$alkylsulfinyl, $(C_1\text{-}C_4)$haloalkylsulfinyl, $(C_1\text{-}C_4)$alkylsulfonyl, $(C_1\text{-}C_4)$haloalkylsulfonyl, $(C_1\text{-}C_4)$alkylsulfonyloxy, $(C_1\text{-}C_4)$haloalkylsulfonyloxy, $(C_1\text{-}C_4)$alkylcarbonyl, $(C_1\text{-}C_4)$haloalkylcarbonyl, aminocarbonyl, $(C_1\text{-}C_4)$alkylaminocarbonyl, di$(C_1\text{-}C_4)$alkylaminocarbonyl, $(C_1\text{-}C_4)$alkylsulfonylamino, $(C_1\text{-}C_4)$alkylamino, di$(C_1\text{-}C_4)$alkylamino, aminosulfonyl, $(C_1\text{-}C_4)$alkylaminosulfonyl or di$(C_1\text{-}C_4)$alkylaminosulfonyl

and

n is 0, 1 or 2.

**3.** Compounds of the formula (Ia) or (Ib) according to Claim 2, in which,

if 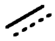 represent exclusively double bonds and hence Aa to Ad together with the carbon atoms adjacent to Aa and Ad in each case form an aromatic ring,

Aa is nitrogen or $C(R^{10})$,
Ab is nitrogen or $C(R^{11})$,
Ac is nitrogen or $C(R^{12})$, and
Ad is nitrogen or $C(R^{13})$,

where not more than two of Aa, Ab, Ac and Ad are nitrogen,

or, if 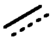 represent exclusively single bonds,

Aa is $C(R^{10})(R^{14})$,
Ab is $C(R^{11})(R^{15})$,
Ac is $C(R^{12})(R^{16})$ and
Ad is $C(R^{13})(R^{17})$,

$R^1$ is $(C_1\text{-}C_4)$alkyl, $(C_1\text{-}C_4)$hydroxyalkyl, $(C_1\text{-}C_4)$haloalkyl, $(C_2\text{-}C_4)$alkenyl, $(C_2\text{-}C_4)$haloalkenyl, $(C_2\text{-}C_4)$alkynyl, $(C_2\text{-}C_4)$haloalkynyl, $(C_3\text{-}C_6)$cycloalkyl, $(C_1\text{-}C_4)$alkylthio-$(C_1\text{-}C_4)$alkyl, $(C_1\text{-}C_4)$alkylsulfinyl-$(C_1\text{-}C_4)$alkyl or $(C_1\text{-}C_4)$alkylsulfonyl-$(C_1\text{-}C_4)$alkyl,

$R^{10}$, $R^{11}$, $R^{12}$, $R^{13}$, $R^{14}$, $R^{15}$, $R^{16}$, $R^{17}$ are independently hydrogen, cyano, halogen, nitro, hydroxyl, amino, tri $(C_1\text{-}C_4)$alkylsilyl, $(C_3\text{-}C_6)$cycloalkyl, $(C_3\text{-}C_6)$cycloalkyl-$(C_3\text{-}C_6)$cycloalkyl, $(C_1\text{-}C_4)$alkyl-$(C_3\text{-}C_6)$cycloalkyl, halo$(C_3\text{-}C_6)$cycloalkyl, $(C_1\text{-}C_4)$alkyl, $(C_1\text{-}C_4)$haloalkyl, $(C_1\text{-}C_4)$cyanoalkyl, $(C_1\text{-}C_4)$alkoxy-$(C_1\text{-}C_4)$alkyl, $(C_2\text{-}C_4)$alkenyl, $(C_2\text{-}C_4)$haloalkenyl, $(C_2\text{-}C_4)$cyanoalkenyl, $(C_2\text{-}C_4)$alkynyl, $(C_2\text{-}C_4)$haloalkynyl, $(C_2\text{-}C_4)$cyanoalkynyl, $(C_1\text{-}C_4)$alkoxy, $(C_1\text{-}C_4)$haloalkoxy, $(C_1\text{-}C_4)$cyanoalkoxy, $(C_1\text{-}C_4)$alkylthio, $(C_1\text{-}C_4)$haloalkylthio, $(C_1\text{-}C_4)$alkylsulfinyl, $(C_1\text{-}C_4)$haloalkylsulfinyl, $(C_1\text{-}C_4)$alkylsulfonyl, $(C_1\text{-}C_4)$haloalkylsulfonyl, $(C_1\text{-}C_4)$alkylsulfonyloxy, $(C_1\text{-}C_4)$alkylcarbonyl, $(C_1\text{-}C_4)$haloalkylcarbonyl, aminocarbonyl, $(C_1\text{-}C_4)$alkylaminocarbonyl, di $(C_1\text{-}C_4)$alkylaminocarbonyl, $(C_3\text{-}C_6)$cycloalkylaminocarbonyl, $(C_1\text{-}C_4)$alkylsulfonylamino, $(C_1\text{-}C_4)$alkylamino, di$(C_1\text{-}C_4)$alkylamino, aminosulfonyl, $(C_1\text{-}C_4)$alkylaminosulfonyl, di $(C_1\text{-}C_4)$alkylaminosulfonyl, $(C_1\text{-}C_4)$alkylcarbamoyl (including-NHCOO$(C_1\text{-}C_4)$alkyl and-N$(C_1\text{-}C_4)$alkylCOO$(C_1\text{-}C_4)$alkyl), $(C_1\text{-}C_4)$alkylcarbonylamino, $(C_1\text{-}C_4)$alkylurea (including -NHCONH$(C_1\text{-}C_4)$alkyl and-NHCON$(C_1\text{-}C_4)$dialkyl)

or are phenyl or hetaryl, each of which is optionally mono- or disubstituted identically or differently, where (in the case of hetaryl) at least one carbonyl group may optionally be present and where possible substituents are in each case as follows: cyano, halogen, $(C_3-C_6)$cycloalkyl, $(C_3-C_6)$cycloalkyl-$(C_3-C_6)$cycloalkyl, $(C_1-C_4)$alkyl-$(C_3-C_6)$cycloalkyl, halo$(C_3-C_6)$cycloalkyl, $(C_1-C_4)$alkyl, $(C_1-C_4)$haloalkyl, $(C_1-C_4)$cyanoalkyl, $(C_2-C_4)$alkenyl, $(C_2-C_4)$haloalkenyl, $(C_2-C_4)$cyanoalkenyl, $(C_2-C_4)$alkynyl, $(C_2-C_4)$haloalkynyl, $(C_2-C_4)$cyanoalkynyl, $(C_1-C_4)$alkoxy, $(C_1-C_4)$haloalkoxy, $(C_1-C_4)$alkylthio, $(C_1-C_4)$haloalkylthio, $(C_1-C_4)$alkylsulfinyl, $(C_1-C_4)$haloalkylsulfinyl, $(C_1-C_4)$alkylsulfonyl, $(C_1-C_4)$haloalkylsulfonyl, $(C_1-C_4)$alkylsulfonyloxy, $(C_1-C_4)$alkylcarbonyl, $(C_1-C_4)$haloalkylcarbonyl, aminocarbonyl, $(C_1-C_4)$alkylaminocarbonyl, di$(C_1-C_4)$alkylaminocarbonyl, $(C_1-C_4)$alkylsulfonylamino, $(C_1-C_4)$alkylamino, di$(C_1-C_4)$alkylamino, aminosulfonyl, $(C_1-C_4)$alkylaminosulfonyl, di$(C_1-C_4)$alkylaminosulfonyl, $(C_1-C_4)$alkylcarbonylamino,
where only one or two of the $R^{10}$, $R^{11}$, $R^{12}$, $R^{13}$, $R^{14}$, $R^{15}$, $R^{16}$, $R^{17}$ radicals are a substituent other than hydrogen, and, if any of $R^{10}$ and $R^{14}$, $R^{11}$ and $R^{15}$, $R^{12}$ and $R^{16}$ or $R^{13}$ and $R^{17}$ are both not hydrogen, $R^{14}$, $R^{15}$, $R^{16}$ and $R^{17}$ are each independently only cyano, halogen, $(C_3-C_6)$cycloalkyl, $(C_3-C_6)$cycloalkyl-$(C_3-C_6)$cycloalkyl, $(C_1-C_4)$alkyl-$(C_3-C_6)$cycloalkyl, halo$(C_3-C_6)$cycloalkyl, $(C_1-C_4)$alkyl, $(C_1-C_4)$haloalkyl or $(C_1-C_4)$cyanoalkyl,
Q is a heteroaromatic 9-membered or 12-membered fused bicyclic or tricyclic ring system from the group of Q1 to Q15, where
$R^4$ is hydrogen, $(C_1-C_4)$alkyl, $(C_3-C_6)$cycloalkyl, $(C_1-C_4)$haloalkyl, $(C_1-C_4)$cyanoalkyl, $(C_1-C_4)$alkoxy-$(C_1-C_4)$alkyl, $(C_1-C_4)$haloalkoxy-$(C_1-C_4)$alkyl, $(C_2-C_4)$alkenyl, $(C_2-C_4)$haloalkenyl, $(C_2-C_4)$alkynyl or $(C_2-C_4)$haloalkynyl and
$R^5$, $R^6$ are independently hydrogen, cyano, halogen, $(C_1-C_4)$alkyl, $(C_1-C_4)$haloalkyl, $(C_2-C_4)$alkenyl, $(C_2-C_4)$haloalkenyl, $(C_2-C_4)$alkynyl, $(C_2-C_4)$haloalkynyl, $(C_3-C_6)$cycloalkyl, $(C_3-C_6)$cycloalkyl-$(C_3-C_6)$cycloalkyl, $(C_1-C_4)$alkyl-$(C_3-C_6)$cycloalkyl, $(C_1-C_4)$haloalkyl-$(C_3-C_6)$cycloalkyl, cyano-$(C_3-C_6)$cycloalkyl, halo$(C_3-C_6)$cycloalkyl, $(C_1-C_4)$alkoxy, $(C_1-C_4)$haloalkoxy, $(C_1-C_4)$alkoxyimino, $(C_1-C_4)$haloalkoxyimino $(C_1-C_4)$alkylthio, $(C_1-C_4)$haloalkylthio, $(C_1-C_4)$alkylsulfinyl, $(C_1-C_4)$haloalkylsulfinyl, $(C_1-C_4)$alkylsulfonyl, $(C_1-C_4)$haloalkylsulfonyl, $(C_1-C_4)$alkylsulfonyloxy, $(C_1-C_4)$haloalkylsulfonyloxy, $(C_1-C_4)$alkylcarbonyl, $(C_1-C_4)$haloalkylcarbonyl, aminocarbonyl, $(C_1-C_4)$alkylaminocarbonyl, di$(C_1-C_4)$alkylaminocarbonyl, $(C_1-C_4)$alkylsulfonylamino, $(C_1-C_4)$alkylamino, di$(C_1-C_4)$alkylamino, aminosulfonyl, $(C_1-C_4)$ alkylaminosulfonyl or di$(C_1-C_4)$alkylaminosulfonyl
and
n is 0, 1 or 2.

**4.** Compounds of the formula (Ia) or (Ib) according to Claim 2, in which,

if 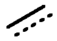 represent exclusively double bonds and hence Aa to Ad together with the carbon atoms adjacent to Aa and Ad in each case form an aromatic ring,

Aa is nitrogen or C ($R^{10}$),
Ab is nitrogen or C($R^{11}$),
Ac is nitrogen or C($R^{12}$), and
Ad is nitrogen or C($R^{13}$),

where not more than two of Aa, Ab, Ac and Ad are nitrogen,

or, if 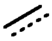 represent exclusively single bonds,

Aa is C($R^{10}$)($R^{14}$),
Ab is C($R^{11}$) ($R^{15}$),
Ac is C($R^{12}$) ($R^{16}$) and
Ad is C($R^{13}$) ($R^{17}$),
$R^1$ is methyl, ethyl, n-propyl, isopropyl, cyclopropyl, n-butyl, isobutyl, tert-butyl, cyclobutyl, fluoromethyl, difluoromethyl, trifluoromethyl, fluoroethyl, difluoroethyl, trifluoroethyl, tetrafluoroethyl or pentafluoroethyl,
$R^{10}$, $R^{11}$, $R^{12}$, $R^{13}$, $R^{14}$, $R^{15}$, $R^{16}$, $R^{17}$ are independently hydrogen, cyano, halogen, $(C_1-C_4)$alkyl, $(C_1-C_4)$ haloalkyl, $(C_1-C_4)$ alkoxy, $(C_1-C_4)$ haloalkoxy, $(C_1-C_4)$ alkylthio, $(C_1-C_4)$ alkylsulfinyl, $(C_1-C_4)$ alkylsulfonyl, $(C_1-C_4)$ haloalkylthio, $(C_1-C_4)$ haloalkylsulfinyl, $(C_1-C_4)$ haloalkylsulfonyl, aminocarbonyl, $(C_1-C_4)$ alkylaminocarbonyl, $(C_3-C_6)$ cycloalkylaminocarbonyl, $(C_1-C_4)$ alkylcarbamoyl (including-NHCOO $(C_1-C_4)$ alkyl and-N$(C_1-C_4)$ alkylCOO $(C_1-C_4)$ alkyl), $(C_1-C_4)$ alkylcarbonylamino or $(C_1-C_4)$ alkylurea (including -NHCONH $(C_1-C_4)$ alkyl and-NHCON $(C_1-C_4)$ dialkyl), where only one or two of the $R^{10}$, $R^{11}$, $R^{12}$, $R^{13}$, $R^{14}$, $R^{15}$, $R^{16}$, $R^{17}$ radicals are a substituent other than hydrogen, and, if any of $R^{10}$ and $R^{14}$, $R^{11}$ and $R^{15}$, $R^{12}$ and $R^{16}$ or $R^{13}$ and $R^{17}$ are

both not hydrogen, $R^{14}$, $R^{15}$, $R^{16}$ and $R^{17}$ are each independently only cyano, halogen, $(C_1-C_4)$ alkyl or $(C_1-C_4)$ haloalkyl,
Q is a heteroaromatic 9-membered or 12-membered fused bicyclic or tricyclic ring system from the group of Q1, Q2, Q3, Q5, Q6, Q8, Q9, Q14 or Q15,

where

$R^4$ is $(C_1-C_4)$ alkyl, $(C_1-C_4)$ haloalkyl, $(C_3-C_6)$ cycloalkyl or $(C_1-C_4)$alkoxy- $(C_1-C_4)$ alkyl,
$R^5$ is cyano, halogen, $(C_1-C_4)$haloalkyl, halo-$(C_3-C_6)$ cycloalkyl, $(C_1-C_4)$haloalkoxy, $(C_1-C_4)$ haloalkylthio, $(C_1-C_4)$ haloalkylsulfinyl, $(C_1-C_4)$ alkylsulfonyl, $(C_1-C_4)$haloalkylsulfonyl, $(C_1-C_4)$ haloalkylcarbonyl or $(C_1-C_4)$ haloalkylsulfonyloxy,
$R^6$ is hydrogen, cyano, halogen, $(C_1-C_4)$alkyl, $(C_1-C_4)$ haloalkyl or $(C_3-C_6)$ cycloalkyl and
n is 0, 1 or 2.

**5.** Compounds of the formula (Ia) or (Ib) according to Claim 2, in which,

if 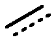 represent exclusively double bonds and hence Aa to Ad together with the carbon atoms adjacent to Aa and Ad in each case form an aromatic ring,

Aa is $C(R^{10})$,
Ab is $C(R^{11})$,
Ac is $C(R^{12})$ and
Ad is $C(R^{13})$,

or, if 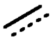 represent exclusively single bonds,

Aa is $C(R^{10})(R^{14})$,
Ab is $C(R^{11})$ $(R^{15})$,
Ac is $C(R^{12})$ $(R^{16})$ and
Ad is $C(R^{13})$ $(R^{17})$,
$R^1$ is ethyl,
$R^{10}$, $R^{11}$, $R^{12}$, $R^{13}$ are independently hydrogen, fluorine, chlorine, bromine, iodine, cyano, methyl, ethyl, trifluoromethyl, trifluoromethoxy, 2,2,2-trifluoroethoxy, acetylamino (methylcarbonylamino), cyclopropylamido (cyclopropylaminocarbonyl), methylcarbamoyl (-NHCOOMe), methylurea (-NHCONHMe) or cyclopropyl, where only one or two of the $R^{10}$, $R^{11}$, $R^{12}$, $R^{13}$ radicals are a substituent other than hydrogen,
$R^{14}$, $R^{15}$, $R^{16}$, $R^{17}$ are independently hydrogen or $(C_1-C_4)$ alkyl,
Q is a heteroaromatic 9-membered fused bicyclic ring system from the group of Q2, Q3 or Q14

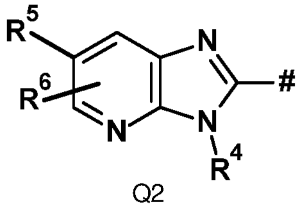
Q2

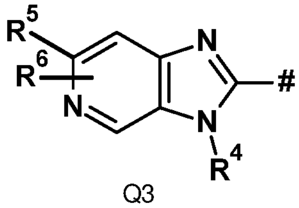
Q3

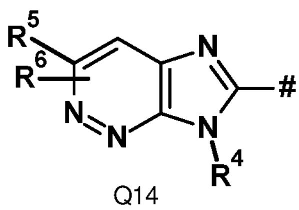
Q14

where

$R^4$ is methyl,
$R^5$ is trifluoromethyl or pentafluoroethyl,
$R^6$ is hydrogen and
n is 0, 1 or 2.

**6.** Compounds of the formula (Ia) or (Ib) according to Claim 2, in which,

if 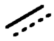 represent exclusively double bonds and hence Aa to Ad together with the carbon atoms adjacent to Aa and Ad in each case form an aromatic ring,

Aa is $C(R^{10})$,
Ab is nitrogen or $C(R^{11})$,
Ac is $C(R^{12})$ and
Ad is $C(R^{13})$, where
$R^{10}$ is hydrogen, chlorine or trifluoromethyl,
$R^{11}$ is hydrogen, chlorine, -NHCOMe or trifluoromethyl,
$R^{12}$ is hydrogen, chlorine or trifluoromethyl,
$R^{13}$ is hydrogen, chlorine or trifluoromethyl,

or, if 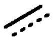 represent exclusively single bonds,
Aa is $C(R^{10})$ $(R^{14})$,
Ab is $C(R^{11})$ $(R^{15})$,
Ac is $C(R^{12})$ $(R^{16})$ and
Ad is $C(R^{13})$ $(R^{17})$ , where
$R^{10}$, $R^{12}$, $R^{13}$, $R^{14}$, $R^{16}$, $R^{17}$ are hydrogen,
$R^{11}$ is hydrogen or methyl and
$R^{15}$ is hydrogen or methyl,
$R^1$ is ethyl,
Q is a heteroaromatic 9-membered fused bicyclic ring system from the group of Q2 or Q3

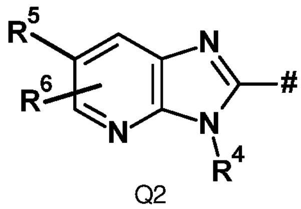
Q2

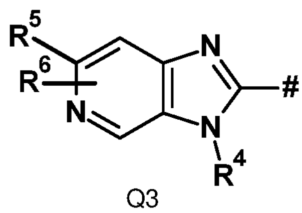
Q3

,

where

$R^4$ is methyl,
$R^5$ is trifluoromethyl or pentafluoroethyl,
$R^6$ is hydrogen and
n is 0, 1 or 2.

**7.** Compounds of the formula (Ia) or (Ib) according to any of Claims 1 to 6, in which
Q is Q2, Q3, Q5, Q6, Q8, Q9, Q14 or Q15,
where

$R^4$ is $(C_1-C_4)$ alkyl, $(C_1-C_4)$ haloalkyl, $(C_3-C_6)$ cycloalkyl or $(C_1-C_4)$ alkoxy- $(C_1-C_4)$ alkyl,
$R^5$ is cyano, halogen, $(C_1-C_4)$ haloalkyl, halo-$(C_3-C_6)$ cycloalkyl, $(C_1-C_4)$ haloalkoxy, $(C_1-C_4)$ haloalkylthio, $(C_1-C_4)$ haloalkylsulfinyl, $(C_1-C_4)$ alkylsulfonyl, $(C_1-C_4)$ haloalkylsulfonyl, $(C_1-C_4)$haloalkylcarbonyl or $(C_1-C_4)$ haloalkylsulfonyloxy,
$R^6$ is hydrogen, cyano, halogen, $(C_1-C_4)$ alkyl, $(C_1-C_4)$ haloalkyl or $(C_3-C_6)$ cycloalkyl and

and Aa, Ab, Ac, Ad, $R^1$, $R^{10}$ $R^{11}$, $R^{12}$, $R^{13}$, $R^{14}$, $R^{15}$, $R^{16}$, $R^{17}$ and n have the definitions according to Claim 1 or Claim 2 or Claim 3 or Claim 5 or Claim 6.

**8.** Compounds of the formula (Ia) or (Ib) according to any of Claims 1 to 6, in which
Q is Q2, Q3 or Q14

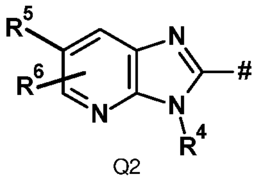

Q2

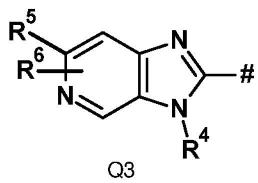

Q3

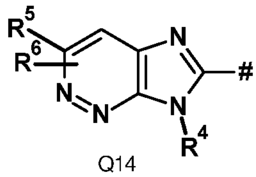

Q14

,

where

$R^4$ is methyl,
$R^5$ is trifluoromethyl or pentafluoroethyl,
$R^6$ is hydrogen

and Aa, Ab, Ac, Ad, $R^1$, $R^{10}$ $R^{11}$, $R^{12}$, $R^{13}$, $R^{14}$, $R^{15}$, $R^{16}$, $R^{17}$ and n have the definitions according to Claim 1 or Claim 2 or Claim 3 or Claim 4 or Claim 6.

**9.** Compounds of the formula (Ia) or (Ib) according to any of Claims 1 to 6, in which
Q is Q2 or Q3

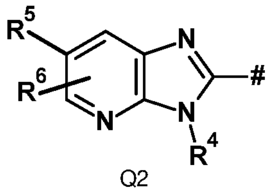

Q2

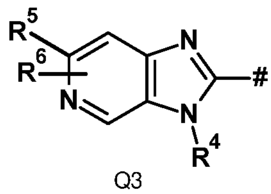

Q3

,

where

$R^4$ is methyl,
$R^5$ is trifluoromethyl or pentafluoroethyl,
$R^6$ is hydrogen

and Aa, Ab, Ac, Ad, $R^1$, $R^{10}$ $R^{11}$, $R^{12}$, $R^{13}$, $R^{14}$, $R^{15}$, $R^{16}$, $R^{17}$ and n have the definitions according to Claim 1 or Claim 2 or Claim 3 or Claim 4 or Claim 5.

**10.** Compounds of the formula (Ia) or (Ib) according to any of Claims 1 to 6, in which $R^1$ is ethyl and Aa, Ab, Ac, Ad, $R^{10}$ $R^{11}$, $R^{12}$, $R^{13}$, $R^{14}$, $R^{15}$, $R^{16}$, $R^{17}$ Q, $R^4$, $R^5$, $R^6$ and n have the definitions according to Claim 1 or Claim 2 or Claim 3 or Claim 4.

**11.** Compounds of the formula (Ia) or (Ib) according to any of Claims 1 to 6, where the formulae (Ia) and (Ib) give rise to the following structures (Ial) to (Ia4) and (Ibl) to (Ib4):

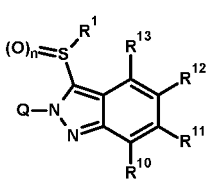

(Ia1)

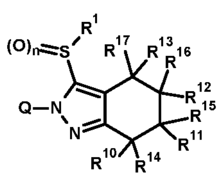

(Ia2)

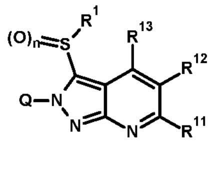

(Ia3)

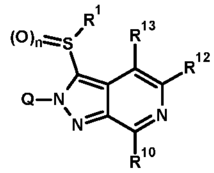

(Ia4)

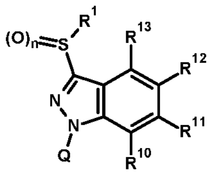 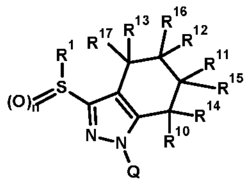 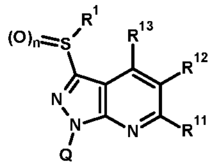 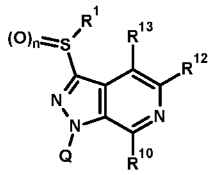

(Ib1) (Ib2) (Ib3) (Ib4)

where $R^1$, $R^{10}$, $R^{11}$, $R^{12}$, $R^{13}$, $R^{14}$, $R^{15}$, $R^{16}$, $R^{17}$, Q, $R^4$, $R^5$, $R^6$ and n have the definitions described in any of Claims 1 to 6.

**12.** Compounds of the formula I-1 to 1-5 and 1-7 to 1-19

| | |
|---|---|
| I-1 | 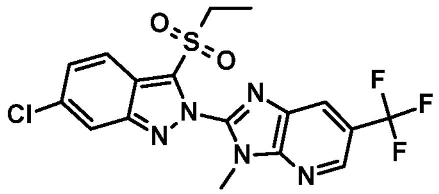 |
| I-2 | 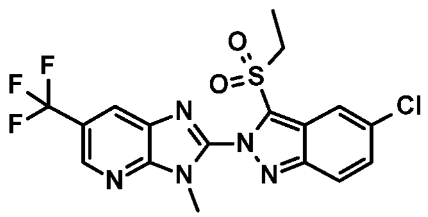 |
| I-3 | 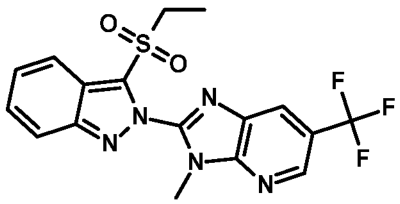 |
| I-4 | 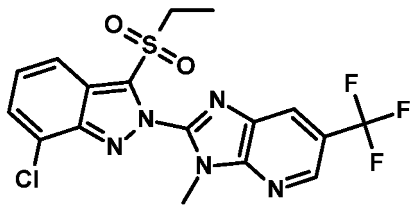 |
| I-5 | 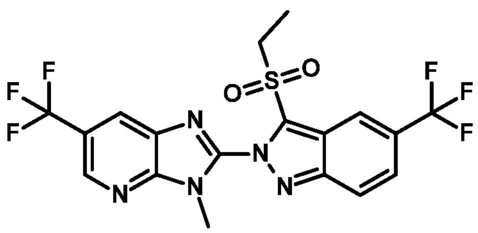 |

(continued)

| | |
|---|---|
| I-7 | 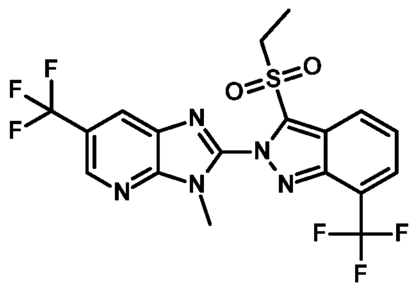 |
| I-8 | 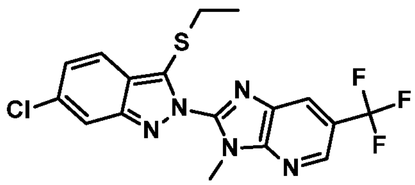 |
| I-9 | 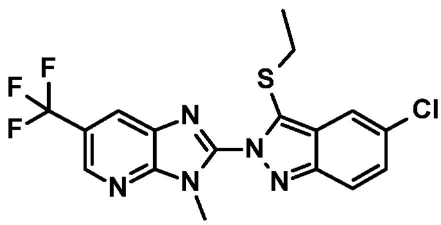 |
| I-10 | 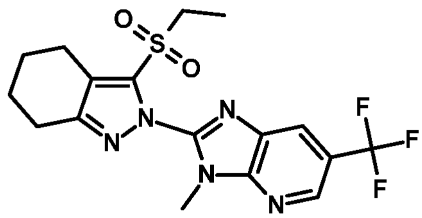 |
| I-11 | 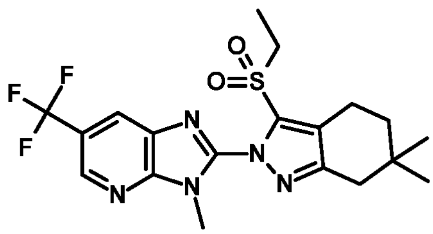 |
| I-12 | 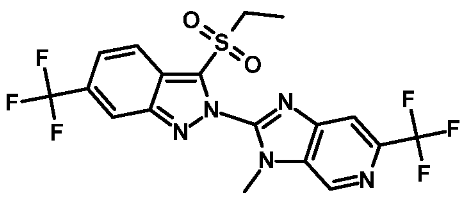 |
| I-13 | 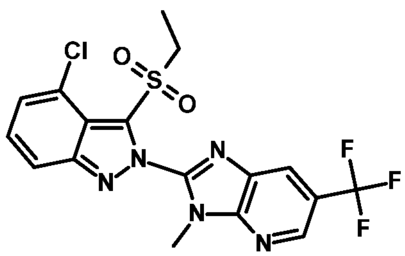 |

(continued)

| | |
|---|---|
| I-14 | 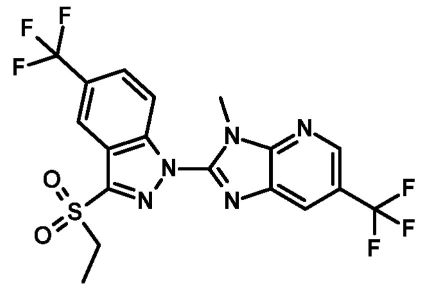 |
| I-15 | 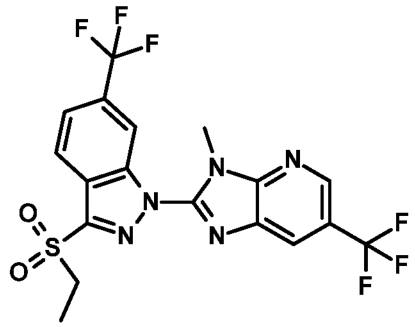 |
| I-16 | 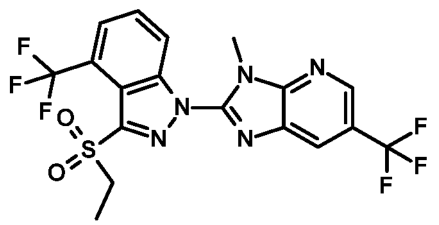 |
| I-17 | 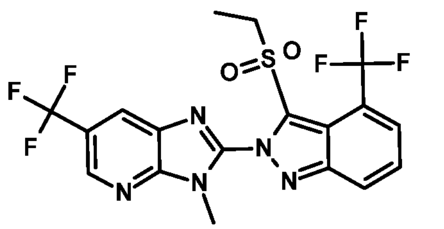 |
| I-18 | 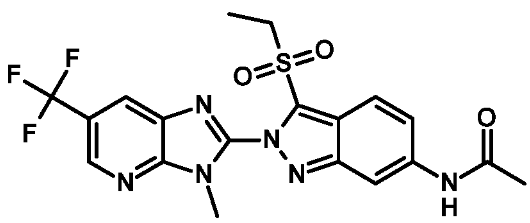 |
| I-19 | 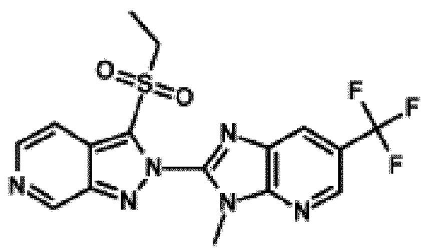 |

**13.** Compounds of the formula (Ia) or (Ib) according to any of Claims 1 to 11,

**characterized in that** if, for the compounds of the formula (Ia),

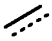 represents exclusively double bonds,

$R^1$ is $C_1$-$C_6$-alkyl,
Aa and Ad are CH,
Ab is $C(R^{11})$,
Ac is $C(R^{12})$,

and

Q is Q2, where
$R^4$ is $C_1$-$C_6$-alkyl,
$R^6$ is hydrogen and
$R^5$ is ($C_1$-$C_6$) haloalkyl, ($C_1$-$C_6$) haloalkylthio, ($C_1$-$C_6$) haloalkylsulfinyl or ($C_1$-$C_6$) haloalkylsulfonyl, at least one of the $R^{11}$ and $R^{12}$ radicals is not hydrogen, halogen or ($C_1$-$C_6$)haloalkyl.

**14.** Compounds of the formula (Ia) or (Ib) according to any of Claims 1 to 11, **characterized in that** if, for the compounds of the formula (Ia),

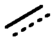 represents exclusively double bonds,
$R^1$ is $C_1$-$C_6$-alkyl,
Aa and Ad are CH,
Ab is $C(R^{11})$,
Ac is $C(R^{12})$,
$R^{11}$ and $R^{12}$ are independently hydrogen, halogen or ($C_1$-$C_6$) haloalkyl,

and

Q is Q2,
$R^4$ is $C_1$-$C_6$-alkyl and
$R^6$ is hydrogen,
$R^5$ is not ($C_1$-$C_6$) haloalkyl, ($C_1$-$C_6$) haloalkylthio, ($C_1$-$C_6$) haloalkylsulfinyl or ($C_1$-$C_6$) haloalkylsulfonyl.

**15.** Compounds of the formula (Ia) or (Ib) according to any of Claims 1 to 11, **characterized in that** compounds of the formula (Y) in which

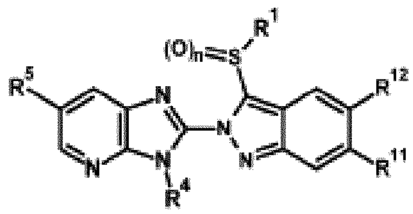

(Y)

$R^1$ is ($C_1$-$C_6$) -alkyl,
$R^4$ is ($C_1$-$C_6$) -alkyl,
$R^5$ is ($C_1$-$C_6$) haloalkyl, ($C_1$-$C_6$) haloalkylthio, ($C_1$-$C_6$) haloalkylsulfinyl or ($C_1$-$C_6$) haloalkylsulfonyl,
$R^{11}$ and $R^{12}$ are independently hydrogen, halogen or ($C_1$-$C_6$) haloalkyl, and
n is 0, 1 or 2

are excluded.

**16.** Compounds of the formula (Ia) according to any of Claims 1 to 11, **characterized in that** 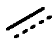 represents exclusively single bonds.

**17.** Agrochemical formulation comprising at least one compound of the formula (Ia) or (Ib) according to any of Claims 1 to 16 and also extenders and/or surfactants.

**18.** Agrochemical formulation according to Claim 17, additionally comprising a further agrochemically active ingredient.

**19.** Method for controlling animal pests, **characterized in that** a compound of the formula (Ia) or (Ib) according to any of Claims 1 to 16 or an agrochemical formulation according to Claim 17 or 18 is allowed to act on the animal pests and/or their habitat, excluding methods of surgical or therapeutic treatment of the human or animal body and diagnostic methods that are performed on the human or animal body.

**20.** Use of compounds of the formula (Ia) or (Ib) according to any of Claims 1 to 16 or an agrochemical formulation according to Claim 17 or 18 for controlling animal pests, excluding methods of surgical or therapeutic treatment of the human or animal body and diagnostic methods that are performed on the human or animal body.

## Revendications

**1.** Composés de la formule (Ia) ou (Ib) :

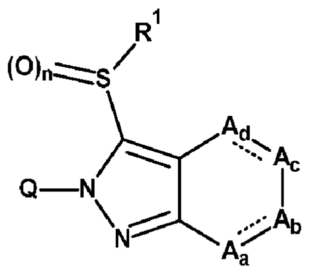

*Ia*

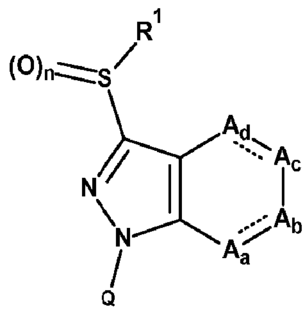

*Ib*

où les 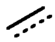 représentent des simples liaisons ou des doubles liaisons,
dans lesquels

dans le cas où les 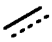 représentent exclusivement des doubles liaisons, et par conséquent Aa à Ad forment un cycle aromatique avec les atomes C voisins respectivement de Aa et Ad,

Aa représente azote ou C($R^{10}$),
Ab représente azote ou C($R^{11}$),
Ac représente azote ou C($R^{12}$) et
Ad représente azote ou C($R^{13}$),
Aa, Ab, Ac et Ad représentant au plus deux azotes,

ou dans le cas où les 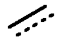 représentent exclusivement des simples liaisons,
Aa représente C($R^{10}$) ($R^{14}$),
Ab représente C($R^{11}$) ($R^{15}$),
Ac représente C($R^{12}$) ($R^{16}$) et
Ad représente C($R^{13}$) ($R^{17}$),
$R^1$ représente alkyle en ($C_1$-$C_6$), halogénoalkyle en ($C_1$-$C_6$), cyanoalkyle en ($C_1$-$C_6$), hydroxyalkyle en ($C_1$-$C_6$), alcoxy en ($C_1$-$C_6$) -alkyle en ($C_1$-$C_6$) , halogénoalcoxy en ($C_1$-$C_6$) -alkyle en ($C_1$-$C_6$), alcényle en ($C_2$-$C_6$) , alcényloxy en ($C_2$-$C_6$) -alkyle en ($C_1$-$C_6$), halogénoalcényloxy en ($C_2$-$C_6$) -alkyle en ($C_1$-$C_6$) , halogénoalcényle en ($C_2$-$C_6$), cyanoalcényle en ($C_2$-$C_6$), alcynyle en ($C_2$-$C_6$), alcynyloxy en ($C_2$-$C_6$)-alkyle en ($C_1$-$C_6$), halogénoalcynyloxy en ($C_2$-$C_6$) -alkyle en ($C_1$-$C_6$), halogénoalcynyle en ($C_2$-$C_6$), cyanoalcynyle en ($C_2$-$C_6$), cycloalkyle en ($C_3$-$C_8$), cycloalkyle en ($C_3$-$C_8$)-cycloalkyle en ($C_3$-$C_8$), alkyle en ($C_1$-$C_6$)-cycloalkyle en ($C_3$-$C_8$), halogénocycloalkyle en ($C_3$-$C_8$), amino, alkylamino en ($C_1$-$C_6$), di-alkylamino en ($C_1$-$C_6$), cycloalkylamino en ($C_3$-$C_8$), alkylcarbonylamino en ($C_1$-$C_6$), alkylthio en ($C_1$-$C_6$) -alkyle en ($C_1$-$C_6$), halogénoalkylthio en ($C_1$-$C_6$) -alkyle en ($C_1$-$C_6$), alkylsulfinyle en ($C_1$-$C_6$) -alkyle en ($C_1$-$C_6$), halogénoalkylsulfinyle en ($C_1$-$C_6$) -alkyle en ($C_1$-$C_6$), alkylsulfonyle en ($C_1$-$C_6$) -alkyle en ($C_1$-$C_6$), halogénoalkylsulfonyle en ($C_1$-$C_6$) -alkyle en ($C_1$-$C_6$), alcoxy en ($C_1$-$C_6$) -alkylthio en ($C_1$-$C_6$) -alkyle en ($C_1$-$C_6$), alcoxy en ($C_1$-$C_6$)-alkylsulfinyle en ($C_1$-$C_6$)-alkyle en ($C_1$-$C_6$), alcoxy en ($C_1$-$C_6$)-alkylsulfonyle en ($C_1$-$C_6$) - alkyle en ($C_1$-$C_6$), alkylcarbonyle en ($C_1$-$C_6$)-alkyle en ($C_1$-$C_6$), halogé-

noalkylcarbonyle en ($C_1$-$C_6$)-alkyle en ($C_1$-$C_6$), alcoxycarbonyle en ($C_1$-$C_6$) -alkyle en ($C_1$-$C_6$), halogénoalcoxycarbonyle en ($C_1$-$C_6$) -alkyle en ($C_1$-$C_6$), alkylsulfonylamino en ($C_1$-$C_6$), aminosulfonyl-alkyle en ($C_1$-$C_6$), alkylaminosulfonyle en ($C_1$-$C_6$)-alkyle en ($C_1$-$C_6$), di-alkylaminosulfonyle en ($C_1$-$C_6$)-alkyle en ($C_1$-$C_6$), ou alkyle en ($C_1$-$C_6$), alcoxy en ($C_1$-$C_6$), alcényle en ($C_2$-$C_6$), alcynyle en ($C_2$-$C_6$) , cycloalkyle en ($C_3$-$C_8$), substitués chacun une fois ou plusieurs fois, de manière identique ou différente, par aryle, hétaryle ou hétérocyclyle, l'aryle, l'hétaryle ou l'hétérocyclyle pouvant chacun éventuellement être substitué une fois ou plusieurs fois, de manière identique ou différente, par halogène, cyano, nitro, hydroxy, amino, carboxy, carbamoyle, aminosulfonyle, alkyle en ($C_1$-$C_6$), cycloalkyle en ($C_3$-$C_6$), alcoxy en ($C_1$-$C_6$), halogénoalkyle en ($C_1$-$C_6$) , halogénoalcoxy en ($C_1$-$C_6$) , alkylthio en ($C_1$-$C_6$), alkylsulfinyle en ($C_1$-$C_6$), alkylsulfonyle en ($C_1$-$C_6$), alkylsulfimino en ($C_1$-$C_6$), alkylsulfimino en ($C_1$-$C_6$)-alkyle en ($C_1$-$C_6$), alkylsulfimino en ($C_1$-$C_6$)-alkylcarbonyle en ($C_2$-$C_6$), alkylsulfoximino en ($C_1$-$C_6$), alkylsulfoximino en ($C_1$-$C_6$)-alkyle en ($C_1$-$C_6$), alkylsulfoximino en ($C_1$-$C_6$)-alkylcarbonyle en ($C_2$-$C_6$) , alcoxycarbonyle en ($C_1$-$C_6$), alkylcarbonyle en ($C_1$-$C_6$), trialkylsilyle en ($C_3$-$C_6$) ou benzyle, ou

$R^1$ représente aryle, hétaryle ou hétérocyclyle, chacun éventuellement substitué une fois ou plusieurs fois, de manière identique ou différente, par halogène, cyano, nitro, hydroxy, amino, carboxy, carbamoyle, alkyle en ($C_1$-$C_6$), cycloalkyle en ($C_3$-$C_8$), alcoxy en ($C_1$-$C_6$), halogénoalkyle en ($C_1$-$C_6$), halogénoalcoxy en ($C_1$-$C_6$), alkylthio en ($C_1$-$C_6$), alkylsulfinyle en ($C_1$-$C_6$), alkylsulfonyle en ($C_1$-$C_6$), alkylsulfimino en ($C_1$-$C_6$), alkylsulfimino en ($C_1$-$C_6$)-alkyle en ($C_1$-$C_6$), alkylsulfimino en ($C_1$-$C_6$) -alkylcarbonyle en ($C_2$-$C_6$), alkylsulfoximino en ($C_1$-$C_6$), alkylsulfoximino en ($C_1$-$C_6$)-alkyle en ($C_1$-$C_6$), alkylsulfoximino en ($C_1$-$C_6$)-alkylcarbonyle en ($C_2$-$C_6$), alcoxycarbonyle en ($C_1$-$C_6$), alkylcarbonyle en ($C_1$-$C_6$), trialkylsilyle en ($C_3$-$C_6$), (=O) (uniquement dans le cas d'hétérocyclyle) ou $(=O)_2$ (uniquement dans le cas d'hétérocyclyle),

$R^{10}$, $R^{11}$, $R^{12}$, $R^{13}$, $R^{14}$, $R^{15}$, $R^{16}$, $R^{17}$ représentent indépendamment les uns des autres hydrogène, cyano, halogène, nitro, hydroxy, amino, tri-alkylsilyle en ($C_1$-$C_6$), cycloalkyle en ($C_3$-$C_8$), cycloalkyle en ($C_3$-$C_8$)-cycloalkyle en ($C_3$-$C_8$), alkyle en ($C_1$-$C_6$)-cycloalkyle en ($C_3$-$C_8$), halogéno-cycloalkyle en ($C_3$-$C_8$), alkyle en ($C_1$-$C_6$), halogénoalkyle en ($C_1$-$C_6$), cyanoalkyle en ($C_1$-$C_6$), hydroxyalkyle en ($C_1$-$C_6$), hydroxycarbonyl-alcoxy en ($C_1$-$C_6$), alcoxycarbonyle en ($C_1$-$C_6$)-alkyle en ($C_1$-$C_6$), alcoxy en ($C_1$-$C_6$) -alkyle en ($C_1$-$C_6$), alcényle en ($C_2$-$C_6$) , halogénoalcényle en ($C_2$-$C_6$), cyanoalcényle en ($C_2$-$C_6$), alcynyle en ($C_2$-$C_6$), halogénoalcynyle en ($C_2$-$C_6$), cyanoalcynyle en ($C_2$-$C_6$), alcoxy en ($C_1$-$C_6$), halogénoalcoxy en ($C_1$-$C_6$), cyanoalcoxy en ($C_1$-$C_6$), alcoxycarbonyle en ($C_1$-$C_6$) -alcoxy en ($C_1$-$C_6$), alcoxy en ($C_1$-$C_6$) -alcoxy en ($C_1$-$C_6$), alkylhydroxyimino en ($C_1$-$C_6$), alcoxyimino en ($C_1$-$C_6$), alkyle en ($C_1$-$C_6$)-alcoxyimino en ($C_1$-$C_6$), halogénoalkyle en ($C_1$-$C_6$)-alcoxyimino en ($C_1$-$C_6$), alkylthio en ($C_1$-$C_6$), halogénoalkylthio en ($C_1$-$C_6$), alcoxy en ($C_1$-$C_6$)-alkylthio en ($C_1$-$C_6$), alkylthio en ($C_1$-$C_6$)-alkyle en ($C_1$-$C_6$), alkylsulfinyle en ($C_1$-$C_6$), halogénoalkylsulfinyle en ($C_1$-$C_6$), alcoxy en ($C_1$-$C_6$)-alkylsulfinyle en ($C_1$-$C_6$), alkylsulfinyle en ($C_1$-$C_6$)-alkyle en ($C_1$-$C_6$), alkylsulfonyle en ($C_1$-$C_6$), halogénoalkylsulfonyle en ($C_1$-$C_6$), alcoxy en ($C_1$-$C_6$)-alkylsulfonyle en ($C_1$-$C_6$), alkylsulfonyle en ($C_1$-$C_6$)-alkyle en ($C_1$-$C_6$), alkylsulfonyloxy en ($C_1$-$C_6$), alkylcarbonyle en ($C_1$-$C_6$), alkylthiocarbonyle en ($C_1$-$C_6$), halogénoalkylcarbonyle en ($C_1$-$C_6$), alkylcarbonyloxy en ($C_1$-$C_6$), alcoxycarbonyle en ($C_1$-$C_6$), halogénoalcoxycarbonyle en ($C_1$-$C_6$), aminocarbonyle, alkylaminocarbonyle en ($C_1$-$C_6$), alkylaminothiocarbonyle en ($C_1$-$C_6$), di-alkylaminocarbonyle en ($C_1$-$C_6$), di-alkylaminothiocarbonyle en ($C_1$-$C_6$), alcénylaminocarbonyle en ($C_2$-$C_6$), di-alcénylaminocarbonyle en ($C_2$-$C_6$), cycloalkylaminocarbonyle en ($C_3$-$C_8$), alkylsulfonylamino en ($C_1$-$C_6$), alkylamino en ($C_1$-$C_6$), di-alkylamino en ($C_1$-$C_6$), aminosulfonyle, alkylaminosulfonyle en ($C_1$-$C_6$), di-alkylaminosulfonyle en ($C_1$-$C_6$), alkylsulfoximino en ($C_1$-$C_6$), aminothiocarbonyle, alkylaminothiocarbonyle en ($C_1$-$C_6$), di-alkylaminothiocarbonyle en ($C_1$-$C_6$), cycloalkylamino en ($C_3$-$C_8$), alkylcarbamoyle en ($C_1$-$C_6$) (comprend -NHCOO-alkyle en ($C_1$-$C_6$), -N-alkyle en ($C_1$-$C_6$)-COO-alkyle en ($C_1$-$C_6$), -OCONH-alkyle en ($C_1$-$C_6$) ou -OCON-dialkyle en ($C_1$-$C_6$)), alkylcarbonylamino en ($C_1$-$C_6$) (alkyle en ($C_1$-$C_6$)-CONH), alkylurée en ($C_1$-$C_6$) (comprend -NHCONH-alkyle en ($C_1$-$C_6$) et -NHCON-dialkyle en ($C_1$-$C_6$)) ou

aryle ou hétaryle, chacun éventuellement substitué une fois ou plusieurs fois, de manière identique ou différente, (dans le cas d'hétaryle) éventuellement au moins un groupe carbonyle pouvant être contenu, et des substituants envisagés étant respectivement : cyano, carboxyle, halogène, nitro, hydroxy, amino, tri-alkylsilyle en ($C_1$-$C_6$), cycloalkyle en ($C_3$-$C_8$), cycloalkyle en ($C_3$-$C_8$)-cycloalkyle en ($C_3$-$C_8$), alkyle en ($C_1$-$C_6$) -cycloalkyle en ($C_3$-$C_8$), halogéno-cycloalkyle en ($C_3$-$C_8$), alkyle en ($C_1$-$C_6$), halogénoalkyle en ($C_1$-$C_6$), cyanoalkyle en ($C_1$-$C_6$), hydroxyalkyle en ($C_1$-$C_6$), hydroxycarbonyle-alcoxy en ($C_1$-$C_6$), alcoxycarbonyle en ($C_1$-$C_6$)-alkyle en ($C_1$-$C_6$), alcoxy en ($C_1$-$C_6$)-alkyle en ($C_1$-$C_6$), alcényle en ($C_2$-$C_6$), halogénoalcényle en ($C_2$-$C_6$), cyanoalcényle en ($C_2$-$C_6$) , alcynyle en ($C_2$-$C_6$) , halogénoalcynyle en ($C_2$-$C_6$), cyanoalcynyle en ($C_2$-$C_6$), alcoxy en ($C_1$-$C_6$), halogénoalcoxy en ($C_1$-$C_6$), cyanoalcoxy en ($C_1$-$C_6$), alcoxycarbonyle en ($C_1$-$C_6$) -alcoxy en ($C_1$-$C_6$), alcoxy en ($C_1$-$C_6$)-alcoxy en ($C_1$-$C_6$), alkylhydroxyimino en ($C_1$-$C_6$) , alcoxyimino en ($C_1$-$C_6$), alkyle en ($C_1$-$C_6$)-alcoxyimino en ($C_1$-$C_6$), halogénoalkyle en ($C_1$-$C_6$)-alcoxyimino en ($C_1$-$C_6$), alkylthio en ($C_1$-$C_6$), halogénoalkylthio en ($C_1$-$C_6$), alcoxy en ($C_1$-$C_6$) -alkylthio en ($C_1$-$C_6$), alkylthio en ($C_1$-$C_6$) -alkyle en ($C_1$-$C_6$), alkylsulfinyle en ($C_1$-$C_6$), halogénoalkylsulfinyle en ($C_1$-$C_6$), alcoxy en ($C_1$-$C_6$)-alkylsulfinyle en ($C_1$-$C_6$), alkylsulfinyle en ($C_1$-$C_6$) -alkyle en ($C_1$-$C_6$), alkylsulfonyle en ($C_1$-$C_6$), halogénoalkylsulfonyle en ($C_1$-$C_6$), alcoxy en ($C_1$-$C_6$)-alkylsulfonyle en

($C_1$-$C_6$), alkylsulfonyle en ($C_1$-$C_6$) -alkyle en ($C_1$-$C_6$), alkylsulfonyloxy en ($C_1$-$C_6$), alkylcarbonyle en ($C_1$-$C_6$), halogénoalkylcarbonyle en ($C_1$-$C_6$), alkylcarbonyloxy en ($C_1$-$C_6$), alcoxycarbonyle en ($C_1$-$C_6$), halogénoalcoxycarbonyle en ($C_1$-$C_6$),aminocarbonyle, alkylaminocarbonyle en ($C_1$-$C_6$),di-alkylaminocarbonyle en ($C_1$-$C_6$),alcénylaminocarbonyle en ($C_2$-$C_6$) , di-alcénylaminocarbonyle en ($C_2$-$C_6$), cycloalkylaminocarbonyle en ($C_3$-$C_8$), alkylsulfonylamino en ($C_1$-$C_6$), alkylamino en ($C_1$-$C_6$), di-alkylamino en ($C_1$-$C_6$), aminosulfonyle, alkylaminosulfonyle en ($C_1$-$C_6$), di-alkylaminosulfonyle en ($C_1$-$C_6$), alkylsulfoximino en ($C_1$-$C_6$), aminothiocarbonyle, alkylaminothiocarbonyle en ($C_1$-$C_6$), di-alkylaminothiocarbonyle en ($C_1$-$C_6$), cycloalkylamino en ($C_3$-$C_8$)ou alkylcarbonylamino en ($C_1$-$C_6$),

uniquement un ou deux des radicaux $R^{10}$, $R^{11}$, $R^{12}$, $R^{13}$, $R^{14}$, $R^{15}$, $R^{16}$ et $R^{17}$ représentant un substituant différent de l'hydrogène,

et dans le cas où $R^{10}$ et $R^{14}$, $R^{11}$ et $R^{15}$, $R^{12}$ et $R^{16}$ ou $R^{13}$ et $R^{17}$ sont respectivement tous les deux différents de l'hydrogène, $R^{14}$, $R^{15}$, $R^{16}$ et $R^{17}$ représentant chacun indépendamment les uns des autres uniquement cyano, halogène, cycloalkyle en ($C_3$-$C_8$), cycloalkyle en ($C_3$-$C_8$)-cycloalkyle en ($C_3$-$C_8$), alkyle en ($C_1$-$C_6$)-cycloalkyle en ($C_3$-$C_8$), halogéno-cycloalkyle en ($C_3$-$C_8$), alkyle en ($C_1$-$C_6$), halogénoalkyle en ($C_1$-$C_6$) ou cyanoalkyle en ($C_1$-$C_6$), Q représente un système cyclique bicyclique ou tricyclique annelé, partiellement saturé ou saturé, hétérocyclique ou hétéroaromatique, à 8, 9, 11 ou 12 chaînons, au moins un groupe carbonyle pouvant éventuellement être contenu et le système cyclique étant éventuellement substitué une fois ou plusieurs fois, de manière identique ou différente, et les substituants pouvant être choisis indépendamment les uns des autres parmi cyano, halogène, nitro, hydroxy, amino, tri-alkylsilyle en ($C_1$-$C_6$), cycloalkyle en ($C_3$-$C_8$), cycloalkyle en ($C_3$-$C_8$)-cycloalkyle en ($C_3$-$C_8$), alkyle en ($C_1$-$C_6$) -cycloalkyle en ($C_3$-$C_8$), halogéno-cycloalkyle en ($C_3$-$C_8$), alkyle en ($C_1$-$C_6$), halogénoalkyle en ($C_1$-$C_6$), cyanoalkyle en ($C_1$-$C_6$), hydroxyalkyle en ($C_1$-$C_6$), hydroxycarbonyl-alcoxy en ($C_1$-$C_6$), alcoxycarbonyle en ($C_1$-$C_6$)-alkyle en ($C_1$-$C_6$), alcoxy en ($C_1$-$C_6$)-alkyle en ($C_1$-$C_6$), alcényle en ($C_2$-$C_6$), halogénoalcényle en ($C_2$-$C_6$), cyanoalcényle en ($C_2$-$C_6$) , alcynyle en ($C_2$-$C_6$) , alcynyloxy en ($C_2$-$C_6$)-alkyle en ($C_1$-$C_4$), halogénoalcynyle en ($C_2$-$C_6$), cyanoalcynyle en ($C_2$-$C_6$), alcoxy en ($C_1$-$C_6$), halogénoalcoxy en ($C_1$-$C_6$), halogénoalcoxy en ($C_1$-$C_6$)-alkyle en ($C_1$-$C_6$), alcényloxy en ($C_2$-$C_6$) -alkyle en ($C_1$-$C_6$), halogénoalcényloxy en ($C_2$-$C_6$) -alkyle en ($C_1$-$C_6$), cyanoalcoxy en ($C_1$-$C_6$), alcoxycarbonyle en ($C_1$-$C_6$)-alcoxy en ($C_1$-$C_6$), alcoxy en ($C_1$-$C_6$) -alcoxy en ($C_1$-$C_6$), alkylhydroxyimino en ($C_1$-$C_6$), alcoxyimino en ($C_1$-$C_6$), alkyle en ($C_1$-$C_6$) -alcoxyimino en ($C_1$-$C_6$), halogénoalkyle en ($C_1$-$C_6$) -alcoxyimino en ($C_1$-$C_6$), alkylthio en ($C_1$-$C_6$), halogénoalkylthio en ($C_1$-$C_6$), alcoxy en ($C_1$-$C_6$)-alkylthio en ($C_1$-$C_6$), alkylthio en ($C_1$-$C_6$) -alkyle en ($C_1$-$C_6$), alkylsulfinyle en ($C_1$-$C_6$), halogénoalkylsulfinyle en ($C_1$-$C_6$), alcoxy en ($C_1$-$C_6$)-alkylsulfinyle en ($C_1$-$C_6$), alkylsulfinyle en ($C_1$-$C_6$) -alkyle en ($C_1$-$C_6$), alkylsulfonyle en ($C_1$-$C_6$), halogénoalkylsulfonyle en ($C_1$-$C_6$), alcoxy en ($C_1$-$C_6$)-alkylsulfonyle en ($C_1$-$C_6$), alkylsulfonyle en ($C_1$-$C_6$) -alkyle en ($C_1$-$C_6$), alkylsulfonyloxy en ($C_1$-$C_6$), alkylcarbonyle en ($C_1$-$C_6$), alkylcarbonyle en ($C_1$-$C_6$) -alkyle en ($C_1$-$C_6$) , alkylthiocarbonyle en ($C_1$-$C_6$), halogénoalkylcarbonyle en ($C_1$-$C_6$), alkylcarbonyloxy en ($C_1$-$C_6$), alcoxycarbonyle en ($C_1$-$C_6$), halogénoalcoxycarbonyle en ($C_1$-$C_6$), aminocarbonyle, alkylaminocarbonyle en ($C_1$-$C_6$), alkylaminothiocarbonyle en ($C_1$-$C_6$), di-alkylaminocarbonyle en ($C_1$-$C_6$), di-alkylaminothiocarbonyle en ($C_1$-$C_6$), alcénylaminocarbonyle en ($C_2$-$C_6$), di-alcénylaminocarbonyle en ($C_2$-$C_6$), cycloalkylaminocarbonyle en ($C_3$-$C_8$), alkylsulfonylamino en ($C_1$-$C_6$), alkylamino en ($C_1$-$C_6$), di-alkylamino en ($C_1$-$C_6$), aminosulfonyle, alkylaminosulfonyle en ($C_1$-$C_6$), di-alkylaminosulfonyle en ($C_1$-$C_6$), alkylsulfoximino en ($C_1$-$C_6$), aminothiocarbonyle, alkylaminothiocarbonyle en ($C_1$-$C_6$), di-alkylaminothiocarbonyle en ($C_1$-$C_6$), cycloalkylamino en ($C_3$-$C_8$)et alkylcarbonylamino en ($C_1$-$C_6$),

ou les substituants pouvant être choisis indépendamment les uns des autres parmi phényle et un cycle hétéroaromatique à 5 ou 6 chaînons, le phényle ou le cycle pouvant éventuellement être substitué une fois ou plusieurs fois, de manière identique ou différente, par alkyle en ($C_1$-$C_6$), alcényle en ($C_2$-$C_6$), alcynyle en ($C_2$-$C_6$), cycloalkyle en ($C_3$-$C_6$) , haloalkyle en ($C_1$-$C_6$), haloalcényle en ($C_2$-$C_6$), haloalcynyle en ($C_2$-$C_6$), halocycloalkyle en ($C_3$-$C_6$), halogène, CN, alcoxy en ($C_1$-$C_4$), haloalcoxy en ($C_1$-$C_4$) ,

n représente 0, 1 ou 2.

**2.** Composés de la formule (Ia) ou (Ib) selon la revendication 1, dans lesquels dans le cas où les 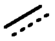 représentent exclusivement des doubles liaisons, et par conséquent Aa à Ad forment un cycle aromatique avec les atomes C voisins respectivement de Aa et Ad,

Aa représente azote ou C($R^{10}$),
Ab représente azote ou C($R^{11}$),
Ac représente azote ou C($R^{12}$) et
Ad représente azote ou C($R^{13}$),
Aa, Ab, Ac et Ad représentant au plus deux azotes,

ou dans le cas où les  représentent exclusivement des simples liaisons,

Aa représente C($R^{10}$) ($R^{14}$),
Ab représente C($R^{11}$) ($R^{15}$),
Ac représente C($R^{12}$) ($R^{16}$) et
Ad représente C($R^{13}$) ($R^{17}$),
$R^1$ représente alkyle en ($C_1$-$C_4$), hydroxyalkyle en ($C_1$-$C_4$), halogénoalkyle en ($C_1$-$C_4$), cyanoalkyle en ($C_1$-$C_4$), alcoxy en ($C_1$-$C_4$) -alkyle en ($C_1$-$C_4$) , halogénoalcoxy en ($C_1$-$C_4$) -alkyle en ($C_1$-$C_4$), alcényle en ($C_2$-$C_4$), alcényloxy en ($C_2$-$C_4$)-alkyle en ($C_1$-$C_4$), halogénoalcényloxy en ($C_2$-$C_4$)-alkyle en ($C_1$-$C_4$) , halogénoalcényle en ($C_2$-$C_4$), cyanoalcényle en ($C_2$-$C_4$), alcynyle en ($C_2$-$C_4$), alcynyloxy en ($C_2$-$C_4$)-alkyle en ($C_1$-$C_4$), halogénoalcynyloxy en ($C_2$-$C_4$)-alkyle en ($C_1$-$C_4$), halogénoalcynyle en ($C_2$-$C_4$), cyanoalcynyle en ($C_2$-$C_4$), cycloalkyle en ($C_3$-$C_6$), cycloalkyle en ($C_3$-$C_6$)-cycloalkyle en ($C_3$-$C_6$), alkyle en ($C_1$-$C_4$)-cycloalkyle en ($C_3$-$C_6$), halogénocycloalkyle en ($C_3$-$C_6$), alkylamino en ($C_1$-$C_4$), di-alkylamino en ($C_1$-$C_4$), cycloalkylamino en ($C_3$-$C_6$), alkylcarbonylamino en ($C_1$-$C_4$), alkylthio en ($C_1$-$C_4$)-alkyle en ($C_1$-$C_4$), halogénoalkylthio en ($C_1$-$C_4$)-alkyle en ($C_1$-$C_4$), alkylsulfinyle en ($C_1$-$C_4$)-alkyle en ($C_1$-$C_4$), halogénoalkylsulfinyle en ($C_1$-$C_4$)-alkyle en ($C_1$-$C_4$), alkylsulfonyle en ($C_1$-$C_4$) -alkyle en ($C_1$-$C_4$), alkylcarbonyle en ($C_1$-$C_4$) -alkyle en ($C_1$-$C_4$), halogénoalkylcarbonyle en ($C_1$-$C_4$) -alkyle en ($C_1$-$C_4$) ou alkylsulfonylamino en ($C_1$-$C_4$),
$R^{10}$, $R^{11}$, $R^{12}$, $R^{13}$, $R^{14}$, $R^{15}$, $R^{16}$, $R^{17}$ représentent indépendamment les uns des autres hydrogène, cyano, halogène, nitro, hydroxy, amino, tri-alkylsilyle en ($C_1$-$C_4$), cycloalkyle en ($C_3$-$C_6$) , cycloalkyle en ($C_3$-$C_6$)-cycloalkyle en ($C_3$-$C_6$), alkyle en ($C_1$-$C_4$)-cycloalkyle en ($C_3$-$C_6$), halogéno-cycloalkyle en ($C_3$-$C_6$), alkyle en ($C_1$-$C_4$), halogénoalkyle en ($C_1$-$C_4$), cyanoalkyle en ($C_1$-$C_4$), hydroxyalkyle en ($C_1$-$C_4$), alcoxy en ($C_1$-$C_4$)-alkyle en ($C_1$-$C_4$), alcényle en ($C_2$-$C_4$), halogénoalcényle en ($C_2$-$C_4$), cyanoalcényle en ($C_2$-$C_4$), alcynyle en ($C_2$-$C_4$), halogénoalcynyle en ($C_2$-$C_4$), cyanoalcynyle en ($C_2$-$C_4$), alcoxy en ($C_1$-$C_4$), halogénoalcoxy en ($C_1$-$C_4$), cyanoalcoxy en ($C_1$-$C_4$), alcoxy en ($C_1$-$C_4$) -alcoxy en ($C_1$-$C_4$), alkylhydroxyimino en ($C_1$-$C_4$), alcoxyimino en ($C_1$-$C_4$), alkyle en ($C_1$-$C_4$) -alcoxyimino en ($C_1$-$C_4$), halogénoalkyle en ($C_1$-$C_4$)-alcoxyimino en ($C_1$-$C_4$), alkylthio en ($C_1$-$C_4$), halogénoalkylthio en ($C_1$-$C_4$), alkylthio en ($C_1$-$C_4$)-alkyle en ($C_1$-$C_4$), alkylsulfinyle en ($C_1$-$C_4$), halogénoalkylsulfinyle en ($C_1$-$C_4$), alkylsulfinyle en ($C_1$-$C_4$) -alkyle en ($C_1$-$C_4$), alkylsulfonyle en ($C_1$-$C_4$), halogénoalkylsulfonyle en ($C_1$-$C_4$), alkylsulfonyle en ($C_1$-$C_4$) -alkyle en ($C_1$-$C_4$), alkylsulfonyloxy en ($C_1$-$C_4$), alkylcarbonyle en ($C_1$-$C_4$), halogénoalkylcarbonyle en ($C_1$-$C_4$), aminocarbonyle, aminothiocarbonyle, alkylaminocarbonyle en ($C_1$-$C_4$), di-alkylaminocarbonyle en ($C_1$-$C_4$), cycloalkylaminocarbonyle en ($C_3$-$C_6$), alkylsulfonylamino en ($C_1$-$C_4$), alkylamino en ($C_1$-$C_4$), di-alkylamino en ($C_1$-$C_4$), aminosulfonyle, alkylaminosulfonyle en ($C_1$-$C_4$), di-alkylaminosulfonyle en ($C_1$-$C_4$), aminothiocarbonyle, alkylcarbamoyle en ($C_1$-$C_4$) (comprend -NHCOO-alkyle en ($C_1$-$C_4$), -N-alkyle en ($C_1$-$C_4$) -COO-alkyle en ($C_1$-$C_4$), -OCONH-alkyle en ($C_1$-$C_4$) ou - OCON-dialkyle en ($C_1$-$C_4$)), alkylcarbonylamino en ($C_1$-$C_4$), alkylurée en ($C_1$-$C_4$) (comprend -NHCONH-alkyle en ($C_1$-$C_4$) et -NHCON-dialkyle en ($C_1$-$C_4$)),
ou représentent phényle ou hétaryle, chacun éventuellement substitué une fois ou deux fois, de manière identique ou différente, (dans le cas d'hétaryle) éventuellement au moins un groupe carbonyle pouvant être contenu, et des substituants envisagés étant respectivement : cyano, halogène, nitro, acétyle, amino, cycloalkyle en ($C_3$-$C_6$), cycloalkyle en ($C_3$-$C_6$)-cycloalkyle en ($C_3$-$C_6$), alkyle en ($C_1$-$C_4$)-cycloalkyle en ($C_3$-$C_6$), halogénocycloalkyle en ($C_3$-$C_6$), alkyle en ($C_1$-$C_4$), halogénoalkyle en ($C_1$-$C_4$), cyanoalkyle en ($C_1$-$C_4$), hydroxyalkyle en ($C_1$-$C_4$), alcoxy en ($C_1$-$C_4$)-alkyle en ($C_1$-$C_4$), alcényle en ($C_2$-$C_4$), halogénoalcényle en ($C_2$-$C_4$), cyanoalcényle en ($C_2$-$C_4$), alcynyle en ($C_2$-$C_4$), halogénoalcynyle en ($C_2$-$C_4$), cyanoalcynyle en ($C_2$-$C_4$), alcoxy en ($C_1$-$C_4$), halogénoalcoxy en ($C_1$-$C_4$), cyanoalcoxy en ($C_1$-$C_4$), alcoxy en ($C_1$-$C_4$) -alcoxy en ($C_1$-$C_4$), alkylhydroxyimino en ($C_1$-$C_4$), alcoxyimino en ($C_1$-$C_4$), alkyle en ($C_1$-$C_4$) -alcoxyimino en ($C_1$-$C_4$), halogénoalkyle en ($C_1$-$C_4$)-alcoxyimino en ($C_1$-$C_4$), alkylthio en ($C_1$-$C_4$), halogénoalkylthio en ($C_1$-$C_4$), alkylthio en ($C_1$-$C_4$)-alkyle en ($C_1$-$C_4$), alkylsulfinyle en ($C_1$-$C_4$), halogénoalkylsulfinyle en ($C_1$-$C_4$), alkylsulfinyle en ($C_1$-$C_4$) -alkyle en ($C_1$-$C_4$), alkylsulfonyle en ($C_1$-$C_4$), halogénoalkylsulfonyle en ($C_1$-$C_4$), alkylsulfonyle en ($C_1$-$C_4$) -alkyle en ($C_1$-$C_4$), alkylsulfonyloxy en ($C_1$-$C_4$), alkylcarbonyle en ($C_1$-$C_4$), halogénoalkylcarbonyle en ($C_1$-$C_4$), aminocarbonyle, alkylaminocarbonyle en ($C_1$-$C_4$), di-alkylaminocarbonyle en ($C_1$-$C_4$), alkylsulfonylamino en ($C_1$-$C_4$), alkylamino en ($C_1$-$C_4$), di-alkylamino en ($C_1$-$C_4$), aminosulfonyle, alkylaminosulfonyle en ($C_1$-$C_4$), di-alkylaminosulfonyle en ($C_1$-$C_4$) ou alkylcarbonylamino en ($C_1$-$C_4$),
uniquement un ou deux des radicaux $R^{10}$, $R^{11}$, $R^{12}$, $R^{13}$, $R^{14}$, $R^{15}$, $R^{16}$ et $R^{17}$ représentant un substituant différent de l'hydrogène,
et dans le cas où $R^{10}$ et $R^{14}$, $R^{11}$ et $R^{15}$, $R^{12}$ et $R^{16}$ ou $R^{13}$ et $R^{17}$ sont respectivement tous les deux différents de l'hydrogène, $R^{14}$, $R^{15}$, $R^{16}$ et $R^{17}$ représentant chacun indépendamment les uns des autres uniquement cyano, halogène, cycloalkyle en ($C_3$-$C_6$), cycloalkyle en ($C_3$-$C_6$)-cycloalkyle en ($C_3$-$C_6$), alkyle en ($C_1$-$C_4$) -cycloalkyle en ($C_3$-$C_6$), halogéno-cycloalkyle en ($C_3$-$C_6$), alkyle en ($C_1$-$C_4$), halogénoalkyle en ($C_1$-$C_4$) ou cyanoalkyle en ($C_1$-$C_4$), Q représente un système cyclique bicyclique ou tricyclique annelé hétéroaromatique, à 9 chaînons ou 12 chaînons, de la série Q1 à Q15 :

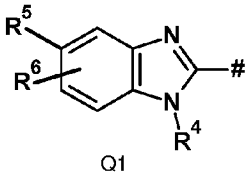

Q1

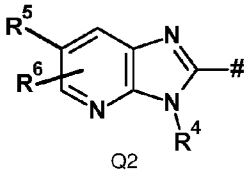

Q2

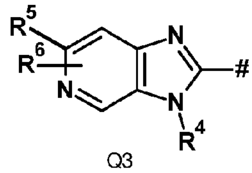

Q3

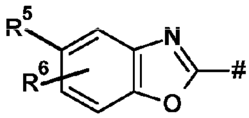

Q4

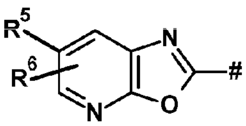

Q5

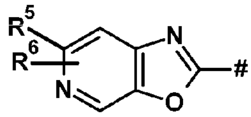

Q6

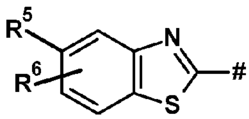

Q7

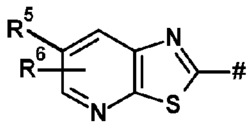

Q8

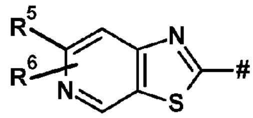

Q9

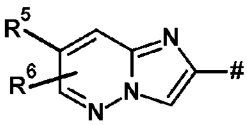

Q10

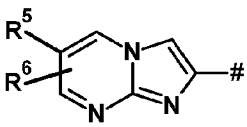

Q11

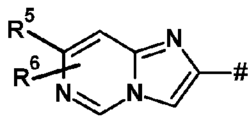

Q12

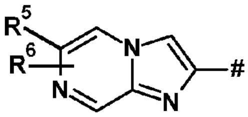

Q13

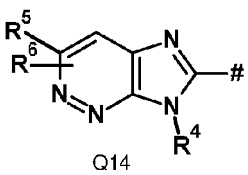

Q14

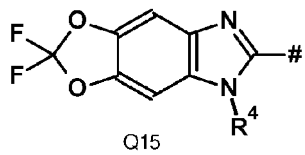

Q15

où

$R^4$ représente hydrogène, alkyle en ($C_1$-$C_4$), halogénoalkyle en ($C_1$-$C_4$), cyanoalkyle en ($C_1$-$C_4$), hydroxyalkyle en ($C_1$-$C_4$), alcoxy en ($C_1$-$C_4$)-alkyle en ($C_1$-$C_4$) , halogénoalcoxy en ($C_1$-$C_4$) -alkyle en ($C_1$-$C_4$), alcényle en ($C_2$-$C_4$), alcényloxy en ($C_2$-$C_4$)-alkyle en ($C_1$-$C_4$), halogénoalcényloxy en ($C_2$-$C_4$) -alkyle en ($C_1$-$C_4$), halogénoalcényle en (CrC$_4$), cyanoalcényle en ($C_2$-$C_4$) , alcynyle en ($C_2$-$C_4$) , halogénoalcynyle en ($C_2$-$C_4$) ou cycloalkyle en ($C_3$-$C_6$), et

$R^5$, $R^6$ représentent indépendamment l'un de l'autre hydrogène, cyano, halogène, alkyle en ($C_1$-$C_4$), halogénoalkyle en ($C_1$-$C_4$), alcényle en ($C_2$-$C_4$) , halogénoalcényle en ($C_2$-$C_4$), alcynyle en ($C_2$-$C_4$), halogénoalcynyle en ($C_2$-$C_4$) , cycloalkyle en ($C_3$-$C_6$) , cycloalkyle en ($C_3$-$C_6$)-cycloalkyle en($C_3$-$C_6$), alkyle en ($C_1$-$C_4$) -cycloalkyle en ($C_3$-$C_6$), halogénoalkyle en ($C_1$-$C_4$)-cycloalkyle en ($C_3$-$C_6$), cyano-cycloalkyle en ($C_3$-$C_6$), halogéno-cycloalkyle en ($C_3$-$C_6$) , alcoxy en ($C_1$-$C_4$) , halogénoalcoxy en ($C_1$-$C_4$), alcoxyimino en ($C_1$-$C_4$) , halogénoalcoxyimino en ($C_1$-$C_4$), alkylthio en ($C_1$-$C_4$), halogénoalkylthio en ($C_1$-$C_4$), alkylsulfinyle en ($C_1$-$C_4$), halogénoalkylsulfinyle en ($C_1$-$C_4$), alkylsulfonyle en ($C_1$-$C_4$), halogénoalkylsulfonyle en ($C_1$-$C_4$), alkylsulfonyloxy en ($C_1$-$C_4$), halogénoalkylsulfonyloxy en ($C_1$-$C_4$), alkylcarbonyle en ($C_1$-$C_4$), halogénoalkylcarbonyle en ($C_1$-$C_4$), aminocarbonyle, alkylaminocarbonyle en ($C_1$-$C_4$), di-alkylaminocarbonyle en ($C_1$-$C_4$), alkylsulfonylamino en ($C_1$-$C_4$), alkylamino en ($C_1$-$C_4$), di-alkylamino en ($C_1$-$C_4$), aminosulfonyle, alkylaminosulfonyle en ($C_1$-$C_4$)ou di-alkylaminosulfonyle en ($C_1$-$C_4$),

et

n représente 0, 1 ou 2.

**3.** Composés de la formule (Ia) ou (Ib) selon la revendication 2, dans lesquels

dans le cas où les  représentent exclusivement des doubles liaisons, et par conséquent Aa à Ad forment un

cycle aromatique avec les atomes C voisins respectivement de Aa et Ad,

Aa représente azote ou $C(R^{10})$,
Ab représente azote ou $C(R^{11})$ ,
Ac représente azote ou $C(R^{12})$ et
Ad représente azote ou $C(R^{13})$,
Aa, Ab, Ac et Ad représentant au plus deux azotes,

ou dans le cas où les 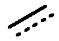 représentent exclusivement des simples liaisons,
Aa représente $C(R^{10})(R^{14})$ ,
Ab représente $C(R^{11})(R^{15})$ ,
Ac représente $C(R^{12})(R^{16})$ et
Ad représente $C(R^{13})(R^{17})$ ,
$R^1$ représente alkyle en ($C_1$-$C_4$), hydroxyalkyle en ($C_1$-$C_4$), halogénoalkyle en ($C_1$-$C_4$), alcényle en ($C_2$-$C_4$) , halogénoalcényle en ($C_2$-$C_4$) , alcynyle en ($C_2$-$C_4$) , halogénoalcynyle en ($C_2$-$C_4$) , cycloalkyle en ($C_3$-$C_6$) , alkylthio en ($C_1$-$C_4$)-alkyle en ($C_1$-$C_4$), alkylsulfinyle en ($C_1$-$C_4$)-alkyle en ($C_1$-$C_4$)ou alkylsulfonyle en ($C_1$-$C_4$)-alkyle en ($C_1$-$C_4$),
$R^{10}$, $R^{11}$, $R^{12}$, $R^{13}$, $R^{14}$, $R^{15}$, $R^{16}$, $R^{17}$ représentent indépendamment les uns des autres hydrogène, cyano, halogène, nitro, hydroxy, amino, tri-alkylsilyle en ($C_1$-$C_4$), cycloalkyle en ($C_3$-$C_6$), cycloalkyle en ($C_3$-$C_6$)-cycloalkyle en ($C_3$-$C_6$), alkyle en ($C_1$-$C_4$) -cycloalkyle en ($C_3$-$C_6$), halogéno-cycloalkyle en ($C_3$-$C_6$), alkyle en ($C_1$-$C_4$), halogénoalkyle en ($C_1$-$C_4$), cyanoalkyle en ($C_1$-$C_4$) , alcoxy en ($C_1$-$C_4$)-alkyle en ($C_1$-$C_4$), alcényle en ($C_2$-$C_4$) , halogénoalcényle en ($C_2$-$C_4$), cyanoalcényle en ($C_2$-$C_4$) , alcynyle en ($C_2$-$C_4$) , halogénoalcynyle en ($C_2$-$C_4$) , cyanoalcynyle en ($C_2$-$C_4$) , alcoxy en ($C_1$-$C_4$), halogénoalcoxy en ($C_1$-$C_4$), cyanoalcoxy en ($C_1$-$C_4$), alkylthio en ($C_1$-$C_4$), halogénoalkylthio en ($C_1$-$C_4$), alkylsulfinyle en ($C_1$-$C_4$), halogénoalkylsulfinyle en ($C_1$-$C_4$), alkylsulfonyle en ($C_1$-$C_4$), halogénoalkylsulfonyle en ($C_1$-$C_4$), alkylsulfonyloxy en ($C_1$-$C_4$), alkylcarbonyle en ($C_1$-$C_4$), halogénoalkylcarbonyle en ($C_1$-$C_4$) , aminocarbonyle, alkylaminocarbonyle en ($C_1$-$C_4$), di-alkylaminocarbonyle en ($C_1$-$C_4$), cycloalkylaminocarbonyle en ($C_3$-$C_6$), alkylsulfonylamino en ($C_1$-$C_4$), alkylamino en ($C_1$-$C_4$), di-alkylamino en ($C_1$-$C_4$), aminosulfonyle, alkylaminosulfonyle en ($C_1$-$C_4$), di-alkylaminosulfonyle en ($C_1$-$C_4$), alkylcarbamoyle en ($C_1$-$C_4$)(comprend -NHCOO-alkyle en ($C_1$-$C_4$)et -N-alkyle en ($C_1$-$C_4$)-COO-alkyle en ($C_1$-$C_4$)), alkylcarbonylamino en ($C_1$-$C_4$), alkylurée en ($C_1$-$C_4$) (comprend -NHCONH-alkyle en ($C_1$-$C_4$) et -NHCON-dialkyle en ($C_1$-$C_4$)),
ou représentent phényle ou hétaryle, chacun éventuellement substitué une fois ou deux fois, de manière identique ou différente, (dans le cas d'hétaryle) éventuellement au moins un groupe carbonyle pouvant être contenu et, des substituants envisagés étant respectivement : cyano, halogène, cycloalkyle en ($C_3$-$C_6$), cycloalkyle en ($C_3$-$C_6$) -cycloalkyle en ($C_3$-$C_6$), alkyle en ($C_1$-$C_4$)-cycloalkyle en ($C_3$-$C_6$) , halogéno-cycloalkyle en ($C_3$-$C_6$), alkyle en ($C_1$-$C_4$), halogénoalkyle en ($C_1$-$C_4$), cyanoalkyle en ($C_1$-$C_4$), alcényle en ($C_2$-$C_4$) , halogénoalcényle en ($C_2$-$C_4$), cyanoalcényle en ($C_2$-$C_4$) , alcynyle en ($C_2$-$C_4$) , halogénoalcynyle en ($C_2$-$C_4$) , cyanoalcynyle en ($C_2$-$C_4$) , alcoxy en ($C_1$-$C_4$), halogénoalcoxy en ($C_1$-$C_4$), alkylthio en ($C_1$-$C_4$), halogénoalkylthio en ($C_1$-$C_4$), alkylsulfinyle en ($C_1$-$C_4$), halogénoalkylsulfinyle en ($C_1$-$C_4$), alkylsulfonyle en ($C_1$-$C_4$), halogénoalkylsulfonyle en ($C_1$-$C_4$), alkylsulfonyloxy en ($C_1$-$C_4$), alkylcarbonyle en ($C_1$-$C_4$), halogénoalkylcarbonyle en ($C_1$-$C_4$), aminocarbonyle, alkylaminocarbonyle en ($C_1$-$C_4$), di-alkylaminocarbonyle en ($C_1$-$C_4$), alkylsulfonylamino en ($C_1$-$C_4$), alkylamino en ($C_1$-$C_4$), di-alkylamino en ($C_1$-$C_4$), aminosulfonyle, alkylaminosulfonyle en ($C_1$-$C_4$), di-alkylaminosulfonyle en ($C_1$-$C_4$), alkylcarbonylamino en ($C_1$-$C_4$),
uniquement un ou deux des radicaux $R^{10}$, $R^{11}$, $R^{12}$, $R^{13}$, $R^{14}$, $R^{15}$, $R^{16}$, $R^{17}$ représentant un substituant différent de l'hydrogène,
et dans le cas où $R^{10}$ et $R^{14}$, $R^{11}$ et $R^{15}$, $R^{12}$ et $R^{16}$ ou $R^{13}$ et $R^{17}$ sont respectivement tous les deux différents de l'hydrogène, $R^{14}$, $R^{15}$, $R^{16}$ et $R^{17}$ représentant chacun indépendamment les uns des autres uniquement cyano, halogène, cycloalkyle en ($C_3$-$C_6$), cycloalkyle en ($C_3$-$C_6$)-cycloalkyle en ($C_3$-$C_6$), alkyle en ($C_1$-$C_4$) -cycloalkyle en ($C_3$-$C_6$), halogéno-cycloalkyle en ($C_3$-$C_6$), alkyle en ($C_1$-$C_4$), halogénoalkyle en ($C_1$-$C_4$) ou cyanoalkyle en ($C_1$-$C_4$), Q représente un système cyclique bicyclique ou tricyclique annelé hétéroaromatique, à 9 chaînons ou 12 chaînons, de la série Q1 à Q15,
où
$R^4$ représente hydrogène, alkyle en ($C_1$-$C_4$), cycloalkyle en ($C_3$-$C_6$), halogénoalkyle en ($C_1$-$C_4$), cyanoalkyle en ($C_1$-$C_4$), alcoxy en ($C_1$-$C_4$)-alkyle en ($C_1$-$C_4$), halogénoalcoxy en ($C_1$-$C_4$)-alkyle en ($C_1$-$C_4$), alcényle en ($C_2$-$C_4$) , halogénoalcényle en ($C_2$-$C_4$) , alcynyle en ($C_2$-$C_4$) ou halogénoalcynyle en ($C_2$-$C_4$) , et $R^5$, $R^6$ représentent indépendamment l'un de l'autre hydrogène, cyano, halogène, alkyle en ($C_1$-$C_4$), halogénoalkyle en ($C_1$-$C_4$), alcényle en ($C_2$-$C_4$) , halogénoalcényle en ($C_2$-$C_4$) , alcynyle en ($C_2$-$C_4$) , halogénoalcynyle en ($C_2$-$C_4$) , cycloalkyle en ($C_3$-$C_6$), cycloalkyle en ($C_3$-$C_6$)-cycloalkyle en($C_3$-$C_6$), alkyle en ($C_1$-$C_4$) -cycloalkyle en ($C_3$-$C_6$),

halogénoalkyle en ($C_1$-$C_4$)-cycloalkyle en ($C_3$-$C_6$), cyano-cycloalkyle en ($C_3$-$C_6$), halogéno-cycloalkyle en ($C_3$-$C_6$) , alcoxy en ($C_1$-$C_4$), halogénoalcoxy en ($C_1$-$C_4$), alcoxyimino en ($C_1$-$C_4$), halogénoalcoxyimino en ($C_1$-$C_4$), alkylthio en ($C_1$-$C_4$), halogénoalkylthio en ($C_1$-$C_4$), alkylsulfinyle en ($C_1$-$C_4$), halogénoalkylsulfinyle en ($C_1$-$C_4$), alkylsulfonyle en ($C_1$-$C_4$), halogénoalkylsulfonyle en ($C_1$-$C_4$), alkylsulfonyloxy en ($C_1$-$C_4$), halogénoalkylsulfonyloxy en ($C_1$-$C_4$), alkylcarbonyle en ($C_1$-$C_4$), halogénoalkylcarbonyle en ($C_1$-$C_4$), aminocarbonyle, alkylaminocarbonyle en ($C_1$-$C_4$), di-alkylaminocarbonyle en ($C_1$-$C_4$), alkylsulfonylamino en ($C_1$-$C_4$), alkylamino en ($C_1$-$C_4$), di-alkylamino en ($C_1$-$C_4$), aminosulfonyle, alkylaminosulfonyle en ($C_1$-$C_4$)ou di-alkylaminosulfonyle en ($C_1$-$C_4$),
et
n représente 0, 1 ou 2.

**4.** Composés de la formule (Ia) ou (Ib) selon la revendication 2, dans lesquels

dans le cas où les 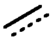 représentent exclusivement des doubles liaisons, et par conséquent Aa à Ad forment un cycle aromatique avec les atomes C voisins respectivement de Aa et Ad,

Aa représente azote ou C($R^{10}$),
Ab représente azote ou C($R^{11}$) ,
Ac représente azote ou C($R^{12}$) et
Ad représente azote ou C($R^{13}$),
Aa, Ab, Ac et Ad représentant au plus deux azotes,

ou dans le cas où les 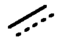 représentent exclusivement des simples liaisons,
Aa représente C($R^{10}$) ($R^{14}$) ,
Ab représente C($R^{11}$) ($R^{15}$) ,
Ac représente C($R^{12}$)($R^{16}$) et
Ad représente C($R^{13}$)($R^{17}$) ,
$R^1$ représente méthyle, éthyle, n-propyle, i-propyle, cyclopropyle, n-butyle, i-butyle, tert.-butyle, cyclobutyle, fluorométhyle, difluorométhyle, trifluorométhyle, fluoroéthyle, difluoroéthyle, trifluoroéthyle, tétrafluoroéthyle ou pentafluoroéthyle,
$R^{10}$, $R^{11}$, $R^{12}$, $R^{13}$, $R^{14}$, $R^{15}$, $R^{16}$, $R^{17}$ représentent indépendamment les uns des autres hydrogène, cyano, halogène, alkyle en ($C_1$-$C_4$), halogénoalkyle en ($C_1$-$C_4$), alcoxy en ($C_1$-$C_4$), halogénoalcoxy en ($C_1$-$C_4$), alkylthio en ($C_1$-$C_4$), alkylsulfinyle en ($C_1$-$C_4$), alkylsulfonyle en ($C_1$-$C_4$), halogénoalkylthio en ($C_1$-$C_4$), halogénoalkylsulfinyle en ($C_1$-$C_4$) , halogénoalkylsulfonyle en ($C_1$-$C_4$) , aminocarbonyle, alkylaminocarbonyle en ($C_1$-$C_4$), cycloalkylaminocarbonyle en ($C_3$-$C_6$), alkylcarbamoyle en ($C_1$-$C_4$)(comprend -NHCOO-alkyle en ($C_1$-$C_4$)et -N-alkyle en ($C_1$-$C_4$)-COO-alkyle en ($C_1$-$C_4$)), alkylcarbonylamino en ($C_1$-$C_4$)ou alkylurée en ($C_1$-$C_4$) (comprend -NHCONH-alkyle en ($C_1$-$C_4$) et -NHCON-dialkyle en ($C_1$-$C_4$)),
uniquement un ou deux des radicaux $R^{10}$, $R^{11}$, $R^{12}$, $R^{13}$, $R^{14}$, $R^{15}$, $R^{16}$, $R^{17}$ représentant un substituant différent de l'hydrogène,
et dans le cas où $R^{10}$ et $R^{14}$, $R^{11}$ et $R^{15}$, $R^{12}$ et $R^{16}$ ou $R^{13}$ et $R^{17}$ sont respectivement tous les deux différents de l'hydrogène, $R^{14}$, $R^{15}$, $R^{16}$ et $R^{17}$ représentant chacun indépendamment les uns des autres uniquement cyano, halogène, alkyle en ($C_1$-$C_4$)ou halogénoalkyle en ($C_1$-$C_4$), Q représente un système cyclique bicyclique ou tricyclique annelé hétéroaromatique, à 9 chaînons ou 12 chaînons, de la série Q2, Q3, Q5, Q6, Q8, Q9, Q14 ou Q15,
où
$R^4$ représente alkyle en ($C_1$-$C_4$), halogénoalkyle en ($C_1$-$C_4$), cycloalkyle en ($C_3$-$C_6$) ou alcoxy en ($C_1$-$C_4$)-alkyle en ($C_1$-$C_4$),
$R^5$ représente cyano, halogène, halogénoalkyle en ($C_1$-$C_4$), halogéno-cycloalkyle en ($C_3$-$C_6$), halogénoalcoxy en ($C_1$-$C_4$), halogénoalkylthio en ($C_1$-$C_4$), halogénoalkylsulfinyle en ($C_1$-$C_4$), alkylsulfonyle en ($C_1$-$C_4$), halogénoalkylsulfonyle en ($C_1$-$C_4$), halogénoalkylcarbonyle en ($C_1$-$C_4$)ou halogénoalkylsulfonyloxy en ($C_1$-$C_4$),
$R^6$ représente hydrogène, cyano, halogène, alkyle en ($C_1$-$C_4$), halogénoalkyle en ($C_1$-$C_4$) ou cycloalkyle en ($C_3$-$C_6$), et
n représente 0, 1 ou 2.

**5.** Composés de la formule (Ia) ou (Ib) selon la revendication 2, dans lesquels

dans le cas où les 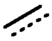 représentent exclusivement des doubles liaisons, et par conséquent Aa à Ad forment un cycle aromatique avec les atomes C voisins respectivement de Aa et Ad,

Aa représente $C(R^{10})$,
Ab représente $C(R^{11})$ ,
Ac représente $C(R^{12})$ et
Ad représente $C(R^{13})$,

ou dans le cas ou les 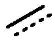 représentent exclusivement des simples liaisons,
Aa représente $C(R^{10})$ $(R^{14})$ ,
Ab représente $C(R^{11})$ $(R^{15})$ ,
Ac représente $C(R^{12})(R^{16})$ et
Ad représente $C(R^{13})(R^{17})$ ,
$R^1$ représente éthyle,
$R^{10}$, $R^{11}$, $R^{12}$, $R^{13}$ représentent indépendamment les uns des autres hydrogène, fluor, chlore, brome, iode, cyano, méthyle, éthyle, trifluorométhyle, trifluorométhoxy, 2,2,2-trifluoroéthoxy, acétylamino (méthylcarbonylamino), cyclopropylamido (cyclopropylaminocarbonyle), méthylcarbamoyle (-NHCOOMe), méthylurée (-NHCONHMe) ou cyclopropyle, uniquement un ou deux des radicaux $R^{10}$, $R^{11}$, $R^{12}$, $R^{13}$ représentant un substituant différent de l'hydrogène, $R^{14}$, $R^{15}$, $R^{16}$, $R^{17}$ représentent indépendamment les uns des autres hydrogène ou alkyle en ($C_1$-$C_4$),
Q représente un système cyclique bicyclique annelé hétéroaromatique à 9 chaînons de la série Q2, Q3 ou Q14,

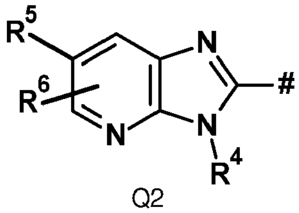
Q2

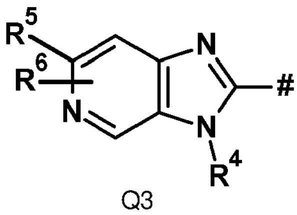
Q3

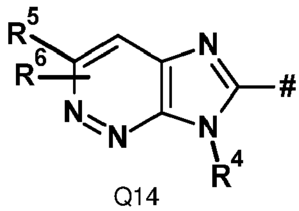
Q14

où
$R^4$ représente méthyle,
$R^5$ représente trifluorométhyle ou pentafluoroéthyle,
$R^6$ représente hydrogène et
n représente 0, 1 ou 2.

6. Composés de la formule (Ia) ou (Ib) selon la revendication 2, dans lesquels

dans le cas où les 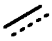 représentent exclusivement des doubles liaisons, et par conséquent Aa à Ad forment un cycle aromatique avec les atomes C voisins respectivement de Aa et Ad,

Aa représente $C(R^{10})$,
Ab représente azote ou $C(R^{11})$,
Ac représente $C(R^{12})$ et
Ad représente $C(R^{13})$,
$R^{10}$ représentant hydrogène, chlore ou trifluorométhyle,
$R^{11}$ représentant hydrogène, chlore, -NHCOMe ou trifluorométhyle,
$R^{12}$ représentant hydrogène, chlore ou trifluorométhyle,
$R^{13}$ représentant hydrogène, chlore ou trifluorométhyle,

ou dans le cas où les 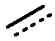 représentent exclusivement des simples liaisons,
Aa représente $C(R^{10})$ $(R^{14})$ ,
Ab représente $C(R^{11})$ $(R^{15})$ ,
Ac représente $C(R^{12})(R^{16})$ et
Ad représente $C(R^{13})(R^{17})$ ,
$R^{10}$, $R^{12}$, $R^{13}$, $R^{14}$, $R^{16}$, $R^{17}$ représentant hydrogène,
$R^{11}$ représentant hydrogène ou méthyle, et
$R^{15}$ représentant hydrogène ou méthyle,
$R^1$ représente éthyle,
Q représente un système cyclique bicyclique annelé hétéroaromatique à 9 chaînons de la série Q2 ou Q3

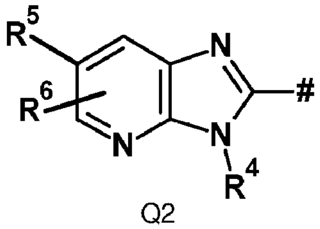
Q2

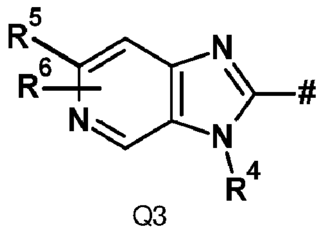
Q3

où
$R^4$ représente méthyle,
$R^5$ représente trifluorométhyle ou pentafluoroéthyle,
$R^6$ représente hydrogène et
n représente 0, 1 ou 2.

**7.** Composés de la formule (Ia) ou (Ib) selon l'une quelconque des revendications 1 à 6, dans lesquels
Q représente Q2, Q3, Q5, Q6, Q8, Q9, Q14 ou Q15,
où
$R^4$ représente alkyle en ($C_1$-$C_4$), halogénoalkyle en ($C_1$-$C_4$), cycloalkyle en ($C_3$-$C_6$) ou alcoxy en ($C_1$-$C_4$)-alkyle en ($C_1$-$C_4$),
$R^5$ représente cyano, halogène, halogénoalkyle en ($C_1$-$C_4$), halogéno-cycloalkyle en ($C_3$-$C_6$), halogénoalcoxy en ($C_1$-$C_4$), halogénoalkylthio en ($C_1$-$C_4$), halogénoalkylsulfinyle en ($C_1$-$C_4$), alkylsulfonyle en ($C_1$-$C_4$), halogénoalkylsulfonyle en ($C_1$-$C_4$), halogénoalkylcarbonyle en ($C_1$-$C_4$) ou halogénoalkylsulfonyloxy en ($C_1$-$C_4$),
$R^6$ représente hydrogène, cyano, halogène, alkyle en ($C_1$-$C_4$), halogénoalkyle en ($C_1$-$C_4$) ou cycloalkyle en ($C_3$-$C_6$) et Aa, Ab, Ac, Ad, $R^1$, $R^{10}$, $R^{11}$, $R^{12}$, $R^{13}$, $R^{14}$, $R^{15}$, $R^{16}$, $R^{17}$ et n ont les significations selon la revendication 1 ou la revendication 2 ou la revendication 3 ou la revendication 5 ou la revendication 6.

**8.** Composés de la formule (Ia) ou (Ib) selon l'une quelconque des revendications 1 à 6, dans lesquels
Q représente Q2, Q3 ou Q14,

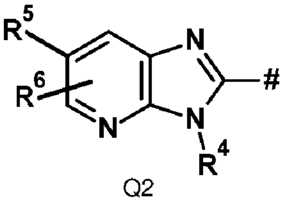
Q2

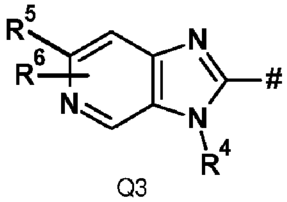
Q3

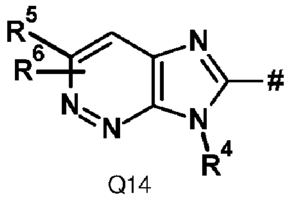
Q14

où

$R^4$ représente méthyle,
$R^5$ représente trifluorométhyle ou pentafluoroéthyle,
$R^6$ représente hydrogène,
et Aa, Ab, Ac, Ad, $R^1$, $R^{10}$, $R^{11}$, $R^{12}$, $R^{13}$, $R^{14}$, $R^{15}$, $R^{16}$, $R^{17}$ et n ont les significations selon la revendication 1 ou la revendication 2 ou la revendication 3 ou la revendication 4 ou la revendication 6.

**9.** Composés de la formule (Ia) ou (Ib) selon l'une quelconque des revendications 1 à 6, dans lesquels
Q représente Q2 ou Q3,

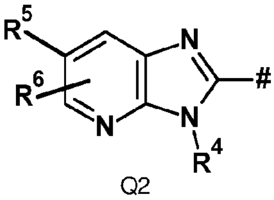
Q2

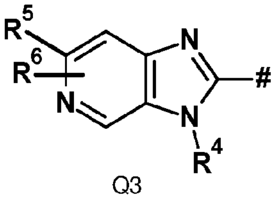
Q3

où

$R^4$ représente méthyle,
$R^5$ représente trifluorométhyle ou pentafluoroéthyle,
$R^6$ représente hydrogène,
et Aa, Ab, Ac, Ad, $R^1$, $R^{10}$, $R^{11}$, $R^{12}$, $R^{13}$, $R^{14}$, $R^{15}$, $R^{16}$, $R^{17}$ et n ont les significations selon la revendication 1 ou la revendication 2 ou la revendication 3 ou la revendication 4 ou la revendication 5.

**10.** Composés de la formule (Ia) ou (Ib) selon l'une quelconque des revendications 1 à 6, dans lesquels $R^1$ représente éthyle, et Aa, Ab, Ac, Ad, $R^{10}$, $R^{11}$, $R^{12}$, $R^{13}$, $R^{14}$, $R^{15}$, $R^{16}$, $R^{17}$, Q, R4, $R^5$, $R^6$ et n ont les significations selon la revendication 1 ou la revendication 2 ou la revendication 3 ou la revendication 4.

**11.** Composés de la formule (Ia) ou (Ib) selon l'une quelconque des revendications 1 à 6, dans lesquels les structures (Ia1) à (Ia4) et (Ibl) à (Ib4) sont obtenues pour les formules (Ia) et (Ib),

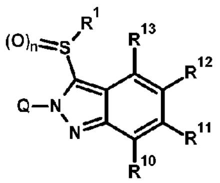
(Ia1)

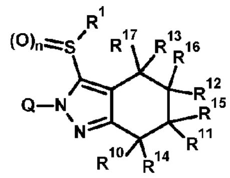
(Ia2)

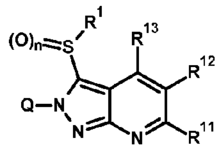
(Ia3)

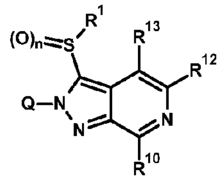
(Ia4)

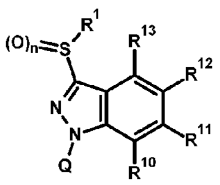
(Ib1)

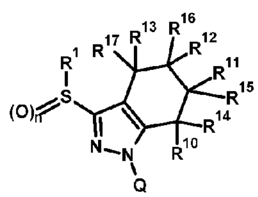
(Ib2)

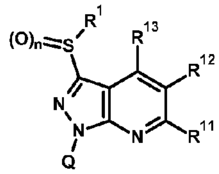
(Ib3)

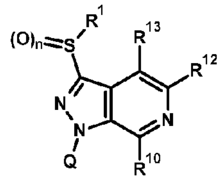
(Ib4)

où $R^1$, $R^{10}$, $R^{11}$, $R^{12}$, $R^{13}$, $R^{14}$, $R^{15}$, $R^{16}$, $R^{17}$, Q, $R^4$, $R^5$, $R^6$ et n ont les significations décrites dans l'une quelconque des revendications 1 à 6.

**12.** Composés des formules I-1 à I-5 et I-7 à I-19

| | |
|---|---|
| I-1 | 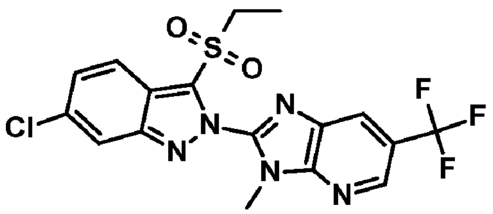 |
| I-2 | 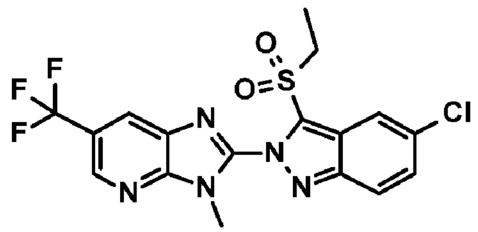 |

(suite)

| | |
|---|---|
| I-3 | 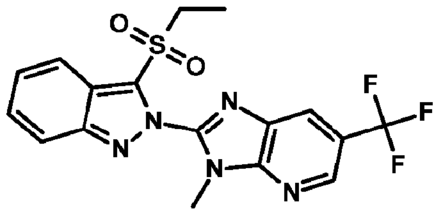 |
| I-4 | 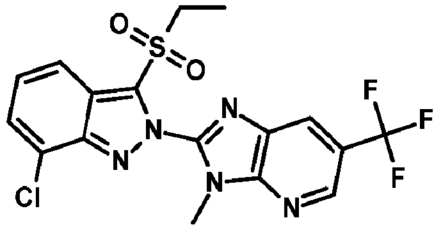 |
| I-5 | 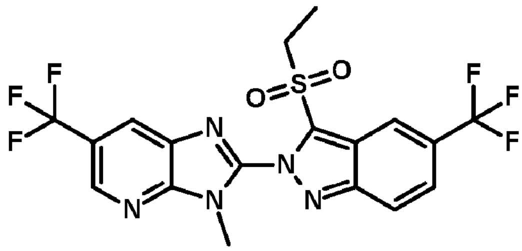 |
| I-7 | 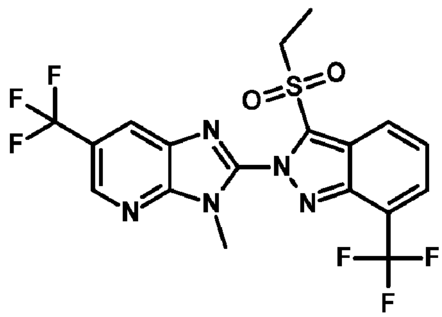 |
| I-8 | 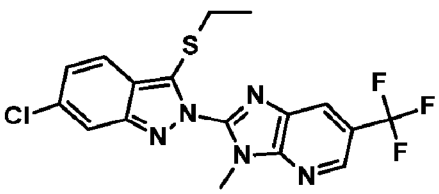 |
| I-9 | 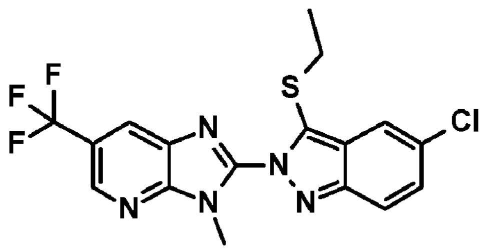 |

(suite)

| | |
|---|---|
| I-10 | 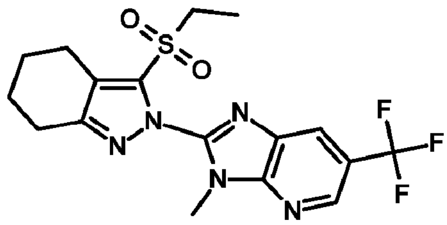 |
| I-11 | 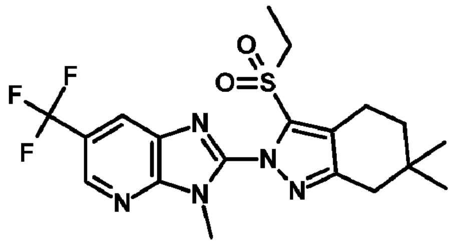 |
| I-12 | 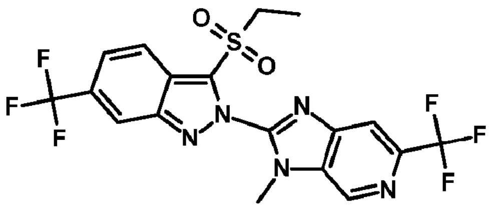 |
| I-13 | 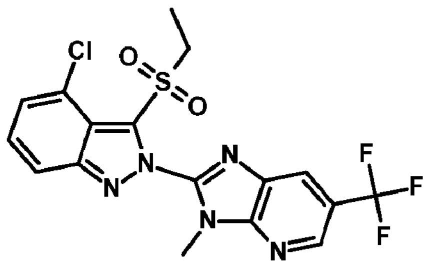 |
| I-14 | 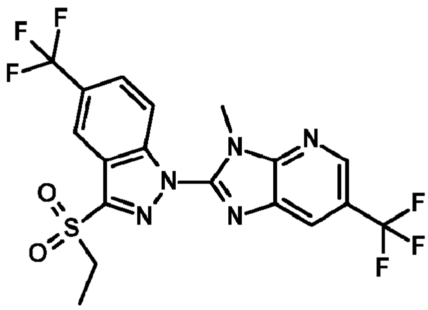 |
| I-15 | 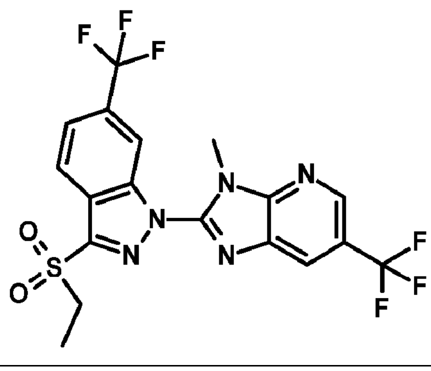 |

(suite)

| | |
|---|---|
| I-16 | 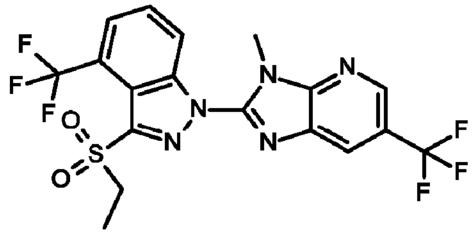 |
| I-17 | 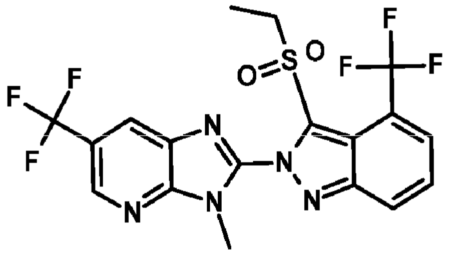 |
| I-18 | 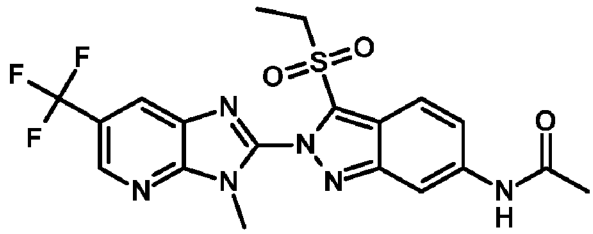 |
| I-19 | 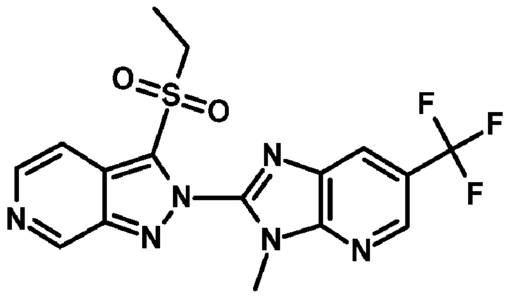 |

13. Composés de la formule (Ia) ou (Ib) selon l'une quelconque des revendications 1 à 11, **caractérisés en ce que**, dans le cas où, pour les composés de la formule (Ia) ,

les 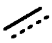 représentent exclusivement des doubles liaisons,

$R^1$ représente alkyle en $C_1$-$C_6$,
Aa et Ad représentent CH,
Ab représente $C(R^{11})$ ,
Ac représente $C(R^{12})$

et

Q représente Q2,
$R^4$ représente alkyle en $C_1$-$C_6$,
$R^6$ représente hydrogène et
$R^5$ représente halogénoalkyle en ($C_1$-$C_6$), halogénoalkylthio en ($C_1$-$C_6$), halogénoalkylsulfinyle en ($C_1$-$C_6$) ou halogénoalkylsulfonyle en ($C_1$-$C_6$),
au moins un des radicaux $R^{11}$ ou $R^{12}$ ne représente pas hydrogène, halogène ou halogénoalkyle en ($C_1$-$C_6$).

14. Composés de la formule (Ia) ou (Ib) selon l'une quelconque des revendications 1 à 11, **caractérisés en ce que**,

dans le cas où, pour les composés de la formule (Ia) ,

les 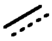 représentent exclusivement des doubles liaisons,

$R^1$ représente alkyle en $C_1$-$C_6$,
Aa et Ad représentent CH,
Ab représente $C(R^{11})$,
Ac représente $C(R^{12})$ ,
$R^{11}$ et $R^{12}$ représentent indépendamment l'un de l'autre hydrogène, halogène ou halogénoalkyle en ($C_1$-$C_6$)

et

Q représente Q2,
$R^4$ représente alkyle en $C_1$-$C_6$ et
$R^6$ représente hydrogène,
$R^5$ ne représente pas halogénoalkyle en ($C_1$-$C_6$), halogénoalkylthio en ($C_1$-$C_6$), halogénoalkylsulfinyle en ($C_1$-$C_6$) ou halogénoalkylsulfonyle en ($C_1$-$C_6$) .

**15.** Composés de la formule (Ia) ou (Ib) selon l'une quelconque des revendications 1 à 11, **caractérisés en ce que** les composés de la formule (Y), dans lesquels

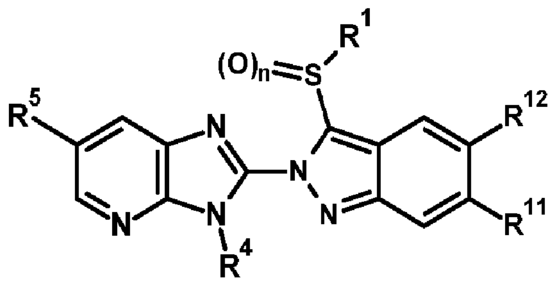

(Y)

$R^1$ représente alkyle en ($C_1$-$C_6$),

$R^4$ représente alkyle en ($C_1$-$C_6$),
$R^5$ représente halogénoalkyle en ($C_1$-$C_6$), halogénoalkylthio en ($C_1$-$C_6$), halogénoalkylsulfinyle en ($C_1$-$C_6$) ou halogénoalkylsulfonyle en ($C_1$-$C_6$),
$R^{11}$ et $R^{12}$ représentent chacun indépendamment l'un de l'autre hydrogène, halogène ou halogénoalkyle en ($C_1$-$C_6$), et
n représente 0, 1 ou 2,
sont exclus.

**16.** Composés de la formule (Ia) selon l'une quelconque des revendications 1 à 11, **caractérisés en ce que** les

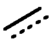 représentent exclusivement des simples liaisons.

**17.** Formulation agrochimique contenant au moins un composé de la formule (Ia) ou (Ib) selon l'une quelconque des revendications 1 à 16, ainsi que des extendeurs et/ou des substances tensioactives.

**18.** Formulation agrochimique selon la revendication 17, contenant en outre un agent actif agrochimique supplémentaire.

**19.** Procédé de lutte contre des animaux nuisibles, **caractérisé en ce qu'**on laisse agir un composé de la formule (Ia) ou (Ib) selon l'une quelconque des revendications 1 à 16 ou une formulation agrochimique selon l'une quelconque des revendications 17 et 18 sur les animaux nuisibles et/ou leur habitat, des procédés de traitement chirurgical ou thérapeutique du corps humain ou animal et des procédés de diagnostic, qui sont réalisés sur le corps humain ou animal, étant exclus.

**20.** Utilisation de composés de la formule (Ia) ou (Ib) selon l'une quelconque des revendications 1 à 16 ou de formulations

agrochimiques selon l'une quelconque des revendications 17 et 18 pour lutter contre des animaux nuisibles, des utilisations dans des procédés de traitement chirurgical ou thérapeutique du corps humain ou animal et des procédés de diagnostic, qui sont réalisés sur le corps humain ou animal, étant exclues.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2010125985 A
- WO 2012074135 A
- WO 2016162318 A
- WO 2017093180 A
- WO 2017072039 A
- WO 2017125340 A
- WO 2017144341 A
- EP 16184163
- EP 16189445
- EP 16163912
- WO 2016107742 A
- WO 2016091731 A
- WO 2016129684 A
- WO 2016142326 A
- WO 2016142327 A
- WO 2017155103 A
- US 200918132 A1
- US 3966760 A1
- US 2012214837 A1
- WO 2015116882 A1
- WO 201171725 A1
- US 201631875 A1
- WO 201019899 A1
- WO 2016039411 A
- WO 201323184 A1
- WO 2009147431 A1
- WO 200910530 A1
- WO 200589763 A1
- US 200625633 B
- US 2006111591 A
- US 2820062 A
- WO 2009134750 A1
- WO 2006116412 A2
- WO 201359928 A1
- EP 1466527 A1
- KR 20161508 A
- US 20150225397 A1
- WO 2009134750 A
- WO 2006043635 A
- WO 2003106457 A
- WO 2006003494 A
- WO 2010052161 A
- EP 2647626 A
- WO 2004099160 A
- JP 2010018586 A
- WO 2012029672 A
- WO 2013144213 A
- WO 2010051926 A
- CN 103232431
- WO 2013050317 A1
- WO 2013162715 A2
- WO 2013162716 A2
- US 20140213448 A1
- CN 101337937 A
- CN 103109816 A
- WO 2012034403 A1
- WO 2011085575 A1
- CN 101337940 A
- CN 101715774 A
- CN 103524422 A
- CN 102391261 A
- US 20140275503 A1
- WO 2007040280 A1
- WO 2007040282 A1
- WO 2015058021 A1
- WO 2015058028 A1
- US 201594329 A1


### In der Beschreibung aufgeführte Nicht-Patentliteratur


- *Chemical Communications,* 2011, vol. 47, 10133
- *Journal of Organic Chemistry,* 1995, vol. 60 (7), 2254-2256
- *Beispiel Bioorganic and medicinal chemistry letters,* 2002, vol. 12 (16), 2221-2224
- *Journal of Medicinal Chemistry,* 1999, vol. 42 (1), 50-59
- *European Journal of Medicinal Chemistry,* 2014, vol. 78, 35-42
- *Journal of Heterocyclic Chemistry,* 1995, vol. 32 (2), 467-471
- *Journal of Heterocyclic Chemistry,* 1997, vol. 34 (3), 717-727
- *Synlett,* 2016, vol. 27 (12), 1798-1802
- *Chemical and pharmaceutical bulletin,* 1979, vol. 27 (2), 410-418
- *Chemical Communications,* 2000, vol. 13, 1163-1164
- *Journal of the American Chemical Society,* 1922, vol. 44, 1329

- *Bioorganic and Medicinal Chemistry Letters,* 2010, vol. 20, 2770-2775
- *Organic and Biomolecular Chemistry,* 2007, vol. 5 (16), 2567-2571
- *Tetrahedron,* 1984, vol. 40, 1051
- *Journal of Organic Chemistry,* 2006, vol. 71 (16), 6149-6156
- rganic Chemistry Including Medicinal Chemistry. *Indian Journal of Chemistry,* 1991, vol. 30 (3), 306-312
- *Justus Liebigs Annalen der Chemie,* 1927, vol. 453, 228
- *Beispiel Bioorganic Chemistry,* 2015, vol. 60, 19-29
- *European Journal of Medicinal Chemistry,* 2014, vol. 75, 492-500
- *Arzneimittel-Forschung/Drug Research,* 1977, vol. 27 (1), 82-89
- Crop Life International und in Pesticide Specifications, Manual on development and use of FAO and WHO specifications for pesticides. FAO Plant Production and Protection Papers - 173, prepared by the FAO/WHO Joint Meeting on Pesticide Specifications. 2004
- **BAUR et al.** *Pesticide Science,* 1997, vol. 51, 131-152
- The Pesticide Manual. British Crop Protection Council, 2012
- *CHEMICAL ABSTRACTS,* 885026-50-6
- *CHEMICAL ABSTRACTS,* 637360-23-7
- *CHEMICAL ABSTRACTS,* 872999-66-1
- *CHEMICAL ABSTRACTS,* 1225292-17-0
- *CHEMICAL ABSTRACTS,* 1440516-42-6
- *CHEMICAL ABSTRACTS,* 792914-58-0
- *CHEMICAL ABSTRACTS,* 1204776-60-2
- *CHEMICAL ABSTRACTS,* 1363400-41-2
- *CHEMICAL ABSTRACTS,* 1461743-15-6
- *CHEMICAL ABSTRACTS,* 1226889-14-0
- *CHEMICAL ABSTRACTS,* 1449220-44-3
- *CHEMICAL ABSTRACTS,* 1332628-83-7
- *CHEMICAL ABSTRACTS,* 1477923-37-7
- *CHEMICAL ABSTRACTS,* 1105672-77-2
- *CHEMICAL ABSTRACTS,* 1232543-85-9
- *CHEMICAL ABSTRACTS,* 1268277-22-0
- *CHEMICAL ABSTRACTS,* 1233882-22-8
- *CHEMICAL ABSTRACTS,* 1108184-52-6
- *CHEMICAL ABSTRACTS,* 1542271-46-4
- *CHEMICAL ABSTRACTS,* 1370358-69-2
- *CHEMICAL ABSTRACTS,* 1181213-14-8
- *CHEMICAL ABSTRACTS,* 1253850-56-4
- *CHEMICAL ABSTRACTS,* 933798-27-7
- *CHEMICAL ABSTRACTS,* 934001-66-8
- *CHEMICAL ABSTRACTS,* 1477919-27-9
- Pesticide Manual. British Crop Protection Council
- *CHEMICAL ABSTRACTS,* 129531-12-0
- *CHEMICAL ABSTRACTS,* 71526-07-3
- *CHEMICAL ABSTRACTS,* 52836-31-4
- **R. WEGLER.** Chemie der Pflanzenschutz- und Schädlingsbekämpfungsmittel. Springer Verlag, 1970, vol. 2, 401-412
- Research Disclosure Database. 564025